# EUROPEAN PATENT APPLICATION

(11) **EP 3 889 267 A1**
(43) Date of publication of application: **06.10.2021**
(21) Application number: 20167881.0
(22) Date of filing: 03.04.2020
(51) Int. Cl.: C12N 15/82, C07K 14/01

(54) **(BE-)CURTOVIRUS REPLICON-MEDIATED GENOME EDITING IN PLANTS**

(71) Applicant: KWS SAAT SE & Co. KGaA, 37574 Einbeck (DE); Institut für Zuckerrübenforschung des Vereins der Zuckerindustrie e.V., 37079 Göttingen (DE)
(72) Inventor: SCHUMANN, Nadine, 37574 Einbeck (DE); NIESSEN, Markus, 30880 Laatzen (DE); EINI, Omid, 37079 Göttingen (DE); VARRELMANN, Mark, 37079 Göttingen (DE)
(74) Representative: Eisenführ Speiser

(57) **Abstract**

The present invention relates to the technical field of targeted modification of a nucleotide sequence of interest in the genome of a plant. More particularly, the invention relates to the provision of new viral replicons for delivering genome editing tools to plant cells recalcitrant for transformation for, in particular, achieving homology-directed repair (HDR)-mediated genome editing suitable for, *inter alia*, plant cells or plants of the species of the family *Amaranthacea*, preferably a cell of a plant from the genus *Beta* or *Spinacia.* The invention thus describes a method for increased efficiency of genome editing, in particular HDR-mediated genome editing, in a plant or a plant cell by providing specific viral replicons facilitating the modification of transformation recalcitrant plants and plant cells.

## Description

### Technical Field

The present invention relates to the technical field of targeted modification of a nucleotide sequence of interest in the genome of a plant. More particularly, the invention relates to the provision of new viral replicons for delivering genome editing tools to plant cells recalcitrant for transformation for, in particular, achieving homology-directed repair (HDR)-mediated genome editing suitable for, *inter alia,* plant cells or plants of the species of the family *Amaranthacea,* preferably a cell of a plant from the genus *Beta* or *Spinacia.* The invention thus describes a method for increased efficiency of genome editing, in particular HDR-mediated genome editing, in a plant or a plant cell by providing specific viral replicons facilitating the modification of transformation recalcitrant plants and plant cells.

### Background of the Invention

While for many crops the use of CRISPR-based genome editing (GE) is described already, there are several crop plants for which only little is known about the successful and efficient methods for such genome editing, in particular complex modifications like HDR-mediated genome editing. For instances, a plant of the species *Beta vulgaris* (such as for example sugar beet, fodder beet, red beet, or chard) is well known as being very recalcitrant in transgenic modifications. There are only very few established methods for transformation, mainly based on *Agrobacterium-mediated* transformation.

The common way to modify a nucleotide sequence of interest in the genome of e.g. sugar beet is to stably transform plants via *Agrobacterium-mediated* delivery of a T-DNA vector expressing a nucleotide sequence of interest, which may be a gene conferring an important trait or a gene silencing cassette. Particularly, in sugar beet stable transformation and subsequent regeneration of transgenic plants is amongst the aforementioned technical hurdles extremely labor-intensive, time-consuming and cost-intensive. Moreover, the *Agrobacterium-mediated* construct insertion into the plant genome does not occur in a targeted way, but randomly which is not desired for obtaining a predictable GE outcome. Therefore, positional effects appear frequently. In contrast to that, efficient GE mediated by site-specific nucleases might allow targeted modification of a nucleotide sequence in the genome of sugar beet and it simultaneously may expand the potential applications, but this requires the provision of new tools for providing the relevant GE tools to a recalcitrant, i.e., difficult to transform or transfect, target plant.

Technically the most difficult and challenging way of targeted genome editing in any crop is the HDR-mediated approach. This approach allows targeted insertion of larger nucleotide molecules, e.g. entire genes, regulatory elements or the replacement of one allele for another which for example carries one or more individual amino acid substitution. Previous studies in other crops revealed that HDR-mediated genome editing in plants encounters generally two main barriers: 1) efficient HDR-mediated genome modification requires a large amount of exogenous repair template to outcompete the sister chromatid, which is usually used as repair template under natural conditions; 2) when transforming terminally differentiated/non-dividing cells, the pathway for HDR may not be available.

Both requirements for efficient HDR-mediated genome modification cannot be reached by conventional DNA delivery methods like (binary) vectors or other physical delivery. Zaidi & Mansoor (2017) (Viral vectors for plant genome engineering. Frontiers in plant science, 8, 539) reviewed the potential use of Geminivirus replicons (GVRs) as suitable delivery tools for GE components to induce HDR. Until today, however, GVRs have been used only in plants which are known to be easy transformable and regenerable like *Arabidopsis thaliana, Solanum lycopersicum, Nicotiana tabacum, Solanum tuberosum, Nicotiana benthamiana, Triticum aestivum* and *Oryza sativa.* Additionally, a further limitation is the fact that viruses have a very specific plant tropism and the number of Geminiviruses tested positively for transformation purposes yet are very limited.

Zaidi & Mansoor report on three different virus strains, namely BeYDV, CaLCuV and WDV. All these viruses, as naturally occurring, have a specific genome architecture and are not specific in tropism for recalcitrant plants like *Beta vulgaris,* or other plants in the family *Amaranthaceae.* The authors highlighted particularly that regenerating plants transformed with Geminivirus vectors has generally proven extremely difficult, mainly because Geminivirus proteins interact with several cellular proteins to facilitate viral replication that in turn compromises the integrity of host cell. Therefore, establishing new viral replicons based on DNA viruses is usually associated with several hurdles to be overcome and a suitable replicon based on a new virus has to be constructed case-by-case taking into consideration the genome structure and pathogenicity mechanism of the virus and the target plant(s) of interest to be transformed.

Lozano-Durán (2016) (Geminiviruses for biotechnology: the art of parasite taming. New Phytologist, 210(1), 58-64.) indicated that another pleiotropic effect of constitutive replicon expression might be an increase in off-target modifications, generally not desired for precise GE in plants.

EP 2 861 737 B1 disclosed a method to increase the efficiency of GE events in plants, which are based on homology-directed repair. However, the only virus used is the Mastrevirus bean yellow dwarf virus (BeYDV) and, again, only plant species which are known to be susceptible for genetic modification and simple to recover, namely *Arabidopsis thaliana* and *Nicotiana tabacum,* are used. Therefore, the approach disclosed is not suitable for efficiently transforming and modifying recalcitrant dicotyledonous plants, as already transformation with the disclosed viruses may be associated with problems due to incompatibility of the relevant host to be transformed and later the host genome to be modified.

Since a *Beta vulgaris* plant is not a natural host for the Mastrevirus BeYDV, in sugar beet BeYDV-based GVRs cannot replicate and interact with host factors as efficient as in the model plants *Nicotiana tabacum* or *Arabidopsis.* Moreover, the family of *Geminiviridae* is highly diverse in structure and function which otherwise necessarily entails that findings for one specific virus from a genus or species attributed to the *Geminiviridae* family cannot easily be applied to another genus due to large differences in genome organization and pathology.

For example, Bean Yellow Dwarf Virus (BeYDV) which has been used for genome editing (Baltes et al. 2014. DNA Replicons for Plant Genome Engineering. The Plant Cell 26 (1):151-163. doi:10.1105/tpc.113.119792*.)* is a member of Mastreviruses. The genome of this Geminivirus encodes four genes (Rep, RepA, V1 and V2) and contains a long internal region (LIR) and a short internal region (SIR). Baltes *et al.* kept the LIR, SIR and Rep and RepA sequences in the BeYDV replicon.

In contrast, Beet curly top iran virus (BCTIV) is a new and not yet detailed studied member of Becurtoviruses with a unique nanonucleotide in the LIR and a rather different member of the family *Geminiviridae.* This virus encodes five genes (V1, V2 and V3 on the virion-sense strand and Rep and C2 genes on the complementary-sense strand). The genome sequence and structure of the virion-sense stand of Becurtoviruses is similar to Curtoviruses while the complementary-sense strand has similarity to the Mastreviruses.

Beet severe curly top virus (BSCTV) and Beet curly top virus (BCTV) are members of Curtoviruses. Curtoviruses are monopartite Geminiviruses that are vectored by leafhoppers, often in a species-specific manner and rather different to Mastreviruses. BCTV encodes usually 7 genes (V1, V2, V3 on the virion-sense strand and Rep, C2, C3 and C4 on the complementary-sense strand) without SIR sequence.

Still, at date little is known about (Be-)Curtovirus replicon architecture allowing efficient strategies for replication and expression of GE tools and having a tropism for recalcitrant plants, e.g. in the family *Amaranthaceae.*

Thus, there is a great need of providing new viral replicons for safely and efficiently transforming recalcitrant plants and new methods for HDR-mediated modification of nucleic acids of interest in particular for such plant species, which are recalcitrant for genetic modifications and where the use of these methods is not limited to very less and commercially not applicable genotypes.

### Summary of the Invention

To address the above objectives, the present invention thus provides, in a first aspect, a method for the targeted modification of at least one genomic target sequence in a plant cell preferably from a plant of the family *Amaranthaceae* or *Solanaceae,* preferably a cell of a plant from the genus *Beta* or *Spinacia,* more preferably a cell of a plant of the species *Beta vulgaris* or *Spinacia oleracea,* wherein the method comprises the following steps: (a) introducing into the cell (i) at least one viral replicon, or a sequence encoding the same, the replicon being preferably constructed based on a Becurtovirus, or a Curtovirus genome, wherein the at least one viral replicon further comprises: (ii) at least one genome editing system comprising at least one nucleic acid guided or site-specific nuclease, nickase or an inactivated nuclease, or a sequence encoding the same, and optionally, in the case a CRISPR system is used, at least one guide molecule, or a sequence encoding the same; (iii) and, optionally at least one repair template, or a sequence encoding the same; (b) cultivating the cell under conditions allowing the expression and/or assembly of the at least one genome editing system and optionally the at least one guide molecule and/or optionally the at least one repair template; and (c) obtaining at least one modified cell; and (d) optionally obtaining at least one plant, plant tissue, organ, or seed regenerated from the at least one modified cell.

In a further aspect, a plant, plant cell, tissue, organ, orseed obtainable by or obtained by a method according to the first aspect is provided.

In yet another aspect, a viral replicon for plant transformation is provided, wherein the viral replicon is encoded by a sequence according to SEQ ID NO: 4 or 6, or a sequence having at least sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

In yet a further aspect, an expression construct is provided comprising or encoding: (i) at least one viral replicon as defined in the above aspect and (ii) a vector, preferably a binary vector, more preferably a binary vector as defined in the above aspect, and (iii) at least one component of at least one genome editing system, wherein the genome editing system comprises or encodes: (a) at least one nucleic acid guided or site-specific nuclease, nickase or an inactivated nuclease, or at least one site-directed nuclease, nickase or an inactivated nuclease, and optionally at least one nuclear or organellar localization sequence; and optionally (b) at least one guide molecule for guiding the at least one nucleic acid guided nuclease, nickase or an inactivated nuclease and optionally for activating at least one reverse transcriptase; and (c) optionally at least one repair template; and (d) optionally at least one reverse transcriptase; and (iv) wherein the expression construct comprises or encodes at least one regulatory sequence, wherein the regulatory sequence is selected from the group consisting of a core promoter sequence, a proximal promoter sequence, a cis regulatory sequence, a trans regulatory sequence, a locus control sequence, an insulator sequence, a silencer sequence, an enhancer sequence, a terminator sequence, an intron sequence, and/or any combination thereof.

In a further aspect, a use of a viral replicon optimized in tropism for a plant or plant cell of interest, or a use of an expression construct according to the above aspect is provided, for stimulating homology-directed repair by reverting a host plant cell into the S-phase of the cell cycle, and/or for increasing homology-directed repair-based genome editing.

### Brief description of Drawings

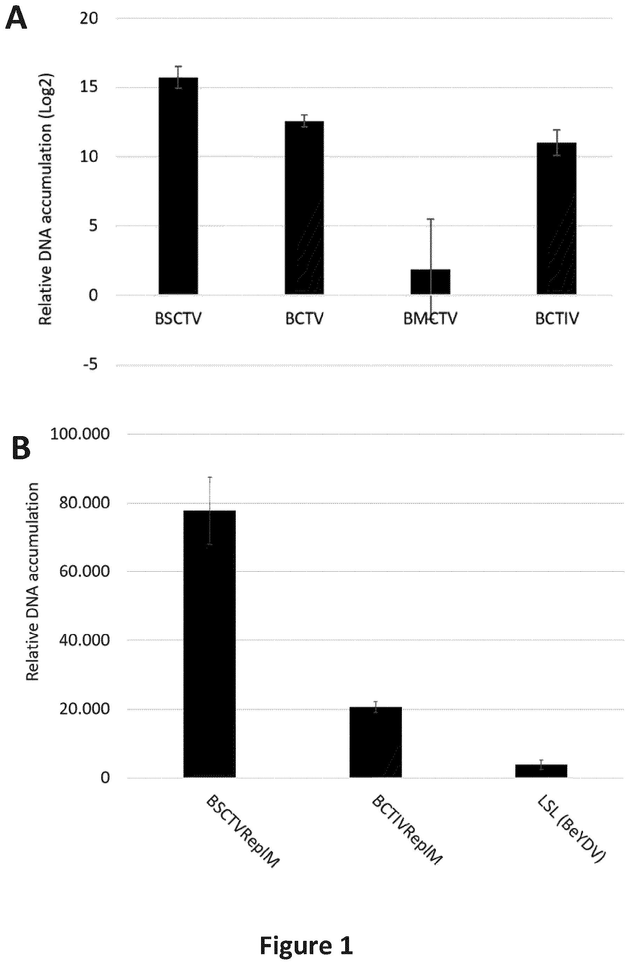

**Figure 1** shows viral DNA levels of full-length clones (A) and replicons (B).
**Figure 1** **A** shows virus accumulation in sugar beet (SB) cotyledons six days post inoculation. Cotyledons of sugar beet seedlings were syringe-infiltrated with full-length clones of different SB-infecting Geminiviruses (BSCTV, BCTV, BMCTV, BCTIV). DNA levels were determined via qPCR six days post infiltration (dpi). Four biological replicates were tested per construct. Error bars represent standard deviations. **Figure 1B** shows replication intensities of GVRs in sugar beet. Cotyledons of sugar beet seedlings were syringe-infiltrated with GVRs derived from BSCTV (BSCTVRepIM), BCTIV (BCTIVRepIM) or BeYDV (LSL). DNA levels were determined via qPCR ten days post infiltration using virus specific primers. A reference gene from *Beta vulgaris* was used for normalization. Three biological replicates were tested per replicon. Error bars represent standard deviations.

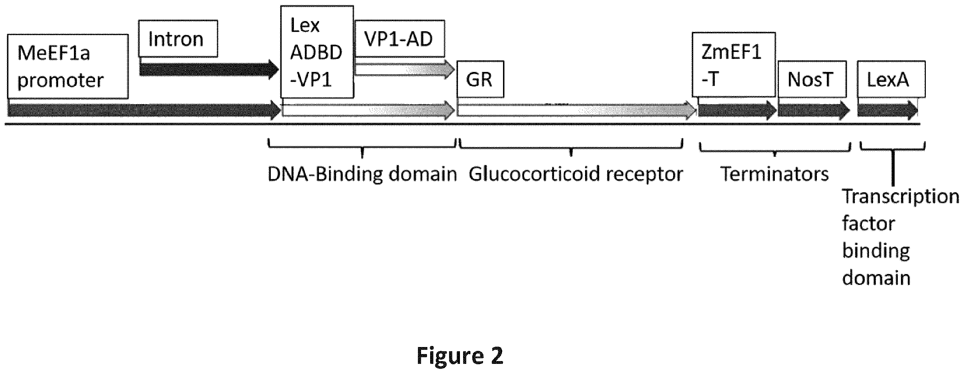

**Figure 2** shows a schematic view of the dexamethasone (DEX)-inducible promoter system.

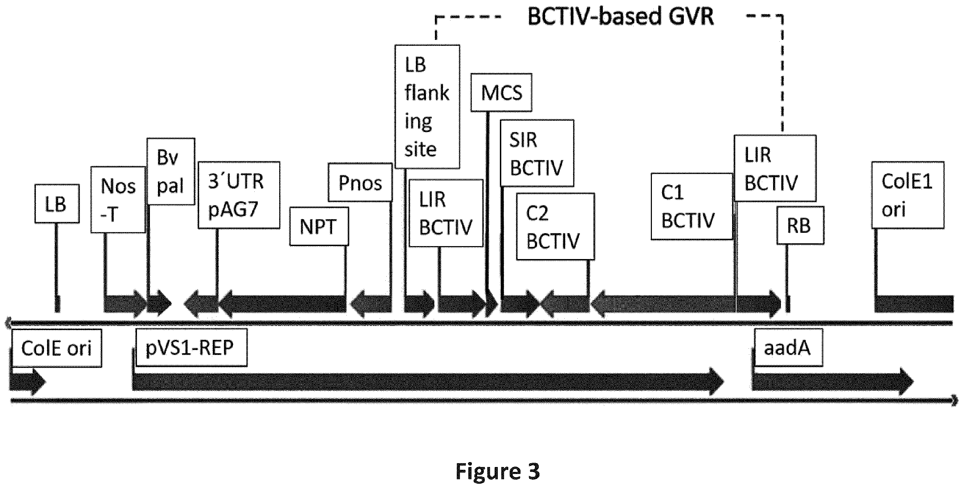

**Figure 3** shows a schematic view of the binary vector pZFNnptll containing the BCTIV-based replicon (BCTIV-Rep/MCS in pZFNnptll).

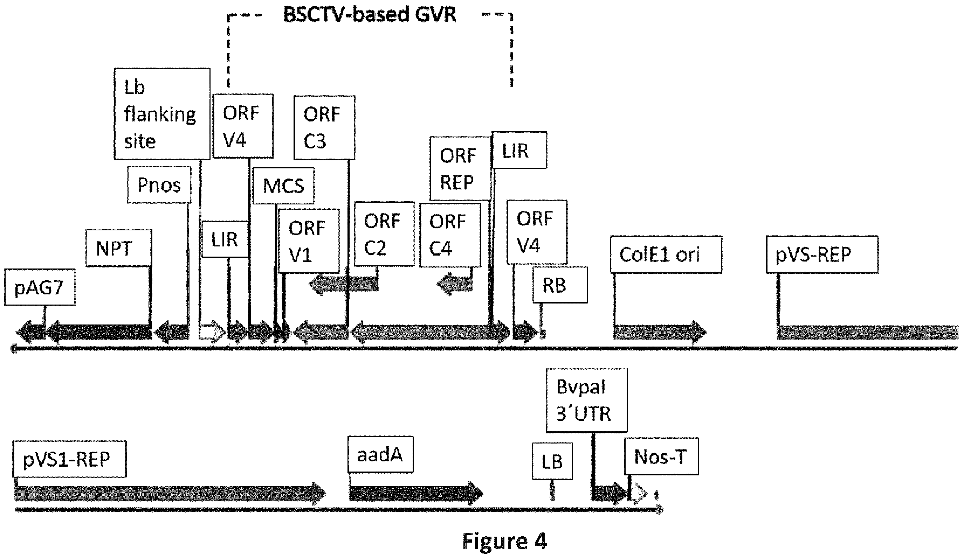

**Figure 4** shows a schematic view of the binary vector pZFNnptll containing the BSCTV-based replicon (BSCTV-Rep/MCS in pZFNnptll).

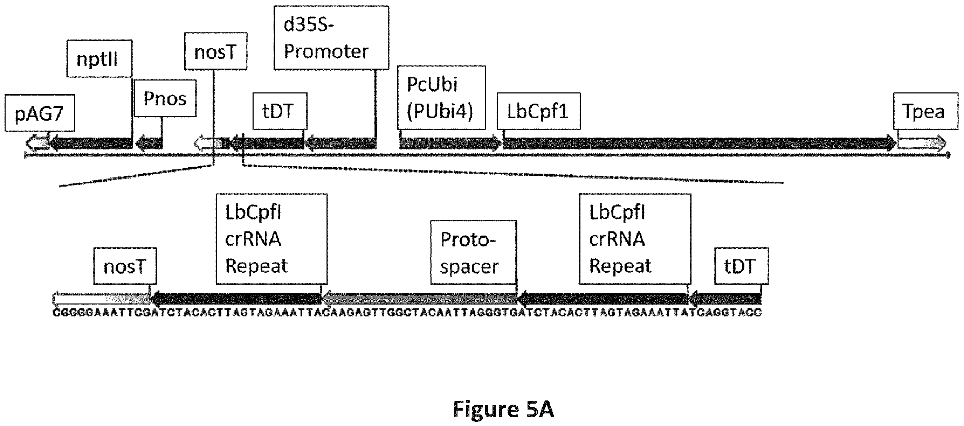

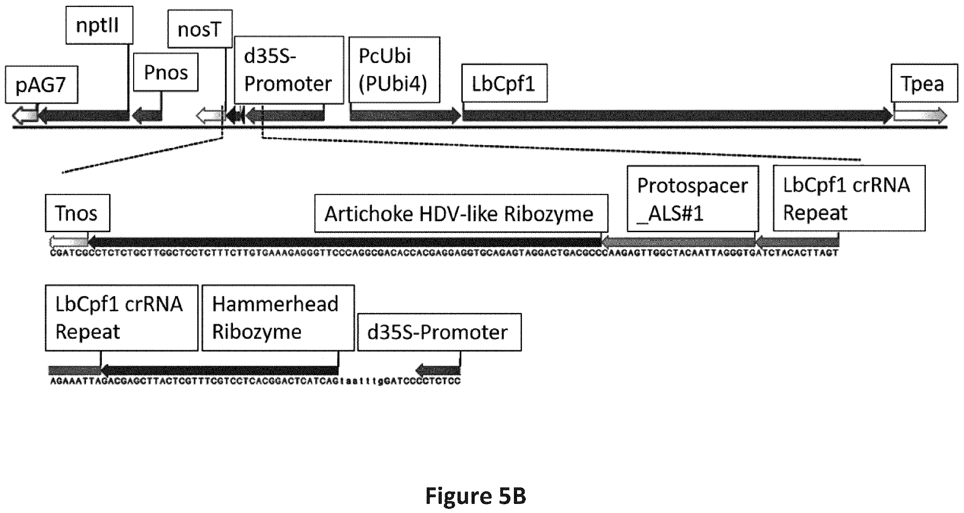

**Figure 5** shows the design of the binary vectors used for *in planta* proof of LbCpf1 mediated genome editing. (**A**) The LbCpf1_tDTcrRNA_ALS#1 (SEQ ID NO: 25) construct harbors expression cassettes for nptll, LbCpf1 and crRNA_ALS#1, which is directed against the target region BvALS#1. The crRNA is embedded in a Pol II-driven RNA transcript as described by Zhong et al., 2017 (doi: 10.1038/nchembio.2410). (B) The LbCpf1_RibocrRNA_ALS#1 (SEQ ID NO: 26) construct harbors expression cassettes for nptll, LbCpf1 and a crRNA_ALS#1, which is directed against the target region BvALS#1. The crRNA is produced from a Pol II promoter using the ribozyme-based strategy.

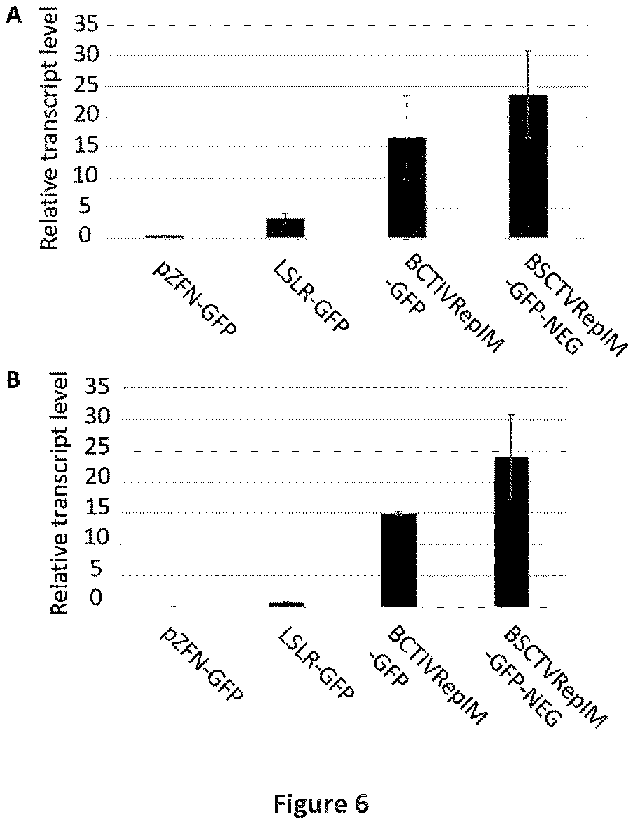

**Figure 6** shows GFP transcript levels in sugar beet. In sugar beet BCTIV-GVR and BSCTV-GVRs outperform BeYDV-GVR in terms of heterologous gene expression. Cotyledons of sugar beet seedlings were syringe-infiltrated with GVRs derived from BeYDV (LSLR), BCTIV (BCTIVRepIM) or BSCTV (BSCTVRepIM), which contain a 35S::GFP expression cassette. 35S::GFP expressed via a binary vector (pZFN-GFP) was used as non-GVR control. Transcript levels of GFP were measured via qRT-PCR four days (A) or six days (B) post infiltration. GFP expression was normalized against a reference gene from sugar beet. Three biological repeats were tested. Error bars represent standard deviations.

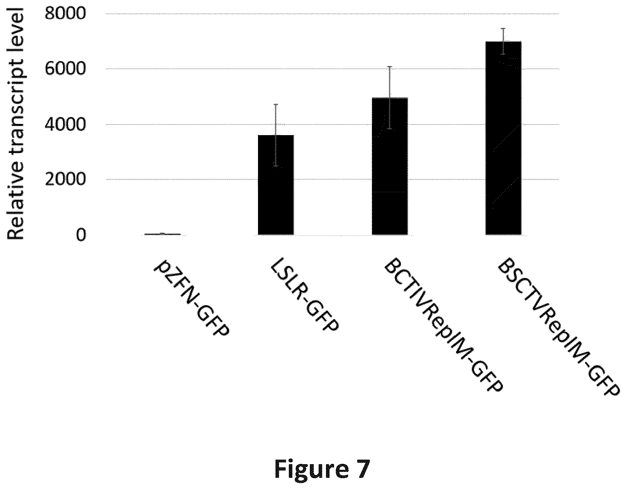

**Figure 7** shows GFP transcript levels in *Nicotiana benthamiana.- Nicotiana benthamiana* leaves were syringe-infiltrated with GVRs derived from BeYDV (LSLR), BCTIV (BCTIVRepIM) or BSCTV (BSCTVRepIM), which contain a 35S::GFP expression cassette. 35S::GFP expressed via a binary vector (pZFN-GFP) was used as non-GVR control. Transcript level of GFP were measured via qRT-PCR six days post infiltration. GFP expression was normalized against a reference gene from *N. benthamiana.* Three biological replicates were tested. Error bar represent standard deviations.

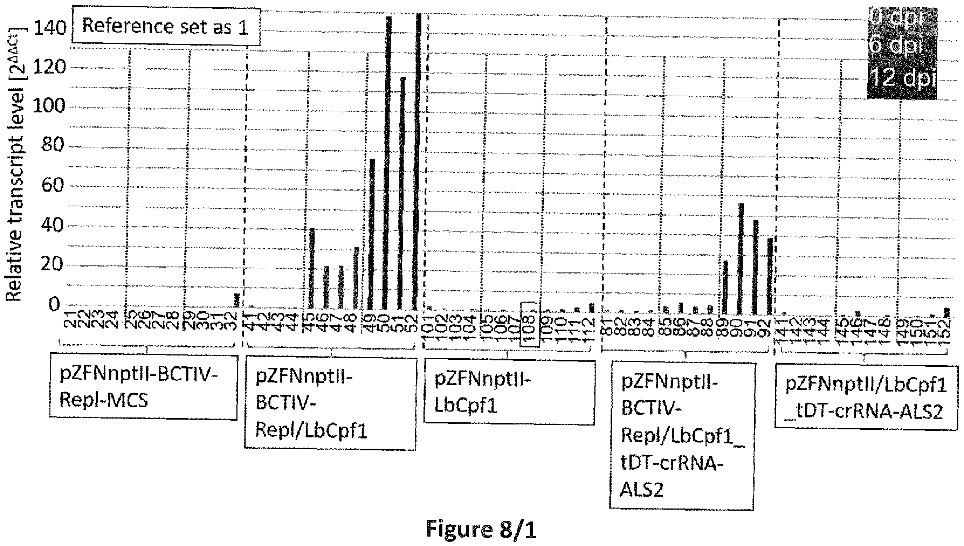

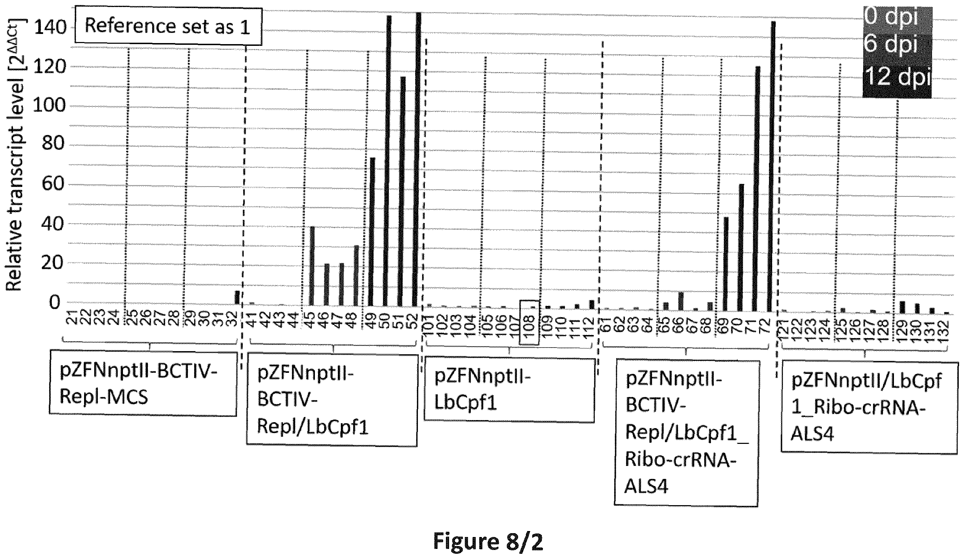

**Figure 8** shows that transcription of LbCpf1 is increased in BCTIV-GVRs compared to the corresponding binary vector controls. **Figure 8****/****1** shows relative transcription levels for the LbCpf1_tDTcrRNA construct. The samples with the numbers 21, 22, 23, 24, 41, 42, 43, 44, 101, 102, 103, 104, 81, 82, 83, 84, 141, 142, 143 and 144 represent relative transcription levels zero days post infiltration (dpi). The samples with the numbers 25, 26, 27, 28, 45, 46, 47, 48, 105, 106, 107, 108, 85, 86, 87, 88, 145, 146, 147, 148 represent relative transcription levels 6 dpi. The samples 29, 30, 31, 32, 49, 50, 51, 52, 109, 110, 111, 112, 89, 90, 91, 92, 149, 150, 151, and 152 represent relative transcription levels 12 dpi. Sample 108 (boxed) was used as a reference (set as 1). **Figure 8****/****2** shows relative transcription levels for the LbCpf1_Ribo_crRNA construct. The samples with the numbers 21, 22, 23, 24, 41, 42, 43, 44, 101, 102, 103, 104, 61, 62, 63, 64, 121, 122, 123 and 124 represent relative transcription levels 0 dpi. The samples with the numbers 25, 26, 27, 28, 45, 46, 47, 48, 105, 106, 107, 108, 65, 66, 67, 68, 125, 126, 127, 128 represent relative transcription levels 6 dpi. The samples 29, 30, 31, 32, 49, 50, 51, 52, 109, 110, 111, 112, 69, 70, 71, 72, 129, 130, 131, and 132 represent relative transcription levels 12 dpi. Sample 108 (boxed) was used as a reference (set as 1).

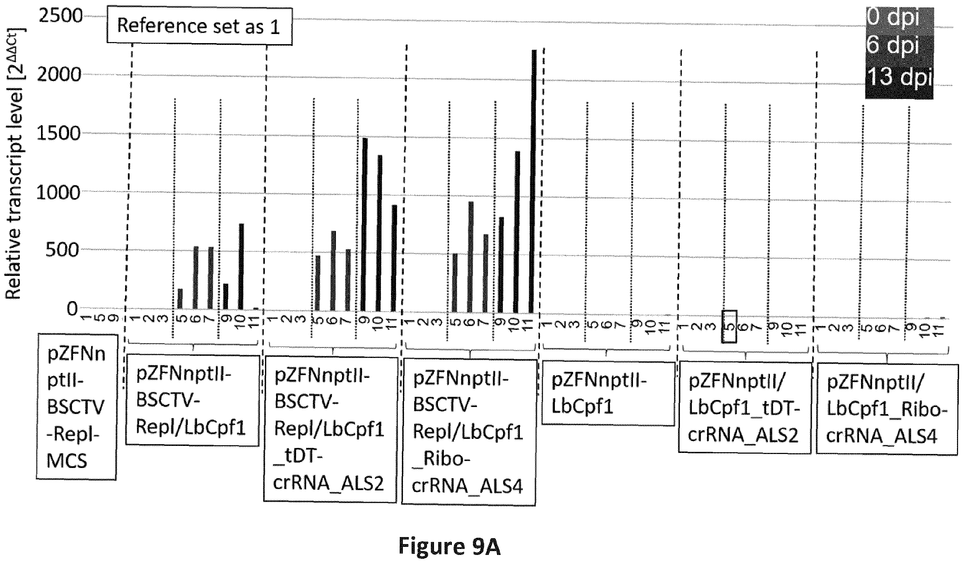

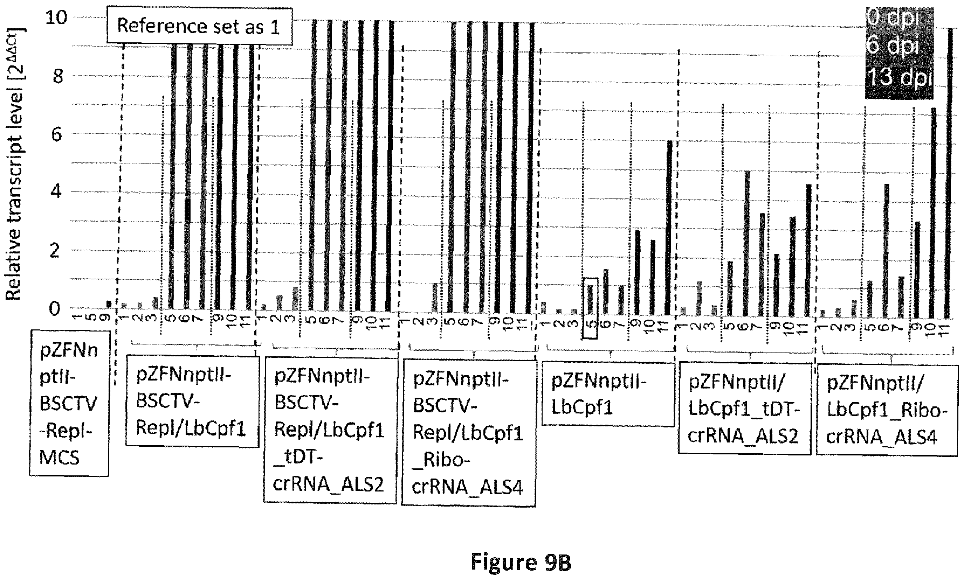

**Figure 9** shows transcription of LbCpf1 is increased in plant tissue infiltrated with BSCTV-GVRs compared to the corresponding binary vector controls. Cotyledons of sugar beet seedlings were syringe-infiltrated with GVRs derived from BSCTV (pZFNnptII-BSCTV-RepI), which contain genome editing components (LbCpf1 or LbCpf1+crRNA). GE components expressed via a binary vector (pZFNnptII) were used as non-GVR controls. Transcript levels of LbCpf1 were measured via qRT-PCR zero (sample numbers: 1, 2, 3), six (sample numbers: 5, 6, 7) and 13 (sample numbers: 9, 10, 11) days post infiltration (dpi). Three biological replicates per construct and harvesting time point were tested. Figure A and B show the same data, but the x-axes differ to show in B that LbCpf1 transcript level mediated by the binary vector controls are expressed at a very low level compared to the BSCTV-GVR constructs.

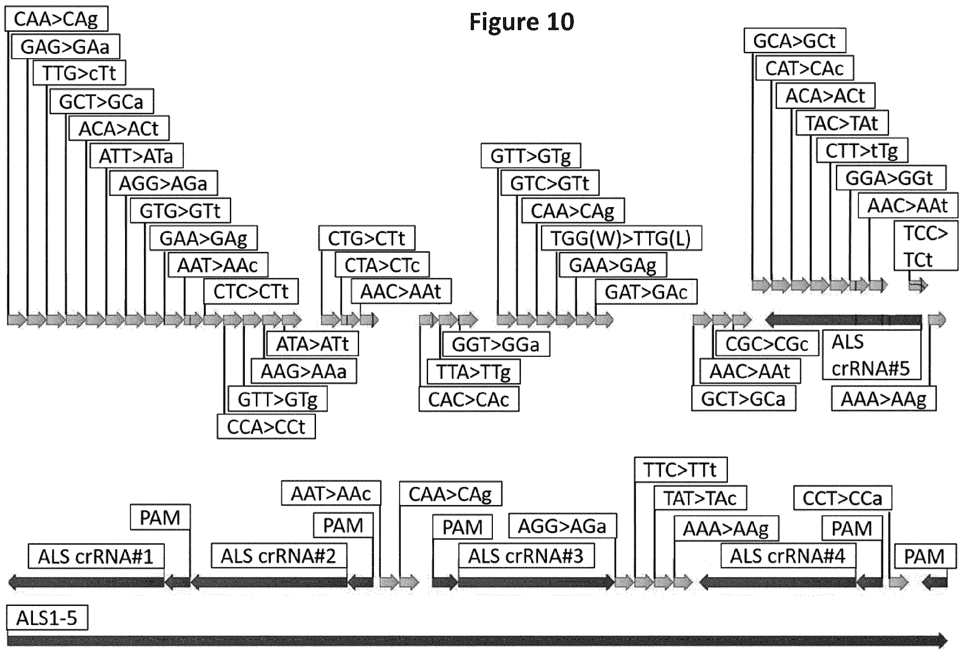

**Figure 10** shows the repair template for BvALS. Schematic view of the double-stranded repair template for introducing an amino acid exchange (Trp-Leu) in BvALS, which confers herbicide resistance towards sulfuronylurea. Templates: Double stranded DNA, Core template = 144 bp, Whole core template mutated with 44 SNPs creating silent mutations + 1 sense mutation conferring herbicide resistance against sulfonylurea, Homologous arms: 2x100 bp or 2x500 bp (or 2x1000 bp).

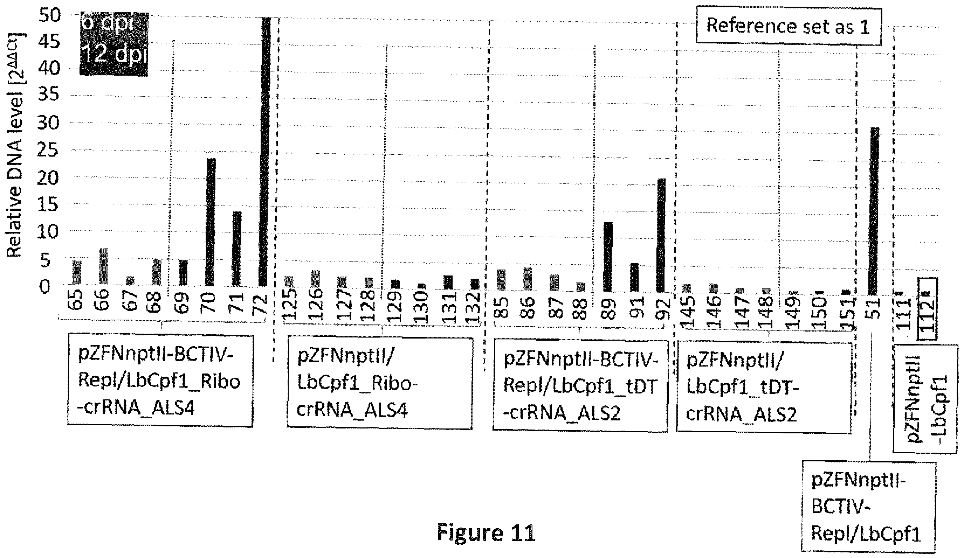

**Figure 11** shows increased DNA levels (from the LbCpf1-encoding gene) in BCTIV-GVRs infiltrated plants compared to the corresponding binary vector controls at 6 dpi and 12 dpi, respectively. Cotyledons of sugar beet seedlings were syringe-infiltrated with GVRs derived from BCTIV (pZFNnptII-BCTIV-RepI), which contain genome editing components (LbCpf1, LbCpf1+tDT-crRNA_ALS2 or LbCpf1+Ribo-crRNA_ALS4). GE components expressed via a binary vector (pZFNnptll) were used as non-GVR controls. DNA level were measured via qPCR using LbCpf1-specific primer at 6 (sample numbers 65, 66, 67, 68, 125, 126, 127, 128, 85, 86, 87, 88, 145, 146, 147, 148) and 12 (sample numbers 69, 70, 71, 72, 129, 130, 131, 132, 89, 91, 92, 149, 150, 151, 51, 111, 112) days post infiltration (dpi). Each bar represents one biological replicate per construct and harvesting time point. Sample 112 (boxed) was used as a reference (set as 1).

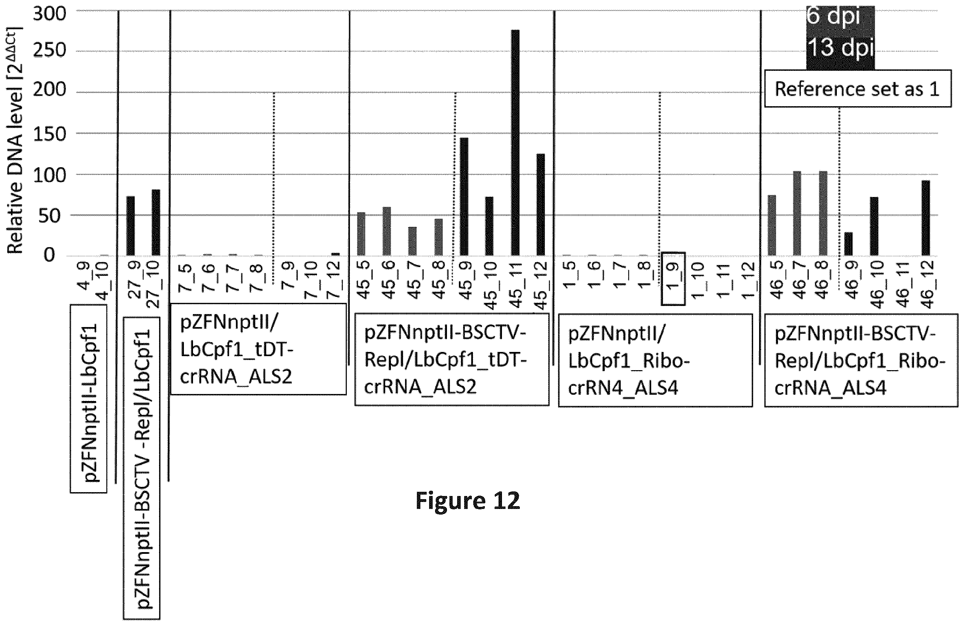

**Figure 12** shows increased DNA levels (from the LbCpf1-encoding gene) in BSCTV-GVRs infiltrated plants compared to the corresponding binary vector controls. Cotyledons of sugar beet seedlings were syringe-infiltrated with GVRs derived from BSCTV (pZFNnptII-BSCTV-RepI), which contain genome editing components (LbCpf1, LbCpf1+tDT-crRNA_ALS2 or LbCpf1+Ribo-crRNA_ALS4). GE components expressed via a binary vector (pZFNnptll) were used as non-GVR controls. DNA level were measured via qPCR using LbCpf1-specific primers at 6 (sample numbers 4_9, 4_10, 7_5 - 7_8, 45_5 - 45_8, 1_5 - 1_8, 46_5 - 46_8) and 13 (27_9, 27_10, 7_9, 7_10_7_12, 45_9 - 45_12, 1_9 - 1_12, 46_9_46_12) days post infiltration (dpI). Each bar represents one biological replicate per construct and harvesting time point. Sample 1_9 (boxed) was used as a reference (set as 1).

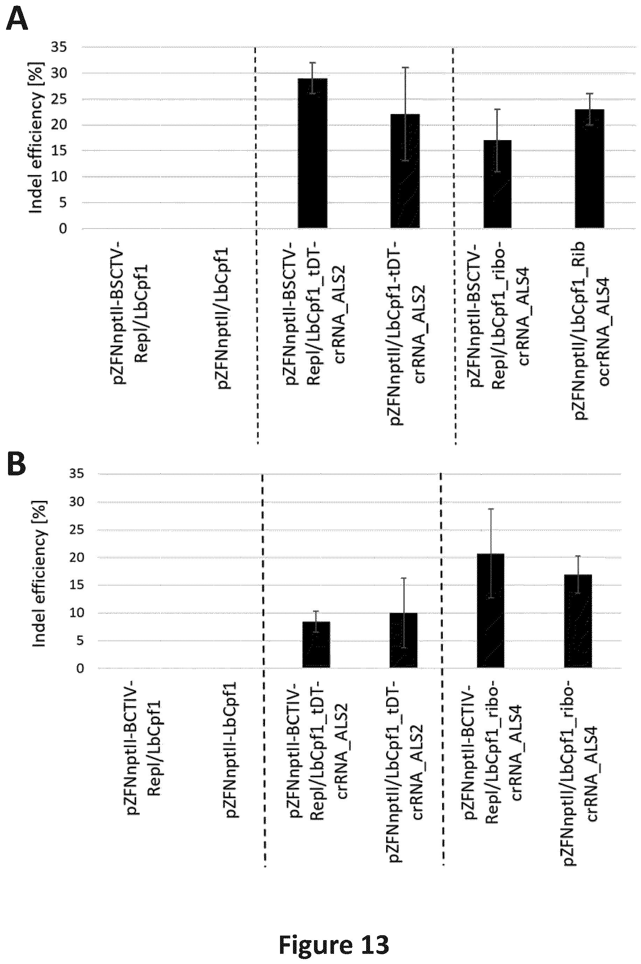

**Figure 13** shows indel efficiencies comparing plants infiltrated with the BSCTV_GVRs (A), the BCTIV -GVRs (B) and the respective binary vector controls. (**A**) Cotyledons of sugar beet seedlings were syringe-infiltrated with GVRs derived from BSCTV (pZFNnptII-BSCTV-RepI), which contain genome editing components (LbCpf1, LbCpf1+tDT-crRNA_ALS2 or LbCpf1+Ribo-crRNA_ALS4). GE components expressed via a binary vector (pZFNnptll) were used as non-GVR controls. Indel efficiencies were determined via ddPCR from leaf samples harvested 13 days post infiltration (dpI). Error bars represent standard deviation. n=2 for constructs lacking a crRNA (non-edited controls); n=3-4 for constructs harboring a crRNA. (**B**) Cotyledons of sugar beet seedlings were syringe-infiltrated with GVRs derived from BCTIV (pZFNnptII-BCTIV-RepI), which contain genome editing components (LbCpf1, LbCpf1+tDT-crRNA_ALS2 or LbCpf1+Ribo-crRNA_ALS4). GE components expressed via a binary vector (pZFNnptll) were used as non-GVR controls. Indel efficiencies were determined via NGS analysis from leaf samples harvested 12 days post infiltration (dpI). Error bars represent standard deviation. n=2 for constructs lacking the crRNA (non-edited controls); n=3-4 for constructs harboring a crRNA.

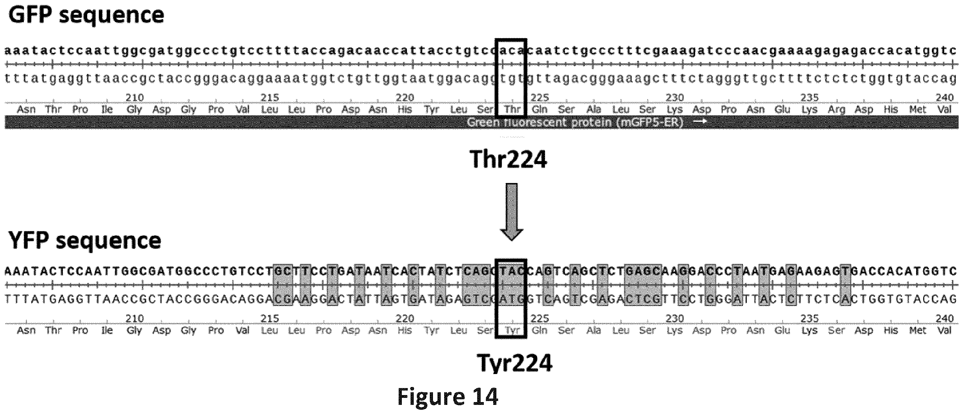

**Figure 14** shows sequence sections from GFP (upper panel) and YFP (lower panel), respectively. The nucleotides in boxes are synonymous mutations that are introduced by the donor template into the GFP sequence. The non-synonymous mutations that lead to a conversion of Thr224 into Tyr224 (T224Y) result in conversion of GFP into YFP.

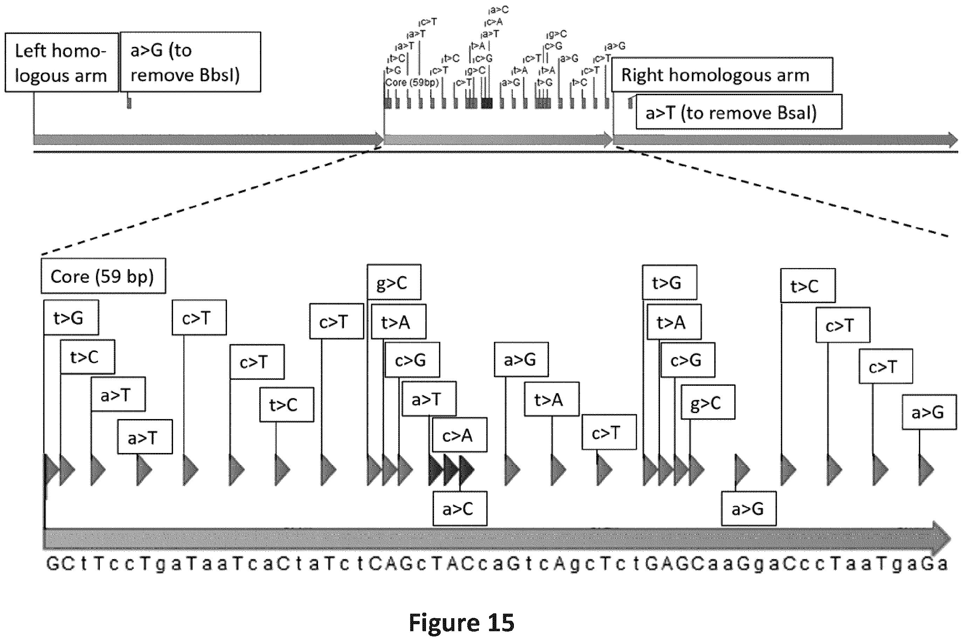

**Figure 15** shows the repair template (RT) that consists of two homologous arms, which are complementary to the target region in mGFP5-ER. The "core" of the RT includes 23 synonymous mutations, which avoid binding and cutting of the CRISPR complex after RT integration. In addition, the "core" includes 3 non-synonymous mutations, which lead to the desired T224Y amino acid exchange.

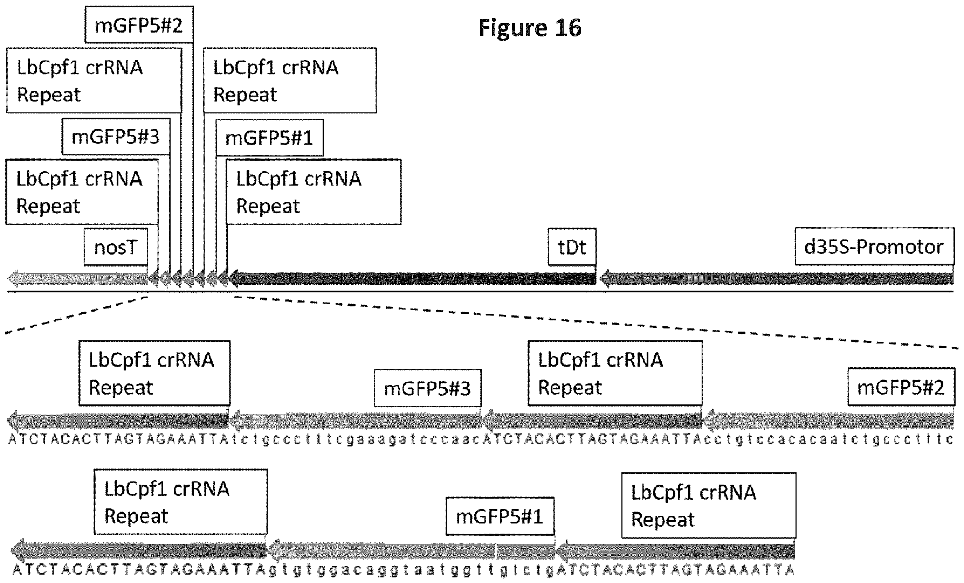

**Figure 16** shows the crRNA expression cassette, which is part of the construct *pZFNnptII-LbCpf1_tDT-crRNA_mGFP#1+2+3_GFPtoYFP template* (SEQ ID NO: 32).

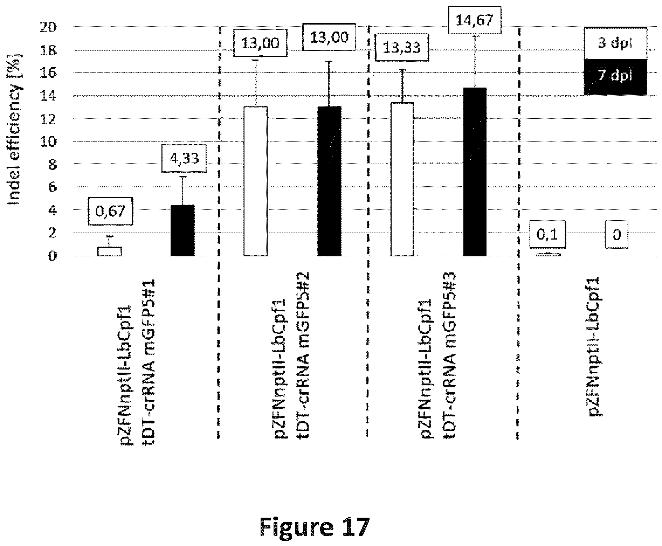

**Figure 17** shows SDN-1 editing efficiency in infiltrated tobacco leaves. *Nicotiana benthamiana* leaves were syringe-infiltrated with pZFNnptll/LbCpf1_tDT-crRNA_mGFP#1, #2 or #3. pZFNnptII/LbCpf1 without any crRNAs was used as a negative control. Indel efficiency was determined via ddPCR from leaf samples harvested 3 and 7 days post infiltration (dpI). Error bars represent standard deviation (n=3).

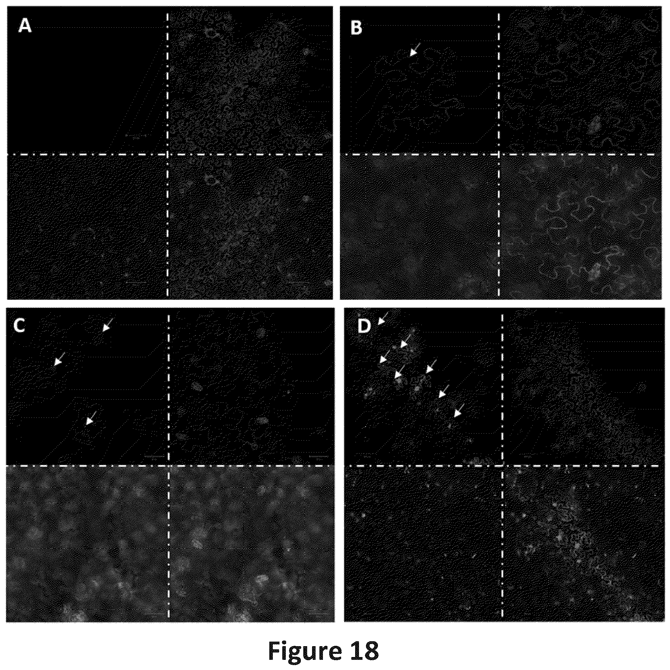

**Figure 18** shows homology-directed repair (HDR) after the induction of CRISPR/Cas-mediated double-stand breaks (DSBs) in GFP transgenic *Nicotiana benthamiana* (16c) using a Beet severe curly top virus replicon (BSCTVRepI) as a delivery vector. Epifluorescence of leaf tissues are shown after excitation with 488-nm laser light for green fluorescent protein (GFP) in the top right panel and 514-nm laser light (top left panel) in the spectral un-mixing for the yellow fluorescent protein (YFP) using confocal laser microscopy. For each treatment a bright field of a leaf (bottom left) and a merge merged image (bottom right) is shown. Images were taken at 7 days after agroinoculation. pZFN-BSCTVRepI-LbCpf1 (A) which contains only the nuclease but not the crRNA and donor repair template was used as a control. Conversion of transgene GFP to YFP by homologous recombination occurs after changing a single amino acid mutation (Thr224Tyr) in GFP. GFP to YFP conversion is shown in B, C and D (marked with arrows) transgenic plants which have been inoculated with pZFN-LbCpf1-tDT-crRNA_mGFP#1+2+3-GFPYFP-RT (B), pZFN-BSCTV-RepI-LbCpf1-tDT-crRNA_mGFP#1+2+3-GFPYFP-RT (C) or pZFN-BSCTVRepl-tDT-crRNA_mGFP#1+2+3-GFPYFP-RT200-Out-lbcpf1 (D), respectively.

**Brief description of Sequences**

| **SEQ ID NO:** | **Name** | **Description** |
|---|---|---|
| 1 | ABA inducible promoter 2xS -2xD | ABA inducible promoter for the inducible expression of the viral replication initiator protein (Rep) for improved callus development and/or shoot regeneration |
| 2 | ABA inducible promoter 4D | ABA inducible promoter for the inducible expression of the viral replication initiator protein (Rep) for improved callus development and/or shoot regeneration |
| 3 | Dexamethasone inducible system | Dexamethasone inducible system for the inducible expression of the viral replication initiator protein (Rep) for improved callus development and/or shoot regeneration |
| 4 | BCTIV replicon | BCTIV replicon sequence |
| 5 | pZFNnptll-BCTIV-Rep/MCS | Full sequence of binary vector pZFNnptll (for Agrobacterium-mediated transformation) including the BCTIV replicon |
| 6 | BSCTV replicon | BSCTV replicon sequence |
| 7 | pZFNnptll-BSCTV-Rep/MCS | Full sequence of the binary vector pZFNnptll including the BSCTV replicon |
| 8 | pZFNnptll-BCTIV-Repl-35S-GFP | Full sequence of the binary vector pZFNnptll (for Agrobacterium-mediated transformation) including the BCTIV replicon and an expression cassette for GFP |
| 9 | pZFNnptll-BSCTV-RepI-35S-GFP | Full sequence of binary vector pZFNnptll (for Agrobacterium-mediated transformation) including the BSCTV replicon and an expression cassette for GFP |
| 10 | pZFNnptII-BeYDV RepI-35S-GFP | Full sequence of binary vector pZFNnptll (for Agrobacterium-mediated transformation) including the BeYDV replicon and an expression cassette for GFP (control) |
| 11 | pFZNnptII-35-GFP | Full sequence of binary vector pZFNnptll (for Agrobacterium-mediated transformation) including an expression cassette for GFP (control) |
| 12 | pZFNnptII-BCTIV-RepI/LbCpf1_RibocrR NA_ALS4_RT_ALS-mutated_2x100 | Binary vector for the Agrobacterium-mediated - transformation coding for the replicon (BCTIV) including GE components (SSN, crRNA and the repair template (ALS)) and 2^{∗}100 nt homology arms; crRNA is produced from a Pol II promoter using the ribozyme-based strategy (He et al. 2017) |
| 13 | pZFNnptII-BCTIV-RepI/LbCpf1_tDT-crRNA_ALS2_RT_AL S-mutated_2x100 | Binary vector for Agrobacterium-mediated - transformation coding for the replicon (BCTIV) including GE components (SSN, crRNA and the repair template (ALS)) and 2^{∗}100 nt homology arms; the crRNA is embedded in a Pol II-driven RNA transcript as described before in Zhong et al., 2017. This transcript includes an mRNA encoding the fluorescent protein tDT. |
| 14 | pZFNnptII-BSCTV-RepI/LbCpf1_RibocrR NA_ALS4_RT_ALS-mutated_2x100 | Binary vector for Agrobacterium-mediated - transformation coding for the replicon (BSCTV) including GE components (SSN, crRNA and the repair template (ALS)) and 2^{∗}100 nt homology arms; crRNA is produced from a Pol II promoter using the ribozyme-based strategy (He et al. 2017). |
| 15 | pZFNnptII-BSCTV-RepI/LbCpf1_tDT-crRNA_ALS2_RT_AL S-mutated_2x100 | Binary vector for Agrobacterium-mediated - transformation coding for the replicon (BSCTV) including GE components (SSN, crRNA and the repair template (ALS)) and 2^{∗}100 nt homology arms; the crRNA is embedded in a Pol II-driven RNA transcript as described before in Zhong et al., 2017. This transcript includes an mRNA encoding the fluorescent protein tDT. |
| 16 | crRNA_ALS#1 | Selected protospacer within the target gene BvALS for PAM TTTC |
| 17 | crRNA_ALS#2 | Selected protospacer within the target gene BvALS for PAM TTTA |
| 18 | crRNA_ALS#3 | Selected protospacer within the target gene BvALS for PAM TTTA |
| 19 | crRNA_ALS#4 | Selected protospacer within the target gene BvALS for PAM TTTC |
| 20 | crRNA_ALS#5 | Selected protospacer within the target gene BvALSfor PAM TTTG |
| 21 | RT_ALS-mutated_2x100bp arms_HindIII | Full sequence of the ALS repair template |
| 22 | BCTIV virus sequence | NCBI Reference Sequence: KP410285.1 |
| 23 | BSCTV virus sequence | NCBI Reference Sequence: U02311.1 |
| 24 | LbCpf1 | Nuclease sequence optimized for *Arabidopsis thaliana* |
| 25 | LbCpf1_tDTcrRNA_AL S#1 | Construct (comprising the fragment shown in Figure 5 A (SEQ ID NO: 29)) |
| 26 | LbCpf1_RibocrRNA_A LS#1 | Construct (comprising the fragment shown in Figure 5 B (SEQ ID NO: 30)) |
| 27 | BMCTV | NCBI Reference Sequence: U56975.1 |
| 28 | BCTV | NCBI Reference Sequence: M24597.2 |
| 29 | mGFP5-ER CDS | GFP coding sequence as expressed in the transgenic *Nicotiana benthamiana* 16c line (Philips et al., 2017, Figure 14). PAM sequence 1 comprises bps 645 - 648, crRNA_mGFP5#1 comprises bps 650-673, PAM sequence 2 comprises bps 687 - 690, crRNA_mGFP#2 comprises bps 663-686, PAM sequence 3 comprises bps 700 - 703 and crRNA_mGFP#2 comprises bps 676-699. The desired mutation (T224Y) is at position 670-672. |
| 30 | mGFP5-ER PRT | GFP protein sequence as expressed in the transgenic *Nicotiana benthamiana* 16c line (Philips et al., 2017 (The widely used *Nicotiana benthamiana* 16c line has an unusual T-DNA integration pattern including a transposon sequence. *PLoS ONE* 12(2): e0171311. https://doi.org/10.1371/journal.pone.0171311), Figure 14). |
| 31 | GFP to YFP repair template | GFP to YFP repair template (Figure 15) |
| 32 | pZFNnptII/LbCpf1_tDT - crRNA_mGFP#1+2+3 _GFP toYFP template | Binary vector without replicon sequences (non GVR control) including the expression cassettes for nptll, LbCpf1 and tDT-crRNA_mGFP#1+2+3, directed against three target regions in mGFP5-ER (Figure 16). The crRNA is embedded in a Pol II-driven RNA transcript as described by Zhong et al., 2017 |
| | | (doi: 10.1038/nchembio.2410). The binary vector additionally encodes for the repair template for GFP to YFP exchange (Figure 15). |
| 33 | pZFNnptII-BSCTV-RepI/LbCpf1_tDT-crRNA_mGFP#1+2+3 _GFPtoYFP Template | Binary vector enconding an nptll expression cassette and a viral replicon sequence derived from Beet severe curly top virus (BSCTV). LbCpf1, tDT-crRNA_mGFP#1+2+3 and the RT for GFP to YFP exchange (Figure 15) are located within the viral replicon sequence. The crRNA tDT-crRNA_mGFP#1+2+3 is directed against three target regions in mGFP5 (Figure 16) and embedded in a Pol II-driven RNA transcript as described by Zhong et al., 2017. |
| 34 | pZFNnptII-BSCTV-RepI/ tDT-crRNA_mGFP#1+2+3-RT GFPtoYFP200-Out LbCpf1 | Binary vector enconding an nptll expression cassette and a viral replicon sequence derived from Beet severe curly top virus (BSCTV). Repair template (Figure 15) and tDT-crRNA_mGFP#1+2+3 are located within the viral replicon sequence expression cassettes. This GVR construct is characterized by a LbCpf1 expression cassette, which is located outside the viral replicon, but still within the T-DNA. |

### Detailed Description

The present invention is based on the relevant finding that plants known to be recalcitrant to transformation can be conveniently modified via genome editing/engineering (GE) in case new viral replicons are provided, specifically designed and optimized to (i) have a tropism for the relevant plant, (ii) to be safe and efficient in their use and in replication, (iii) have the advantage of being cargos to deliver GE tools, and (iv) have additional advantages in specifically interacting with a plant cell cycle so that desired and site-specific HDR events are strongly favored.

In a first aspect, a method for the targeted modification of at least one genomic target sequence in a plant cell preferably from a plant of the family *Amaranthacea* or *Solanaceae,* preferably a cell of a plant from the genus *Beta* or *Spinacia,* more preferably a cell of a plant of the species *Beta vulgaris* (e.g., sugar beet; SB) or *Spinacia oleracea* (spinach) is provided, wherein the method comprises the following steps: (a) introducing into the cell (i) at least one viral replicon, or a sequence encoding the same, the replicon being preferably constructed based on a Becurtovirus, or a Curtovirus genome, wherein the at least one viral replicon further comprises: (ii) at least one genome editing system comprising at least one nucleic acid guided nuclease, nickase or an inactivated nuclease, or a sequence encoding the same, and optionally, in case a CRISPR system is used, at least one guide molecule, or a sequence encoding the same; (iii) and, optionally at least one repair template, or a sequence encoding the same; (b) cultivating the cell under conditions allowing the expression and/or assembly of the at least one genome editing system and the at least one guide molecule and/or optionally at least one repair template; and (c) obtaining at least one modified cell; and (d) optionally obtaining at least one plant, plant tissue, organ, or seed regenerated from the at least one modified cell.

The present invention hereby solves the problem to provide methods and systems for increasing the efficiency of targeted modification of a nucleotide sequence of interest in the genome of a plant or a plant cell recalcitrant to modifications with state of the art methods and thus provides efficient and robust methods to regenerate new plants from the transformed plant cells, or plant material.

Since the agronomically highly relevant *Beta vulgaris* plant is not a natural host for the Mastrevirus BeYDV, in sugar beet BeYDV-based GVRs do not replicate and cannot interact with host factors as efficient as in model plants, e.g., *Nicotiana tabacum* or *Arabidopsis.* Therefore, no suitable sugar beet Geminivirus-based replicons can be obtained starting from Geminiviruses *per se* having a low tropism or infectious and replicative potential in this commercially relevant plant. This was confirmed by quantifying and comparing the replication intensities of GVRs based on BeYDV, BSCTV (Beet severe curly top virus) and BCTIV (Beet curly top iran virus) in sugar beet as shown in the below examples.

According to the disclosure provided herein, the limitations of using BeYDV-based replicons in *Beta vulgaris,* particularly in sugar beet, are overcome by using GVRs, derived from Geminiviruses from different viral genera. The present invention thus specifically provides replicons of two viruses for the heterologous expression of genome editing components in *Beta vulgaris,* namely:
(i) Beet curly top iran virus (BCTIV, SEQ ID NO: 22), belonging to the genus Becurtovirus; and
(ii) Beet severe curly top virus (BSCTV, SEQ ID NO: 23), belonging to the genus Curtovirus.

Using GVRs derived from BSCTV or BCTIV instead of BeYDV leads to a significantly increased replication intensity in *Beta vulgaris* leading to high expression levels of the heterologous sequence. This heterologous sequence can for instance encode GE components as well as optionally a repair template and thereby might enhance HDR-mediated genome editing efficiency. Furthermore, GVRs can revert host cells into the S-phase of the cell cycle, which stimulates homology-dependent recombination and thus increases efficiency additionally.

In particular, species like for example *Beta vulgaris* (such as sugar beet, fodder beet, red beet, or chard) are well known as being very recalcitrant in transgenic modifications. The same applies for other dicotyledonous plants, in particular plants within the taxonomic family of *Amaranthacea.* After establishing suitable viral replicons for recalcitrant plants, the inventors found that certain of these replicons can have a broad spectrum of tropism for other target plants, e.g., non-crop plants. This indicates, for example, that the use of Geminivirus replicons (GVRs) derived from sugar beet-infecting viruses is not restricted to sugar beet but can be extended to other non-crop species like tobacco (*Nicotiana benthamiana*) belonging to the family *Solanaceae*)*.* To achieve one of the main objects of the present invention to establish suitable viral vectors for recalcitrant plants, it is thus necessary to first establish a new viral replicon very specific for the recalcitrant plant species, as usual viral vectors, including GVRs, will not be efficient. The viral replicons established may additionally be suitable to transform other target plants effectively, particularly those target plants known to be easy or relatively easy to transform or transfect.

In one embodiment, the target plant to be transformed according to the methods of the present invention is thus selected from a plant belonging to the family *Amaranthacea* or *Solanaceae.* In preferred embodiments, the methods disclosed herein are thus genotype-independent based on the preceding efforts to provide new GVRs based on specific (Be-)Curtoviruses.

A genomic target sequence of interest according to any of the embodiments described herein may be an endogenous or isolated nucleic acid region of a plant cell or organism including any organellar genomic target sequence.

In a preferred embodiment, the at least one viral replicon, or a sequence encoding the same, may be a replicon being constructed based on a Becurtovirus, or a Curtovirus genome. It was surprisingly found that completely new Geminivirus-based replicons (GVRs) derived from BSCTV or BCTIV instead of BeYDV lead to a significantly increased replication intensity in *Beta vulgaris* and other *Amaranthacea* plants leading to high expression levels of the heterologous sequence, which for instances encodes GE components as well as optionally a repair template and thereby might enhance HDR-mediated genome editing efficiency. Furthermore, this specific GVRs can revert host cells into the S-phase of the cell cycle, which stimulates homology-dependent recombination and thus increases efficiency additionally. A viral replicon as used herein thus refers to an expression construct based on a specific naturally occurring virus and comprising a DNA molecule or RNA molecule, or a region of DNA or RNA, that can replicate from a single origin of replication, wherein the viral replicon maintains relevant characteristics of the native virus, in particular regarding the plant tropism and its replication efficiency and further beneficial effects, but is modified to be used as expression construct and does not comprise all virulence factors of the native virus.

As introduced above, Beet curly top iran virus (BCTIV) is a new and not yet detailed studied member of Becurtoviruses with a unique nanonucleotide in the LIR and a rather different member of the family *Geminiviridae.* This virus encodes five genes (V1, V2 and V3 on the virion-sense strand and Rep and C2 genes on the complementary-sense strand). The genome sequence and structure of the virion-sense stand of Becurtoviruses is similar to Curtoviruses while the complementary-sense strand has similarity to the Mastreviruses. Beet severe curly top virus (BSCTV) and Beet curly top virus (BCTV) are members of Curtoviruses. Curtoviruses are monopartite Geminiviruses that are vectored by leafhoppers, often in a species-specific manner and rather different to Mastreviruses. BCTV encodes usually 7 genes (V1, V2, V3 on virion-sense strand and Rep, C2, C3 and C4 on the complementary-sense strand) without SIR sequence. Therefore, Becurtovirus and Curtovirus are different in genomic architecture to other Geminivirus replicon systems and thus need certain optimizations to be suitable at all for use as viral replicons, as comparable replicons are not available at date. Obviously, specific adaptations within the genomic structure need to be made to (i) guarantee general functionality of a new viral replicon and (ii) to maintain and improve the replication efficiency of the respective viral replicon, which is easily disturbed when modifying and re-purposing a naturally occurring virus.

According to the findings presented herein, heterologous expression of nucleic acid material for the purpose of GE is achieved using a specifically designed viral replicon derived from, in particular, a *Beta vulgaris* infecting Geminivirus (GV), or particularly modified variants of said GV. Such modified GV variants have been demonstrated to be suitable for heterologous expression of a nucleotide sequence of interest *in planta.* Thereby, the artificially created Geminivirus replicons (GVRs) of the present invention replicate efficiently inside host cells and therefore produce high amounts of replicons, in turn producing high expression of genome editing effector molecules cloned into the respective GVR.

Additionally, in certain embodiments, this can help to increase the abundance of donor or repair templates available for HDR, thereby considerably enhancing the targeting efficiency and accuracy.

In addition to rolling circle replication, GVRs as studied and constructed herein, replicate inside host cells also via homologous recombination (HR)-dependent replication, which may revert host plant cells to the S-phase. The S-phase-like endocycle is favorable for HR if, for example, supplemented with site-specific nucleases and complementary donor template.

In one embodiment of the invention, the viral replicon may be provided as single-component replicon for BCTIV, BSCTV or BCTV. For example, a BCTIV replicon may contain LIR, SIR and Rep, or a BCTV or BSCTV replicon may contain LIR and Rep, but no SIR sequence. For the specific replicons newly established and tested, the inventors further found that HDR-based GE efficiency can be significantly increased by using the specific (Be-)Curtovirus GVR, as these viral replicons were constructed in a way maintaining their effect of reverting a plant target cell in the S-phase of the cell cycle, which is highly favorable for HDR to occur, and which stimulates HDR-events.

In one embodiment, a viral replicon according to the present invention may thus be used to revert a host cell of interest into the S-phase and/or to improve HDR-mediated GE efficiency by specifically combining the effects inherent to the new viral replicons of interest as cargo and the GE inserts inserted into these GVR tools. This observation is supported by recent studies done in *Arabidopsis thaliana* infected with the Geminivirus *Euphorbia yellow mosaic virus* (EuYMV) *(*Richter, K. S., et al. (2016). "The recombination mediator RAD51D promotes geminiviral infection." Virology 493: 113-127.).

In a further aspect, there is thus provided a viral replicon optimized for plant transformation and based on the relevant findings on new GVRs based on (Be-)curtovirus genomes as disclosed herein, wherein the viral replicon may be encoded by a sequence according to SEQ ID NO: 4 or 6, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

In one embodiment, BCTIV- and BSCTV-GVRs may thus be individually equipped with various GE system components or tools, which are necessary to mediate SDN-1 events and optionally with at least one repair template to mediate SDN-2/3 kind modifications.

In certain embodiments, introduction of at least one viral replicon may be achieved by biological or physical means, including direct application to a cell, transfection, transformation, including transformation by *Agrobacterium spp.,* preferably by *Agrobacterium tumefaciens,* biolistic bombardment, transfection using chemical agents, including polyethylene glycol transfection, or any combination thereof. In a preferred embodiment, the viral replicon may be integrated into a vector suitable for *Agrobacterium-mediated* plant (cell) transformation such as a binary vector. In another preferred embodiment, the viral replicon may be integrated into a vector suitable for transformation via biolistic bombardment; this is not necessarily a binary vector.

In certain embodiments, the at least one viral replicon, or the sequence encoding the same, may be constructed based on a Beet curly top iran virus Becurtovirus genome, a Spinach Curly Top Arizona Virus Becurtovirus genome, a Beet severe curly top virus Curtovirus genome, a Beet curly top virus Curtovirus genome, a Beet mild curly top virus Curtovirus genome, or a Spinach severe curly top virus Curtovirus genome, preferably a genome selected from SEQ ID NOs: 22, 23, 27 or 28, or a genome from a homologous virus to the respective sequences belonging to the genus Becurtovirus or Curtovirus, wherein a homologous virus may be understood as a naturally occurring plant infecting virus having a comparable genome architecture as a Becurtovirus or Curtovirus and thus belonging to the same genus of Becurtovirus or Curtovirus, but having certain sequence variations.

The at least one viral replicon according to the various aspects and embodiments disclosed herein may be encoded by a sequence according to SEQ ID NO: 4 or 6, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

Whenever the present disclosure relates to the percentage of the homology or identity of nucleic acid or amino acid sequences to each other over the entire length of the sequences to be compared to each other, wherein these identity or homology values define those as obtained by using the EMBOSS Water Pairwise Sequence Alignments (nucleotide) program (www.ebi.ac.uk/Tools/psa/emboss_water/nucleotide.html) for nucleic acid sequences or the EMBOSS Water Pairwise Sequence Alignments (protein) program (www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

Generally, any suitable delivery method to introduce the components (i) or (ii) and optionally (iii) according to step (a) of the methods of the present invention into a cell can be applied, depending on the target cell. The term "introducing" as used herein thus implies a functional transport of a biomolecule or genetic construct (DNA, RNA, single- or double-stranded, protein, comprising natural and/or synthetic components, or a mixture thereof) into a cell or into a cellular compartment of interest, e.g. the nucleus or an organelle, or into the cytoplasm, which allows the transcription and/or translation and/or the catalytic activity and/or binding activity, including the binding of a nucleic acid molecule to another nucleic acid molecule, including DNA or RNA, or the binding of a protein to a target structure within the cell, and/or the catalytic activity of an enzyme such introduced, optionally after transcription and/or translation.

A variety of delivery techniques may be suitable according to the methods of the present invention for introducing the components (i) or (ii) and optionally (iii) into a cell, in particular a plant cell, the delivery methods being known to the skilled person, e.g. by choosing direct delivery techniques ranging from polyethylene glycol (PEG) treatment of protoplasts, procedures like electroporation, microinjection, silicon carbide fiber whisker technology, viral vector mediated approaches and particle bombardment. A common biological means, and a preferred cargo according to the present invention, is transformation with *Agrobacterium spp.* which has been used for decades for a variety of different plant materials. Viral vector mediated plant transformation represents a further strategy for introducing genetic material into a cell of interest.

In certain embodiments according to the various aspects of the present invention the at least one viral replicon comprising the at least one genome editing system may thus be introduced into the cell by transformation or transfection, preferably by *Agrobacterium-mediated* transformation, biolistic bombardment, micro- or nanoparticle delivery, or by chemical transfection. Specific vectors have been constructed for use according to the present disclosure and optimized for efficiently delivering the viral replicons and the encoded sequences into a cell of interest.

In one embodiment, there may thus be provided at least one viral replicon comprising at least one genome editing system, wherein the replicon is introduced into the cell by *Agrobacterium-*mediated transformation, wherein the at least one viral replicon is inserted into a binary vector, preferably wherein the binary vector comprising the at least one viral replicon has a sequence of SEQ ID NOs: 5 or 7 to 15, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

Further vectors, e.g., artificial vectors suitable as shuttle vectors, so-called because they are able to replicate in multiple hosts, e.g., *E. coli* and *Agrobacterium* comprising a transfer DNA (T-DNA) binary system necessary for agrobacterium transformation may be used and are available to the skilled person.

In preferred embodiments, the methods for the targeted modification of at least one genomic target sequence in a plant cell are used to genome editing (GE) purposes. The GE strategy may be an SDN-1 strategy exclusively introducing GE tools of interest, but no further foreign nucleic acid material. In other embodiments, SDN-2/3 strategies additionally introducing at least one repair template or donor template, said terms being used interchangeably herein, may be desired. Both strategies have been shown to be compatible with the specific viral replicons designed.

The at least one genome editing system (GE system) according to the present disclosure may preferably comprise at least one nucleic acid guided nuclease, nickase or an inactivated nuclease, or a sequence encoding the same, and at least one guide molecule, or a sequence encoding the same.

A "(targeted) modification" of at least one genomic target sequence in the context of the present invention as mediated by a GE system of interest refers to any change of a (nucleic acid) sequence that results in at least one difference in the (nucleic acid) sequence distinguishing it from the original sequence. The at least one "(targeted) modification" can be introduced as a consequence of the endogenous repair mechanism (e.g., as a consequence of NHEJ and other known endogenous repair mechanisms) after the at least one GE system introduced in a targeted way was active, or as a direct consequence of the at least one GE system, e.g., a base editor system, or another fusion system, or as a consequence of a repair template (RT) introduced at the site of a single- or double stranded break as introduced by at least one GE system. The genomic target sequence to be modified in a targeted manner can be present in the nuclear, or in the plastid genome, including, but not being limited thereto, the mitochondrial, and the chloroplast genome. Further the genomic target sequence to be modified may be present in a cell as a target nucleic acid sequence present in a cell extra-chromosomally, for example, as autonomously replicating plasmid, or as nucleic acid plasmid transiently introduced into a cell, but not (fully) integrated into the genome of a target cell. In particular, a modification can be achieved by insertion or addition of one or more nucleotide(s), or substitution or deletion of one or more nucleotide(s) of the original sequence, e.g., a targeted point mutation as mediated by a single-nucleotide exchange, or any combination of these. Specifically, this implies that a targeted modification can comprise more than one modification mediated simultaneously, for example, more than one point mutation, wherein the individual point mutations can be at least one synonymous mutation (resulting in a difference in the nucleic acid sequence, yet not in an exchange of the encoded amino acid sequence, when the sequence encodes an amino acid sequence) together with at least one non-synonymous mutation. Further, a targeted modification can comprise introducing at least one point mutation (synonymous and/or non-synonymous) together with either one of a deletion and/or an insertion in one and the same experiment, i.e., simultaneously. A targeted modification is introduced using site-specific tools such as at least one nucleic acid guided nuclease, or a variant thereof (including a nickase, or a nuclease-dead variant) which recognizes and/or cuts the target at a specific location. If two or more different guide nucleic acid sequences are used, it is possible to target multiple sites in the genomic target region and introduce "multiple modifications". The "multiple modifications" may also be made, for example, in the same gene, or the "multiple modifications" may be made into regions of the genome not directly connected, e.g., modifications in different chromosomes, and the like. More than one, or "multiple modifications" can be introduced, by way of example, by providing a repair template (RT) harboring all desired modifications in the form of at least one point mutation and, optionally, the capacity to mediate an insertion or deletion in addition to the point mutations. To this end, more than one guide RNA or crRNA may be used when "multiple modifications" are of interest, or one one guide RNA or crRNA may be used together with a RT carrying more than one modification to be effected.

A preferred GE system according to the present disclosure is preferably based on a CRISPR system comprising at least one CRISPR nuclease and at least one guide RNA.

A "site-directed nuclease" or "site-specific nuclease" can be a single stranded DNA break (SSB) inducing enzyme or a double stranded DNA break (DSB) inducing enzyme, which preferably recognizes a predetermined site in the genome of targeted cell.

A "CRISPR nuclease", as used herein, is a specific form of a site-directed nuclease and refers to any nucleic acid guided nuclease, which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. CRISPR nucleases also comprise mutants or catalytically active fragments or fusions of a naturally occurring CRISPR effector sequences, or the respective sequences encoding the same. A CRISPR nuclease may in particular also refer to a CRISPR nickase or even a nuclease-dead variant of a CRISPR polypeptide having endonucleolytic function in its natural environment. The CRISPR nucleases include CRISPR/Cas systems, including CRISPR/Cas9 systems, CRISPR/Cpf1 systems, CRISPR/C2C2 systems, CRISPR/CasX systems, CRISPR/CasY systems, CRISPR/Cmr systems, CRISPR/MAD systems, including, for example, CRISPR/MAD7 systems and CRISPR/MAD2 systems, CRISPR/CasZ systems and/or any combination, variant, or catalytically active fragment thereof. A nuclease may be a DNAse and/or an RNAse, in particular taking into consideration that certain CRISPR effector nucleases have RNA cleavage activity alone, or in addition to the DNA cleavage activity.

A "CRISPR system" is thus to be understood as a combination of a CRISPR nuclease or CRISPR effector, or a nickase or a nuclease-dead variant of said nuclease, or a functional active fragment or variant thereof together with the cognate guide RNA (or pegRNA or crRNA) guiding the relevant CRISPR nuclease.

A nucleic acid guided nuclease, or site-specific or site-directed nuclease, of any kind as delivered with a viral replicon according to the present invention thus recognizes a certain protospacer adjacent motif (PAM) at the target site, which is required to be present for the nuclease to cut the target site. The "PAM specificity" of a nuclease defines, which PAM(s) the nuclease recognizes. For example, certain variations of a PAM or different PAMs may result in cleavage. The different variants or different PAMs may provide a varying degree of nuclease activity at a target site. In the context of the present invention, a nuclease is considered to "recognize" a certain PAM, when the indel percentage at a certain site with the PAM, normalized by transformation efficiency in the system used, is at least 10%, preferably at least 20%, at least 30%, at least 40%, at least 50% or at least 60%. In this context, a "higher activity" of a nuclease refers to a higher indel percentage at a certain target site compared to another nuclease. An "indel" refers to an insertion or a deletion of one or more nucleotides at the target site, which is due to site specific nuclease activity at the target site. The frequency with which indels occur at the target site can be used as a measure for site specific nuclease activity.

In preferred embodiments, the at least one nucleic acid guided nuclease, nickase or an inactivated nuclease, or a sequence encoding the same, of the genome editing system may thus be selected from the group consisting of a CRISPR/Cas system, preferably from a CRISPR/Cfp1 system, a CRISPR/MAD7 system, a CRISPR/MAD2 system, a CRISPR/Cas9 system, a CRISPR/CasX system, a CRISPR/CasY system, or a CRISPR/Csm system, or any combination, variant, or catalytically active fragment thereof.

In certain embodiments, the GE system may be a base editor (BE) system. Base editors, including CBEs (base editors mediating C to T conversion) and ABEs (adenine base editors mediating A to G conversion), are powerful tools to introduce direct and programmable mutations without the need for double-stranded cleavage (Komor et al., Nature, 2016, 533(7603), 420-424; Gaudelli et al., Nature, 2017, 551, 464-471). In general, base editors are composed of at least one DNA targeting module and a catalytic domain that deaminates cytidine or adenine. All four transitions of DNA (A→T to G→C and C→G to T→A) are possible as long as the base editors can be guided to the target site. Originally developed for application in mammalian cell systems, both BEs and ABEs have been optimized and applied in plant cell systems. Efficient base editing has been shown in multiple plant species (Zong et al., Nature Biotechnology, vol. 25, no. 5, 2017, 438-440*;* Yan et al., Molecular Plant, vol. 11, 4, 2018, 631-634*;* Hua et al., Molecular Plant, vol. 11, 4, 2018, 627-630). Base editors have been used to introduce specific, directed substitutions in genomic sequences with known or predicted phenotypic effects in plants and animals. But they have not been used for directed mutagenesis targeting multiple sites within a genetic locus or several loci to identify novel or optimized traits.

Base editing as detailed above, does not cut the double-stranded DNA but instead uses the CRISPR targeting machinery to shuttle an additional enzyme to a desired sequence, where it converts a single nucleotide into another. Many genetic traits in plants and certain susceptibility to diseases caused by plant pathogens are caused by a single nucleotide change, so base editing offers a powerful alternative for GE. But the method has intrinsic limitations and is said to introduce off-target mutations, which are generally not desired for high precision GE.

In yet another embodiment, the GE system may be a Prime Editing system (Anzalone et al. (2019). Search-and-replace genome editing without double-strand breaks (DSBs) or donor DNA. Nature, 1-1.). In contrast to Base Editing, Prime Editing (PE) systems steer around the shortcomings of earlier CRISPR based GE techniques by heavily modifying the Cas9 protein and the guide RNA. The altered Cas9 only "nicks" a single strand of the double helix, instead of cutting both. The new guide RNA, called a pegRNA (prime editing extended guide RNA), contains an RNA template for a new DNA sequence, to be added to the genome at the target location. That requires a second protein, attached to Cas9 or a different CRISPR effector nuclease: a reverse transcriptase enzyme, which can make a new DNA strand from the RNA template and insert it at the nicked site. To this end, an additional level of specificity is introduced into the GE system in view of the fact that a further step of target specific nucleic acid::nucleic acid hybridization is required. This may significantly reduce off-target effects. Further, the PE system may significantly increase the targeting range of a respective GE system in view of the fact that BEs cannot cover all intended nucleotide transitions/mutations (C→A, C→G, G→C, G→T, A→C, A→T, T→A, and T→G) due to the very nature of the respective systems, and the transitions as supported by BEs may require DSBs in many cell types and organisms.

In certain embodiments according to the various aspects of the present invention, the at least one GE system thus may further comprise at least one PE system and plant host compatible reverse transcriptase, preferably a modified reverse transcriptase, wherein the reverse transcriptase is preferably used in combination with a nucleic acid guided nickase, and/or at least one cytidine or adenine deaminase, preferably wherein the at least one cytidine or adenine deaminase is independently selected from an apolipoprotein B mRNA-editing complex (APOBEC) family deaminase, preferably a rat-derived APOBEC, an activation-induced cytidine deaminase (AID), an ACF1/ASE deaminase, an ADAT family deaminase, an ADAR2 deaminase, or a PmCDA1 deaminase, a TadA derived deaminase, and/or a transposon, or a sequence encoding the aforementioned at least one enzyme, or any combination, variant, or catalytically active fragment thereof.

Any nucleic acid sequence of the GE systems disclosed herein may be "codon optimized" for the codon usage of a plant target cell of interest. This means that the sequence is adapted to the preferred codon usage in the organism that it is to be expressed in, i.e. a "target cell of interest". If a nucleic acid sequence is expressed in a heterologous system, codon optimization increases the translation efficiency significantly.

A GE system, in particular a CRISPR system, comprises at least one nucleic acid guided nuclease and at least one "guide molecule" or "guide nucleic acid sequence" (usually called and abbreviated as guide RNA, crRNA, crRNA+tracrRNA, gRNA, sgRNA), which recognizes a target sequence to be cut by the nuclease. The at least one "guide nucleic acid sequence" or "guide molecule" comprises a "scaffold region" and a "target region". The "scaffold region" is a sequence, to which the nucleic acid guided nuclease binds to form a targetable nuclease complex. The scaffold region may comprise direct repeats, which are recognized and processed by the nucleic acid guided nuclease to provide mature crRNA. A pegRNA may comprise a further region within the guide molecule, the so-called "primer-binding site". The "target region" defines the complementarity to the target site, which is intended to be cleaved. A crRNA as used herein may thus be used interchangeably herein with the term guide RNA in case it unifies the effects of meanwhile well-established CRISPR nuclease guide RNA functionalities. Certain CRISPR nucleases, e.g., Cas9, may be used by providing two individual guide nucleic acid sequences in the form of a tracrRNA and a crRNA, which may be provided separately, or linked via covalent or non-covalent bonds/interactions. The guide RNA may also be a pegRNA of a Prime Editing system as further disclosed above.

A "target region/sequence" defines the complementarity to the target site, which is intended to be cleaved, and a "genomic target region/sequence" is a region in the genome, including the organellar genome, of the target cell, which is to be modified using the GE system with a viral replicon of the present invention. The target region can be an endogenous sequence, e.g. an endogenous target gene, or an isolated nucleic acid region, which is not part of the genome of the target cell but e.g. present on a plasmid or an artificial chromosome.

A "repair template" (RT) or "donor template/nucleic acid" represents a single-stranded or double-stranded nucleic acid sequence, which can be provided during any genome editing causing a double-strand or single-strand DNA break to assist the targeted repair of said DNA break by providing a RT as template of known sequence assisting homology-directed repair. The RT may comprise "symmetric or asymmetric homology arms", which provide homology to the sequences flanking the double-strand break introduced by the nuclease and thus promote error-free homology directed repair. The repair template may also comprise at least one chemically modified base or backbone, wherein a "chemically modified base" is present in the repair template, when at least one nucleobase has been modified to carry one or more substituent(s) or label(s) or one or more nucleotide(s) carry a molecule other than a nucleobase instead of a nucleobase. A "chemically modified back bone" is present in the repair template, for example, when the phosphate back bone carries at least one modification such as e.g. a phosphorothioate bond, or, for example, when at least one nucleobase has been modified to carry one or more substituent(s) or label(s) or one or more nucleotide(s) carry a molecule other than a nucleobase instead of a nucleobase. A repair template can thus be provided during any genome editing causing a double-strand and/or single-strand DNA break to assist the targeted repair of said DNA break by providing a RT as template of known sequence assisting homology-directed repair. For example, a repair template can be provided as individual molecule, or a sequence encoding the same, or it may be associated with a guide RNA of a CRISPR nuclease, wherein the association can be of non-covalent nature (complementary base pairing), or the molecules can be physically attached to each other by a covalent bond. The latter embodiment may increase the availability of the at least one repair template of interest at the target site in a genome, where a targeted double- or single-stranded break was introduced which is to be repaired.

In certain embodiments, the at least one repair template may comprise symmetric or asymmetric homology arms and/or the at least one repair template may comprise at least one chemically modified base and/or backbone.

In certain embodiments, it may be preferably to add a repair template to the GE system to obtain a GE event in a highly targeted way for SDN-2/3 settings. Therefore, the at least one genome editing system may comprise at least one repair template, and wherein the at least one repair template may comprise or encode a double- and/or single-stranded nucleic acid sequence, and/or wherein the at least one repair template may be part of the at least one guide molecule.

In another aspect according to the present invention, what is provided may be a plant, plant cell, tissue, organ, or seed obtainable by or obtained by a method according to any of the methods for the targeted modification of at least one genomic target sequence in a plant cell as disclosed herein. As obtaining at least one plant, plant tissue, organ, or seed by regenerating the same from at least one cell modified herein is readily available to the skilled person in the field of plant biotechnology and breeding, the plant material modified in a targeted manner can be used to obtain a whole plant organism, or a part thereof, carrying the desired modification, preferably in an inheritable manner. "Regenerating / regeneration" in this context is to be understood broadly and refers to any kind of *in planta, in vitro,* or a combined *in vivo*/*ex vivo* cultivation of at least one modified plant cell, tissue, organ, or material.

Suitable conditions to cultivate plant cells and plant material as modified according to the methods of the present invention in vitro and in vivo are disclosed herein and are known to the skilled person.

To avoid negative effects on the callus development and/or shoot regeneration, which steps may in certain embodiments be desired for a regeneration step, an inducible promoter system may be used for the expression of the viral replication initiator protein (Rep). Such inducible systems include but are not limited to inducible promoter systems such as the abscisic acid-inducible promoter (SEQ ID NOs: 1 and 2) or a glucocorticoid receptor-based system inducible by dexamethasone (Figure 2, SEQ ID NO: 3). Further inducible systems compatible for use in plant cells are known to the skilled person.

In certain embodiments, the methods of the present invention may be performed by transiently introducing at least one viral replicon, or a sequence encoding the same and/or at least one genome editing system comprising at least one nucleic acid guided nuclease, nickase or an inactivated nuclease, or a sequence encoding the same, and at least one guide molecule, or a sequence encoding the same and/or and, optionally at least one repair template, or a sequence encoding the same, whereas in other embodiments, the tools may be introduced in a stable way. A combination of transient and stable transformations is possible in view of the fact that at least two components are inserted into the at least one cell to be modified. "Transient" implies that the tools are only temporarily expressed and afterwards degraded by the cell, whereas "stable" implies that at least one of the tools is integrated into the nuclear and/or organellar genome of the cell to be modified.

In one aspect, an expression construct is provided, i.e., a nucleic acid cassette comprising several elements for transcribing and/or translating encoded elements in at least one target cell of interest, which may comprise or encode: (i) at least one viral replicon based on a (Be-)Curtovirus as disclosed according to the various aspects and embodiments herein, and (ii) a vector, preferably a (binary) vector as defined above suitable for *Agrobacterium-mediated* transformation, and (iii) at least one component of at least one a genome editing system, wherein the genome editing system comprises or encodes: (a) at least one nucleic acid guided or site-specific nuclease, nickase or an inactivated nuclease, or at least one site-directed nuclease, nickase or an inactivated nuclease, and optionally at least one nuclear or organellar localization sequence; and (b) at least one guide molecule for guiding the at least one nucleic acid guided nuclease, nickase or an inactivated nuclease and optionally for activating at least one reverse transcriptase; and (c) optionally at least one repair template; and (d) optionally at least one reverse transcriptase; and (iv) wherein the expression construct comprises or encodes at least one regulatory sequence, wherein the regulatory sequence is selected from the group consisting of a core promoter sequence, a proximal promoter sequence, a cis regulatory sequence, a trans regulatory sequence, a locus control sequence, an insulator sequence, a silencer sequence, an enhancer sequence, a terminator sequence, an intron sequence, and/or any combination thereof.

In one embodiment of the expression construct described above, the construct comprises or encodes at least one regulatory sequence, wherein the regulatory sequence is selected from the group consisting of a core promoter sequence, a proximal promoter sequence, a cis regulatory sequence, a trans regulatory sequence, a locus control sequence, an insulator sequence, a silencer sequence, an enhancer sequence, a terminator sequence, an intron sequence, and/or any combination thereof.

In another embodiment of the expression construct described above, the regulatory sequence comprises or encodes at least one promoter selected from the group consisting of ZmUbi1, BdUbi10, ZmEf1, a double 35S promoter, a rice U6 (OsU6) promoter, a rice actin promoter, a maize U6 promoter, PcUbi4, Nos promoter, AtUbi10, BdEF1, MeEF1, HSP70, EsEF1, MdHMGR1, or a combination thereof.

In a further embodiment of the expression construct described above, the at least one intron is selected from the group consisting of a ZmUbi1 intron, an FL intron, a BdUbi10 intron, a ZmEf1 intron, a AdH1 intron, a BdEF1 intron, a MeEF1 intron, an EsEF1 intron, and a HSP70 intron.

In one embodiment of the expression construct according to any of the embodiments described above, the construct comprises or encodes a combination of a ZmUbi1 promoter and a ZmUbi1 intron, a ZmUbi1 promoter and FL intron, a BdUbi10 promoter and a BdUbi10 intron, a ZmEf1 promoter and a ZmEf1 intron, a double 35S promoter and a AdH1 intron, or a double 35S promoter and a ZmUbi1 intron, a BdEF1 promoter and BdEF1 intron, a MeEF1 promoter and a MeEF1 intron, a HSP70 promoter and a HSP70 intron, or of an EsEF1 promoter and an EsEF1 intron.

In addition, the expression construct may comprise at least one terminator, which mediates transcriptional termination at the end of the expression construct or the components thereof and release of the transcript from the transcriptional complex.

In one embodiment of the expression construct according to any of the embodiments described above, the regulatory sequence may comprise or encode at least one terminator selected from the group consisting of nosT, a double 35S terminator, a ZmEf1 terminator, an AtSac66 terminator, an octopine synthase (ocs) terminator, or a pAG7 terminator, or a combination thereof. A variety of further suitable promoter and/or terminator sequences for use in expression constructs for different plant cells are well known to the skilled person in the relevant field.

A "nuclear/organellar localization signal/sequence" (abbreviated as NLS/OLS, respectively) refers to an amino acid sequence, or a sequence encoding the same, which is usually added at the C-terminus and/or the N-terminus of a polypeptide or protein, or the sequence encoding the same, which causes the polypeptide or protein to be imported into the cellular nucleus or organelle by nuclear transport or intracellular trafficking in a host cell of interest. More than one NLS or OLS sequence may be present in a target protein, usually a GE system effector nuclease, nickase or nuclease-dead variant thereof. Suitable NLS and OLS sequences for different plant cells are described in the literature.

In one embodiment, the expression construct may additionally comprise or encode at least one self-cleaving ribozyme, preferably at least one hammerhead ribozyme and/or at least one hepatitis-delta virus (HDV) ribozyme and/or at least one artichoke, sunflower or rice HDV-like ribozyme.

A "self-cleaving ribozyme" is an RNA molecule that is capable to catalyse its own cleavage at a specific site. Upon transcription, self-cleaving ribozymes fold into a specific structure, sometimes requiring the presence of certain metal cations, which induces cleavage of the phosphodiester backbone at a certain position. A number of ribozymes are known, which can be used in a variety of settings. These ribozymes may be particularly suitable according to the present disclosure to "activate" encoded GE system components, e.g., various guide RNAs etc., by cleaving and thus activating these crRNAs/pegRNAs/guide RNAs to make them available for interaction with a CRISPR effector protein of interest which is turn targeted to a target site in the plant genome to be modified in a site-directed way.

The expression construct may thus also comprise at least one ribozyme, which upon transcription cleaves the (RNA) transcript at one or more predetermined location(s). If placed strategically, the ribozyme(s) release and activate the components of the expression construct from the transcript. For example, the sequence encoding the CRISPR nuclease (or variant thereof) may be flanked by a ribozyme at the 5'- and at the 3'-end. The guide nucleic acid sequence(s) may be included between ribozyme and nuclease sequence and can be processed by the certain CRISPR nucleases (e.g., Cpf1 variants and MAD7-type nucleases having an intrinsic RNA-cleavage activity in addition to the DNA-cleavage activity) to provide mature crRNA/pegRNA/guide RNA in turn needed for guidance of the CRISPR effector nuclease or variant thereof.

In yet another embodiment, the expression construct may additionally comprise or encode a sequence for an RNA polymerase II (Pol II) promoter. RNA polymerase II promoters, which constitute the majority of the characterized promoters, cannot be directly used for CRISPR guide RNA production *in vivo* because of: (1) The primary transcripts of RNA polymerase II promoters undergo extensive processing such as 5'-end capping, 3'-end polyadenylation, and splicing out of the introns. Some of the modifications may thus render the designed guide RNA non-functional; and (2) the mature RNA molecules are transported into cytosol, thus they are physically separated from the intended targets that are located in the nucleus (Zhang et al., doi:10.21769/BioProtoc.2148). That may be the reason why production of guide RNA *in vivo* using U6 and U3 snRNA promoters has long been the dominant method (Gao and Zhao, Self-processing of ribozyme-flanked RNAs into guide RNAs in vitro and in vivo for CRISPR-mediated genome editing. J. Integr. Plant Biol. 2014; 56(4):343-349).

As it is known for certain CRISPR systems and effectors, e.g. the Cpf1 system, Cpf1 and orthologs thereof, including LBCpf1 and AsCpf1 have RNase activities that can excise multiple CRISPR RNAs (crRNAs) from a single Pol II-driven RNA transcript (Zhong et al., 2017, *supra*)*.* Therefore, crRNA excised from a Pol II-driven mRNA transcript may be used in the certain embodiments in the replicons and/or expression constructs and/or binary vectors disclosed herein to effectively provide said crRNAs as guiding RNAs to the cognate CRISPR effector.

According to certain embodiments, a construct or replicon according to the present invention may either comprise at least one self-cleaving ribozyme encoding sequence and at least one RNA pol II promoter, or it may comprise both elements.

According to certain embodiments, a DNA-dependent RNA polymerase type II promoter may thus be used, preferably together with at least one sequence encoding at least one self-cleaving ribozyme to further enhance the activation of the GE system components as encoded by a viral replicon and/or an expression construct according to the present invention. Suitable promoters are available in the prior art and can be combined as replicon and/or expression construct element based on the disclosure provided herein.

Exemplary viral replicons, expression constructs and vectors combining the above elements in various forms to be suitable for the methods disclosed herein are disclosed in the Examples section and are exemplified in the sequence listing. As it will be apparent to the skilled person, certain elements of the replicons, expression constructs and/or vectors are readily exchangeable and can (or must) be adapted for use in a plant cell of interest, whereas in particular, the overall structure of the (Be-)Curtovirus replicon is relevant to obtain certain effects as disclosed herein.

The terms "plant" or "plant cell" as used herein refer to a plant organism, a plant organ, differentiated and undifferentiated plant tissues, plant cells, seeds, and derivatives and progeny thereof. Plant cells include without limitation, for example, cells from seeds, from mature and immature embryos, meristematic tissues, seedlings, callus tissues in different differentiation states, leaves, flowers, roots, shoots, male or female gametophytes, sporophytes, pollen, pollen tubes and microspores, protoplasts, macroalgae and microalgae. The different eukaryotic cells, for example, animal cells, fungal cells or plant cells, can have any degree of ploidity, i.e. they may either be haploid, diploid, tetraploid, hexaploid or polyploid.

The family of *Amaranthaceae* the preferred plants or plant cells according to the present disclosure belong to comprises a number of relevant food and crop plants. For example, the family comprises the genus *Beta,* comprising *Beta vulgaris* ssp. *vulgaris* var. *vulgaris* (chard), *Beta vulgaris* ssp. *vulgaris* var. *conditiva* (beetroot / red beet), *Beta vulgaris* ssp. *vulgaris* var. *crassa*/*alba* (fodder beet) and *Beta vulgaris* subsp. *vulgaris* var. *altissima* (sugar beet in a narrower sense), the genus *Amaranthus,* the genus *Chenopodium,* including *C. quinoa,* or the genus *Spinacia* including *Spinacia oleracea.* In certain embodiments, the viral replicons identified herein may be optimized for use in a usually recalcitrant plant of the family *Amaranthacea,* but the replicons may also be suitable for transforming less or not recalcitrant plants as illustrated with the enclosed Examples.

In yet a further aspect, there is provided a use of a viral replicon as disclosed herein based on a (Be-)Curtovirus, or a use of an expression construct as disclosed herein in various aspects and embodiment, for stimulating homology-directed repair by reverting a host plant cell into the S-phase of the cell cycle, and/or for increasing homology-directed repair-based genome editing.

This aspect is highly desirable in classical plant breeding as well as plant biotechnology. Thereby genetic variation in a specific genomic region, or in a specific nucleotide sequence of interest may be increased, a complete allele or a part like a domain, or an exonic region of a gene of interest may be replaced, or the elimination of undesired genetic components/material closely linked a nucleotide sequence of interest in the genome of a plant (so-called linkage drag), which cannot be easily segregated out by conventional breeding, can be obtained.

The present invention is further described with reference to the following non-limiting Examples.

### Examples

### Example 1: Selection of sugar beet infecting Geminivirus strain for replicon construction

Full length clones (FLC) of four SB-infecting Geminivirus strains (BSCTV (Beet severe curly top virus), BCTV (Beet Curly Top Virus), BMCTV (Beet Mild Curly Top Virus), BCTIV (Beet curly top iran virus)) of different genera have been used for *Agrobacterium-mediated* transformation of sugar beet seedlings to compare viral replication intensity *in planta.* This initial step was necessary to identify new viruses suitable at all for subsequent viral replicon design in view of the fact that viral replicons for certain dicot target plants like sugar beet and spinach are not readily available and cannot be simply derived from exiting viral replicons having specificity for other target plants.

Highest replication intensity was demonstrated for BSCTV, medium replication level was measured for BCTV and BCTIV and relatively low replication level were shown for BMCTV (Figure 1 A). Based on the experimental results, BSCTV and BCTIV were selected for GVR construction. BSCTV and BCTIV belong to different viral genera than BeYDV.

The design for the GVR construct of BSCTV and BCTIV was adapted and optimized to produce functional replicon constructs in an iterative manner. Additionally, it was demonstrated that not only the DNA levels of the FLC but also the viral DNA levels of the replicons are increased when using the BSCTV-based and the BCTIV-based replicons in comparison to the BeYDV-based replicon (Figure 1B).

Different Geminivirus genera differ in their genomic structure and gene composition. Therefore, for each viral genus, specific adaptations within the genomic structure need to be made to (i) guarantee general functionality of the GVRs and (ii) further improve the replication efficiency of the respective GVR.

### Example 2: Inducible expression of the viral replication initiator protein (Rep) for improved callus development and/or shoot regeneration

To avoid negative effects on the callus development and/or shoot regeneration in *Beta vulgaris,* preferably in sugar beet, an inducible promoter system may be used for the expression of the viral replication initiator protein (Rep). Such inducible systems include but are not limited to inducible promoter systems such as the abscisic acid-inducible promoter (SEQ ID NOs: 1 and 2) or a glucocorticoid receptor-based system inducible by dexamethasone (Figure 2, SEQ ID NO: 3).

### Example 3: Replicon design

For viral replicon construction, the BCTIV full length clone (FLC, SEQ ID NO: 22) was used as starting material. Using the BCTIV FLC as a template, a PCR-based technique was applied to amplify the viral genome without a 1,043 bp fragment which covers the viral ORFs for proteins V1, V2 and V3. Thereby a viral replicon is created lacking ORFs V1, V2 and V3. A multi(ple) cloning site (MCS) was introduced into the BCTIV replicon. The final BCTIV replicon thus constructed (SEQ ID NO: 4) contains a repeat of long intergenic region (LIR), small intergenic region (SIR) and *C1* (Rep) and *C2* (TrAP) genes, which play a role in the virus replication and DNA accumulation.

To allow *Agrobacterium-mediated* delivery of BCTIV-GVR into plants (which represents one option for introducing the viral replicon efficiently), the replicon was introduced into the T-DNA of binary vector pZFNnptll via *NcoI*/*PmeI* sites (see Figure 3, SEQ ID NO: 5).

Further introduction strategies are possible for the developed viral replicons, depending on the experiment and the target material to be treated of interest.

To have further candidates for viral replicon construction, the BSCTV full length clone (SEQ ID NO: 23) was used as further starting material. Using the BSCTV FLC as a template, a PCR-based technique was applied to amplify the viral genome without a 1,002 bp fragment which covers the viral ORFs for proteins R2 and R3 and parts of ORF R1 and R4. Thereby a viral replicon is created lacking R2 and R3 and parts of R1 and R4. A multi(ple) cloning site (MCS) was introduced into the BSCTV replicon. The final BSCTV replicon (SEQ ID NO: 6) contains a repeat of LIR and all complementary sense genes including *C1, C2, C3* and *C4.* To allow *Agrobacterium-mediated* delivery of BSCTV-GVR into plants, the replicon was introduced into the T-DNA of binary vector pZFNnptll via *NcoI*/*PmeI* sites (see Figure 4, SEQ ID NO: 7).

### Example 4: Introduction of heterologous sequences (e.g. nucleotide sequence of interest or repair template) into the replicons

An expression cassette for GVR-mediated transgene expression (for example 35S::GFP) can be introduced via different cloning sites (for example Spel-sites) present in the MCS of pZFNnptll-BCTIV-Rep/MCS and pZFNnptll-BSCTV-Rep/MCS, respectively (SEQ ID NO: 8 and SEQ ID NO: 9). Genome Editing (GE) components (LbCpf1 (SEQ ID NO: 24), crRNA (SEQ ID NOs: 16 to 20), and repair templates (ALS as example, SEQ ID NO: 21)) were introduced into pZFNnptll-BCTIV-Rep/MCS (using SEQ ID NO: 5, producing SEQ ID NOs: 12 and 13) and pZFNnptll-BSCTV-Rep/MCS (using SEQ ID NO: 7, producing SEQ ID NOs: 14 and 15) using the following cloning strategy: first, a pcUbi4::LbCpf1 expression cassette was introduced via *Xma*I/*Apa*I sites of the MCS into pZFNnptll-BCTIV-Rep/MCS and via *Xma*I/*Hind*III into pZFNnptll-BSCTV-Rep/MCS. In a next step the d35S::crRNA expression cassette was introduced via *Sfi*I sites which are present in the MCS of the viral replicons. Finally, the repair template is inserted using a unique *Hind*III site. When pZFNnptll-BCTIV-Rep/MCS is used for inserting the repair template the *Hind*III site is located outside of the 3' end of the Tpea terminator, which is part of the pcUbi4::LbCpf1 expression cassette. Therefore, the *Hind*III site only becomes available after pcUbi4::LbCpf1 expression cassette has been introduced into the viral replicons.

### Example 5: Binary vector pZFNnptll, GE components (LbCpf1 and crRNAs) and Agrobacterium tumefaciens strain

The viral replicons are embedded in a binary vector named pZFNnptII. This binary vector includes a nptll resistance cassette for *in planta* selection. To mediate GE in a sugar beet, the target gene, the PcUbi4::Cpf1 expression cassette and a crRNA, which targets a target region within the target gene, were introduced into the viral replicons. In a first version (SEQ ID NOs: 13 and 15, Figure 5A) the crRNA is embedded in a Pol II-driven RNA transcript as described before in Zhong et al., 2017. This transcript includes an mRNA encoding the fluorescent protein tDT (Figure 5A).

The second crRNA version (as included in SEQ ID NOs: 12 and 14) produces the crRNA from a Pol II promoter using the ribozyme-based strategy (He *et al.,* 2017). Thereby, the crRNA production unit is composed of three parts: the 5'-end encoding the Hammerhead (HH) ribozyme, the middle part encoding the crRNA, and the 3'-end encoding the Artichoke HDV-like ribozyme (Figure 5B). The primary transcripts will undergo self-cleavage catalyzed by the intrinsic nuclease activities of the ribozymes to release the mature and desired crRNA.

The binary vectors were transformed into agrobacterium strain AGL1-1. Notably, other agrobacterium strains available may be used as well.

### Example 6: GFP expression

Next, the constructs as established (cf. Examples 4 and 5) were subjected to several rounds of tests. First, green fluorescent protein (GFP) expression levels (transcript level) were compared between a BCTIV-based replicon (SEQ ID NO: 8), a BSCTV-based replicon (SEQ ID NO: 9), a BeYDV-based replicon (SEQ ID NO: 10) and a standard T-DNA vector without viral replicon elements (SEQ ID NO: 11). Therefore, an expression cassette of 1,843 bp in size, which expresses the GFP under the 35S promoter, was cloned into GVRs derived from BSCTV, BCTIV and BeYDV. The binary vector pZFNnptll, which includes the 35S::GFP expression cassette, was included as a non-GVR control. The constructs were infiltrated into cotyledons of sugar beet seedlings or tobacco leaves and GFP transcript levels were measured using qRT-PCR (reference gene for sugar beet: GAPDH) after four and six days of infiltration in sugar beet (Figure 6) and after six days of infiltration in tobacco (Figure 7).

In sugar beet, the viral replicons derived from BSCTV and BCTIV clearly outperform BeYDV-GVR and the binary vector control in term of heterologous gene expression. GFP transcript level are strongly increased when expressed by BSCTV or BCTIV-based GVRs compared to the binary vector control and BeYDV-GVR (Figure 6).

In tobacco, BCTIV-GVR and BSCTV-GVR express the GFP gene at a significantly higher levels compared to the binary vector control and BeYDV-GVR at 6 dpi (Figure 7).

This indicates that the use of GVRs derived from sugar beet-infecting viruses is not restricted to sugar beet but can be extended to other non-crop species like tobacco.

### Example 7: Expression of GE components (SDN-1)

First, a nucleic acid guided site-specific nuclease (SSN) and a cognate crRNA (SDN-1 technique not relying on adding exogenous nucleic acid material in the form of a repair template) was further tested based on the specific viral replicons tested.

Therefore, a PcUbi4::Cpf1 expression cassette of ∼5.2 kb in size and/or a d35S::crRNA expression cassette of 1.1-1.7 kb in size, which targets one of five different target regions within the target gene acetolactate synthase (BvALS, SEQ ID NOs: 16 to 20) were introduced into the BCTIV-replicon and the BSCTV-replicon (Figure 5) to mediate genome editing in a sugar beet target gene. For each of the five protospacer two different crRNA versions were tested (tDTcrRNA_ALS1 - ALS5 (e. g. SEQ ID NOs: 13 and 15) or RibocrRNA_ALS1 - ALS5 (SEQ ID NOs: 12 and 14)). The GVRs harboring either only the LbCpf1 expression cassette or both, the LbCpf1- and the crRNA-cassette have been infiltrated into cotyledons of sugar beet seedlings using agrobacteria. As non-GVR controls, the corresponding binary vectors without the replicon have been included. Transcript and DNA levels of LbCpf1 were quantified by qRT-PCR and qPCR at three different time points post inoculation.

*Agrobacterium*-mediated infiltration of sugar beet (SB) seedlings with BCTIV-derived GVRs revealed that in comparison to the binary vector controls, LbCpf1 transcription (**Figure 8** shows RT-PCR results) and DNA levels (**Figure 9** shows qPCR results) are strongly increased when expression is mediated by BCTIV-GVR. However, increasing size of the inserted heterologous sequence seems to negatively affect replication ability and/or transcription level of the BCTIV-GVR. The BCTIV-GVR including only the pcUbi4::LbCpf1 expression cassette of ∼5.2 kb in size shows a higher transcript level than the BCTIV-GVRs including both the LbCpf1 and the crRNA expression cassettes.

*Agrobacterium-mediated* infiltration of SB seedlings with BSCTV-derived GVRs revealed that LbCpf1 transcript and DNA levels are dramatically increased compared to the binary vector controls (Figure 9). For BSCTV-GVR, increasing size of the heterologous insertions seems to not impair replicon function. BSCTV-GVRs including both the LbCpf1 and the crRNA expression cassettes show as high transcript levels as the BSCTV-GVR only harboring LbCpf1.

Next, SDN-1 (indel) efficiencies were determined. Cotyledons of sugar beet seedlings were infiltrated with different GVR constructs (derived from BSCTV and BCTIV) containing either only the LbCpf1 expression cassette (= negative control for SDN-1 events, since no crRNA is available for target recognition) or both the LbCpf1 and the crRNA cassette enabling the quantification of SDN-1. For each GVR construct the respective binary vector without replicon was included as a control. Infiltrated SB leaf explants were harvested 13 (BSCTV-based GVR, Figure 13A) or 12 (BCTIV-based GVR, Figure 13B) days after infiltration (dpi). DNA was extracted and SDN-1 (indel) efficiencies were determined via ddPCR (droplet digital PCR).

### Example 8: Expression of GE components (SDN-2/3)

Next, SSN + crRNA + repair template (SDN-2/3) approaches can be tested with the above viral replicons and binary vector systems established. HDR-based genome editing efficiencies in sugar beet are increased by GVR-mediated delivery of (i) site-specific nucleases (SSNs) from the CRISPR/Cas type, (ii) sgRNA and (iii) a repair template (e. g. SEQ ID NO: 21 for BvALS, Figure 10).

To further improve the efficiency of HDR-mediated GE, the GVR is equipped with a repair template, which will be integrated into the genomic target region during homologous recombination. Since GVRs replicate efficiently inside host cells they produce high amounts of replicons, in turn producing high expression of SSNs and increasing the abundance of donor or repair templates available for HDR, thereby considerably enhancing the targeting efficiency. Therefore, BSCTV- and BCTIV-GVR constructs are equipped with the HDR-based GE components LbCpf1, the crRNA and the repair template. The constructs are used for agrobacterium-mediated infiltration of sugar beet leaf explants.

Leaf samples are taken 10 days post infiltration (dpi) and they are used for DNA extraction and subsequent NGS analysis to quantify efficiencies of HDR events.

### Example 9: GVR-based vector system for HDR-based GE in tobacco

Efficiency of GVR-based GE was tested in the transgenic tobacco line *Nicotiana benthamiana* 16c (Philips et al., 2017), which expresses an optimized *Aequorea victoria* green fluorescent protein (mGFP5-ER, codon optimized and equipped with additional regulatory sequences for the expression in plants (Philips et al., 2017)) constitutively. A single amino acid exchange (T224Y) in GFP causes an altered light emission spectrum that turns GFP to YFP (yellow fluorescent protein (Wachter et al. 1998; Figure 14, SEQ ID NO: 29 and SEQ ID NO: 30)).

The repair template that enables the GFP to YFP mutation consists of two homologous arms, which are complementary to the target region in mGFP5-ER. Two nucleotides within the homologous arms were adjusted, to remove *Bsa*I and *Bbs*I restriction sites (Figure 15).

The "core" of the RT (Figure 15, lower panel) includes 23 synonymous mutations, which avoid binding and cutting of the CRISPR complex after RT integration. In addition, the "core" includes 3 non-synonymous mutations (dark grey), which lead to the desired T224Y amino acid exchange (Figure 15, SEQ ID NO: 31).

### Design and cloning of vector constructs

### 1. pZFNnptII/LbCpf1_tDT-crRNA_mGFP#1+2+3_GFPtoYFP template

A binary vector, which does not include viral replicon sequences was used as non GVR control. The binary vector includes expression cassettes for nptll, LbCpf1 and tDT-crRNA_mGFP#1+2+3, which are directed against three target regions in mGFP5 (Figure 15, SEQ ID NO: 32). The crRNA is embedded in a Pol II-driven RNA transcript as described by Zhong et al., 2017. In addition, the binary vector includes the repair template described in Figure 15.

### 2. pZFNnptII-BSCTV-RepI/LbCpf1_tDT-crRNA_mGFP#1+2+3_GFPtoYFP template

A binary vector, which includes a nptll expression cassette and a viral replicon sequence derived from Beet severe curly top virus (BSCTV, SEQ ID NO: 33). LbCpf1 and tDT-crRNA_mGFP#1+2+3 are located within the viral replicon sequence expression cassettes. The crRNA tDT-crRNA_mGFP#1+2+3 are directed against three target regions in mGFP5-ER (Figure 16) and embedded in a Pol II-driven RNA transcript as described by Zhong et al., 2017. In addition, the viral replicon includes the repair template described in Figure 15.

### 3. pZFNnptII-BSCTV-RepI/tDT-crRNA-mGFP#1+2+3-RTGFPtoYFP200-Out LbCpf1

A binary vector, which includes a nptll expression cassette and a viral replicon sequence derived from Beet severe curly top virus (BSCTV, SEQ ID NO: 34). The expression cassette for a tDT-crRNA_mGFP#1+2+3 (Figure 16) and the repair template (Figure 15) are located within the viral replicon. The GVR construct is characterized by an LbCpf1 expression cassette that is located outside the viral replicon, but still within the T-DNA.

### Results and data analysis

### 1. Proof of functionality of the selected crRNAs in infiltrated tobacco leaves

Three crRNAs targeting mGFP5-ER have been selected. For general proof of functionality, each crRNA was cloned separately in the binary vector pZFNnptII-LbCpf1. Leaves of *Nicotiana benthamiana* 16c were syringe-infiltrated with these constructs and leaf samples were harvested 3 dpi and 7 dpi for DNA extraction. SDN-1 efficiencies were quantified by ddPCR. Functionality of all three crRNAs was confirmed (Figure 17). At 7 dpi all selected crRNAs showed considerable SDN-1 efficiency (4-14%).

For enabling the HDR-mediated aa exchange via a GVR-based approach, all three crRNAs were combined in a multiplex construct (tDT-crRNA-mGFP#1+2+3) and cloned into the viral replicon sequence of the binary vector pZFNnptII-BSCTV-RepI/LbCpf1 (to generate the sequence in SEQ ID NO: 33) or pZFNnptll-BSCTV-Repl/Out LbCpf1 (to generate the sequence in SEQ ID NO: 34).

### 2. Proof of HDR-based genome editing in infiltrated tobacco leaves

Leaves of *Nicotiana benthamiana* 16c transgenic line have been syringe infiltrated with either pZFNnptII/LbCpf1_tDT-crRNA_mGFP#1+2+3_GFPtoYFP template (SEQ ID NO: 32), pZFNnptll-BSCTV-Rept/LbCpf1_tDT-crRNA_mGFP#1+2+3_GFPtoYFP_temptate (SEQ ID NO: 33) or *pZFNnptII-BSCTV-RepI*/*tDT-crRNA*-mGFP#1+2+3-*RTGFPtoYFP200-Out LbCpf1* (SEQ ID NO: 34). Delivery of all three constructs resulted in the conversion of GFP into YFP in a certain amounts of infiltrated tobacco cells (Figure 18). Shift from GFP to YFP fluorescence was visualized using confocal laser microscopy. GFP and YFP fluorescence was detected after excitation with 488-nm and 514-nm later light, respectively. All three binary vector constructs mediate HDR-based genome editing in tobacco. pZFN-BSCTVRepI-LbCpf1, which contains only the nuclease but no crRNA and donor repair template, was used as negative control (Figure 18 A) and do not show cells with YFP expression.

## Claims

1. A method for the targeted modification of at least one genomic target sequence in a plant cell from a plant of the family *Amaranthacea,* preferably a cell of a plant from the genus *Beta* or *Spinacia,* more preferably a cell of a plant of the species *Beta vulgaris* or *Spinacia oleracea,* wherein the method comprises the following steps:
(a) introducing into the cell
(i) at least one viral replicon, or a sequence encoding the same, the replicon being constructed based on a Becurtovirus, or a Curtovirus genome, wherein the at least one viral replicon further comprises:
(ii) at least one genome editing system comprising at least one nucleic acid guided nuclease, nickase or an inactivated nuclease, or a sequence encoding the same, and at least one guide molecule, or a sequence encoding the same;
(iii) and, optionally at least one repairtemplate, or a sequence encoding the same;
(b) cultivating the cell under conditions allowing the expression and/or assembly of the at least one genome editing system and the at least one guide molecule and/or optionally the at least one repair template; and
(c) obtaining at least one modified cell; and
(d) optionally obtaining at least one plant, plant tissue, organ, or seed regenerated from the at least one modified cell.

2. The method of claim 1, wherein the at least one viral replicon, or the sequence encoding the same, is constructed based on a Beet curly top iran virus Becurtovirus genome, a Spinach Curly Top Arizona Virus Becurtovirus, a Beet severe curly top virus Curtovirus genome, a Beet Curly Top Virus Curtovirus genome, a Beet Mild Curly Top Virus Curtovirus genome, or a Spinach Severe Curly Top Virus Curtovirus, preferably a genome selected from SEQ ID NOs: 22, 23, 27 or 28, or a genome from a homologous virus to the respective sequences belonging to the genus Becurtovirus or Curtovirus.

3. The method of any of the preceding claims, wherein the at least one viral replicon is encoded by a sequence according to SEQ ID NO: 4 or 6, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

4. The method of any of the preceding claims, wherein the at least one viral replicon comprising the at least one genome editing system is introduced into the cell by transformation or transfection, preferably by *Agrobacterium-*mediated transformation, biolistic bombardment, micro- or nanoparticle delivery, or by chemical transfection.

5. The method of claim 4, wherein the at least one viral replicon comprising the at least one genome editing system is introduced into the cell by *Agrobacterium-*mediated transformation, wherein the at least one viral replicon is inserted into a binary vector, preferably wherein the binary vector comprising the at least one viral replicon has a sequence of SEQ ID NOs: 5 or 7 to 15, or a sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

6. The method of any of the preceding claims, wherein the at least one nucleic acid guided nuclease, nickase or an inactivated nuclease, or a sequence encoding the same, of the genome editing system is selected from the group consisting of a CRISPR/Cas system, preferably from a CRISPR/Cfp1 system, a CRISPR/MAD7 system, a CRISPR/MAD2 system, a CRISPR/Cas9 system, a CRISPR/CasX system, a CRISPR/CasY system, or a CRISPR/Csm system, or any combination, variant, or catalytically active fragment thereof.

7. The method of any of the preceding claims, wherein the at least one genome editing system further comprises at least one reverse transcriptase, preferably a modified reverse transcriptase, wherein the reverse transcriptase is preferably used in combination with a nucleic acid guided nickase, and/or at least one cytidine or adenine deaminase, preferably wherein the at least one cytidine or adenine deaminase is independently selected from an apolipoprotein B mRNA-editing complex (APOBEC) family deaminase, preferably a rat-derived APOBEC, an activation-induced cytidine deaminase (AID), an ACF1/ASE deaminase, an ADAT family deaminase, an ADAR2 deaminase, or a PmCDA1 deaminase, a TadA derived deaminase, and/or a transposon, or a sequence encoding the aforementioned at least one enzyme, or any combination, variant, or catalytically active fragment thereof.

8. The method of any of the preceding claims, wherein the at least one genome editing system comprises at least one repair template, and wherein the at least one repair template comprises or encodes a double- and/or single-stranded nucleic acid sequence, and/or wherein the at least one repair template is part of the at least one guide molecule.

9. The method of claim 8, wherein the at least one repair template comprises symmetric or asymmetric homology arms and/or wherein the at least one repair template comprises at least one chemically modified base and/or backbone.

10. A plant, plant cell, tissue, organ, or seed obtainable by or obtained by a method according to any of the preceding claims.

11. A viral replicon for plant transformation, wherein the viral replicon is encoded by a sequence according to SEQ ID NO: 4 or 6, or a sequence having at least sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto.

12. An expression construct comprising or encoding:
(i) at least one viral replicon as defined in claim 11, and
(ii) a vector, preferably a vector as defined in claim 5, and
(iii) at least one component of at least one a genome editing system, wherein the genome editing system comprises or encodes:
(a) at least one nucleic acid guided nuclease, nickase or an inactivated nuclease, or at least one site-directed nuclease, nickase or an inactivated nuclease, and optionally at least one nuclear or organellar localization sequence; and
(b) at least one guide molecule for guiding the at least one nucleic acid guided nuclease, nickase or an inactivated nuclease and optionally for activating at least one reverse transcriptase; and
(c) optionally at least one repair template; and
(d) optionally at least one reverse transcriptase; and
(iv) wherein the expression construct comprises or encodes at least one regulatory sequence, wherein the regulatory sequence is selected from the group consisting of a core promoter sequence, a proximal promoter sequence, a cis regulatory sequence, a trans regulatory sequence, a locus control sequence, an insulator sequence, a silencer sequence, an enhancer sequence, a terminator sequence, an intron sequence, and/or any combination thereof.

13. The expression construct of claim 12, wherein the expression construct additionally comprises or encodes at least one self-cleaving ribozyme, preferably at least one hammerhead ribozyme and/or at least one hepatitis-delta virus (HDV) ribozyme and/or at least one artichoke, sunflower or rice HDV-like ribozyme.

14. The expression construct of claim 12 or 13, wherein the expression construct additionally comprises or encodes a sequence for an RNA polymerase II promoter.

15. A use of a viral replicon according to claim 11, or a use of an expression construct of any of claims 12 to 14, for stimulating homology-directed repair by reverting a host plant cell into the S-phase of the cell cycle, and/or for increasing homology-directed repair-based genome editing.


SEQUENCE LISTING

<110>  KWS SAAT SE & Co. KGaA
       Institut für Zuckerrübenforschung des Vereins der
       Zuckerindustrie e.V.

<120>  (Be-)Curtovirus replicon-mediated genome editing in plants

<130>  KWS0355EP

<150>  US 62/926,366
<151>  2019-10-25

<160>  34

<170>  PatentIn version 3.5

<210>  1
<211>  199
<212>  DNA
<213>  Artificial Sequence

<220>
<223>  ABA inducible promoter 2xS -2xD - ABA inducible promoter for the
       inducible expression of the viral replication initiator protein
       (Rep) for improved callus development and/or shoot regeneration

<400>  1
ctagtcagcc accaaacagg acccagaatt ctagtcagcc accaaagagg acccagaatt      60

ctagttacaa ttcaaacatt gttcaaacaa ggaacctcta gttacaattc aaacattgtt     120

caaacaagga acctctagag tcgaccgcaa gacccttcct ctatataagg aagttcattt     180

catttggaga ggacacgct                                                  199


<210>  2
<211>  212
<212>  DNA
<213>  Artificial Sequence

<220>
<223>  ABA inducible promoter 4D - ABA inducible promoter for the
       inducible expression of the viral replication initiator protein
       (Rep) for improved callus development and/or shoot regeneration

<400>  2
tacaattcaa acattgttca aacaaggaac ctctagttac aattcaaaca ttgttcaaac      60

aaggaacctc tagttacaat tcaaacattg ttcaaacaag gaacctctag ttacaattca     120

aacattgttc aaacaaggaa cctctagagt cgaccgcaag acccttcctc tatataagga     180

agttcatttc atttggagag gacacgctcg ag                                   212


<210>  3
<211>  3527
<212>  DNA
<213>  Artificial Sequence

<220>

<223>  Dexamethasone inducible system - Dexamethasone inducible system
      for the inducible expression of the viral replication initiator
      protein (Rep) for improved callus development and/or shoot
      regeneration

<400>  3
aatttaaatt attaaattta agtaaattta tttgtaataa ttaatttaat tcggcgttgt      60

ttaaactcgt tacctctctg tccttatcct tttgctgaac aagaaaaaaa tggaaaaaat     120

tacagctaat tgtttttctt tttttataat ttatttttat ttttatgttt taaatttgca     180

gttttaatag caacggcata gacacttagt ttgcaagcaa ttgggacaaa ctcgtcacac     240

tttgtcttgc gcttgaaggt tagggcaagg gcaaaaccgt aagataaaat attcgccgcc     300

ttcgagtaac gagtaaccct agccctttta gcgctataaa tattggcata ttgcctctgc     360

ttctctcagt tcgcatcccc ttttcttcgc tgccatagtg cgtgctcatt tcctaagcct     420

ttcttaatct ccgccgccgt tgctgcctcc tccttctcct cagtctctcc aggtcagtca     480

ctgatttctt agattccaaa tcgtgtcgtg atttagctct cctgctcgat atagtttaat     540

taccttttat tttctttgat taacatcaga tctatttgct ctttcaataa ctataagttt     600

aatatatttt gtttggactg tttagttgat tttgcgattt catagtcttc atatcgattt     660

tttccccgtt ttatgcttta aatcgtcgta aagttggtct gtgctttgca atcagttttc     720

tgtacttggg ttgtggttcc gtttggatat ttttctgata gaccttgtaa aatgttttca     780

tatgagtttt ttaatcagtc ttttgttctt ggatctatag ttcaagtatc ttgtcttgtt     840

ctcgtttctg tatggtatgt ttattgccta ccatcaatgg gttttttact gtttagtatg     900

tcactattct ttattttaac ttaatttttt tttgtttatt actgaatttc ttttatgctt     960

gcttttcaga ttaaacagcg gccgcaacaa tgaaagcgtt aacggccagg caacaagagg    1020

tgtttgatct catccgtgat cacatcagcc agacaggtat gccgccgacg cgtgcggaaa    1080

tcgcgcagcg tttggggttc cgttccccaa acgcggctga agaacatctg aaggcgctgg    1140

cacgcaaagg cgttattgaa attgtttccg gcgcatcacg cgggattcgt ctgttgcagg    1200

aagaggaaga agggttgccg ctggtaggtc gtgtggctgc cggtgaaccg gaggcttctt    1260

ctggatcttc accaccacac tctcaggaaa acccacctga acacggagga gatatgggag    1320

gagcacctgc tgaggaaatt ggaggagagg ctgctgatga tttcatgttc gctgaggata    1380

ctttcccttc tctccctgat ttcccatgcc tttcttctcc atcatcttca accttctcat    1440

caaactcatc ttcaaactct tcatctgctt ataccaacac cgctggtaga gctggaggag    1500

agccatctga acctgctagt gctggagagg gattcgatgc tctcgatgat attgatcaac    1560

tcctcgattt cgcttcactc tctatgcctt gggattctga gccattccct tctgctggag    1620

acatgagagc tgccaacctt tggccaagcc cgctcatgat caaacgctct aagaagaaca    1680

gcctggcctt gtccctgacg gccgaccaga tggtcagtgc cttgttggat gctgagcccc    1740

ccatactcta ttccgagtat gatcctacca gacccttcag tgaagcttcg atgatgggct 1800

tactgaccaa cctggcagac agggagctgg ttcacatgat caactgggcg aagagggtgc 1860

caggctttgt ggatttgacc ctccatgatc aggtccacct tctagaatgt gcctggctag 1920

agatcctgat gattggtctc gtctggcgct ccatggagca cccagtgaag ctactgtttg 1980

ctcctaactt gctcttggac aggaaccagg gaaaatgtgt agagggcatg gtggagatct 2040

tcgacatgct gctggctaca tcatctcggt tccgcatgat gaatctgcag ggagaggagt 2100

ttgtgtgcct caaatctatt attttgctta attctggagt gtacacattt ctgtccagca 2160

ccctgaagtc tctggaagag aaggaccata tccaccgagt cctggacaag atcacagaca 2220

ctttgatcca cctgatggcc aaggcaggcc tgaccctgca gcagcagcac cagcggctgg 2280

cccagctcct cctcatcctc tcccacatca ggcacatgag taacaaaggc atggagcatc 2340

tgtacagcat gaagtgcaag aacgtggtgc ccctctatga cctgctgctg gagatgctgg 2400

acgcccaccg cctacatgcg cccactagcc gtggaggggc atccgtggag gagacggacc 2460

aaagccactt ggccactgcg ggctctactt catcgcattc cttgcaaaag tattacatca 2520

cgggggaggc agagggtttc cctgccacag tctgagagct ccctggaatt ccagagatgt 2580

acatctatct gtctggtgct gtgcttgctt gttggttacc tcgtggaagc ttaaaatatc 2640

tcgtcttgtt gacatccttg cagttgtcgc gtgaacaagt aatctatgat ggcaatgttt 2700

gcctggtcat gaagtattat tactttcgtt gaaaccgtga tgctattaag acttaatttt 2760

tttatggaac atcgaatgta aggcatacat ggttatgagt tttgtactac cctttgcagt 2820

gtgttcctgt ttaccttttg tgctgatgat tcgcttgtct ttggtcgacc cggatgatcc 2880

ccgatcgttc aaacatttgg caataaagtt tcttaagatt gaatcctgtt gccggtcttg 2940

cgatgattat catataattt ctgttgaatt acgttaagca tgtaataatt aacatgtaat 3000

gcatgacgtt atttatgaga tgggttttta tgattagagt cccgcaatta tacatttaat 3060

acgcgataga aaacaaaata tagcgcgcaa actaggataa attatcgcgc gcggtgtcat 3120

ctatgttact agatcgggga attgatcccc cctcgacagc ttgcatgcca gcttgggctg 3180

caggtcgagg ctaaaaaact aatcgcatta tcatcccctc gacgtactgt acatataacc 3240

actggtttta tatacagcag tactgtacat ataaccactg gttttatata cagcagtcga 3300

cgtactgtac atataaccac tggttttata tacagcagta ctgtacatat aaccactggt 3360

tttatataca gcagtcgagg taagattaga tatggatatg tatatggata tgtatatggt 3420

ggtaatgcca tgtaatatgc tcgactctag gatcttcgca agacccttcc tctatataag 3480

gaagttcatt tcatttggag aggacacgct gaagctagtc gactcta 3527

<210> 4

<211>  2160
<212>  DNA
<213>  Artificial Sequence

<220>
<223>  BCTIV replicon sequence

<400>  4
gtttgttttt gtttccctct tccgtacaat gaatgaaaag aaattgtata tatgagagga      60

agaggaactt aagaggaatg aggaactgaa gagggaggta gagggataaa taggtaaacg     120

cggataagat tccccgcgtt tacctttcca cgtggcgtat gcagagtggg cggacggttg     180

agtggggaac acgctttact ttaatttaaa gtaaagtagc actaagtggg acccactttt     240

tatttcttta aagacttaat tattaagtct tctagatgcg tatattatac gtataaacta     300

gtggccttgg cggccggcgc gccggcctta agggccaccc gggagtactg ggcccggtac     360

cactagtatt gtagggctta tttctattga ttattagaaa tgcccaatga tgttgaagtt     420

taataaaaaa tttattatta aatttgtttg tgtaagattg aaatgtagat atgtaaatat     480

ctagtataat tgccttggaa aacaaaaaac gaaaaaatga ttgccggaaa acgaaaaaaa     540

aacccctcag aaattagacc cccggaaaag caatattttt atattaatct gcagcccatc     600

cacgaagtgg atgttacata tttcgaagaa ataacttaac tataccaaac ttcatcactt     660

gctaagtaat atacatcgca attagcgtaa aaccaatctt tcatagattc actcatacaa     720

ggtatccagt ccatatctgg attagtaata gcaatacaag gtcttccgcc tggaatgact     780

aagtttggtt tatgttttat ccaaacacta taatcttcgt tacatccgat aatagattta     840

aacaactcct gttgtatttt ttgatagcgg atatcgtcta ttatatggta tgtagtgtgg     900

tctatatcgt atagagacca atctaatacc atgccagaca tatagtggtg tcggcctaga     960

gatcttgccc actgggtctt tcctgttctg gatggtccgc atatgtagag acttcttggt    1020

cggactggtc tgttgtctgg ctcctggaca aattaagtac gttagtgtct tcactacatg    1080

ctagatcgcg actaagtcga tctaggtgga agtattcttc aagagtcatt tgtctgtctt    1140

gaagaattgc tcccctgcaa ttctcacata tttcgtaatg taaacaatct gaacttacga    1200

aatatatatt ttgttccgcc cactctttga tagtgtcagg tagcctagag aactccgtcc    1260

atctgggtgt gtaaggttgc acaactggcc tgaaatgata gttggcgaag gatagaatgg    1320

aaggccatct gagaacgaaa tctgttgggc aggactctcg tatgttactg aagaactcct    1380

ccttggtgtt ggatgaatca agaattcgtc tccattccac gttgctgttg ccaatactgt    1440

ttggagagcg ccctcccagt cgaagctctc caaatgttct agggctcgtg tcctccttgg    1500

tgatatactc gaatgctcgc tttggggcgt tgagtccgtc aattcttggg tgaatcgttc    1560

gaccgtctgg gagtatgatg ttgaaaaaca ggttcccatt gacgtcgagt ttcttgtcac    1620

actggataat acagtgtgtg tggaatcctc cttcaggttg ttcttcagta ggctgatgat    1680

gttctagtga tactgcaagg aatagaatca catatgattc taataaggtt ttcagtttct 1740

cgaatatata atctttaaaa tcccccccta tttgggaata tgttagaaaa gcattttttt 1800

tttgaaaacg taaataaccg ggttggttag gggtaggtct gtttctgttt ggagcacgtg 1860

gcatgtttgt ttttgtttcc ctcttccgta caatgaatga aaagaaattg tatatatgag 1920

aggaagagga acttaagagg aatgaggaac tgaagaggga ggtagaggga taaataggta 1980

aacgcggata agattccccg cgtttacctt tccacgtggc gtatgcagag tgggcggacg 2040

gttgagtggg gaacacgctt tactttaatt taaagtaaag tagcactaag tgggacccac 2100

tttttatttc tttaaagact taattattaa gtcttctaga tgcgtatatt atacgtataa 2160

<210> 5
<211> 11808
<212> DNA
<213> Artificial Sequence

<220>
<223> pZFNnptII-BCTIV-Rep/MCS - full sequence of binary vector
 pZFNnptII (for Agro transformation) including the BCTIV replicon

<400> 5
aacgtcggct cgattgtacc tgcgttcaaa tactttgcga tcgtgttgcg cgcctgcccg 60

gtgcgtcggc tgatctcacg gatcgactgc ttctctcgca acgccatccg acggatgatg 120

tttaaaagtc ccatgtggat cactccgttg ccccgtcgct caccgtgttg gggggaaggt 180

gcacatggct cagttctcaa tggaaattat ctgcctaacc ggctcagttc tgcgtagaaa 240

ccaacatgca agctccaccg ggtgcaaagc ggcagcggcg gcaggatata ttcaattgta 300

aatggcttca tgtccgggaa atctacatgg atcagcaatg agtatgatgg tcaatatgga 360

gaaaaagaaa gagtaattac caattttttt tcaattcaaa aatgtagatg tccgcagcgt 420

tattataaaa tgaaagtaca ttttgataaa acgacaaatt acgatccgtc gtatttatag 480

gcgaaagcaa taaacaaatt attctaattc ggaaatcttt atttcgacgt gtctacattc 540

acgtccaaat gggggcttag atgagaaact tcacgatcgg ctctagttcg agcgatctag 600

taacatagat gacaccgcgc gcgataattt atcctagttt gcgcgctata ttttgttttc 660

tatcgcgtat taaatgtata attgcgggac tctaatcata aaaacccatc tcataaataa 720

cgtcatgcat tacatgttaa ttattacatg cttaacgtaa ttcaacagaa attatatgat 780

aatcatcgca agaccggcaa caggattcaa tcttaagaaa ctttattgcc aaatgtttga 840

acgatcgggg aaattcgttt ctatgcttca attctgctct atagatgttc ttctagtctt 900

aacttttaga aggcacaaca tttgaaatgt tggtaatttt tctgctgggt agtgaaccat 960

tgtaatttgt gtttgcttat attatccgtt gggtttctct atgttatctg tggggccata 1020

tcccagaact ggttgagtcg gtccaacacc tgggtgccaa tcatgtcgat ggtggggtat 1080

ggtccggatt gaaagaaata tagtttaaat atttattgat aaaataacaa gtcaggtatt 1140

atagtccaag caaaaacata aatttattga tgcaagttta aattcagaaa tatttcaata 1200

actgattata tcagctggta cattgccgta gatgaaagac tgagtgcgat attatgtgta 1260

atacataaat tgatgatata gctagcttag ctcatcgggt cagaagaact cgtcaagaag 1320

gcgatagaag gcgatgcgct gcgaatcggg agcggcgata ccgtaaagca cgaggaagcg 1380

gtcagcccat tcgccgccaa gctcttcagc aatatcacgg gtagccaacg ctatgtcctg 1440

atagcggtcc gccacaccca gccggccaca gtcgatgaat ccagaaaagc ggccattttc 1500

caccatgata ttcggcaagc aggcatcgcc atgagtcacg acgagatcct cgccgtcggg 1560

catgcgcgcc ttgagcctgg cgaacagttc ggctggcgcg agcccctgat gctcttcgtc 1620

cagatcatcc tgatcgacaa gaccggcttc catccgagta cgtgctcgct cgatgcgatg 1680

tttcgcttgg tggtcgaatg ggcaggtagc cggatcaagc gtatgcagcc gccgcattgc 1740

atcagccatg atggatactt tctcggcagg agcaaggtga gatgacagga gatcctgccc 1800

cggcacttcg cccaatagca gccagtccct tcccgcttca gtgacaacgt cgagcacagc 1860

tgcgcaagga acgcccgtcg tggccagcca cgatagccgc gctgcctcgt cctgaagttc 1920

attcagggca ccggacaggt cggtcttgac aaaaagaacc gggcgcccct gcgctgacag 1980

ccggaacacg gcggcatcag agcagccgat tgtctgttgt gcccagtcat agccgaatag 2040

cctctccacc caagcggccg gagaacctgc gtgcaatcca tcttgttcaa tccaagctcc 2100

cattttctgt gattttggat gtgtttttgt atggattgag agtgaatatg agactctaat 2160

tggataccga ggggaattta tggaacgtca gtggagcatt tttgacaaga aatatttgct 2220

agctgatagt gaccttaggc gacttttgaa cgcgcaataa tggtttctga cgtatgtgct 2280

tagctcatta aactccagaa acccgcggct gagtggctcc ttcaacgttg cggttctgtc 2340

agttccaaac gtaaaacggc ttgtcccgcg tcatcggcgg gggtcattcc ggactgtggg 2400

gccatatccc agaactggtt gagtcggtcc aacacctggg tgccaatcat gtcgatggtg 2460

gggtatggcc aatttttttt caattcaaaa atgtagatgt ccgcagcgtt attataaaat 2520

gaaagtacat tttgataaaa cgacaaatta cgatccgtcg tatttatagg cgaaagcaat 2580

aaacaaatta ttctaattcg gaaatcttta tttcgacgtg tctacattca cgtccaaatg 2640

ggggcttaga tgagaaactt cacgatcggc tctagaggcc atgggtttgt ttttgtttcc 2700

ctcttccgta caatgaatga aaagaaattg tatatatgag aggaagagga acttaagagg 2760

aatgaggaac tgaagaggga ggtagaggga taaataggta aacgcggata agattccccg 2820

cgtttacctt tccacgtggc gtatgcagag tgggcggacg gttgagtggg gaacacgctt 2880

tactttaatt taaagtaaag tagcactaag tgggacccac tttttatttc tttaaagact 2940

taattattaa gtcttctaga tgcgtatatt atacgtataa actagtggcc ttggcggccg 3000

gcgcgccggc cttaagggcc acccgggagt actgggcccg gtaccactag tattgtaggg 3060

cttatttcta ttgattatta gaaatgccca atgatgttga agtttaataa aaaatttatt 3120

attaaatttg tttgtgtaag attgaaatgt agatatgtaa atatctagta taattgcctt 3180

ggaaaacaaa aaacgaaaaa atgattgccg gaaaacgaaa aaaaaacccc tcagaaatta 3240

gacccccgga aaagcaatat ttttatatta atctgcagcc catccacgaa gtggatgtta 3300

catatttcga agaaataact taactatacc aaacttcatc acttgctaag taatatacat 3360

cgcaattagc gtaaaaccaa tctttcatag attcactcat acaaggtatc cagtccatat 3420

ctggattagt aatagcaata caaggtcttc cgcctggaat gactaagttt ggtttatgtt 3480

ttatccaaac actataatct tcgttacatc cgataataga tttaaacaac tcctgttgta 3540

ttttttgata gcggatatcg tctattatat ggtatgtagt gtggtctata tcgtatagag 3600

accaatctaa taccatgcca gacatatagt ggtgtcggcc tagagatctt gcccactggg 3660

tctttcctgt tctggatggt ccgcatatgt agagacttct tggtcggact ggtctgttgt 3720

ctggctcctg gacaaattaa gtacgttagt gtcttcacta catgctagat cgcgactaag 3780

tcgatctagg tggaagtatt cttcaagagt catttgtctg tcttgaagaa ttgctcccct 3840

gcaattctca catatttcgt aatgtaaaca atctgaactt acgaaatata tattttgttc 3900

cgcccactct ttgatagtgt caggtagcct agagaactcc gtccatctgg gtgtgtaagg 3960

ttgcacaact ggcctgaaat gatagttggc gaaggataga atggaaggcc atctgagaac 4020

gaaatctgtt gggcaggact ctcgtatgtt actgaagaac tcctccttgg tgttggatga 4080

atcaagaatt cgtctccatt ccacgttgct gttgccaata ctgtttggag agcgccctcc 4140

cagtcgaagc tctccaaatg ttctagggct cgtgtcctcc ttggtgatat actcgaatgc 4200

tcgctttggg gcgttgagtc cgtcaattct tgggtgaatc gttcgaccgt ctgggagtat 4260

gatgttgaaa aacaggttcc cattgacgtc gagtttcttg tcacactgga taatacagtg 4320

tgtgtggaat cctccttcag gttgttcttc agtaggctga tgatgttcta gtgatactgc 4380

aaggaataga atcacatatg attctaataa ggttttcagt ttctcgaata tataatcttt 4440

aaaatccccc cctatttggg aatatgttag aaaagcattt tttttttgaa aacgtaaata 4500

accgggttgg ttaggggtag gtctgtttct gtttggagca cgtggcatgt ttgtttttgt 4560

ttccctcttc cgtacaatga atgaaaagaa attgtatata tgagaggaag aggaacttaa 4620

gaggaatgag gaactgaaga gggaggtaga gggataaata ggtaaacgcg gataagattc 4680

cccgcgttta cctttccacg tggcgtatgc agagtgggcg gacggttgag tggggaacac 4740

gctttacttt aatttaaagt aaagtagcac taagtgggac ccacttttta tttctttaaa 4800

gacttaatta ttaagtcttc tagatgcgta tattatacgt ataagtttaa actatcagtg 4860

tttgacagga tatattggcg ggtaaaccta agagaaaaga gcgtttatta gaataatcgg 4920

atatttaaaa gggcgtgaaa aggtttatcc gttcgtccat ttgtatgtgc atgccaacca 4980

cagggttccc ctcgggagtg cttggcattc cgtgcgataa tgacttctgt tcaaccaccc 5040

aaacgtcgga aagcctgacg acggagcagc attccaaaaa gatcccttgg ctcgtctggg 5100

tcggctagaa ggtcgagtgg gctgctgtgg cttgatccct caacgcggtc gcggacgtag 5160

cgcagcgccg aaaaatcctc gatcgcaaat ccgacgctgt cgaaaagcgt gatctgcttg 5220

tcgctctttc ggccgacgtc ctggccagtc atcacgcgcc aaagttccgt cacaggatga 5280

tctggcgcga gttgctggat ctcgccttca atccgggtct gtggcgggaa ctccacgaaa 5340

atatccgaac gcagcaagat atcgcggtgc atctcggtct tgcctgggca gtcgccgccg 5400

acgccgttga tgtggacgcc gaaaaggatc taggtgaaga tcctttttga taatctcatg 5460

accaaaatcc cttaacgtga gttttcgttc cactgagcgt cagaccccgt agaaaagatc 5520

aaaggatctt cttgagatcc tttttttctg cgcgtaatct gctgcttgca aacaaaaaaa 5580

ccaccgctac cagcggtggt ttgtttgccg gatcaagagc taccaactct ttttccgaag 5640

gtaactggct tcagcagagc gcagatacca aatactgttc ttctagtgta gccgtagtta 5700

ggccaccact tcaagaactc tgtagcaccg cctacatacc tcgctctgct aatcctgtta 5760

ccagtggctg ctgccagtgg cgataagtcg tgtcttaccg ggttggactc aagacgatag 5820

ttaccggata aggcgcagcg gtcgggctga acggggggtt cgtgcacaca gcccagcttg 5880

gagcgaacga cctacaccga actgagatac ctacagcgtg agctatgaga aagcgccacg 5940

cttcccgaag ggagaaaggc ggacaggtat ccggtaagcg gcagggtcgg aacaggagag 6000

cgcacgaggg agcttccagg gggaaacgcc tggtatcttt atagtcctgt cgggtttcgc 6060

cacctctgac ttgagcgtcg atttttgtga tgctcgtcag gggggcggag cctatggaaa 6120

aacgccagca acgcggcctt tttacggttc ctggcctttt gctggccttt tgctcacatg 6180

ttctttcctg cgttatcccc tgattctgtg gataaccgat taccgccttt gagtgagctg 6240

ataccgctcg ccgcagccga acgaccgagc gcagcgagtc agtgagcgag gaagcggaag 6300

agcgcctgat gcggtatttt ctccttacgc atctgtgcgg tatttcacac cgcatatggt 6360

gcactctcag tacaatctgc tctgatgccg catagttaag ccagtataca ctccgctatc 6420

gctacgtgac tgggtcatgg ctgcgccccg acacccgcca acacccgctg acgcgccctg 6480

acgggcttgt ctgctcccgg catccgctta cagacaagct gtgaccgtct ccgggagctg 6540

catgtgtcag aggttttcac cgtcatcacc gaaacgcgcg aggcaggggt acgtcgaggt 6600

cgatccaacc cctccgctgc tatagtgcag tcggcttctg acgttcagtg cagccgtctt 6660

ctgaaaacga catgtcgcac aagtcctaag ttacgcgaca ggctgccgcc ctgccctttt 6720

cctggcgttt tcttgtcgcg tgttttagtc gcataaagta gaatacttgc gactagaacc 6780

ggagacatta cgccatgaac aagagcgccg ccgctggcct gctgggctat gcccgcgtca 6840

gcaccgacga ccaggacttg accaaccaac gggccgaact gcacgcggcc ggctgcacca 6900

agctgttttc cgagaagatc accggcacca ggcgcgaccg cccggagctg gccaggatgc 6960

ttgaccacct acgccctggc gacgttgtga cagtgaccag gctagaccgc ctggcccgca 7020

gcacccgcga cctactggac attgccgagc gcatccagga ggccggcgcg ggcctgcgta 7080

gcctggcaga gccgtgggcc gacaccacca cgccggccgg ccgcatggtg ttgaccgtgt 7140

tcgccggcat tgccgagttc gagcgttccc taatcatcga ccgcacccgg agcgggcgcg 7200

aggccgccaa ggcgcgaggc gtgaagtttg gcccccgccc taccctcacc ccggcacaga 7260

tcgcgcacgc ccgcgagctg atcgaccagg aaggccgcac cgtgaaagag gcggctgcac 7320

tgcttggcgt gcatcgctcg accctgtacc gcgcacttga gcgcagcgag gaagtgacgc 7380

ccaccgaggc caggcggcgc ggtgccttcc gtgaggacgc attgaccgag gccgacgccc 7440

tggcggccgc cgagaatgaa cgccaagagg aacaagcatg aaaccgcacc aggacggcca 7500

ggacgaaccg tttttcatta ccgaagagat cgaggcggag atgatcgcgg ccgggtacgt 7560

gttcgagccg cccgcgcacg tctcaaccgt gcggctgcat gaaatcctgg ccggtttgtc 7620

tgatgccaag ctcgcggcct ggccggcgag cttggccgct gaagaaaccg agcgccgccg 7680

tctaaaaagg tgatgtgtat ttgagtaaaa cagcttgcgt catgcggtcg ctgcgtatat 7740

gatgcgatga gtaaataaac aaatacgcaa ggggaacgca tgaaggttat cgctgtactt 7800

aaccagaaag gcgggtcagg caagacgacc atcgcaaccc atctagcccg cgccctgcaa 7860

ctcgccgggg ccgatgttct gttagtcgat tccgatcccc agggcagtgc ccgcgattgg 7920

gcggccgtgc gggaagatca accgctaacc gttgtcggca tcgaccgccc gacgattgac 7980

cgcgacgtga aggccatcgg ccggcgcgac ttcgtagtga tcgacggagc gccccaggcg 8040

gcggacttgg ctgtgtccgc gatcaaggca gccgacttcg tgctgattcc ggtgcagcca 8100

agcccttacg acatatgggc caccgccgac ctggtggagc tggttaagca gcgcattgag 8160

gtcacggatg gaaggctaca agcggccttt gtcgtgtcgc gggcgatcaa aggcacgcgc 8220

atcggcggtg aggttgccga ggcgctggcc gggtacgagc tgcccattct tgagtcccgt 8280

atcacgcagc gcgtgagcta cccaggcact gccgccgccg gcacaaccgt tcttgaatca 8340

gaacccgagg gcgacgctgc ccgcgaggtc caggcgctgg ccgctgaaat taaatcaaaa 8400

ctcatttgag ttaatgaggt aaagagaaaa tgagcaaaag cacaaacacg ctaagtgccg 8460

gccgtccgag cgcacgcagc agcaaggctg caacgttggc cagcctggca gacacgccag 8520

ccatgaagcg ggtcaacttt cagttgccgg cggaggatca caccaagctg aagatgtacg 8580

cggtacgcca aggcaagacc attaccgagc tgctatctga atacatcgcg cagctaccag 8640

agtaaatgag caaatgaata aatgagtaga tgaattttag cggctaaagg aggcggcatg 8700

gaaaatcaag aacaaccagg caccgacgcc gtggaatgcc ccatgtgtgg aggaacgggc 8760

ggttggccag gcgtaagcgg ctgggttgtc tgccggccct gcaatggcac tggaaccccc 8820

aagcccgagg aatcggcgtg agcggtcgca aaccatccgg cccggtacaa atcggcgcgg 8880

cgctgggtga tgacctggtg gagaagttga aggcggcgca ggccgcccag cggcaacgca 8940

tcgaggcaga agcacgcccc ggtgaatcgt ggcaagcggc cgctgatcga atccgcaaag 9000

aatcccggca accgccggca gccggtgcgc cgtcgattag gaagccgccc aagggcgacg 9060

agcaaccaga ttttttcgtt ccgatgctct atgacgtggg cacccgcgat agtcgcagca 9120

tcatggacgt ggccgttttc cgtctgtcga agcgtgaccg acgagctggc gaggtgatcc 9180

gctacgagct tccagacggg cacgtagagg tttccgcagg gccggccggc atggcgagtg 9240

tgtgggatta cgacctggta ctgatggcgg tttcccatct aaccgaatcc atgaaccgat 9300

accgggaagg gaagggagac aagcccggcc gcgtgttccg tccacacgtt gcggacgtac 9360

tcaagttctg ccggcgagcc gatggcggaa agcagaaaga cgacctggta gaaacctgca 9420

ttcggttaaa caccacgcac gttgccatgc agcgtacgaa gaaggccaag aacggccgcc 9480

tggtgacggt atccgagggt gaagccttga ttagccgcta caagatcgta aagagcgaaa 9540

ccgggcggcc ggagtacatc gagatcgagc tagctgattg gatgtaccgc gagatcacag 9600

aaggcaagaa cccggacgtg ctgacggttc accccgatta ctttttgatc gatcccggca 9660

tcggccgttt tctctaccgc ctggcacgcc gcgccgcagg caaggcagaa gccagatggt 9720

tgttcaagac gatctacgaa cgcagtggca gcgccggaga gttcaagaag ttctgtttca 9780

ccgtgcgcaa gctgatcggg tcaaatgacc tgccggagta cgatttgaag gaggaggcgg 9840

ggcaggctgg cccgatccta gtcatgcgct accgcaacct gatcgagggc gaagcatccg 9900

ccggttccta atgtacggag cagatgctag ggcaaattgc cctagcaggg gaaaaaggtc 9960

gaaaaggtct ctttcctgtg gatagcacgt acattgggaa cccaaagccg tacattggga 10020

accggaaccc gtacattggg aacccaaagc cgtacattgg gaaccggtca cacatgtaag 10080

tgactgatat aaaagagaaa aaaggcgatt tttccgccta aaactcttta aaacttatta 10140

aaactcttaa aacccgcctg gcctgtgcat aactgtctgg ccagcgcaca gccgaagagc 10200

tgcaaaaagc gcctaccctt cggtcgctgc gctccctacg ccccgccgct tcgcgtcggc 10260

ctatcgcggc cgctggccgc tcaaaaatgg ctggcctacg gccaggcaat ctaccagggc 10320

gcggacaagc cgcgccgtcg ccactcgacc gccggcgccc acatcaaggc accggtgggt 10380

atgcctgacg atgcgtggag accgaaacct tgcgctcgtt cgccagccag gacagaaatg 10440

cctcgacttc gctgctgccc aaggttgccg ggtgacgcac accgtggaaa cggatgaagg 10500

cacgaaccca gtggacataa gcctgttcgg ttcgtaagct gtaatgcaag tagcgtatgc 10560

gctcacgcaa ctggtccaga accttgaccg aacgcagcgg tggtaacggc gcagtggcgg 10620

ttttcatggc ttgttatgac tgtttttttg gggtacagtc tatgcctcgg gcatccaagc 10680

agcaagcgcg ttacgccgtg ggtcgatgtt tgatgttatg gagcagcaac gatgttacgc 10740

agcagggcag tcgccctaaa acaaagttaa acatcatgag ggaagcggtg atcgccgaag 10800

tatcgactca actatcagag gtagttggcg tcatcgagcg ccatctcgaa ccgacgttgc 10860

tggccgtaca tttgtacggc tccgcagtgg atggcggcct gaagccacac agtgatattg 10920

atttgctggt tacggtgacc gtaaggcttg atgaaacaac gcggcgagct ttgatcaacg 10980

accttttgga aacttcggct tcccctggag agagcgagat tctccgcgct gtagaagtca 11040

ccattgttgt gcacgacgac atcattccgt ggcgttatcc agctaagcgc gaactgcaat 11100

ttggagaatg gcagcgcaat gacattcttg caggtatctt cgagccagcc acgatcgaca 11160

ttgatctggc tatcttgctg acaaaagcaa gagaacatag cgttgccttg gtaggtccag 11220

cggcggagga actctttgat ccggttcctg aacaggatct atttgaggcg ctaaatgaaa 11280

ccttaacgct atggaactcg ccgcccgact gggctggcga tgagcgaaat gtagtgctta 11340

cgttgtcccg catttggtac agcgcagtaa ccggcaaaat cgcgccgaag gatgtcgctg 11400

ccgactgggc aatggagcgc ctgccggccc agtatcagcc cgtcatactt gaagctagac 11460

aggcttatct tggacaagaa gaagatcgct tggcctcgcg cgcagatcag ttggaagaat 11520

ttgtccacta cgtgaaaggc gagatcacca aggtagtcgg caaataatgt ctaacaattc 11580

gttcaagccg acgccgcttc gcggcgcggc ttaactcaag cgttagatgc actaagcaca 11640

taattgctca cagccaaact atcaggtcaa gtctgctttt attattttta agcgtgcata 11700

ataagcccta cacaaattgg gagatatatc atgaaaggct ggctttttct tgttatcgca 11760

atagttggcg aagtaatcgc aacatagctt gcttggtcgt tccgcgtg 11808

<210> 6
<211> 2566
<212> DNA
<213> Artificial Sequence

<220>
<223> BSCTV replicon sequence

<400> 6
ccaatttttt ttcaattcaa aaatgtagat gtccgcagcg ttattataaa atgaaagtac 60

attttgataa aacgacaaat tacgatccgt cgtatttata ggcgaaagca ataaacaaat 120

tattctaatt cggaaatctt tatttcgacg tgtctacatt cacgtccaaa tgggggctta 180

gatgagaaac ttcacgatcg gctctagagg ccatgggtcg acattgaatc gggctctctt 240

caaatcccct atcaattggg tgctttgggt gctcttaaat accaccaagg ggccatccgc 300

attaatatta ccggatggcc cccaaaaaat acgtggccca atgaaaatat gccacgtgga 360

aagctaaaga tgttgatgtg atggatttgg gcgggaaact tcctgaagaa gttttcattc 420

ccgccccttt ttttgactta gtcaaaaaag tagccgacat actgtttgaa ataaagttaa 480

aagtagatca ttgtggtccc cacaagaaac ttgctaagca agtttcgaat tatttctgtt 540

taaatacgtt atttttacat gtatatgtac ttataaacta gtggccttgg cggccggcgc 600

gccggcctta agggccaccc gggagtacta agcttactag tcggataata ctaatatgta 660

tgccactttg tttggcaatt gtagatgtta tttttattaa taaaaatatt atttttttaa 720

tacaatttca ttgcaacact ctggttctga attacactag atacaaaatt aaattcttca 780

tacaggacat gatttaaacc tctaattaca ttattaatac aaattaaacc taaattatcc 840

aagtatatct ctagtctctt tttcaagact ttcaaaaaac ggtccccagt taatgtcctg 900

tgcgatccag tgatcgtcaa atctatccag cacttgtgaa gattcaagta tttgcggagg 960

ttgtggttga atcttatctg gactttcagt tgataaactg gcccggaacg gaagaagtcc 1020

tggagtttga ggtacaatgg attcggtacc aagtccacgg gtatggaatt cgtcgcttgt 1080

tgcagcgtga tgggttcctc tgtgcgaaaa tccctgatta cattcatgat gaattgtgaa 1140

atgacactta caaggaagtt tgatcttgcg aggacgcttc ttgattgtta tcaaagagag 1200

gacttgtgag tttggcgaag actgaatttt gtaatgtcca ggacctaagg gcttcatttt 1260

ctgatttatt gaggaagtcc tggtaagagc tgccttcgcc tggattgcat aatataatac 1320

tgggaatacc acctttaatg acacgtggtt ttccatactt taagtttgtc tgccactctc 1380

tttgtgcgcc tataaggtgc ttccaatgct tcatctttaa gtaattggga tctacgtcat 1440

caatgacgtt atacagaaca ttatcattat aagtttttaa actaaaatct aaatgaccag 1500

taatataatt atgtggacct aaacacctgg cccacattgt tttaccagtt cttgaatcac 1560

cctctatgat taaactatta tatctaaaag gccgcgcagc gggatccaaa ccaaaataag 1620

aatcggccca ctcttgaata atttctggaa ctctagtgaa agaagaaaga ggaaaaggtg 1680

gttgataaag atctggagga ggaagaaaaa tagcctctaa gttaggtttg aggttgtgat 1740

actgaaaaat atatttttct gggagtttct cccttattat ctgcagagct tctaatgcat 1800

tacctgcatt taatgcctct gctgcagcat cattagccgt ctgttgacct ccgcgtgcag 1860

atcttccatc gacctgaaat tcaccccagt cgatgtgatc tccgtccttt gagacgtagg 1920

acttgacgtc ggaactggat ttagctccct gaatattgca gtggaattgt ttgctggtac 1980

ttcgatgttg cagatcgaag taacgggcat tacggatctg gacttttcct tcgaattgaa 2040

taagggcatg cagatgtggt tccccatttt catgtaattc tctgcagatg cgaatatatt 2100

ttttattcga aggtgtattt atagcgagga gctgttctaa ggcgtcttct ttggttactg 2160

aacattgagg gtatgtaagg aaaaaatttt tggctttttt gtaaaaaggc atattgaatc 2220

gggctctctt caaatcccct atcaattggg tgctttgggt gctcttaaat accaccaagg 2280

ggccatccgc attaatatta ccggatggcc cccaaaaaat acgtggccca atgaaaatat 2340

gccacgtgga aagctaaaga tgttgatgtg atggatttgg gcgggaaact tcctgaagaa 2400

gttttcattc ccgccccttt ttttgactta gtcaaaaaag tagccgacat actgtttgaa 2460

ataaagttaa aagtagatca ttgtggtccc cacaagaaac ttgctaagca agtttcgaat 2520

tatttctgtt taaatacgtt atttttacat gtatatgtac ttataa 2566

<210> 7
<211> 12004
<212> DNA
<213> Artificial Sequence

<220>
<223> pZFNnptII-BSCTV-Rep/MCS - full sequence of binary vector
 pZFNnptII including the BSCTV replicon

<400> 7
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg ggtcgacatt gaatcgggct 1620

ctcttcaaat cccctatcaa ttgggtgctt tgggtgctct taaataccac caagggggca 1680

tccgcattaa tattaccgga tggcccccaa aaaatacgtg gcccaatgaa aatatgccac 1740

gtggaaagct aaagatgttg atgtgatgga tttgggcggg aaacttcctg aagaagtttt 1800

cattcccgcc cctttttttg acttagtcaa aaaagtagcc gacatactgt ttgaaataaa 1860

gttaaaagta gatcattgtg gtccccacaa gaaacttgct aagcaagttt cgaattattt 1920

ctgtttaaat acgttatttt tacatgtata tgtacttata aactagtggc cttggcggcc 1980

ggcgcgccgg ccttaagggc cacccgggag tactaagctt actagtcgga taatactaat 2040

atgtatgcca ctttgtttgg caattgtaga tgttattttt attaataaaa atattatttt 2100

tttaatacaa tttcattgca acactctggt tctgaattac actagataca aaattaaatt 2160

cttcatacag gacatgattt aaacctctaa ttacattatt aatacaaatt aaacctaaat 2220

tatccaagta tatctctagt ctctttttca agactttcaa aaaacggtcc ccagttaatg 2280

tcctgtgcga tccagtgatc gtcaaatcta tccagcactt gtgaagattc aagtatttgc 2340

ggaggttgtg gttgaatctt atctggactt tcagttgata aactggcccg gaacggaaga 2400

agtcctggag tttgaggtac aatggattcg gtaccaagtc cacgggtatg gaattcgtcg 2460

cttgttgcag cgtgatgggt tcctctgtgc gaaaatccct gattacattc atgatgaatt 2520

gtgaaatgac acttacaagg aagtttgatc ttgcgaggac gcttcttgat tgttatcaaa 2580

gagaggactt gtgagtttgg cgaagactga attttgtaat gtccaggacc taagggcttc 2640

attttctgat ttattgagga agtcctggta agagctgcct tcgcctggat tgcataatat 2700

aatactggga ataccacctt taatgacacg tggttttcca tactttaagt ttgtctgcca 2760

ctctctttgt gcgcctataa ggtgcttcca atgcttcatc tttaagtaat tgggatctac 2820

gtcatcaatg acgttataca gaacattatc attataagtt tttaaactaa aatctaaatg 2880

accagtaata taattatgtg gacctaaaca cctggcccac attgttttac cagttcttga 2940

atcaccctct atgattaaac tattatatct aaaaggccgc gcagcgggat ccaaaccaaa 3000

ataagaatcg gcccactctt gaataatttc tggaactcta gtgaaagaag aaagaggaaa 3060

aggtggttga taaagatctg gaggaggaag aaaaatagcc tctaagttag gtttgaggtt 3120

gtgatactga aaaatatatt tttctgggag tttctccctt attatctgca gagcttctaa 3180

tgcattacct gcatttaatg cctctgctgc agcatcatta gccgtctgtt gacctccgcg 3240

tgcagatctt ccatcgacct gaaattcacc ccagtcgatg tgatctccgt cctttgagac 3300

gtaggacttg acgtcggaac tggatttagc tccctgaata ttgcagtgga attgtttgct 3360

ggtacttcga tgttgcagat cgaagtaacg ggcattacgg atctggactt ttccttcgaa 3420

ttgaataagg gcatgcagat gtggttcccc attttcatgt aattctctgc agatgcgaat 3480

atatttttta ttcgaaggtg tatttatagc gaggagctgt tctaaggcgt cttctttggt 3540

tactgaacat tgagggtatg taaggaaaaa atttttggct tttttgtaaa aaggcatatt 3600

gaatcgggct ctcttcaaat cccctatcaa ttgggtgctt tgggtgctct taaataccac 3660

caaggggcca tccgcattaa tattaccgga tggcccccaa aaaatacgtg gcccaatgaa 3720

aatatgccac gtggaaagct aaagatgttg atgtgatgga tttgggcggg aaacttcctg 3780

aagaagtttt cattcccgcc cctttttttg acttagtcaa aaaagtagcc gacatactgt 3840

ttgaaataaa gttaaaagta gatcattgtg gtccccacaa gaaacttgct aagcaagttt 3900

cgaattattt ctgtttaaat acgttatttt tacatgtata tgtacttata agtttaaacg 3960

ggccctatca gtgtttgaca ggatatattg gcgggtaaac ctaagagaaa agagcgttta 4020

ttagaataat cggatattta aaagggcgtg aaaaggttta tccgttcgtc catttgtatg 4080

tgcatgccaa ccacagggtt cccctcggga gtgcttggca ttccgtgcga taatgacttc 4140

tgttcaacca cccaaacgtc ggaaagcctg acgacggagc agcattccaa aaagatccct 4200

tggctcgtct gggtcggcta gaaggtcgag tgggctgctg tggcttgatc cctcaacgcg 4260

gtcgcggacg tagcgcagcg ccgaaaaatc ctcgatcgca aatccgacgc tgtcgaaaag 4320

cgtgatctgc ttgtcgctct ttcggccgac gtcctggcca gtcatcacgc gccaaagttc 4380

cgtcacagga tgatctggcg cgagttgctg gatctcgcct tcaatccggg tctgtggcgg 4440

gaactccacg aaaatatccg aacgcagcaa gatatcgcgg tgcatctcgg tcttgcctgg 4500

gcagtcgccg ccgacgccgt tgatgtggac gccgaaaagg atctaggtga agatcctttt 4560

tgataatctc atgaccaaaa tcccttaacg tgagttttcg ttccactgag cgtcagaccc 4620

cgtagaaaag atcaaaggat cttcttgaga tccttttttt ctgcgcgtaa tctgctgctt 4680

gcaaacaaaa aaaccaccgc taccagcggt ggtttgtttg ccggatcaag agctaccaac 4740

tctttttccg aaggtaactg gcttcagcag agcgcagata ccaaatactg ttcttctagt 4800

gtagccgtag ttaggccacc acttcaagaa ctctgtagca ccgcctacat acctcgctct 4860

gctaatcctg ttaccagtgg ctgctgccag tggcgataag tcgtgtctta ccgggttgga 4920

ctcaagacga tagttaccgg ataaggcgca gcggtcgggc tgaacggggg gttcgtgcac 4980

acagcccagc ttggagcgaa cgacctacac cgaactgaga tacctacagc gtgagctatg 5040

agaaagcgcc acgcttcccg aagggagaaa ggcggacagg tatccggtaa gcggcagggt 5100

cggaacagga gagcgcacga gggagcttcc agggggaaac gcctggtatc tttatagtcc 5160

tgtcgggttt cgccacctct gacttgagcg tcgatttttg tgatgctcgt caggggggcg 5220

gagcctatgg aaaaacgcca gcaacgcggc ctttttacgg ttcctggcct tttgctggcc 5280

ttttgctcac atgttctttc ctgcgttatc ccctgattct gtggataacc gattaccgcc 5340

tttgagtgag ctgataccgc tcgccgcagc cgaacgaccg agcgcagcga gtcagtgagc 5400

gaggaagcgg aagagcgcct gatgcggtat tttctcctta cgcatctgtg cggtatttca 5460

caccgcatat ggtgcactct cagtacaatc tgctctgatg ccgcatagtt aagccagtat 5520

acactccgct atcgctacgt gactgggtca tggctgcgcc ccgacacccg ccaacacccg 5580

ctgacgcgcc ctgacgggct tgtctgctcc cggcatccgc ttacagacaa gctgtgaccg 5640

tctccgggag ctgcatgtgt cagaggtttt caccgtcatc accgaaacgc gcgaggcagg 5700

ggtacgtcga ggtcgatcca acccctccgc tgctatagtg cagtcggctt ctgacgttca 5760

gtgcagccgt cttctgaaaa cgacatgtcg cacaagtcct aagttacgcg acaggctgcc 5820

gccctgccct tttcctggcg ttttcttgtc gcgtgtttta gtcgcataaa gtagaatact 5880

tgcgactaga accggagaca ttacgccatg aacaagagcg ccgccgctgg cctgctgggc 5940

tatgcccgcg tcagcaccga cgaccaggac ttgaccaacc aacgggccga actgcacgcg 6000

gccggctgca ccaagctgtt ttccgagaag atcaccggca ccaggcgcga ccgcccggag 6060

ctggccagga tgcttgacca cctacgccct ggcgacgttg tgacagtgac caggctagac 6120

cgcctggccc gcagcacccg cgacctactg gacattgccg agcgcatcca ggaggccggc 6180

gcgggcctgc gtagcctggc agagccgtgg gccgacacca ccacgccggc cggccgcatg 6240

gtgttgaccg tgttcgccgg cattgccgag ttcgagcgtt ccctaatcat cgaccgcacc 6300

cggagcgggc gcgaggccgc caaggcgcga ggcgtgaagt ttggcccccg ccctaccctc 6360

accccggcac agatcgcgca cgcccgcgag ctgatcgacc aggaaggccg caccgtgaaa 6420

gaggcggctg cactgcttgg cgtgcatcgc tcgaccctgt accgcgcact tgagcgcagc 6480

gaggaagtga cgcccaccga ggccaggcgg cgcggtgcct tccgtgagga cgcattgacc 6540

gaggccgacg ccctggcggc cgccgagaat gaacgccaag aggaacaagc atgaaaccgc 6600

accaggacgg ccaggacgaa ccgtttttca ttaccgaaga gatcgaggcg gagatgatcg 6660

cggccgggta cgtgttcgag ccgcccgcgc acgtctcaac cgtgcggctg catgaaatcc 6720

tggccggttt gtctgatgcc aagctcgcgg cctggccggc gagcttggcc gctgaagaaa 6780

ccgagcgccg ccgtctaaaa aggtgatgtg tatttgagta aaacagcttg cgtcatgcgg 6840

tcgctgcgta tatgatgcga tgagtaaata aacaaatacg caaggggaac gcatgaaggt 6900

tatcgctgta cttaaccaga aaggcgggtc aggcaagacg accatcgcaa cccatctagc 6960

ccgcgccctg caactcgccg gggccgatgt tctgttagtc gattccgatc cccagggcag 7020

tgcccgcgat tgggcggccg tgcgggaaga tcaaccgcta accgttgtcg gcatcgaccg 7080

cccgacgatt gaccgcgacg tgaaggccat cggccggcgc gacttcgtag tgatcgacgg 7140

agcgccccag gcggcggact tggctgtgtc cgcgatcaag gcagccgact tcgtgctgat 7200

tccggtgcag ccaagccctt acgacatatg ggccaccgcc gacctggtgg agctggttaa 7260

gcagcgcatt gaggtcacgg atggaaggct acaagcggcc tttgtcgtgt cgcgggcgat 7320

caaaggcacg cgcatcggcg gtgaggttgc cgaggcgctg gccgggtacg agctgcccat 7380

tcttgagtcc cgtatcacgc agcgcgtgag ctacccaggc actgccgccg ccggcacaac 7440

cgttcttgaa tcagaacccg agggcgacgc tgcccgcgag gtccaggcgc tggccgctga 7500

aattaaatca aaactcattt gagttaatga ggtaaagaga aaatgagcaa aagcacaaac 7560

acgctaagtg ccggccgtcc gagcgcacgc agcagcaagg ctgcaacgtt ggccagcctg 7620

gcagacacgc cagccatgaa gcgggtcaac tttcagttgc cggcggagga tcacaccaag 7680

ctgaagatgt acgcggtacg ccaaggcaag accattaccg agctgctatc tgaatacatc 7740

gcgcagctac cagagtaaat gagcaaatga ataaatgagt agatgaattt tagcggctaa 7800

aggaggcggc atggaaaatc aagaacaacc aggcaccgac gccgtggaat gccccatgtg 7860

tggaggaacg ggcggttggc caggcgtaag cggctgggtt gtctgccggc cctgcaatgg 7920

cactggaacc cccaagcccg aggaatcggc gtgagcggtc gcaaaccatc cggcccggta 7980

caaatcggcg cggcgctggg tgatgacctg gtggagaagt tgaaggcggc gcaggccgcc 8040

cagcggcaac gcatcgaggc agaagcacgc cccggtgaat cgtggcaagc ggccgctgat 8100

cgaatccgca aagaatcccg gcaaccgccg gcagccggtg cgccgtcgat taggaagccg 8160

cccaagggcg acgagcaacc agattttttc gttccgatgc tctatgacgt gggcacccgc 8220

gatagtcgca gcatcatgga cgtggccgtt ttccgtctgt cgaagcgtga ccgacgagct 8280

ggcgaggtga tccgctacga gcttccagac gggcacgtag aggtttccgc agggccggcc 8340

ggcatggcga gtgtgtggga ttacgacctg gtactgatgg cggtttccca tctaaccgaa 8400

tccatgaacc gataccggga agggaaggga gacaagcccg gccgcgtgtt ccgtccacac 8460

gttgcggacg tactcaagtt ctgccggcga gccgatggcg gaaagcagaa agacgacctg 8520

gtagaaacct gcattcggtt aaacaccacg cacgttgcca tgcagcgtac gaagaaggcc 8580

aagaacggcc gcctggtgac ggtatccgag ggtgaagcct tgattagccg ctacaagatc 8640

gtaaagagcg aaaccgggcg gccggagtac atcgagatcg agctagctga ttggatgtac 8700

cgcgagatca cagaaggcaa gaacccggac gtgctgacgg ttcaccccga ttactttttg 8760

atcgatcccg gcatcggccg ttttctctac cgcctggcac gccgcgccgc aggcaaggca 8820

gaagccagat ggttgttcaa gacgatctac gaacgcagtg gcagcgccgg agagttcaag 8880

aagttctgtt tcaccgtgcg caagctgatc gggtcaaatg acctgccgga gtacgatttg 8940

aaggaggagg cggggcaggc tggcccgatc ctagtcatgc gctaccgcaa cctgatcgag 9000

ggcgaagcat ccgccggttc ctaatgtacg gagcagatgc tagggcaaat tgccctagca 9060

ggggaaaaag gtcgaaaagg tctctttcct gtggatagca cgtacattgg gaacccaaag 9120

ccgtacattg ggaaccggaa cccgtacatt gggaacccaa agccgtacat tgggaaccgg 9180

tcacacatgt aagtgactga tataaaagag aaaaaaggcg atttttccgc ctaaaactct 9240

ttaaaactta ttaaaactct taaaacccgc ctggcctgtg cataactgtc tggccagcgc 9300

acagccgaag agctgcaaaa agcgcctacc cttcggtcgc tgcgctccct acgccccgcc 9360

gcttcgcgtc ggcctatcgc ggccgctggc cgctcaaaaa tggctggcct acggccaggc 9420

aatctaccag ggcgcggaca agccgcgccg tcgccactcg accgccggcg cccacatcaa 9480

ggcaccggtg ggtatgcctg acgatgcgtg gagaccgaaa ccttgcgctc gttcgccagc 9540

caggacagaa atgcctcgac ttcgctgctg cccaaggttg ccgggtgacg cacaccgtgg 9600

aaacggatga aggcacgaac ccagtggaca taagcctgtt cggttcgtaa gctgtaatgc 9660

aagtagcgta tgcgctcacg caactggtcc agaaccttga ccgaacgcag cggtggtaac 9720

ggcgcagtgg cggttttcat ggcttgttat gactgttttt ttggggtaca gtctatgcct 9780

cgggcatcca agcagcaagc gcgttacgcc gtgggtcgat gtttgatgtt atggagcagc 9840

aacgatgtta cgcagcaggg cagtcgccct aaaacaaagt taaacatcat gagggaagcg 9900

gtgatcgccg aagtatcgac tcaactatca gaggtagttg gcgtcatcga gcgccatctc 9960

gaaccgacgt tgctggccgt acatttgtac ggctccgcag tggatggcgg cctgaagcca 10020

cacagtgata ttgatttgct ggttacggtg accgtaaggc ttgatgaaac aacgcggcga 10080

gctttgatca acgacctttt ggaaacttcg gcttcccctg gagagagcga gattctccgc 10140

gctgtagaag tcaccattgt tgtgcacgac gacatcattc cgtggcgtta tccagctaag 10200

cgcgaactgc aatttggaga atggcagcgc aatgacattc ttgcaggtat cttcgagcca 10260

gccacgatcg acattgatct ggctatcttg ctgacaaaag caagagaaca tagcgttgcc 10320

ttggtaggtc cagcggcgga ggaactcttt gatccggttc ctgaacagga tctatttgag 10380

gcgctaaatg aaaccttaac gctatggaac tcgccgcccg actgggctgg cgatgagcga 10440

aatgtagtgc ttacgttgtc ccgcatttgg tacagcgcag taaccggcaa aatcgcgccg 10500

aaggatgtcg ctgccgactg ggcaatggag cgcctgccgg cccagtatca gcccgtcata 10560

cttgaagcta gacaggctta tcttggacaa gaagaagatc gcttggcctc gcgcgcagat 10620

cagttggaag aatttgtcca ctacgtgaaa ggcgagatca ccaaggtagt cggcaaataa 10680

tgtctaacaa ttcgttcaag ccgacgccgc ttcgcggcgc ggcttaactc aagcgttaga 10740

tgcactaagc acataattgc tcacagccaa actatcaggt caagtctgct tttattattt 10800

ttaagcgtgc ataataagcc ctacacaaat tgggagatat atcatgaaag gctggctttt 10860

tcttgttatc gcaatagttg gcgaagtaat cgcaacatag cttgcttggt cgttccgcgt 10920

gaacgtcggc tcgattgtac ctgcgttcaa atactttgcg atcgtgttgc gcgcctgccc 10980

ggtgcgtcgg ctgatctcac ggatcgactg cttctctcgc aacgccatcc gacggatgat 11040

gtttaaaagt cccatgtgga tcactccgtt gccccgtcgc tcaccgtgtt gggggaaggt 11100

tgcacatggc tcagttctca atggaaatta tctgcctaac cggctcagtt ctgcgtagaa 11160

accaacatgc aagctccacc gggtgcaaag cggcagcggc ggcaggatat attcaattgt 11220

aaatggcttc atgtccggga aatctacatg gatcagcaat gagtatgatg gtcaatatgg 11280

agaaaaagaa agagtaatta ccaatttttt ttcaattcaa aaatgtagat gtccgcagcg 11340

ttattataaa atgaaagtac attttgataa aacgacaaat tacgatccgt cgtatttata 11400

ggcgaaagca ataaacaaat tattctaatt cggaaatctt tatttcgacg tgtctacatt 11460

cacgtccaaa tgggggctta gatgagaaac ttcacgatcg gctctagttc gagcgatcta 11520

gtaacataga tgacaccgcg cgcgataatt tatcctagtt tgcgcgctat attttgtttt 11580

ctatcgcgta ttaaatgtat aattgcggga ctctaatcat aaaaacccat ctcataaata 11640

acgtcatgca ttacatgtta attattacat gcttaacgta attcaacaga aattatatga 11700

taatcatcgc aagaccggca acaggattca atcttaagaa actttattgc caaatgtttg 11760

aacgatcggg gaaattcgtt tctatgcttc aattctgctc tatagatgtt cttctagtct 11820

taacttttag aaggcacaac atttgaaatg ttggtaattt ttctgctggg tagtgaacca 11880

ttgtaatttg tgtttgctta tattatccgt tgggtttctc tatgttatct gtggggccat 11940

atcccagaac tggttgagtc ggtccaacac ctgggtgcca atcatgtcga tggtggggta 12000

tggt 12004

<210> 8
<211> 13628
<212> DNA
<213> Artificial Sequence

<220>
<223> pZFNnptII-BCTIV-Repl-35S-GFP - full sequence of binary vector
      pZFNnptII (for Agro transformation) including the BCTIV replicon
      and an expression cassette for GFP

<400> 8
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg ggtttgtttt tgtttccctc 1620

ttccgtacaa tgaatgaaaa gaaattgtat atatgagagg aagaggaact taagaggaat 1680

gaggaactga agagggaggt agagggataa ataggtaaac gcggataaga ttccccgcgt 1740

ttacctttcc acgtggcgta tgcagagtgg gcggacggtt gagtggggaa cacgctttac 1800

tttaatttaa agtaaagtag cactaagtgg gacccacttt ttatttcttt aaagacttaa 1860

ttattaagtc ttctagatgc gtatattata cgtataaact agtggccttg gcggccggcg 1920

cgcccggtcc ccagatttgc cttttcaatt tcagaaagaa tgctaaccca cagatggtta 1980

gagaggctta cgcagcaggt ctcatcaaga cgatctaccc gagcaataat ctccaggaaa 2040

tcaaatacct tcccaagaag gttaaagatg cagtcaaaag attcaggact aactgcatca 2100

agaacacaga gaaagatata tttctcaaga tcagaagtac tattccagta tggacgattc 2160

aaggcttgct tcacaaacca aggcaagtaa tagagattgg agtctctaaa aaggtagttc 2220

ccactgaatc aaaggccatg gagtcaaaga ttcaaataga ggacctaaca gaactcgccg 2280

taaagactgg cgaacagttc atacagagtc tcttacgact caatgacaag aagaaaatct 2340

tcgtcaacat ggtggagcac gacacacttg tctactccaa aaatatcaaa gatacagtct 2400

cagaagacca aagggcaatt gagacttttc aacaaagggt aatatccgga aacctcctcg 2460

gattccattg cccagctatc tgtcacttta ttgtgaagat agtggaaaag gaaggtggct 2520

cctacaaatg ccatcattgc gataaaggaa aggccatcgt tgaagatgcc tctgccgaca 2580

gtggtcccaa agatggaccc ccacccacga ggagcatcgt ggaaaaagaa gacgttccaa 2640

ccacgtcttc aaagcaagtg gattgatgtg atatctccac tgacgtaagg gatgacgcac 2700

aatcccacta tccttcgcaa gacccttcct ctatataagg aagttcattt catttggaga 2760

gaacacgggg gactctagaa tggtgagtaa aggagaagaa cttttcactg gagttgtccc 2820

aattcttgtt gaattagatg gtgatgttaa tgggcacaaa ttttctgtca gtggagaggg 2880

tgaaggtgat gcaacatacg gaaaacttac ccttaaattt atttgcacta ctggaaaact 2940

acctgttcca tggccaaccc tggtcaccac cctgacctac ggcgtgcagt gcttctcccg 3000

ttaccctgat catatgaagc ggcacgactt cttcaagagc gccatgcctg agggatacgt 3060

gcaggagagg accatcttct tcaaggacga cgggaactac aagacacgtg ctgaagtcaa 3120

gtttgaggga gacaccctcg tcaacaggat cgagcttaag ggaatcgatt tcaaggagga 3180

cggaaacatc ctcggccaca agttggaata caactacaac tcccacaacg tatacatcat 3240

ggccgacaag caaaagaacg gcatcaaagc caacttcaag acccgccaca acatcgaaga 3300

cggcggcgtg caactcgctg atcattatca acaaaatact ccaattggcg atggccctgt 3360

ccttttacca gacaaccatt acctgtccac acaatctgcc ctttcgaaag atcccaacga 3420

aaagagagac cacatggtcc ttcttgagtt tgtaacagct gctgggatta cacatggcat 3480

ggatgaacta tacaaataaa gctcgaattt ccccgatcgt tcaaacattt ggcaataaag 3540

tttcttaaga ttgaatcctg ttgccggtct tgcgatgatt atcatataat ttctgttgaa 3600

ttacgttaag catgtaataa ttaacatgta atgcatgacg ttatttatga gatgggtttt 3660

tatgattaga gtcccgcaat tatacattta atacgcgata gaaaacaaaa tatagcgcgc 3720

aaactaggat aaattatcgc gcgcggtgtc atctatgtta ctagatcggg cccggtacca 3780

ctagtgtaat tgtagggctt atttctattg attattagaa atgcccaatg atgttgaagt 3840

ttaataaaaa atttattatt aaatttgttt gtgtaagatt gaaatgtaga tatgtaaata 3900

tctagtataa ttgccttgga aaacaaaaaa cgaaaaaatg attgccggaa aacgaaaaaa 3960

aaacccctca gaaattagac ccccggaaaa gcaatatttt tatattaatc tgcagcccat 4020

ccacgaagtg gatgttacat atttcgaaga aataacttaa ctataccaaa cttcatcact 4080

tgctaagtaa tatacatcgc aattagcgta aaaccaatct ttcatagatt cactcataca 4140

aggtatccag tccatatctg gattagtaat agcaatacaa ggtcttccgc ctggaatgac 4200

taagtttggt ttatgtttta tccaaacact ataatcttcg ttacatccga taatagattt 4260

aaacaactcc tgttgtattt tttgatagcg gatatcgtct attatatggt atgtagtgtg 4320

gtctatatcg tatagagacc aatctaatac catgccagac atatagtggt gtcggcctag 4380

agatcttgcc cactgggtct ttcctgttct ggatggtccg catatgtaga gacttcttgg 4440

tcggactggt ctgttgtctg gctcctggac aaattaagta cgttagtgtc ttcactacat 4500

gctagatcgc gactaagtcg atctaggtgg aagtattctt caagagtcat ttgtctgtct 4560

tgaagaattg ctcccctgca attctcacat atttcgtaat gtaaacaatc tgaacttacg 4620

aaatatatat tttgttccgc ccactctttg atagtgtcag gtagcctaga gaactccgtc 4680

catctgggtg tgtaaggttg cacaactggc ctgaaatgat agttggcgaa ggatagaatg 4740

gaaggccatc tgagaacgaa atctgttggg caggactctc gtatgttact gaagaactcc 4800

tccttggtgt tggatgaatc aagaattcgt ctccattcca cgttgctgtt gccaatactg 4860

tttggagagc gccctcccag tcgaagctct ccaaatgttc tagggctcgt gtcctccttg 4920

gtgatatact cgaatgctcg ctttgggcgg ttgagtccgt caattcttgg gtgaatcgtt 4980

cgaccgtctg ggagtatgat gttgaaaaac aggttcccat tgacgtcgag tttcttgtca 5040

cactggataa tacagtgtgt gtggaatcct ccttcaggtt gttcttcagt aggctgatga 5100

tgttctagtg atactgcaag gaatagaatc acatatgatt ctaataaggt tttcagtttc 5160

tcgaatatat aatctttaaa atccccccct atttgggaat atgttagaaa agcatttttt 5220

ttttgaaaac gtaaataacc gggttggtta ggggtaggtc tgtttctgtt tggagcacgt 5280

ggcatgtttg tttttgtttc cctcttccgt acaatgaatg aaaagaaatt gtatatatga 5340

gaggaagagg aacttaagag gaatgaggaa ctgaagaggg aggtagaggg ataaataggt 5400

aaacgcggat aagattcccc gcgtttacct ttccacgtgg cgtatgcaga gtgggcggac 5460

ggttgagtgg ggaacacgct ttactttaat ttaaagtaaa gtagcactaa gtgggaccca 5520

ctttttattt ctttaaagac ttaattatta agtcttctag atgcgtatat tatacgtata 5580

agtttaaact atcagtgttt gacaggatat attggcgggt aaacctaaga gaaaagagcg 5640

tttattagaa taatcggata tttaaaaggg cgtgaaaagg tttatccgtt cgtccatttg 5700

tatgtgcatg ccaaccacag ggttcccctc gggagtgctt ggcattccgt gcgataatga 5760

cttctgttca accacccaaa cgtcggaaag cctgacgacg gagcagcatt ccaaaaagat 5820

cccttggctc gtctgggtcg gctagaaggt cgagtgggct gctgtggctt gatccctcaa 5880

cgcggtcgcg gacgtagcgc agcgccgaaa aatcctcgat cgcaaatccg acgctgtcga 5940

aaagcgtgat ctgcttgtcg ctctttcggc cgacgtcctg gccagtcatc acgcgccaaa 6000

gttccgtcac aggatgatct ggcgcgagtt gctggatctc gccttcaatc cgggtctgtg 6060

gcgggaactc cacgaaaata tccgaacgca gcaagatatc gcggtgcatc tcggtcttgc 6120

ctgggcagtc gccgccgacg ccgttgatgt ggacgccgaa aaggatctag gtgaagatcc 6180

tttttgataa tctcatgacc aaaatccctt aacgtgagtt ttcgttccac tgagcgtcag 6240

accccgtaga aaagatcaaa ggatcttctt gagatccttt ttttctgcgc gtaatctgct 6300

gcttgcaaac aaaaaaacca ccgctaccag cggtggtttg tttgccggat caagagctac 6360

caactctttt tccgaaggta actggcttca gcagagcgca gataccaaat actgttcttc 6420

tagtgtagcc gtagttaggc caccacttca agaactctgt agcaccgcct acatacctcg 6480

ctctgctaat cctgttacca gtggctgctg ccagtggcga taagtcgtgt cttaccgggt 6540

tggactcaag acgatagtta ccggataagg cgcagcggtc gggctgaacg gggggttcgt 6600

gcacacagcc cagcttggag cgaacgacct acaccgaact gagataccta cagcgtgagc 6660

tatgagaaag cgccacgctt cccgaaggga gaaaggcgga caggtatccg gtaagcggca 6720

gggtcggaac aggagagcgc acgagggagc ttccaggggg aaacgcctgg tatctttata 6780

gtcctgtcgg gtttcgccac ctctgacttg agcgtcgatt tttgtgatgc tcgtcagggg 6840

ggcggagcct atggaaaaac gccagcaacg cggccttttt acggttcctg gccttttgct 6900

ggccttttgc tcacatgttc tttcctgcgt tatcccctga ttctgtggat aaccgattac 6960

cgcctttgag tgagctgata ccgctcgccg cagccgaacg accgagcgca gcgagtcagt 7020

gagcgaggaa gcggaagagc gcctgatgcg gtattttctc cttacgcatc tgtgcggtat 7080

ttcacaccgc atatggtgca ctctcagtac aatctgctct gatgccgcat agttaagcca 7140

gtatacactc cgctatcgct acgtgactgg gtcatggctg cgccccgaca cccgccaaca 7200

cccgctgacg cgccctgacg ggcttgtctg ctcccggcat ccgcttacag acaagctgtg 7260

accgtctccg ggagctgcat gtgtcagagg ttttcaccgt catcaccgaa acgcgcgagg 7320

caggggtacg tcgaggtcga tccaacccct ccgctgctat agtgcagtcg gcttctgacg 7380

ttcagtgcag ccgtcttctg aaaacgacat gtcgcacaag tcctaagtta cgcgacaggc 7440

tgccgccctg cccttttcct ggcgttttct tgtcgcgtgt tttagtcgca taaagtagaa 7500

tacttgcgac tagaaccgga gacattacgc catgaacaag agcgccgccg ctggcctgct 7560

gggctatgcc cgcgtcagca ccgacgacca ggacttgacc aaccaacggg ccgaactgca 7620

cgcggccggc tgcaccaagc tgttttccga gaagatcacc ggcaccaggc gcgaccgccc 7680

ggagctggcc aggatgcttg accacctacg ccctggcgac gttgtgacag tgaccaggct 7740

agaccgcctg gcccgcagca cccgcgacct actggacatt gccgagcgca tccaggaggc 7800

cggcgcgggc ctgcgtagcc tggcagagcc gtgggccgac accaccacgc cggccggccg 7860

catggtgttg accgtgttcg ccggcattgc cgagttcgag cgttccctaa tcatcgaccg 7920

cacccggagc gggcgcgagg ccgccaaggc gcgaggcgtg aagtttggcc cccgccctac 7980

cctcaccccg gcacagatcg cgcacgcccg cgagctgatc gaccaggaag gccgcaccgt 8040

gaaagaggcg gctgcactgc ttggcgtgca tcgctcgacc ctgtaccgcg cacttgagcg 8100

cagcgaggaa gtgacgccca ccgaggccag gcggcgcggt gccttccgtg aggacgcatt 8160

gaccgaggcc gacgccctgg cggccgccga gaatgaacgc caagaggaac aagcatgaaa 8220

ccgcaccagg acggccagga cgaaccgttt ttcattaccg aagagatcga ggcggagatg 8280

atcgcggccg ggtacgtgtt cgagccgccc gcgcacgtct caaccgtgcg gctgcatgaa 8340

atcctggccg gtttgtctga tgccaagctc gcggcctggc cggcgagctt ggccgctgaa 8400

gaaaccgagc gccgccgtct aaaaaggtga tgtgtatttg agtaaaacag cttgcgtcat 8460

gcggtcgctg cgtatatgat gcgatgagta aataaacaaa tacgcaaggg gaacgcatga 8520

aggttatcgc tgtacttaac cagaaaggcg ggtcaggcaa gacgaccatc gcaacccatc 8580

tagcccgcgc cctgcaactc gccggggccg atgttctgtt agtcgattcc gatccccagg 8640

gcagtgcccg cgattgggcg gccgtgcggg aagatcaacc gctaaccgtt gtcggcatcg 8700

accgcccgac gattgaccgc gacgtgaagg ccatcggccg gcgcgacttc gtagtgatcg 8760

acggagcgcc ccaggcggcg gacttggctg tgtccgcgat caaggcagcc gacttcgtgc 8820

tgattccggt gcagccaagc ccttacgaca tatgggccac cgccgacctg gtggagctgg 8880

ttaagcagcg cattgaggtc acggatggaa ggctacaagc ggcctttgtc gtgtcgcggg 8940

cgatcaaagg cacgcgcatc ggcggtgagg ttgccgaggc gctggccggg tacgagctgc 9000

ccattcttga gtcccgtatc acgcagcgcg tgagctaccc aggcactgcc gccgccggca 9060

caaccgttct tgaatcagaa cccgagggcg acgctgcccg cgaggtccag gcgctggccg 9120

ctgaaattaa atcaaaactc atttgagtta atgaggtaaa gagaaaatga gcaaaagcac 9180

aaacacgcta agtgccggcc gtccgagcgc acgcagcagc aaggctgcaa cgttggccag 9240

cctggcagac acgccagcca tgaagcgggt caactttcag ttgccggcgg aggatcacac 9300

caagctgaag atgtacgcgg tacgccaagg caagaccatt accgagctgc tatctgaata 9360

catcgcgcag ctaccagagt aaatgagcaa atgaataaat gagtagatga attttagcgg 9420

ctaaaggagg cggcatggaa aatcaagaac aaccaggcac cgacgccgtg gaatgcccca 9480

tgtgtggagg aacgggcggt tggccaggcg taagcggctg ggttgtctgc cggccctgca 9540

atggcactgg aacccccaag cccgaggaat cggcgtgagc ggtcgcaaac catccggccc 9600

ggtacaaatc ggcgcggcgc tgggtgatga cctggtggag aagttgaagg cggcgcaggc 9660

cgcccagcgg caacgcatcg aggcagaagc acgccccggt gaatcgtggc aagcggccgc 9720

tgatcgaatc cgcaaagaat cccggcaacc gccggcagcc ggtgcgccgt cgattaggaa 9780

gccgcccaag ggcgacgagc aaccagattt tttcgttccg atgctctatg acgtgggcac 9840

ccgcgatagt cgcagcatca tggacgtggc cgttttccgt ctgtcgaagc gtgaccgacg 9900

agctggcgag gtgatccgct acgagcttcc agacgggcac gtagaggttt ccgcagggcc 9960

ggccggcatg gcgagtgtgt gggattacga cctggtactg atggcggttt cccatctaac 10020

cgaatccatg aaccgatacc gggaagggaa gggagacaag cccggccgcg tgttccgtcc 10080

acacgttgcg gacgtactca agttctgccg gcgagccgat ggcggaaagc agaaagacga 10140

cctggtagaa acctgcattc ggttaaacac cacgcacgtt gccatgcagc gtacgaagaa 10200

ggccaagaac ggccgcctgg tgacggtatc cgagggtgaa gccttgatta gccgctacaa 10260

gatcgtaaag agcgaaaccg ggcggccgga gtacatcgag atcgagctag ctgattggat 10320

gtaccgcgag atcacagaag gcaagaaccc ggacgtgctg acggttcacc ccgattactt 10380

tttgatcgat cccggcatcg gccgttttct ctaccgcctg gcacgccgcg ccgcaggcaa 10440

ggcagaagcc agatggttgt tcaagacgat ctacgaacgc agtggcagcg ccggagagtt 10500

caagaagttc tgtttcaccg tgcgcaagct gatcgggtca aatgacctgc cggagtacga 10560

tttgaaggag gaggcggggc aggctggccc gatcctagtc atgcgctacc gcaacctgat 10620

cgagggcgaa gcatccgccg gttcctaatg tacggagcag atgctagggc aaattgccct 10680

agcaggggaa aaaggtcgaa aaggtctctt tcctgtggat agcacgtaca ttgggaaccc 10740

aaagccgtac attgggaacc ggaacccgta cattgggaac ccaaagccgt acattgggaa 10800

ccggtcacac atgtaagtga ctgatataaa agagaaaaaa ggcgattttt ccgcctaaaa 10860

ctctttaaaa cttattaaaa ctcttaaaac ccgcctggcc tgtgcataac tgtctggcca 10920

gcgcacagcc gaagagctgc aaaaagcgcc tacccttcgg tcgctgcgct ccctacgccc 10980

cgccgcttcg cgtcggccta tcgcggccgc tggccgctca aaaatggctg gcctacggcc 11040

aggcaatcta ccagggcgcg gacaagccgc gccgtcgcca ctcgaccgcc ggcgcccaca 11100

tcaaggcacc ggtgggtatg cctgacgatg cgtggagacc gaaaccttgc gctcgttcgc 11160

cagccaggac agaaatgcct cgacttcgct gctgcccaag gttgccgggt gacgcacacc 11220

gtggaaacgg atgaaggcac gaacccagtg gacataagcc tgttcggttc gtaagctgta 11280

atgcaagtag cgtatgcgct cacgcaactg gtccagaacc ttgaccgaac gcagcggtgg 11340

taacggcgca gtggcggttt tcatggcttg ttatgactgt ttttttgggg tacagtctat 11400

gcctcgggca tccaagcagc aagcgcgtta cgccgtgggt cgatgtttga tgttatggag 11460

cagcaacgat gttacgcagc agggcagtcg ccctaaaaca aagttaaaca tcatgaggga 11520

agcggtgatc gccgaagtat cgactcaact atcagaggta gttggcgtca tcgagcgcca 11580

tctcgaaccg acgttgctgg ccgtacattt gtacggctcc gcagtggatg gcggcctgaa 11640

gccacacagt gatattgatt tgctggttac ggtgaccgta aggcttgatg aaacaacgcg 11700

gcgagctttg atcaacgacc ttttggaaac ttcggcttcc cctggagaga gcgagattct 11760

ccgcgctgta gaagtcacca ttgttgtgca cgacgacatc attccgtggc gttatccagc 11820

taagcgcgaa ctgcaatttg gagaatggca gcgcaatgac attcttgcag gtatcttcga 11880

gccagccacg atcgacattg atctggctat cttgctgaca aaagcaagag aacatagcgt 11940

tgccttggta ggtccagcgg cggaggaact ctttgatccg gttcctgaac aggatctatt 12000

tgaggcgcta aatgaaacct taacgctatg gaactcgccg cccgactggg ctggcgatga 12060

gcgaaatgta gtgcttacgt tgtcccgcat ttggtacagc gcagtaaccg gcaaaatcgc 12120

gccgaaggat gtcgctgccg actgggcaat ggagcgcctg ccggcccagt atcagcccgt 12180

catacttgaa gctagacagg cttatcttgg acaagaagaa gatcgcttgg cctcgcgcgc 12240

agatcagttg gaagaatttg tccactacgt gaaaggcgag atcaccaagg tagtcggcaa 12300

ataatgtcta acaattcgtt caagccgacg ccgcttcgcg gcgcggctta actcaagcgt 12360

tagatgcact aagcacataa ttgctcacag ccaaactatc aggtcaagtc tgcttttatt 12420

atttttaagc gtgcataata agccctacac aaattgggag atatatcatg aaaggctggc 12480

tttttcttgt tatcgcaata gttggcgaag taatcgcaac atagcttgct tggtcgttcc 12540

gcgtgaacgt cggctcgatt gtacctgcgt tcaaatactt tgcgatcgtg ttgcgcgcct 12600

gcccggtgcg tcggctgatc tcacggatcg actgcttctc tcgcaacgcc atccgacgga 12660

tgatgtttaa aagtcccatg tggatcactc cgttgccccg tcgctcaccg tgttgggggg 12720

aaggtgcaca tggctcagtt ctcaatggaa attatctgcc taaccggctc agttctgcgt 12780

agaaaccaac atgcaagctc caccgggtgc aaagcggcag cggcggcagg atatattcaa 12840

ttgtaaatgg cttcatgtcc gggaaatcta catggatcag caatgagtat gatggtcaat 12900

atggagaaaa agaaagagta attaccaatt ttttttcaat tcaaaaatgt agatgtccgc 12960

agcgttatta taaaatgaaa gtacattttg ataaaacgac aaattacgat ccgtcgtatt 13020

tataggcgaa agcaataaac aaattattct aattcggaaa tctttatttc gacgtgtcta 13080

cattcacgtc caaatggggg cttagatgag aaacttcacg atcggctcta gttcgagcga 13140

tctagtaaca tagatgacac cgcgcgcgat aatttatcct agtttgcgcg ctatattttg 13200

ttttctatcg cgtattaaat gtataattgc gggactctaa tcataaaaac ccatctcata 13260

aataacgtca tgcattacat gttaattatt acatgcttaa cgtaattcaa cagaaattat 13320

atgataatca tcgcaagacc ggcaacagga ttcaatctta agaaacttta ttgccaaatg 13380

tttgaacgat cggggaaatt cgtttctatg cttcaattct gctctataga tgttcttcta 13440

gtcttaactt ttagaaggca caacatttga aatgttggta atttttctgc tgggtagtga 13500

accattgtaa tttgtgtttg cttatattat ccgttgggtt tctctatgtt atctgtgggg 13560

ccatatccca gaactggttg agtcggtcca acacctgggt gccaatcatg tcgatggtgg 13620

ggtatggt 13628

<210> 9
<211> 13827
<212> DNA
<213> Artificial Sequence

<220>
<223> pZFNnptII-BSCTV-Repl-35S-GFP - full sequence of binary vector
      pZFNnptII (for Agro transformation) including the BSCTV replicon
      and an expression cassette for GFP

<400> 9
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg ggtcgacatt gaatcgggct 1620

ctcttcaaat cccctatcaa ttgggtgctt tgggtgctct taaataccac caaggggcca 1680

tccgcattaa tattaccgga tggcccccaa aaaatacgtg gcccaatgaa aatatgccac 1740

gtggaaagct aaagatgttg atgtgatgga tttgggcggg aaacttcctg aagaagtttt 1800

cattcccgcc cctttttttg acttagtcaa aaaagtagcc gacatactgt ttgaaataaa 1860

gttaaaagta gatcattgtg gtccccacaa gaaacttgct aagcaagttt cgaattattt 1920

ctgtttaaat acgttatttt tacatgtata tgtacttata aactagtggc cttggcggcc 1980

ggcgcgcccg gtccccagat ttgccttttc aatttcagaa agaatgctaa cccacagatg 2040

gttagagagg cttacgcagc aggtctcatc aagacgatct acccgagcaa taatctccag 2100

gaaatcaaat accttcccaa gaaggttaaa gatgcagtca aaagattcag gactaactgc 2160

atcaagaaca cagagaaaga tatatttctc aagatcagaa gtactattcc agtatggacg 2220

attcaaggct tgcttcacaa accaaggcaa gtaatagaga ttggagtctc taaaaaggta 2280

gttcccactg aatcaaaggc catggagtca aagattcaaa tagaggacct aacagaactc 2340

gccgtaaaga ctggcgaaca gttcatacag agtctcttac gactcaatga caagaagaaa 2400

atcttcgtca acatggtgga gcacgacaca cttgtctact ccaaaaatat caaagataca 2460

gtctcagaag accaaagggc aattgagact tttcaacaaa gggtaatatc cggaaacctc 2520

ctcggattcc attgcccagc tatctgtcac tttattgtga agatagtgga aaaggaaggt 2580

ggctcctaca aatgccatca ttgcgataaa ggaaaggcca tcgttgaaga tgcctctgcc 2640

gacagtggtc ccaaagatgg acccccaccc acgaggagca tcgtggaaaa agaagacgtt 2700

ccaaccacgt cttcaaagca agtggattga tgtgatatct ccactgacgt aagggatgac 2760

gcacaatccc actatccttc gcaagaccct tcctctatat aaggaagttc atttcatttg 2820

gagagaacac gggggactct agaatggtga gtaaaggaga agaacttttc actggagttg 2880

tcccaattct tgttgaatta gatggtgatg ttaatgggca caaattttct gtcagtggag 2940

agggtgaagg tgatgcaaca tacggaaaac ttacccttaa atttatttgc actactggaa 3000

aactacctgt tccatggcca accctggtca ccaccctgac ctacggcgtg cagtgcttct 3060

cccgttaccc tgatcatatg aagcggcacg acttcttcaa gagcgccatg cctgagggat 3120

acgtgcagga gaggaccatc ttcttcaagg acgacgggaa ctacaagaca cgtgctgaag 3180

tcaagtttga gggagacacc ctcgtcaaca ggatcgagct taagggaatc gatttcaagg 3240

aggacggaaa catcctcggc cacaagttgg aatacaacta caactcccac aacgtataca 3300

tcatggccga caagcaaaag aacggcatca aagccaactt caagacccgc cacaacatcg 3360

aagacggcgg cgtgcaactc gctgatcatt atcaacaaaa tactccaatt ggcgatggcc 3420

ctgtcctttt accagacaac cattacctgt ccacacaatc tgccctttcg aaagatccca 3480

acgaaaagag agaccacatg gtccttcttg agtttgtaac agctgctggg attacacatg 3540

gcatggatga actatacaaa taaagctcga atttccccga tcgttcaaac atttggcaat 3600

aaagtttctt aagattgaat cctgttgccg gtcttgcgat gattatcata taatttctgt 3660

tgaattacgt taagcatgta ataattaaca tgtaatgcat gacgttattt atgagatggg 3720

tttttatgat tagagtcccg caattataca tttaatacgc gatagaaaac aaaatatagc 3780

gcgcaaacta ggataaatta tcgcgcgcgg tgtcatctat gttactagat cgggcccggt 3840

accactagtc ggataatact aatatgtatg ccactttgtt tggcaattgt agatgttatt 3900

tttattaata aaaatattat ttttttaata caatttcatt gcaacactct ggttctgaat 3960

tacactagat acaaaattaa attcttcata caggacatga tttaaacctc taattacatt 4020

attaatacaa attaaaccta aattatccaa gtatatctct agtctctttt tcaagacttt 4080

caaaaaacgg tccccagtta atgtcctgtg cgatccagtg atcgtcaaat ctatccagca 4140

cttgtgaaga ttcaagtatt tgcggaggtt gtggttgaat cttatctgga ctttcagttg 4200

ataaactggc ccggaacgga agaagtcctg gagtttgagg tacaatggat tcggtaccaa 4260

gtccacgggt atggaattcg tcgcttgttg cagcgtgatg ggttcctctg tgcgaaaatc 4320

cctgattaca ttcatgatga attgtgaaat gacacttaca aggaagtttg atcttgcgag 4380

gacgcttctt gattgttatc aaagagagga cttgtgagtt tggcgaagac tgaattttgt 4440

aatgtccagg acctaagggc ttcattttct gatttattga ggaagtcctg gtaagagctg 4500

ccttcgcctg gattgcataa tataatactg ggaataccac ctttaatgac acgtggtttt 4560

ccatacttta agtttgtctg ccactctctt tgtgcgccta taaggtgctt ccaatgcttc 4620

atctttaagt aattgggatc tacgtcatca atgacgttat acagaacatt atcattataa 4680

gtttttaaac taaaatctaa atgaccagta atataattat gtggacctaa acacctggcc 4740

cacattgttt taccagttct tgaatcaccc tctatgatta aactattata tctaaaaggc 4800

cgcgcagcgg gatccaaacc aaaataagaa tcggcccact cttgaataat ttctggaact 4860

ctagtgaaag aagaaagagg aaaaggtggt tgataaagat ctggaggagg aagaaaaata 4920

gcctctaagt taggtttgag gttgtgatac tgaaaaatat atttttctgg gagtttctcc 4980

cttattatct gcagagcttc taatgcatta cctgcattta atgcctctgc tgcagcatca 5040

ttagccgtct gttgacctcc gcgtgcagat cttccatcga cctgaaattc accccagtcg 5100

atgtgatctc cgtcctttga gacgtaggac ttgacgtcgg aactggattt agctccctga 5160

atattgcagt ggaattgttt gctggtactt cgatgttgca gatcgaagta acgggcatta 5220

cggatctgga cttttccttc gaattgaata agggcatgca gatgtggttc cccattttca 5280

tgtaattctc tgcagatgcg aatatatttt ttattcgaag gtgtatttat agcgaggagc 5340

tgttctaagg cgtcttcttt ggttactgaa cattgagggt atgtaaggaa aaaatttttg 5400

gcttttttgt aaaaaggcat attgaatcgg gctctcttca aatcccctat caattgggtg 5460

ctttgggtgc tcttaaatac caccaagggg ccatccgcat taatattacc ggatggcccc 5520

caaaaaatac gtggcccaat gaaaatatgc cacgtggaaa gctaaagatg ttgatgtgat 5580

ggatttgggc gggaaacttc ctgaagaagt tttcattccc gccccttttt ttgacttagt 5640

caaaaaagta gccgacatac tgtttgaaat aaagttaaaa gtagatcatt gtggtcccca 5700

caagaaactt gctaagcaag tttcgaatta tttctgttta aatacgttat ttttacatgt 5760

atatgtactt ataagtttaa acgggcccta tcagtgtttg acaggatata ttggcgggta 5820

aacctaagag aaaagagcgt ttattagaat aatcggatat ttaaaagggc gtgaaaaggt 5880

ttatccgttc gtccatttgt atgtgcatgc caaccacagg gttcccctcg ggagtgcttg 5940

gcattccgtg cgataatgac ttctgttcaa ccacccaaac gtcggaaagc ctgacgacgg 6000

agcagcattc caaaaagatc ccttggctcg tctgggtcgg ctagaaggtc gagtgggctg 6060

ctgtggcttg atccctcaac gcggtcgcgg acgtagcgca gcgccgaaaa atcctcgatc 6120

gcaaatccga cgctgtcgaa aagcgtgatc tgcttgtcgc tctttcggcc gacgtcctgg 6180

ccagtcatca cgcgccaaag ttccgtcaca ggatgatctg gcgcgagttg ctggatctcg 6240

ccttcaatcc gggtctgtgg cgggaactcc acgaaaatat ccgaacgcag caagatatcg 6300

cggtgcatct cggtcttgcc tgggcagtcg ccgccgacgc cgttgatgtg gacgccgaaa 6360

aggatctagg tgaagatcct ttttgataat ctcatgacca aaatccctta acgtgagttt 6420

tcgttccact gagcgtcaga ccccgtagaa aagatcaaag gatcttcttg agatcctttt 6480

tttctgcgcg taatctgctg cttgcaaaca aaaaaaccac cgctaccagc ggtggtttgt 6540

ttgccggatc aagagctacc aactcttttt ccgaaggtaa ctggcttcag cagagcgcag 6600

ataccaaata ctgttcttct agtgtagccg tagttaggcc accacttcaa gaactctgta 6660

gcaccgccta catacctcgc tctgctaatc ctgttaccag tggctgctgc cagtggcgat 6720

aagtcgtgtc ttaccgggtt ggactcaaga cgatagttac cggataaggc gcagcggtcg 6780

ggctgaacgg ggggttcgtg cacacagccc agcttggagc gaacgaccta caccgaactg 6840

agatacctac agcgtgagct atgagaaagc gccacgcttc ccgaagggag aaaggcggac 6900

aggtatccgg taagcggcag ggtcggaaca ggagagcgca cgagggagct tccaggggga 6960

aacgcctggt atctttatag tcctgtcggg tttcgccacc tctgacttga gcgtcgattt 7020

ttgtgatgct cgtcaggggg gcggagccta tggaaaaacg ccagcaacgc ggccttttta 7080

cggttcctgg ccttttgctg gccttttgct cacatgttct ttcctgcgtt atcccctgat 7140

tctgtggata accgattacc gcctttgagt gagctgatac cgctcgccgc agccgaacga 7200

ccgagcgcag cgagtcagtg agcgaggaag cggaagagcg cctgatgcgg tattttctcc 7260

ttacgcatct gtgcggtatt tcacaccgca tatggtgcac tctcagtaca atctgctctg 7320

atgccgcata gttaagccag tatacactcc gctatcgcta cgtgactggg tcatggctgc 7380

gccccgacac ccgccaacac ccgctgacgc gccctgacgg gcttgtctgc tcccggcatc 7440

cgcttacaga caagctgtga ccgtctccgg gagctgcatg tgtcagaggt tttcaccgtc 7500

atcaccgaaa cgcgcgaggc aggggtacgt cgaggtcgat ccaacccctc cgctgctata 7560

gtgcagtcgg cttctgacgt tcagtgcagc cgtcttctga aaacgacatg tcgcacaagt 7620

cctaagttac gcgacaggct gccgccctgc ccttttcctg gcgttttctt gtcgcgtgtt 7680

ttagtcgcat aaagtagaat acttgcgact agaaccggag acattacgcc atgaacaaga 7740

gcgccgccgc tggcctgctg ggctatgccc gcgtcagcac cgacgaccag gacttgacca 7800

accaacgggc cgaactgcac gcggccggct gcaccaagct gttttccgag aagatcaccg 7860

gcaccaggcg cgaccgcccg gagctggcca ggatgcttga ccacctacgc cctggcgacg 7920

ttgtgacagt gaccaggcta gaccgcctgg cccgcagcac ccgcgaccta ctggacattg 7980

ccgagcgcat ccaggaggcc ggcgcgggcc tgcgtagcct ggcagagccg tgggccgaca 8040

ccaccacgcc ggccggccgc atggtgttga ccgtgttcgc cggcattgcc gagttcgagc 8100

gttccctaat catcgaccgc acccggagcg ggcgcgaggc cgccaaggcg cgaggcgtga 8160

agtttggccc ccgccctacc ctcaccccgg cacagatcgc gcacgcccgc gagctgatcg 8220

accaggaagg ccgcaccgtg aaagaggcgg ctgcactgct tggcgtgcat cgctcgaccc 8280

tgtaccgcgc acttgagcgc agcgaggaag tgacgcccac cgaggccagg cggcgcggtg 8340

ccttccgtga ggacgcattg accgaggccg acgccctggc ggccgccgag aatgaacgcc 8400

aagaggaaca agcatgaaac cgcaccagga cggccaggac gaaccgtttt tcattaccga 8460

agagatcgag gcggagatga tcgcggccgg gtacgtgttc gagccgcccg cgcacgtctc 8520

aaccgtgcgg ctgcatgaaa tcctggccgg tttgtctgat gccaagctcg cggcctggcc 8580

ggcgagcttg gccgctgaag aaaccgagcg ccgccgtcta aaaaggtgat gtgtatttga 8640

gtaaaacagc ttgcgtcatg cggtcgctgc gtatatgatg cgatgagtaa ataaacaaat 8700

acgcaagggg aacgcatgaa ggttatcgct gtacttaacc agaaaggcgg gtcaggcaag 8760

acgaccatcg caacccatct agcccgcgcc ctgcaactcg ccggggccga tgttctgtta 8820

gtcgattccg atccccaggg cagtgcccgc gattgggcgg ccgtgcggga agatcaaccg 8880

ctaaccgttg tcggcatcga ccgcccgacg attgaccgcg acgtgaaggc catcggccgg 8940

cgcgacttcg tagtgatcga cggagcgccc caggcggcgg acttggctgt gtccgcgatc 9000

aaggcagccg acttcgtgct gattccggtg cagccaagcc cttacgacat atgggccacc 9060

gccgacctgg tggagctggt taagcagcgc attgaggtca cggatggaag gctacaagcg 9120

gcctttgtcg tgtcgcgggc gatcaaaggc acgcgcatcg gcggtgaggt tgccgaggcg 9180

ctggccgggt acgagctgcc cattcttgag tcccgtatca cgcagcgcgt gagctaccca 9240

ggcactgccg ccgccggcac aaccgttctt gaatcagaac ccgagggcga cgctgcccgc 9300

gaggtccagg cgctggccgc tgaaattaaa tcaaaactca tttgagttaa tgaggtaaag 9360

agaaaatgag caaaagcaca aacacgctaa gtgccggccg tccgagcgca cgcagcagca 9420

aggctgcaac gttggccagc ctggcagaca cgccagccat gaagcgggtc aactttcagt 9480

tgccggcgga ggatcacacc aagctgaaga tgtacgcggt acgccaaggc aagaccatta 9540

ccgagctgct atctgaatac atcgcgcagc taccagagta aatgagcaaa tgaataaatg 9600

agtagatgaa ttttagcggc taaaggaggc ggcatggaaa atcaagaaca accaggcacc 9660

gacgccgtgg aatgccccat gtgtggagga acgggcggtt ggccaggcgt aagcggctgg 9720

gttgtctgcc ggccctgcaa tggcactgga acccccaagc ccgaggaatc ggcgtgagcg 9780

gtcgcaaacc atccggcccg gtacaaatcg gcgcggcgct gggtgatgac ctggtggaga 9840

agttgaaggc ggcgcaggcc gcccagcggc aacgcatcga ggcagaagca cgccccggtg 9900

aatcgtggca agcggccgct gatcgaatcc gcaaagaatc ccggcaaccg ccggcagccg 9960

gtgcgccgtc gattaggaag ccgcccaagg gcgacgagca accagatttt ttcgttccga 10020

tgctctatga cgtgggcacc cgcgatagtc gcagcatcat ggacgtggcc gttttccgtc 10080

tgtcgaagcg tgaccgacga gctggcgagg tgatccgcta cgagcttcca gacgggcacg 10140

tagaggtttc cgcagggccg gccggcatgg cgagtgtgtg ggattacgac ctggtactga 10200

tggcggtttc ccatctaacc gaatccatga accgataccg ggaagggaag ggagacaagc 10260

ccggccgcgt gttccgtcca cacgttgcgg acgtactcaa gttctgccgg cgagccgatg 10320

gcggaaagca gaaagacgac ctggtagaaa cctgcattcg gttaaacacc acgcacgttg 10380

ccatgcagcg tacgaagaag gccaagaacg gccgcctggt gacggtatcc gagggtgaag 10440

ccttgattag ccgctacaag atcgtaaaga gcgaaaccgg gcggccggag tacatcgaga 10500

tcgagctagc tgattggatg taccgcgaga tcacagaagg caagaacccg gacgtgctga 10560

cggttcaccc cgattacttt ttgatcgatc ccggcatcgg ccgttttctc taccgcctgg 10620

cacgccgcgc cgcaggcaag gcagaagcca gatggttgtt caagacgatc tacgaacgca 10680

gtggcagcgc cggagagttc aagaagttct gtttcaccgt gcgcaagctg atcgggtcaa 10740

atgacctgcc ggagtacgat ttgaaggagg aggcggggca ggctggcccg atcctagtca 10800

tgcgctaccg caacctgatc gagggcgaag catccgccgg ttcctaatgt acggagcaga 10860

tgctagggca aattgcccta gcaggggaaa aaggtcgaaa aggtctcttt cctgtggata 10920

gcacgtacat tgggaaccca aagccgtaca ttgggaaccg gaacccgtac attgggaacc 10980

caaagccgta cattgggaac cggtcacaca tgtaagtgac tgatataaaa gagaaaaaag 11040

gcgatttttc cgcctaaaac tctttaaaac ttattaaaac tcttaaaacc cgcctggcct 11100

gtgcataact gtctggccag cgcacagccg aagagctgca aaaagcgcct acccttcggt 11160

cgctgcgctc cctacgcccc gccgcttcgc gtcggcctat cgcggccgct ggccgctcaa 11220

aaatggctgg cctacggcca ggcaatctac cagggcgcgg acaagccgcg ccgtcgccac 11280

tcgaccgccg gcgcccacat caaggcaccg gtgggtatgc ctgacgatgc gtggagaccg 11340

aaaccttgcg ctcgttcgcc agccaggaca gaaatgcctc gacttcgctg ctgcccaagg 11400

ttgccgggtg acgcacaccg tggaaacgga tgaaggcacg aacccagtgg acataagcct 11460

gttcggttcg taagctgtaa tgcaagtagc gtatgcgctc acgcaactgg tccagaacct 11520

tgaccgaacg cagcggtggt aacggcgcag tggcggtttt catggcttgt tatgactgtt 11580

tttttggggt acagtctatg cctcgggcat ccaagcagca agcgcgttac gccgtgggtc 11640

gatgtttgat gttatggagc agcaacgatg ttacgcagca gggcagtcgc cctaaaacaa 11700

agttaaacat catgagggaa gcggtgatcg ccgaagtatc gactcaacta tcagaggtag 11760

ttggcgtcat cgagcgccat ctcgaaccga cgttgctggc cgtacatttg tacggctccg 11820

cagtggatgg cggcctgaag ccacacagtg atattgattt gctggttacg gtgaccgtaa 11880

ggcttgatga aacaacgcgg cgagctttga tcaacgacct tttggaaact tcggcttccc 11940

ctggagagag cgagattctc cgcgctgtag aagtcaccat tgttgtgcac gacgacatca 12000

ttccgtggcg ttatccagct aagcgcgaac tgcaatttgg agaatggcag cgcaatgaca 12060

ttcttgcagg tatcttcgag ccagccacga tcgacattga tctggctatc ttgctgacaa 12120

aagcaagaga acatagcgtt gccttggtag gtccagcggc ggaggaactc tttgatccgg 12180

ttcctgaaca ggatctattt gaggcgctaa atgaaacctt aacgctatgg aactcgccgc 12240

ccgactgggc tggcgatgag cgaaatgtag tgcttacgtt gtcccgcatt tggtacagcg 12300

cagtaaccgg caaaatcgcg ccgaaggatg tcgctgccga ctgggcaatg gagcgcctgc 12360

cggcccagta tcagcccgtc atacttgaag ctagacaggc ttatcttgga caagaagaag 12420

atcgcttggc ctcgcgcgca gatcagttgg aagaatttgt ccactacgtg aaaggcgaga 12480

tcaccaaggt agtcggcaaa taatgtctaa caattcgttc aagccgacgc cgcttcgcgg 12540

cgcggcttaa ctcaagcgtt agatgcacta agcacataat tgctcacagc caaactatca 12600

ggtcaagtct gcttttatta tttttaagcg tgcataataa gccctacaca aattgggaga 12660

tatatcatga aaggctggct ttttcttgtt atcgcaatag ttggcgaagt aatcgcaaca 12720

tagcttgctt ggtcgttccg cgtgaacgtc ggctcgattg tacctgcgtt caaatacttt 12780

gcgatcgtgt tgcgcgcctg cccggtgcgt cggctgatct cacggatcga ctgcttctct 12840

cgcaacgcca tccgacggat gatgtttaaa agtcccatgt ggatcactcc gttgccccgt 12900

cgctcaccgt gttgggggga aggtgcacat ggctcagttc tcaatggaaa ttatctgcct 12960

aaccggctca gttctgcgta gaaaccaaca tgcaagctcc accgggtgca aagcggcagc 13020

ggcggcagga tatattcaat tgtaaatggc ttcatgtccg ggaaatctac atggatcagc 13080

aatgagtatg atggtcaata tggagaaaaa gaaagagtaa ttaccaattt tttttcaatt 13140

caaaaatgta gatgtccgca gcgttattat aaaatgaaag tacattttga taaaacgaca 13200

aattacgatc cgtcgtattt ataggcgaaa gcaataaaca aattattcta attcggaaat 13260

ctttatttcg acgtgtctac attcacgtcc aaatgggggc ttagatgaga aacttcacga 13320

tcggctctag ttcgagcgat ctagtaacat agatgacacc gcgcgcgata atttatccta 13380

gtttgcgcgc tatattttgt tttctatcgc gtattaaatg tataattgcg ggactctaat 13440

cataaaaacc catctcataa ataacgtcat gcattacatg ttaattatta catgcttaac 13500

gtaattcaac agaaattata tgataatcat cgcaagaccg gcaacaggat tcaatcttaa 13560

gaaactttat tgccaaatgt ttgaacgatc ggggaaattc gtttctatgc ttcaattctg 13620

ctctatagat gttcttctag tcttaacttt tagaaggcac aacatttgaa atgttggtaa 13680

tttttctgct gggtagtgaa ccattgtaat ttgtgtttgc ttatattatc cgttgggttt 13740

ctctatgtta tctgtggggc catatcccag aactggttga gtcggtccaa cacctgggtg 13800

ccaatcatgt cgatggtggg gtatggt 13827

<210> 10
<211> 13700
<212> DNA
<213> Artificial Sequence

<220>
<223> pZFNnptII-BeYDV Repl-35S-GFP - full sequence of binary vector
pZFNnptII (for Agro transformation) including the BeYDV replicon
and an expression cassette for GFP (control)

<400> 10
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg gcggccgcac tagtggatcc 1620

cccgggatgc atgaattcaa gcttatcgat gtcgactatc ggacgagctc aaagtttaac 1680

gcgttagcag aaggcatgtt gttgtgactc cgaggggttg cctcaaactc tatcttataa 1740

ccggcgtgga ggcatggagg caggggtatt ttggtcattt taatagatag tggaaaatga 1800

cgtggaattt acttaaagac gaagtctttg cgacaagggg gggcccacgc cgaatttaat 1860

attaccggcg tggccccccc ttatcgcgag tgctttagca cgagcggtcc agatttaaag 1920

tagaaaattt cccgcccact agggttaaag gtgttcacac tataaaagca tatacgatgt 1980

gatggtattt gatggagcgt atattgtatc aggtatttcc gttggatacg aattattcgt 2040

acgaccctcg gcgcgccgag aacttccaga gctttgggta cgtcacctga tagtttaaac 2100

ggtccccaga tttgcctttt caatttcaga aagaatgcta acccacagat ggttagagag 2160

gcttacgcag caggtctcat caagacgatc tacccgagca ataatctcca ggaaatcaaa 2220

taccttccca agaaggttaa agatgcagtc aaaagattca ggactaactg catcaagaac 2280

acagagaaag atatatttct caagatcaga agtactattc cagtatggac gattcaaggc 2340

ttgcttcaca aaccaaggca agtaatagag attggagtct ctaaaaaggt agttcccact 2400

gaatcaaagg ccatggagtc aaagattcaa atagaggacc taacagaact cgccgtaaag 2460

actggcgaac agttcataca gagtctctta cgactcaatg acaagaagaa aatcttcgtc 2520

aacatggtgg agcacgacac acttgtctac tccaaaaata tcaaagatac agtctcagaa 2580

gaccaaaggg caattgagac ttttcaacaa agggtaatat ccggaaacct cctcggattc 2640

cattgcccag ctatctgtca ctttattgtg aagatagtgg aaaaggaagg tggctcctac 2700

aaatgccatc attgcgataa aggaaaggcc atcgttgaag atgcctctgc cgacagtggt 2760

cccaaagatg gacccccacc cacgaggagc atcgtggaaa aagaagacgt tccaaccacg 2820

tcttcaaagc aagtggattg atgtgatatc tccactgacg taagggatga cgcacaatcc 2880

cactatcctt cgcaagaccc ttcctctata taaggaagtt catttcattt ggagagaaca 2940

cgggggactc tagaatggtg agtaaaggag aagaactttt cactggagtt gtcccaattc 3000

ttgttgaatt agatggtgat gttaatgggc acaaattttc tgtcagtgga gagggtgaag 3060

gtgatgcaac atacggaaaa cttaccctta aatttatttg cactactgga aaactacctg 3120

ttccatggcc aaccctggtc accaccctga cctacggcgt gcagtgcttc tcccgttacc 3180

ctgatcatat gaagcggcac gacttcttca agagcgccat gcctgaggga tacgtgcagg 3240

agaggaccat cttcttcaag gacgacggga actacaagac acgtgctgaa gtcaagtttg 3300

agggagacac cctcgtcaac aggatcgagc ttaagggaat cgatttcaag gaggacggaa 3360

acatcctcgg ccacaagttg gaatacaact acaactccca caacgtatac atcatggccg 3420

acaagcaaaa gaacggcatc aaagccaact tcaagacccg ccacaacatc gaagacggcg 3480

gcgtgcaact cgctgatcat tatcaacaaa atactccaat tggcgatggc cctgtccttt 3540

taccagacaa ccattacctg tccacacaat ctgccctttc gaaagatccc aacgaaaaga 3600

gagaccacat ggtccttctt gagtttgtaa cagctgctgg gattacacat ggcatggatg 3660

aactatacaa ataaagctcg aatttccccg atcgttcaaa catttggcaa taaagtttct 3720

taagattgaa tcctgttgcc ggtcttgcga tgattatcat ataatttctg ttgaattacg 3780

ttaagcatgt aataattaac atgtaatgca tgacgttatt tatgagatgg gtttttatga 3840

ttagagtccc gcaattatac atttaatacg cgatagaaaa caaaatatag cgcgcaaact 3900

aggataaatt atcgcgcgcg gtgtcatcta tgttactaga tcgtgggccc agctccagac 3960

gtcgcggtgg tggctcgagc gcgtagtcct cggtaatatc gcagaacaaa agtacctgat 4020

atcgagtgta cttcaagtca gtgggaaatc aataaaatga ttattttatg aatatatttc 4080

attgtgcaag tagatagaaa ttacatatgt tacataacac acgaaataaa caaaaaaaga 4140

caatccaaaa acaaacaccc caaaaaaaat aatcacttta gataaactcg tatgaggaga 4200

ggcacgttca gtgactcgac gattcccgag caaaaaaagt ctccccgtca cacatgtagt 4260

gggtgacgca attatcttta aagtaatcct tctgttgact tgtcattgat aacatccagt 4320

cttcgtcagg attgcaaaga attatagaag ggatcccacc ttttattttc ttcttttttc 4380

catatttagg gttgacagtg aaatcagact ggcaacctat taattgcttc cacaatggga 4440

cgaacttgaa ggggatgtcg tcgatgatat tataggtggc gtgttcatcg tagttggtga 4500

aatcgatggt accgttccaa tagttgtgtc gtccgagact tctagcccag gtggtctttc 4560

cggtacgagt tggtccgcag atgtagaggc tggggtgtcg gattccattc cttccattgt 4620

ccttgttaaa tcggccatcc attcaaggtc agattgagct tgttggtatg agacaggatg 4680

tatgtaagta taagcgtcta tgcttacatg gtatagatgg gtttccctcc aggagtgtag 4740

atcttcgtgg cagcgaagat ctgattctgt gaagggcgac acatacggtt caggttgtgg 4800

agggaataat ttgttggctg aatattccag ccattgaagc tttgttgccc attcatgagg 4860

gaattcttcc ttgatcatgt caagatattc ctccttagac gttgcagtct ggataatagt 4920

tctccatcgt gcgtcagatt tgcgaggaga aaccttatga tctcggaaat ctcctctggt 4980

tttaatatct ccgtcctttg atatgtaatc aaggacttgt ttagagtttc tagctggctg 5040

gatattaggg tgatttcctt caaaatcgaa aaaagaagga tccctaatac aaggtttttt 5100

atcaagctgg agaagagcat gatagtgggt agtgccatct tgatgaagct cagaagcaac 5160

accaaggaag aaaataagaa aaggtgtgag tttctcccag agaaactgga ataaatcatc 5220

tctttgagat gagcacttgg gataggtaag gaaaacatat ttagattgga gtctgaagtt 5280

cttactagca gaaggcatgt tgttgtgact ccgaggggtt gcctcaaact ctatcttata 5340

accggcgtgg aggcatggag gcaggggtat tttggtcatt ttaatagata gtggaaaatg 5400

acgtggaatt tacttaaaga cgaagtcttt gcgacaaggg ggggcccacg ccgaatttaa 5460

tattaccggc gtggcccccc cttatcgcga gtgctttagc acgagcggtc cagatttaaa 5520

gtagaaaatt tcccgcccac tagggttaaa ggtgttcaca ctataaaagc atatacgatg 5580

tgatggtatt tgatggagcg tatattgtat caggtatttc cgttggatac gaattattcg 5640

tacgaccctc atagtttaaa ctatcagtgt ttgacaggat atattggcgg gtaaacctaa 5700

gagaaaagag cgtttattag aataatcgga tatttaaaag ggcgtgaaaa ggtttatccg 5760

ttcgtccatt tgtatgtgca tgccaaccac agggttcccc tcgggagtgc ttggcattcc 5820

gtgcgataat gacttctgtt caaccaccca aacgtcggaa agcctgacga cggagcagca 5880

ttccaaaaag atcccttggc tcgtctgggt cggctagaag gtcgagtggg ctgctgtggc 5940

ttgatccctc aacgcggtcg cggacgtagc gcagcgccga aaaatcctcg atcgcaaatc 6000

cgacgctgtc gaaaagcgtg atctgcttgt cgctctttcg gccgacgtcc tggccagtca 6060

tcacgcgcca aagttccgtc acaggatgat ctggcgcgag ttgctggatc tcgccttcaa 6120

tccgggtctg tggcgggaac tccacgaaaa tatccgaacg cagcaagata tcgcggtgca 6180

tctcggtctt gcctgggcag tcgccgccga cgccgttgat gtggacgccg aaaaggatct 6240

aggtgaagat cctttttgat aatctcatga ccaaaatccc ttaacgtgag ttttcgttcc 6300

actgagcgtc agaccccgta gaaaagatca aaggatcttc ttgagatcct ttttttctgc 6360

gcgtaatctg ctgcttgcaa acaaaaaaac caccgctacc agcggtggtt tgtttgccgg 6420

atcaagagct accaactctt tttccgaagg taactggctt cagcagagcg cagataccaa 6480

atactgttct tctagtgtag ccgtagttag gccaccactt caagaactct gtagcaccgc 6540

ctacatacct cgctctgcta atcctgttac cagtggctgc tgccagtggc gataagtcgt 6600

gtcttaccgg gttggactca agacgatagt taccggataa ggcgcagcgg tcgggctgaa 6660

cggggggttc gtgcacacag cccagcttgg agcgaacgac ctacaccgaa ctgagatacc 6720

tacagcgtga gctatgagaa agcgccacgc ttcccgaagg gagaaaggcg gacaggtatc 6780

cggtaagcgg cagggtcgga acaggagagc gcacgaggga gcttccaggg ggaaacgcct 6840

ggtatcttta tagtcctgtc gggtttcgcc acctctgact tgagcgtcga tttttgtgat 6900

gctcgtcagg ggggcggagc ctatggaaaa acgccagcaa cgcggccttt ttacggttcc 6960

tggccttttg ctggcctttt gctcacatgt tctttcctgc gttatcccct gattctgtgg 7020

ataaccgatt accgcctttg agtgagctga taccgctcgc cgcagccgaa cgaccgagcg 7080

cagcgagtca gtgagcgagg aagcggaaga gcgcctgatg cggtattttc tccttacgca 7140

tctgtgcggt atttcacacc gcatatggtg cactctcagt acaatctgct ctgatgccgc 7200

atagttaagc cagtatacac tccgctatcg ctacgtgact gggtcatggc tgcgccccga 7260

cacccgccaa cacccgctga cgcgccctga cgggcttgtc tgctcccggc atccgcttac 7320

agacaagctg tgaccgtctc cgggagctgc atgtgtcaga ggttttcacc gtcatcaccg 7380

aaacgcgcga ggcaggggta cgtcgaggtc gatccaaccc ctccgctgct atagtgcagt 7440

cggcttctga cgttcagtgc agccgtcttc tgaaaacgac atgtcgcaca agtcctaagt 7500

tacgcgacag gctgccgccc tgcccttttc ctggcgtttt cttgtcgcgt gttttagtcg 7560

cataaagtag aatacttgcg actagaaccg gagacattac gccatgaaca agagcgccgc 7620

cgctggcctg ctgggctatg cccgcgtcag caccgacgac caggacttga ccaaccaacg 7680

ggccgaactg cacgcggccg gctgcaccaa gctgttttcc gagaagatca ccggcaccag 7740

gcgcgaccgc ccggagctgg ccaggatgct tgaccaccta cgccctggcg acgttgtgac 7800

agtgaccagg ctagaccgcc tggcccgcag cacccgcgac ctactggaca ttgccgagcg 7860

catccaggag gccggcgcgg gcctgcgtag cctggcagag ccgtgggccg acaccaccac 7920

gccggccggc cgcatggtgt tgaccgtgtt cgccggcatt gccgagttcg agcgttccct 7980

aatcatcgac cgcacccgga gcgggcgcga ggccgccaag gcgcgaggcg tgaagtttgg 8040

cccccgccct accctcaccc cggcacagat cgcgcacgcc cgcgagctga tcgaccagga 8100

aggccgcacc gtgaaagagg cggctgcact gcttggcgtg catcgctcga ccctgtaccg 8160

cgcacttgag cgcagcgagg aagtgacgcc caccgaggcc aggcggcgcg gtgccttccg 8220

tgaggacgca ttgaccgagg ccgacgccct ggcggccgcc gagaatgaac gccaagagga 8280

acaagcatga aaccgcacca ggacggccag gacgaaccgt ttttcattac cgaagagatc 8340

gaggcggaga tgatcgcggc cgggtacgtg ttcgagccgc ccgcgcacgt ctcaaccgtg 8400

cggctgcatg aaatcctggc cggtttgtct gatgccaagc tcgcggcctg gccggcgagc 8460

ttggccgctg aagaaaccga gcgccgccgt ctaaaaaggt gatgtgtatt tgagtaaaac 8520

agcttgcgtc atgcggtcgc tgcgtatatg atgcgatgag taaataaaca aatacgcaag 8580

gggaacgcat gaaggttatc gctgtactta accagaaagg cgggtcaggc aagacgacca 8640

tcgcaaccca tctagcccgc gccctgcaac tcgccggggc cgatgttctg ttagtcgatt 8700

ccgatcccca gggcagtgcc cgcgattggg cggccgtgcg ggaagatcaa ccgctaaccg 8760

ttgtcggcat cgaccgcccg acgattgacc gcgacgtgaa ggccatcggc cggcgcgact 8820

tcgtagtgat cgacggagcg ccccaggcgg cggacttggc tgtgtccgcg atcaaggcag 8880

ccgacttcgt gctgattccg gtgcagccaa gcccttacga catatgggcc accgccgacc 8940

tggtggagct ggttaagcag cgcattgagg tcacggatgg aaggctacaa gcggcctttg 9000

tcgtgtcgcg ggcgatcaaa ggcacgcgca tcggcggtga ggttgccgag gcgctggccg 9060

ggtacgagct gcccattctt gagtcccgta tcacgcagcg cgtgagctac ccaggcactg 9120

ccgccgccgg cacaaccgtt cttgaatcag aacccgaggg cgacgctgcc cgcgaggtcc 9180

aggcgctggc cgctgaaatt aaatcaaaac tcatttgagt taatgaggta aagagaaaat 9240

gagcaaaagc acaaacacgc taagtgccgg ccgtccgagc gcacgcagca gcaaggctgc 9300

aacgttggcc agcctggcag acacgccagc catgaagcgg gtcaactttc agttgccggc 9360

ggaggatcac accaagctga agatgtacgc ggtacgccaa ggcaagacca ttaccgagct 9420

gctatctgaa tacatcgcgc agctaccaga gtaaatgagc aaatgaataa atgagtagat 9480

gaattttagc ggctaaagga ggcggcatgg aaaatcaaga acaaccaggc accgacgccg 9540

tggaatgccc catgtgtgga ggaacgggcg gttggccagg cgtaagcggc tgggttgtct 9600

gccggccctg caatggcact ggaaccccca agcccgagga atcggcgtga gcggtcgcaa 9660

accatccggc ccggtacaaa tcggcgcggc gctgggtgat gacctggtgg agaagttgaa 9720

ggcggcgcag gccgcccagc ggcaacgcat cgaggcagaa gcacgccccg gtgaatcgtg 9780

gcaagcggcc gctgatcgaa tccgcaaaga atcccggcaa ccgccggcag ccggtgcgcc 9840

gtcgattagg aagccgccca agggcgacga gcaaccagat tttttcgttc cgatgctcta 9900

tgacgtgggc acccgcgata gtcgcagcat catggacgtg gccgttttcc gtctgtcgaa 9960

gcgtgaccga cgagctggcg aggtgatccg ctacgagctt ccagacgggc acgtagaggt 10020

ttccgcaggg ccggccggca tggcgagtgt gtgggattac gacctggtac tgatggcggt 10080

ttcccatcta accgaatcca tgaaccgata ccgggaaggg aagggagaca agcccggccg 10140

cgtgttccgt ccacacgttg cggacgtact caagttctgc cggcgagccg atggcggaaa 10200

gcagaaagac gacctggtag aaacctgcat tcggttaaac accacgcacg ttgccatgca 10260

gcgtacgaag aaggccaaga acggccgcct ggtgacggta tccgagggtg aagccttgat 10320

tagccgctac aagatcgtaa agagcgaaac cgggcggccg gagtacatcg agatcgagct 10380

agctgattgg atgtaccgcg agatcacaga aggcaagaac ccggacgtgc tgacggttca 10440

ccccgattac tttttgatcg atcccggcat cggccgtttt ctctaccgcc tggcacgccg 10500

cgccgcaggc aaggcagaag ccagatggtt gttcaagacg atctacgaac gcagtggcag 10560

cgccggagag ttcaagaagt tctgtttcac cgtgcgcaag ctgatcgggt caaatgacct 10620

gccggagtac gatttgaagg aggaggcggg gcaggctggc ccgatcctag tcatgcgcta 10680

ccgcaacctg atcgagggcg aagcatccgc cggttcctaa tgtacggagc agatgctagg 10740

gcaaattgcc ctagcagggg aaaaaggtcg aaaaggtctc tttcctgtgg atagcacgta 10800

cattgggaac ccaaagccgt acattgggaa ccggaacccg tacattggga acccaaagcc 10860

gtacattggg aaccggtcac acatgtaagt gactgatata aaagagaaaa aaggcgattt 10920

ttccgcctaa aactctttaa aacttattaa aactcttaaa acccgcctgg cctgtgcata 10980

actgtctggc cagcgcacag ccgaagagct gcaaaaagcg cctacccttc ggtcgctgcg 11040

ctccctacgc cccgccgctt cgcgtcggcc tatcgcggcc gctggccgct caaaaatggc 11100

tggcctacgg ccaggcaatc taccagggcg cggacaagcc gcgccgtcgc cactcgaccg 11160

ccggcgccca catcaaggca ccggtgggta tgcctgacga tgcgtggaga ccgaaacctt 11220

gcgctcgttc gccagccagg acagaaatgc ctcgacttcg ctgctgccca aggttgccgg 11280

gtgacgcaca ccgtggaaac ggatgaaggc acgaacccag tggacataag cctgttcggt 11340

tcgtaagctg taatgcaagt agcgtatgcg ctcacgcaac tggtccagaa ccttgaccga 11400

acgcagcggt ggtaacggcg cagtggcggt tttcatggct tgttatgact gtttttttgg 11460

ggtacagtct atgcctcggg catccaagca gcaagcgcgt tacgccgtgg gtcgatgttt 11520

gatgttatgg agcagcaacg atgttacgca gcagggcagt cgccctaaaa caaagttaaa 11580

catcatgagg gaagcggtga tcgccgaagt atcgactcaa ctatcagagg tagttggcgt 11640

catcgagcgc catctcgaac cgacgttgct ggccgtacat ttgtacggct ccgcagtgga 11700

tggcggcctg aagccacaca gtgatattga tttgctggtt acggtgaccg taaggcttga 11760

tgaaacaacg cggcgagctt tgatcaacga ccttttggaa acttcggctt cccctggaga 11820

gagcgagatt ctccgcgctg tagaagtcac cattgttgtg cacgacgaca tcattccgtg 11880

gcgttatcca gctaagcgcg aactgcaatt tggagaatgg cagcgcaatg acattcttgc 11940

aggtatcttc gagccagcca cgatcgacat tgatctggct atcttgctga caaaagcaag 12000

agaacatagc gttgccttgg taggtccagc ggcggaggaa ctctttgatc cggttcctga 12060

acaggatcta tttgaggcgc taaatgaaac cttaacgcta tggaactcgc cgcccgactg 12120

ggctggcgat gagcgaaatg tagtgcttac gttgtcccgc atttggtaca gcgcagtaac 12180

cggcaaaatc gcgccgaagg atgtcgctgc cgactgggca atggagcgcc tgccggccca 12240

gtatcagccc gtcatacttg aagctagaca ggcttatctt ggacaagaag aagatcgctt 12300

ggcctcgcgc gcagatcagt tggaagaatt tgtccactac gtgaaaggcg agatcaccaa 12360

ggtagtcggc aaataatgtc taacaattcg ttcaagccga cgccgcttcg cggcgcggct 12420

taactcaagc gttagatgca ctaagcacat aattgctcac agccaaacta tcaggtcaag 12480

tctgctttta ttatttttaa gcgtgcataa taagccctac acaaattggg agatatatca 12540

tgaaaggctg gctttttctt gttatcgcaa tagttggcga agtaatcgca acatagcttg 12600

cttggtcgtt ccgcgtgaac gtcggctcga ttgtacctgc gttcaaatac tttgcgatcg 12660

tgttgcgcgc ctgcccggtg cgtcggctga tctcacggat cgactgcttc tctcgcaacg 12720

ccatccgacg gatgatgttt aaaagtccca tgtggatcac tccgttgccc cgtcgctcac 12780

cgtgttgggg ggaaggtgca catggctcag ttctcaatgg aaattatctg cctaaccggc 12840

tcagttctgc gtagaaacca acatgcaagc tccaccgggt gcaaagcggc agcggcggca 12900

ggatatattc aattgtaaat ggcttcatgt ccgggaaatc tacatggatc agcaatgagt 12960

atgatggtca atatggagaa aaagaaagag taattaccaa ttttttttca attcaaaaat 13020

gtagatgtcc gcagcgttat tataaaatga aagtacattt tgataaaacg acaaattacg 13080

atccgtcgta tttataggcg aaagcaataa acaaattatt ctaattcgga aatctttatt 13140

tcgacgtgtc tacattcacg tccaaatggg ggcttagatg agaaacttca cgatcggctc 13200

tagttcgagc gatctagtaa catagatgac accgcgcgcg ataatttatc ctagtttgcg 13260

cgctatattt tgttttctat cgcgtattaa atgtataatt gcgggactct aatcataaaa 13320

acccatctca taaataacgt catgcattac atgttaatta ttacatgctt aacgtaattc 13380

aacagaaatt atatgataat catcgcaaga ccggcaacag gattcaatct taagaaactt 13440

tattgccaaa tgtttgaacg atcggggaaa ttcgtttcta tgcttcaatt ctgctctata 13500

gatgttcttc tagtcttaac ttttagaagg cacaacattt gaaatgttgg taatttttct 13560

gctgggtagt gaaccattgt aatttgtgtt tgcttatatt atccgttggg tttctctatg 13620

ttatctgtgg ggccatatcc cagaactggt tgagtcggtc caacacctgg gtgccaatca 13680

tgtcgatggt ggggtatggt 13700

<210> 11
<211> 11543
<212> DNA
<213> Artificial Sequence

<220>
<223> pFZNnptII-35-GFP - full sequence of binary vector pZFNnptII (for
      Agro transformation) including an expression cassette for GFP
      (control)

<400> 11
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg gcggccggcg cgcccggtcc 1620

ccagatttgc cttttcaatt tcagaaagaa tgctaaccca cagatggtta gagaggctta 1680

cgcagcaggt ctcatcaaga cgatctaccc gagcaataat ctccaggaaa tcaaatacct 1740

tcccaagaag gttaaagatg cagtcaaaag attcaggact aactgcatca agaacacaga 1800

gaaagatata tttctcaaga tcagaagtac tattccagta tggacgattc aaggcttgct 1860

tcacaaacca aggcaagtaa tagagattgg agtctctaaa aaggtagttc ccactgaatc 1920

aaaggccatg gagtcaaaga ttcaaataga ggacctaaca gaactcgccg taaagactgg 1980

cgaacagttc atacagagtc tcttacgact caatgacaag aagaaaatct tcgtcaacat 2040

ggtggagcac gacacacttg tctactccaa aaatatcaaa gatacagtct cagaagacca 2100

aagggcaatt gagacttttc aacaaagggt aatatccgga aacctcctcg gattccattg 2160

cccagctatc tgtcacttta ttgtgaagat agtggaaaag gaaggtggct cctacaaatg 2220

ccatcattgc gataaaggaa aggccatcgt tgaagatgcc tctgccgaca gtggtcccaa 2280

agatggaccc ccacccacga ggagcatcgt ggaaaaagaa gacgttccaa ccacgtcttc 2340

aaagcaagtg gattgatgtg atatctccac tgacgtaagg gatgacgcac aatcccacta 2400

tccttcgcaa gacccttcct ctatataagg aagttcattt catttggaga gaacacgggg 2460

gactctagaa tggtgagtaa aggagaagaa cttttcactg gagttgtccc aattcttgtt 2520

gaattagatg gtgatgttaa tgggcacaaa ttttctgtca gtggagaggg tgaaggtgat 2580

gcaacatacg gaaaacttac ccttaaattt atttgcacta ctggaaaact acctgttcca 2640

tggccaaccc tggtcaccac cctgacctac ggcgtgcagt gcttctcccg ttaccctgat 2700

catatgaagc ggcacgactt cttcaagagc gccatgcctg agggatacgt gcaggagagg 2760

accatcttct tcaaggacga cgggaactac aagacacgtg ctgaagtcaa gtttgaggga 2820

gacaccctcg tcaacaggat cgagcttaag ggaatcgatt tcaaggagga cggaaacatc 2880

ctcggccaca agttggaata caactacaac tcccacaacg tatacatcat ggccgacaag 2940

caaaagaacg gcatcaaagc caacttcaag acccgccaca acatcgaaga cggcggcgtg 3000

caactcgctg atcattatca acaaaatact ccaattggcg atggccctgt ccttttacca 3060

gacaaccatt acctgtccac acaatctgcc ctttcgaaag atcccaacga aaagagagac 3120

cacatggtcc ttcttgagtt tgtaacagct gctgggatta cacatggcat ggatgaacta 3180

tacaaataaa gctcgaattt ccccgatcgt tcaaacattt ggcaataaag tttcttaaga 3240

ttgaatcctg ttgccggtct tgcgatgatt atcatataat ttctgttgaa ttacgttaag 3300

catgtaataa ttaacatgta atgcatgacg ttatttatga gatgggtttt tatgattaga 3360

gtcccgcaat tatacattta atacgcgata gaaaacaaaa tatagcgcgc aaactaggat 3420

aaattatcgc gcgcggtgtc atctatgtta ctagatcggg cccggtaccc gatcagattg 3480

tcgtttcccg ccttcggttt aaactatcag tgtttgacag gatatattgg cgggtaaacc 3540

taagagaaaa gagcgtttat tagaataatc ggatatttaa aagggcgtga aaaggtttat 3600

ccgttcgtcc atttgtatgt gcatgccaac cacagggttc ccctcgggag tgcttggcat 3660

tccgtgcgat aatgacttct gttcaaccac ccaaacgtcg gaaagcctga cgacggagca 3720

gcattccaaa aagatccctt ggctcgtctg ggtcggctag aaggtcgagt gggctgctgt 3780

ggcttgatcc ctcaacgcgg tcgcggacgt agcgcagcgc cgaaaaatcc tcgatcgcaa 3840

atccgacgct gtcgaaaagc gtgatctgct tgtcgctctt tcggccgacg tcctggccag 3900

tcatcacgcg ccaaagttcc gtcacaggat gatctggcgc gagttgctgg atctcgcctt 3960

caatccgggt ctgtggcggg aactccacga aaatatccga acgcagcaag atatcgcggt 4020

gcatctcggt cttgcctggg cagtcgccgc cgacgccgtt gatgtggacg ccgaaaagga 4080

tctaggtgaa gatccttttt gataatctca tgaccaaaat cccttaacgt gagttttcgt 4140

tccactgagc gtcagacccc gtagaaaaga tcaaaggatc ttcttgagat cctttttttc 4200

tgcgcgtaat ctgctgcttg caaacaaaaa aaccaccgct accagcggtg gtttgtttgc 4260

cggatcaaga gctaccaact ctttttccga aggtaactgg cttcagcaga gcgcagatac 4320

caaatactgt tcttctagtg tagccgtagt taggccacca cttcaagaac tctgtagcac 4380

cgcctacata cctcgctctg ctaatcctgt taccagtggc tgctgccagt ggcgataagt 4440

cgtgtcttac cgggttggac tcaagacgat agttaccgga taaggcgcag cggtcgggct 4500

gaacgggggg ttcgtgcaca cagcccagct tggagcgaac gacctacacc gaactgagat 4560

acctacagcg tgagctatga gaaagcgcca cgcttcccga agggagaaag gcggacaggt 4620

atccggtaag cggcagggtc ggaacaggag agcgcacgag ggagcttcca gggggaaacg 4680

cctggtatct ttatagtcct gtcgggtttc gccacctctg acttgagcgt cgatttttgt 4740

gatgctcgtc aggggggcgg agcctatgga aaaacgccag caacgcggcc tttttacggt 4800

tcctggcctt ttgctggcct tttgctcaca tgttctttcc tgcgttatcc cctgattctg 4860

tggataaccg tattaccgcc tttgagtgag ctgataccgc tcgccgcagc cgaacgaccg 4920

agcgcagcga gtcagtgagc gaggaagcgg aagagcgcct gatgcggtat tttctcctta 4980

gcatctgtgc ggtatttcac accgcatatg gtgcactctc agtacaatct gctctgatgc 5040

cgcatagtta agccagtata cactccgcta tcgctacgtg actgggtcat ggctgcgccc 5100

cgacacccgc caacacccgc tgacgcgccc tgacgggctt gtctgctccc ggcatccgct 5160

tacagacaag ctgtgaccgt ctccgggagc tgcatgtgtc agaggttttc accgtcatca 5220

ccgaaacgcg cgaggcaggg gtacgtcgag gtcgatccaa cccctccgct gctatagtgc 5280

agtcggcttc tgacgttcag tgcagccgtc ttctgaaaac gacatgtcgc acaagtccta 5340

agttacgcga caggctgccg ccctgccctt ttcctggcgt tttcttgtcg cgtgttttag 5400

tcgcataaag tagaatactt gcgactagaa ccggagacat tacgccatga acaagagcgc 5460

cgccgctggc ctgctgggct atgcccgcgt cagcaccgac gaccaggact tgaccaacca 5520

acgggccgaa ctgcacgcgg ccggctgcac caagctgttt tccgagaaga tcaccggcac 5580

caggcgcgac cgcccggagc tggccaggat gcttgaccac ctacgccctg gcgacgttgt 5640

gacagtgacc aggctagacc gcctggcccg cagcacccgc gacctactgg acattgccga 5700

gcgcatccag gaggccggcg cgggcctgcg tagcctggca gagccgtggg ccgacaccac 5760

cacgccggcc ggccgcatgg tgttgaccgt gttcgccggc attgccgagt tcgagcgttc 5820

cctaatcatc gaccgcaccc ggagcgggcg cgaggccgcc aaggcgcgag gcgtgaagtt 5880

tggcccccgc cctaccctca ccccggcaca gatcgcgcac gcccgcgagc tgatcgacca 5940

ggaaggccgc accgtgaaag aggcggctgc actgcttggc gtgcatcgct cgaccctgta 6000

ccgcgcactt gagcgcagcg aggaagtgac gcccaccgag gccaggcggc gcggtgcctt 6060

ccgtgaggac gcattgaccg aggccgacgc cctggcggcc gccgagaatg aacgccaaga 6120

ggaacaagca tgaaaccgca ccaggacggc caggacgaac cgtttttcat taccgaagag 6180

atcgaggcgg agatgatcgc ggccgggtac gtgttcgagc cgcccgcgca cgtctcaacc 6240

gtgcggctgc atgaaatcct ggccggtttg tctgatgcca agctcgcggc ctggccggcg 6300

agcttggccg ctgaagaaac cgagcgccgc cgtctaaaaa ggtgatgtgt atttgagtaa 6360

aacagcttgc gtcatgcggt cgctgcgtat atgatgcgat gagtaaataa acaaatacgc 6420

aaggggaacg catgaaggtt atcgctgtac ttaaccagaa aggcgggtca ggcaagacga 6480

ccatcgcaac ccatctagcc cgcgccctgc aactcgccgg ggccgatgtt ctgttagtcg 6540

attccgatcc ccagggcagt gcccgcgatt gggcggccgt gcgggaagat caaccgctaa 6600

ccgttgtcgg catcgaccgc ccgacgattg accgcgacgt gaaggccatc ggccggcgcg 6660

acttcgtagt gatcgacgga gcgccccagg cggcggactt ggctgtgtcc gcgatcaagg 6720

cagccgactt cgtgctgatt ccggtgcagc caagccctta cgacatatgg gccaccgccg 6780

acctggtgga gctggttaag cagcgcattg aggtcacgga tggaaggcta caagcggcct 6840

ttgtcgtgtc gcgggcgatc aaaggcacgc gcatcggcgg tgaggttgcc gaggcgctgg 6900

ccgggtacga gctgcccatt cttgagtccc gtatcacgca gcgcgtgagc tacccaggca 6960

ctgccgccgc cggcacaacc gttcttgaat cagaacccga gggcgacgct gcccgcgagg 7020

tccaggcgct ggccgctgaa attaaatcaa aactcatttg agttaatgag gtaaagagaa 7080

aatgagcaaa agcacaaaca cgctaagtgc cggccgtccg agcgcacgca gcagcaaggc 7140

tgcaacgttg gccagcctgg cagacacgcc agccatgaag cgggtcaact ttcagttgcc 7200

ggcggaggat cacaccaagc tgaagatgta cgcggtacgc caaggcaaga ccattaccga 7260

gctgctatct gaatacatcg cgcagctacc agagtaaatg agcaaatgaa taaatgagta 7320

gatgaatttt agcggctaaa ggaggcggca tggaaaatca agaacaacca ggcaccgacg 7380

ccgtggaatg ccccatgtgt ggaggaacgg gcggttggcc aggcgtaagc ggctgggttg 7440

tctgccggcc ctgcaatggc actggaaccc ccaagcccga ggaatcggcg tgagcggtcg 7500

caaaccatcc ggcccggtac aaatcggcgc ggcgctgggt gatgacctgg tggagaagtt 7560

gaaggcggcg caggccgccc agcggcaacg catcgaggca gaagcacgcc ccggtgaatc 7620

gtggcaagcg gccgctgatc gaatccgcaa agaatcccgg caaccgccgg cagccggtgc 7680

gccgtcgatt aggaagccgc ccaagggcga cgagcaacca gattttttcg ttccgatgct 7740

ctatgacgtg ggcacccgcg atagtcgcag catcatggac gtggccgttt tccgtctgtc 7800

gaagcgtgac cgacgagctg gcgaggtgat ccgctacgag cttccagacg ggcacgtaga 7860

ggtttccgca gggccggccg gcatggcgag tgtgtgggat tacgacctgg tactgatggc 7920

ggtttcccat ctaaccgaat ccatgaaccg ataccgggaa gggaagggag acaagcccgg 7980

ccgcgtgttc cgtccacacg ttgcggacgt actcaagttc tgccggcgag ccgatggcgg 8040

aaagcagaaa gacgacctgg tagaaacctg cattcggtta aacaccacgc acgttgccat 8100

gcagcgtacg aagaaggcca agaacggccg cctggtgacg gtatccgagg gtgaagcctt 8160

gattagccgc tacaagatcg taaagagcga aaccgggcgg ccggagtaca tcgagatcga 8220

gctagctgat tggatgtacc gcgagatcac agaaggcaag aacccggacg tgctgacggt 8280

tcaccccgat tactttttga tcgatcccgg catcggccgt tttctctacc gcctggcacg 8340

ccgcgccgca ggcaaggcag aagccagatg gttgttcaag acgatctacg aacgcagtgg 8400

cagcgccgga gagttcaaga agttctgttt caccgtgcgc aagctgatcg ggtcaaatga 8460

cctgccggag tacgatttga aggaggaggc ggggcaggct ggcccgatcc tagtcatgcg 8520

ctaccgcaac ctgatcgagg gcgaagcatc cgccggttcc taatgtacgg agcagatgct 8580

agggcaaatt gccctagcag gggaaaaagg tcgaaaaggt ctctttcctg tggatagcac 8640

gtacattggg aacccaaagc cgtacattgg gaaccggaac ccgtacattg ggaacccaaa 8700

gccgtacatt gggaaccggt cacacatgta agtgactgat ataaaagaga aaaaaggcga 8760

tttttccgcc taaaactctt taaaacttat taaaactctt aaaacccgcc tggcctgtgc 8820

ataactgtct ggccagcgca cagccgaaga gctgcaaaaa gcgcctaccc ttcggtcgct 8880

gcgctcccta cgccccgccg cttcgcgtcg gcctatcgcg gccgctggcc gctcaaaaat 8940

ggctggccta cggccaggca atctaccagg gcgcggacaa gccgcgccgt cgccactcga 9000

ccgccggcgc ccacatcaag gcaccggtgg gtatgcctga cgatgcgtgg agaccgaaac 9060

cttgcgctcg ttcgccagcc aggacagaaa tgcctcgact tcgctgctgc ccaaggttgc 9120

cgggtgacgc acaccgtgga aacggatgaa ggcacgaacc cagtggacat aagcctgttc 9180

ggttcgtaag ctgtaatgca agtagcgtat gcgctcacgc aactggtcca gaaccttgac 9240

cgaacgcagc ggtggtaacg gcgcagtggc ggttttcatg gcttgttatg actgtttttt 9300

tggggtacag tctatgcctc gggcatccaa gcagcaagcg cgttacgccg tgggtcgatg 9360

tttgatgtta tggagcagca acgatgttac gcagcagggc agtcgcccta aaacaaagtt 9420

aaacatcatg agggaagcgg tgatcgccga agtatcgact caactatcag aggtagttgg 9480

cgtcatcgag cgccatctcg aaccgacgtt gctggccgta catttgtacg gctccgcagt 9540

ggatggcggc ctgaagccac acagtgatat tgatttgctg gttacggtga ccgtaaggct 9600

tgatgaaaca acgcggcgag ctttgatcaa cgaccttttg gaaacttcgg cttcccctgg 9660

agagagcgag attctccgcg ctgtagaagt caccattgtt gtgcacgacg acatcattcc 9720

gtggcgttat ccagctaagc gcgaactgca atttggagaa tggcagcgca atgacattct 9780

tgcaggtatc ttcgagccag ccacgatcga cattgatctg gctatcttgc tgacaaaagc 9840

aagagaacat agcgttgcct tggtaggtcc agcggcggag gaactctttg atccggttcc 9900

tgaacaggat ctatttgagg cgctaaatga aaccttaacg ctatggaact cgccgcccga 9960

ctgggctggc gatgagcgaa atgtagtgct tacgttgtcc cgcatttggt acagcgcagt 10020

aaccggcaaa atcgcgccga aggatgtcgc tgccgactgg gcaatggagc gcctgccggc 10080

ccagtatcag cccgtcatac ttgaagctag acaggcttat cttggacaag aagaagatcg 10140

cttggcctcg cgcgcagatc agttggaaga atttgtccac tacgtgaaag gcgagatcac 10200

caaggtagtc ggcaaataat gtctaacaat tcgttcaagc cgacgccgct tcgcggcgcg 10260

gcttaactca agcgttagat gcactaagca cataattgct cacagccaaa ctatcaggtc 10320

aagtctgctt ttattatttt taagcgtgca taataagccc tacacaaatt gggagatata 10380

tcatgaaagg ctggcttttt cttgttatcg caatagttgg cgaagtaatc gcaacatagc 10440

ttgcttggtc gttccgcgtg aacgtcggct cgattgtacc tgcgttcaaa tactttgcga 10500

tcgtgttgcg cgcctgcccg gtgcgtcggc tgatctcacg gatcgactgc ttctctcgca 10560

acgccatccg acggatgatg tttaaaagtc ccatgtggat cactccgttg ccccgtcgct 10620

caccgtgttg gggggaaggt gcacatggct cagttctcaa tggaaattat ctgcctaacc 10680

ggctcagttc tgcgtagaaa ccaacatgca agctccaccg ggtgcaaagc ggcagcggcg 10740

gcaggatata ttcaattgta aatggcttca tgtccgggaa atctacatgg atcagcaatg 10800

agtatgatgg tcaatatgga gaaaaagaaa gagtaattac caattttttt tcaattcaaa 10860

aatgtagatg tccgcagcgt tattataaaa tgaaagtaca ttttgataaa acgacaaatt 10920

acgatccgtc gtatttatag gcgaaagcaa taaacaaatt attctaattc ggaaatcttt 10980

atttcgacgt gtctacattc acgtccaaat gggggcttag atgagaaact tcacgatcgg 11040

ctctagttcg agcgatctag taacatagat gacaccgcgc gcgataattt atcctagttt 11100

gcgcgctata ttttgttttc tatcgcgtat taaatgtata attgcgggac tctaatcata 11160

aaaacccatc tcataaataa cgtcatgcat tacatgttaa ttattacatg cttaacgtaa 11220

ttcaacagaa attatatgat aatcatcgca agaccggcaa caggattcaa tcttaagaaa 11280

ctttattgcc aaatgtttga acgatcgggg aaattcgttt ctatgcttca attctgctct 11340

atagatgttc ttctagtctt aacttttaga aggcacaaca tttgaaatgt tggtaatttt 11400

tctgctgggt agtgaaccat tgtaatttgt gtttgcttat attatccgtt gggtttctct 11460

atgttatctg tggggccata tcccagaact ggttgagtcg gtccaacacc tgggtgccaa 11520

tcatgtcgat ggtggggtat ggt 11543

<210> 12
<211> 18764
<212> DNA
<213> Artificial Sequence

<220>
<223> pZFNnptII-BCTIV-Repl/LbCpf1_RibocrRNA_ALS4_RT_ALS-mutated_2x100 -
binary vector for Agro-transformation coding for the replicon
(BCTIV) including GE components (SSN, crRNA and the repair
template (ALS)) and 2*100 nt homology arms; crRNA is produced

<400> 12
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg ggtttgtttt tgtttccctc 1620

ttccgtacaa tgaatgaaaa gaaattgtat atatgagagg aagaggaact taagaggaat 1680

gaggaactga agagggaggt agagggataa ataggtaaac gcggataaga ttccccgcgt 1740

ttacctttcc acgtggcgta tgcagagtgg gcggacggtt gagtggggaa cacgctttac 1800

tttaatttaa agtaaagtag cactaagtgg gacccacttt ttatttcttt aaagacttaa 1860

ttattaagtc ttctagatgc gtatattata cgtataaact agtggccttg gcggccgctc 1920

gagcgatcta gtaacataga tgacaccgcg cgcgataatt tatcctagtt tgcgcgctat 1980

attttgtttt ctatcgcgta ttaaatgtat aattgcggga ctctaatcat aaaaacccat 2040

ctcataaata acgtcatgca ttacatgtta attattacat gcttaacgta attcaacaga 2100

aattatatga taatcatcgc aagaccggca acaggattca atcttaagaa actttattgc 2160

caaatgtttg aacgatcgcc tctctgcttg gctcctcttt cttgtgaaag agggttccca 2220

ggcgacacca cgaggaggtg cagagtagga ctgacgccct aaccgggcac atacatacct 2280

tgatctacac ttagtagaaa ttagacgagc ttactcgttt cgtcctcacg gactcatcag 2340

taatttggat cccctctcca aatgaaatga acttccttat atagaggaag ggtcttgcga 2400

aggatagtgg gattgtgcgt catcccttac gtcagtggag atatcacatc aatccacttg 2460

ctttgaagac gtggttggaa cgtcttcttt ttccacgatg ttcctcgtgg gtgggggtcc 2520

atctttggga ccactgtcgg tagaggcatc ttgaacgata gcctttcctt tatcgcaatg 2580

atggcatttg tagaagccat cttccttttc tactgtcctt tcgatgaagt gacagatagc 2640

tgggcaatgg aatccgagga ggtttcccga tattaccctt tgttgaaaag tctcaatagc 2700

cctctggtct tctgagactg tatctttgat attcttggag tagacgagag tgtcgtgctc 2760

caccatgtat cacatcaatc cacttgcttt gaagacgtgg ttggaacgtc ttctttttcc 2820

acgatgttcc tcgtgggtgg gggtccatct ttgggaccac tgtcggtaga ggcatcttga 2880

acgatagcct ttcctttatc gcaatgatgg catttgtaga agccatcttc cttttctact 2940

gtcctttcga tgaagtgaca gatagctggg caatggaatc cgaggaggtt tcccgatatt 3000

accctttgtt gaaaagtctc aatagccctc tggtcttctg aacactagta aggccttaag 3060

ggccacccgg gctgcaggaa ttcgatctgg cacgacaggt ttcccgactg gaaagcgggc 3120

agtgagcgca acgcaattaa tgtgagttag ctcactcatt aggcacccca ggctttacac 3180

tttatgcttc cggctcgtat gttgtgtgga attgtgagcg gataacaatt tcacacagga 3240

aacagctatg acatgattac gaattcaaaa attacggata tgaatatagg catatccgta 3300

tccgaattat ccgtttgaca gctagcaacg attgtacaat tgcttcttta aaaaaggaag 3360

aaagaaagaa agaaaagaat caacatcagc gttaacaaac ggccccgtta cggcccaaac 3420

ggtcatatag agtaacggcg ttaagcgttg aaagactcct atcgaaatac gtaaccgcaa 3480

acgtgtcata gtcagatccc ctcttccttc accgcctcaa acacaaaaat aatcttctac 3540

agcctatata tacaaccccc ccttctatct ctcctttctc acaattcatc atctttcttt 3600

ctctaccccc aattttaaga aatcctctct tctcctcttc attttcaagg taaatctctc 3660

tctctctctc tctctctgtt attccttgtt ttaattaggt atgtattatt gctagtttgt 3720

taatctgctt atcttatgta tgccttatgt gaatatcttt atcttgttca tctcatccgt 3780

ttagaagcta taaatttgtt gatttgactg tgtatctaca cgtggttatg tttatatcta 3840

atcagatatg aatttcttca tattgttgcg tttgtgtgta ccaatccgaa atcgttgatt 3900

tttttcattt aatcgtgtag ctaattgtac gtatacatat ggatctacgt atcaattgtt 3960

catctgtttg tgtttgtatg tatacagatc tgaaaacatc acttctctca tctgattgtg 4020

ttgttacata catagatata gatctgttat atcatttttt ttattaattg tgtatatata 4080

tatgtgcata gatctggatt acatgattgt gattatttac atgattttgt tatttacgta 4140

tgtatatatg tagatctgga ctttttggag ttgttgactt gattgtattt gtgtgtgtat 4200

atgtgtgttc tgatcttgat atgttatgta tgtgcagcga attcggcgcg ccaaacaatg 4260

gcttcctcca tggctcctaa gaagaagagg aaggttagca agctcgagaa gtttaccaac 4320

tgctacagcc tctctaagac cctcaggttc aaggctatcc ctgtgggaaa gacccaagag 4380

aatatcgaca acaagaggct cctcgtcgag gatgagaaga gagctgaaga ttacaagggc 4440

gtgaagaagc tcctcgacag gtactacctc agcttcatca acgatgtgct ccacagcatc 4500

aagctcaaga acctcaacaa ctacatcagc ctcttccgta agaaaaccag gaccgagaaa 4560

gagaacaaag agcttgagaa cctcgagatc aacctccgta aagagatcgc caaggctttc 4620

aagggaaacg agggatacaa gagcctcttc aagaaggata ttatcgagac aatcctgcct 4680

gagttcctgg acgataagga tgagatcgct ctcgtgaaca gcttcaacgg attcactact 4740

gccttcaccg gattcttcga caacagggaa aacatgttca gcgaagaggc caagagcacc 4800

tctatcgctt tcagatgcat caacgagaac ctcacgcgtt acatcagcaa catggacatc 4860

ttcgagaagg tggacgccat cttcgataag cacgaggtgc aagaaatcaa agagaagatc 4920

ctcaacagcg actacgacgt cgaggacttt tttgaagggg agttcttcaa cttcgttctc 4980

acccaagagg gcatcgacgt gtacaacgct attatcggag gattcgtgac cgagtctggg 5040

gagaagatta agggactcaa cgagtacatc aacctgtaca accagaaaac gaagcagaag 5100

ctcccgaagt tcaagccgct ctacaagcag gttctctctg atcgtgagag cctctcattt 5160

tacggtgagg gttacacctc tgacgaggaa gtgcttgagg ttttccgtaa caccctcaac 5220

aagaacagcg agatcttctc gtccatcaag aagttggaga aacttttcaa gaacttcgac 5280

gagtacagca gcgctgggat cttcgttaag aacggacctg ctatcagcac catcagcaag 5340

gatattttcg gcgagtggaa cgtgatcagg gacaagtgga atgctgagta cgatgacatc 5400

cacctcaaga agaaggctgt cgtcactgag aagtacgagg atgacaggcg taagtcgttc 5460

aagaagatcg gctctttcag cctcgagcag cttcaagaat acgctgatgc tgatctcagc 5520

gtggtcgaga agctcaaaga gatcatcatc cagaaggtcg acgagatcta caaggtgtac 5580

gggtcctctg agaagttgtt cgatgctgat ttcgtcctcg agaagagtct gaagaagaac 5640

gacgctgtcg tcgcgatcat gaaggatttg ctcgacagcg tgaagtcctt cgagaactat 5700

atcaaggcct tcttcggaga gggcaaagag actaataggg acgagtcttt ctacggggat 5760

ttcgtgctcg cttacgatat cctcctcaag gtggaccata tctacgacgc catcagaaac 5820

tacgtgaccc agaagcctta cagcaaggac aagttcaagt tgtactttca gaacccgcag 5880

ttcatgggcg gatgggacaa agacaaagag acagattaca gggccaccat cctcaggtac 5940

gggtctaagt actacctggc catcatggac aagaaatacg ccaagtgcct ccaaaagatc 6000

gacaaggatg acgtgaacgg gaactatgag aagatcaact acaagctcct tccgggaccg 6060

aacaagatgc ttcctaaggt gttcttcagc aagaaatgga tggcctacta caacccgtct 6120

gaggacatcc agaaaatcta caagaacggg accttcaaga aaggcgacat gttcaacctc 6180

aacgactgcc acaagctcat cgatttcttc aaggacagca tctcgcgtta cccgaagtgg 6240

tctaacgctt acgactttaa cttcagcgag acagaaaagt acaaggatat cgccgggttc 6300

taccgtgagg ttgaggaaca gggttacaag gttagcttcg agagcgcctc caagaaagag 6360

gttgacaagt tggtcgaaga gggcaagctc tacatgttcc agatctataa caaggacttc 6420

tccgacaaga gccacggaac tcctaacctc catacgatgt acttcaagct gcttttcgac 6480

gagaacaacc acgggcagat cagactttct ggtggtgctg aactcttcat gcgtagggcc 6540

tcactcaaga aagaagagtt ggttgttcac ccggccaact ctccaatcgc taacaagaat 6600

cctgacaacc cgaaaaagac caccacgctg tcttacgacg tctacaagga caaaaggttc 6660

agcgaggacc agtacgagct tcatatcccg atcgctatca acaagtgccc gaagaacatc 6720

ttcaagatca ataccgaggt gagggtgctg ctcaagcacg atgataaccc ttacgtgatc 6780

ggaatcgatc gtggtgagag aaacctcctc tacatcgttg tggtggacgg aaagggaaac 6840

atcgtcgagc agtacagcct gaacgagatt atcaacaatt tcaacggcat caggatcaag 6900

accgactacc actcactcct cgataagaaa gaaaaagagc gtttcgaggc caggcagaac 6960

tggacttcta tcgaaaacat caaagagttg aaggccggct acatctctca ggtggtgcat 7020

aagatctgcg agctggtgga aaagtacgat gctgtgatcg ctcttgagga cctcaactct 7080

gggttcaaga acagtagagt gaaggttgag aagcaggtct accaaaagtt cgagaagatg 7140

ctcatcgaca agctcaacta catggtggac aaaaagagca acccttgcgc taccggtggt 7200

gctcttaagg gataccagat cacgaacaag ttcgagtcct tcaagagcat gagcacccag 7260

aacggcttca tcttctatat ccctgcttgg ctcaccagca agatcgatcc ttctactggt 7320

ttcgtgaacc tgctcaagac caagtacacc tcgatcgccg acagcaagaa gttcatctcg 7380

tctttcgaca ggatcatgta cgtgccggaa gaggatcttt tcgagttcgc tctcgactat 7440

aagaacttca gcaggaccga cgccgactac attaagaagt ggaagctcta ctcctacggg 7500

aaccgtatca ggatcttccg aaatccgaag aaaaacaacg tgttcgactg ggaagaagtg 7560

tgcctcacct ctgcctacaa agaactgttc aacaagtacg gcatcaacta ccagcagggt 7620

gatatcaggg ctcttttgtg cgagcagagc gacaaggcat tctacagctc attcatggcc 7680

ctcatgtctc tcatgctcca gatgaggaac tctatcaccg gaaggaccga tgtggacttc 7740

cttatctctc cggtcaagaa ctctgacggg atcttctacg acagccgtaa ctatgaggct 7800

caagagaacg ctatcctgcc gaagaatgct gatgcaaacg gggcttacaa cattgcgaga 7860

aaggttctct gggctatcgg gcagtttaag aaagcggaag atgagaagct cgacaaggtg 7920

aagatcgcca tctccaacaa agagtggctt gagtacgctc agacctccgt taagcacaag 7980

aggcctgctg ctactaagaa agctggccag gccaaaaaga agaagtgagg cgcgccgagc 8040

tccaggcctc ccagctttcg tccgtatcat cggtttcgac aacgttcgtc aagttcaatg 8100

catcagtttc attgcccaca caccagaatc ctactaagtt tgagtattat ggcattggaa 8160

aagctgtttt cttctatcat ttgttctgct tgtaatttac tgtgttcttt cagtttttgt 8220

tttcggacat caaaatgcaa atggatggat aagagttaat aaatgatatg gtccttttgt 8280

tcattctcaa attattatta tctgttgttt ttactttaat gggttgaatt taagtaagaa 8340

aggaactaac agtgtgatat taaggtgcaa tgttagacat ataaaacagt ctttcacctc 8400

tctttggtta tgtcttgaat tggtttgttt cttcacttat ctgtgtaatc aagtttacta 8460

tgagtctatg atcaagtaat tatgcaatca agttaagtac agtataggct tgagctccct 8520

aggatcaagc tttatggggt ttgggctacc agccgccatt ggagctgcag ttgctcgacc 8580

agatgcagtg gttgtcgata ttgatgggga tggcagtttt attatgaatg ttcaggaact 8640

tgcaactata agagttgaga accttcctgt gaaaattatg cttctcaata accagcactt 8700

gggaatggtg gttcagttgg aggacagatt ttacaaggca aatcgcgctc acacttattt 8760

gggtaatcca tctaagtctg ctgatatctt ccctgatatg ctcaaattcg ctgaggcatg 8820

tgatattcct tctgcccgtg ttagcaacgt ggctgatttg agggccgcca ttcaaaaagc 8880

ttatcgatac cgtcgacctc gagggggggc ccggtaccac tagtattgta gggcttattt 8940

ctattgatta ttagaaatgc ccaatgatgt tgaagtttaa taaaaaattt attattaaat 9000

ttgtttgtgt aagattgaaa tgtagatatg taaatatcta gtataattgc cttggaaaac 9060

aaaaaacgaa aaaatgattg ccggaaaacg aaaaaaaaac ccctcagaaa ttagaccccc 9120

ggaaaagcaa tatttttata ttaatctgca gcccatccac gaagtggatg ttacatattt 9180

cgaagaaata acttaactat accaaacttc atcacttgct aagtaatata catcgcaatt 9240

agcgtaaaac caatctttca tagattcact catacaaggt atccagtcca tatctggatt 9300

agtaatagca atacaaggtc ttccgcctgg aatgactaag tttggtttat gttttatcca 9360

aacactataa tcttcgttac atccgataat agatttaaac aactcctgtt gtattttttg 9420

atagcggata tcgtctatta tatggtatgt agtgtggtct atatcgtata gagaccaatc 9480

taataccatg ccagacatat agtggtgtcg gcctagagat cttgcccact gggtctttcc 9540

tgttctggat ggtccgcata tgtagagact tcttggtcgg actggtctgt tgtctggctc 9600

ctggacaaat taagtacgtt agtgtcttca ctacatgcta gatcgcgact aagtcgatct 9660

aggtggaagt attcttcaag agtcatttgt ctgtcttgaa gaattgctcc cctgcaattc 9720

tcacatattt cgtaatgtaa acaatctgaa cttacgaaat atatattttg ttccgcccac 9780

tctttgatag tgtcaggtag cctagagaac tccgtccatc tgggtgtgta aggttgcaca 9840

actggcctga aatgatagtt ggcgaaggat agaatggaag gccatctgag aacgaaatct 9900

gttgggcagg actctcgtat gttactgaag aactcctcct tggtgttgga tgaatcaaga 9960

attcgtctcc attccacgtt gctgttgcca atactgtttg gagagcgccc tcccagtcga 10020

agctctccaa atgttctagg gctcgtgtcc tccttggtga tatactcgaa tgctcgcttt 10080

ggggcgttga gtccgtcaat tcttgggtga atcgttcgac cgtctgggag tatgatgttg 10140

aaaaacaggt tcccattgac gtcgagtttc ttgtcacact ggataataca gtgtgtgtgg 10200

aatcctcctt caggttgttc ttcagtaggc tgatgatgtt ctagtgatac tgcaaggaat 10260

agaatcacat atgattctaa taaggttttc agtttctcga atatataatc tttaaaatcc 10320

ccccctattt gggaatatgt tagaaaagca tttttttttt gaaaacgtaa ataaccgggt 10380

tggttagggg taggtctgtt tctgtttgga gcacgtggca tgtttgtttt tgtttccctc 10440

ttccgtacaa tgaatgaaaa gaaattgtat atatgagagg aagaggaact taagaggaat 10500

gaggaactga agagggaggt agagggataa ataggtaaac gcggataaga ttccccgcgt 10560

ttacctttcc acgtggcgta tgcagagtgg gcggacggtt gagtggggaa cacgctttac 10620

tttaatttaa agtaaagtag cactaagtgg gacccacttt ttatttcttt aaagacttaa 10680

ttattaagtc ttctagatgc gtatattata cgtataagtt taaactatca gtgtttgaca 10740

ggatatattg gcgggtaaac ctaagagaaa agagcgttta ttagaataat cggatattta 10800

aaagggcgtg aaaaggttta tccgttcgtc catttgtatg tgcatgccaa ccacagggtt 10860

cccctcggga gtgcttggca ttccgtgcga taatgacttc tgttcaacca cccaaacgtc 10920

ggaaagcctg acgacggagc agcattccaa aaagatccct tggctcgtct gggtcggcta 10980

gaaggtcgag tgggctgctg tggcttgatc cctcaacgcg gtcgcggacg tagcgcagcg 11040

ccgaaaaatc ctcgatcgca aatccgacgc tgtcgaaaag cgtgatctgc ttgtcgctct 11100

ttcggccgac gtcctggcca gtcatcacgc gccaaagttc cgtcacagga tgatctggcg 11160

cgagttgctg gatctcgcct tcaatccggg tctgtggcgg gaactccacg aaaatatccg 11220

aacgcagcaa gatatcgcgg tgcatctcgg tcttgcctgg gcagtcgccg ccgacgccgt 11280

tgatgtggac gccgaaaagg atctaggtga agatcctttt tgataatctc atgaccaaaa 11340

tcccttaacg tgagttttcg ttccactgag cgtcagaccc cgtagaaaag atcaaaggat 11400

cttcttgaga tccttttttt ctgcgcgtaa tctgctgctt gcaaacaaaa aaaccaccgc 11460

taccagcggt ggtttgtttg ccggatcaag agctaccaac tctttttccg aaggtaactg 11520

gcttcagcag agcgcagata ccaaatactg ttcttctagt gtagccgtag ttaggccacc 11580

acttcaagaa ctctgtagca ccgcctacat acctcgctct gctaatcctg ttaccagtgg 11640

ctgctgccag tggcgataag tcgtgtctta ccgggttgga ctcaagacga tagttaccgg 11700

ataaggcgca gcggtcgggc tgaacggggg gttcgtgcac acagcccagc ttggagcgaa 11760

cgacctacac cgaactgaga tacctacagc gtgagctatg agaaagcgcc acgcttcccg 11820

aagggagaaa ggcggacagg tatccggtaa gcggcagggt cggaacagga gagcgcacga 11880

gggagcttcc agggggaaac gcctggtatc tttatagtcc tgtcgggttt cgccacctct 11940

gacttgagcg tcgatttttg tgatgctcgt caggggggcg gagcctatgg aaaaacgcca 12000

gcaacgcggc ctttttacgg ttcctggcct tttgctggcc ttttgctcac atgttctttc 12060

ctgcgttatc ccctgattct gtggataacc gattaccgcc tttgagtgag ctgataccgc 12120

tcgccgcagc cgaacgaccg agcgcagcga gtcagtgagc gaggaagcgg aagagcgcct 12180

gatgcggtat tttctcctta cgcatctgtg cggtatttca caccgcatat ggtgcactct 12240

cagtacaatc tgctctgatg ccgcatagtt aagccagtat acactccgct atcgctacgt 12300

gactgggtca tggctgcgcc ccgacacccg ccaacacccg ctgacgcgcc ctgacgggct 12360

tgtctgctcc cggcatccgc ttacagacaa gctgtgaccg tctccgggag ctgcatgtgt 12420

cagaggtttt caccgtcatc accgaaacgc gcgaggcagg ggtacgtcga ggtcgatcca 12480

acccctccgc tgctatagtg cagtcggctt ctgacgttca gtgcagccgt cttctgaaaa 12540

cgacatgtcg cacaagtcct aagttacgcg acaggctgcc gccctgccct tttcctggcg 12600

ttttcttgtc gcgtgtttta gtcgcataaa gtagaatact tgcgactaga accggagaca 12660

ttacgccatg aacaagagcg ccgccgctgg cctgctgggc tatgcccgcg tcagcaccga 12720

cgaccaggac ttgaccaacc aacgggccga actgcacgcg gccggctgca ccaagctgtt 12780

ttccgagaag atcaccggca ccaggcgcga ccgcccggag ctggccagga tgcttgacca 12840

cctacgccct ggcgacgttg tgacagtgac caggctagac cgcctggccc gcagcacccg 12900

cgacctactg gacattgccg agcgcatcca ggaggccggc gcgggcctgc gtagcctggc 12960

agagccgtgg gccgacacca ccacgccggc cggccgcatg gtgttgaccg tgttcgccgg 13020

cattgccgag ttcgagcgtt ccctaatcat cgaccgcacc cggagcgggc gcgaggccgc 13080

caaggcgcga ggcgtgaagt ttggcccccg ccctaccctc accccggcac agatcgcgca 13140

cgcccgcgag ctgatcgacc aggaaggccg caccgtgaaa gaggcggctg cactgcttgg 13200

cgtgcatcgc tcgaccctgt accgcgcact tgagcgcagc gaggaagtga cgcccaccga 13260

ggccaggcgg cgcggtgcct tccgtgagga cgcattgacc gaggccgacg ccctggcggc 13320

cgccgagaat gaacgccaag aggaacaagc atgaaaccgc accaggacgg ccaggacgaa 13380

ccgtttttca ttaccgaaga gatcgaggcg gagatgatcg cggccgggta cgtgttcgag 13440

ccgcccgcgc acgtctcaac cgtgcggctg catgaaatcc tggccggttt gtctgatgcc 13500

aagctcgcgg cctggccggc gagcttggcc gctgaagaaa ccgagcgccg ccgtctaaaa 13560

aggtgatgtg tatttgagta aaacagcttg cgtcatgcgg tcgctgcgta tatgatgcga 13620

tgagtaaata aacaaatacg caaggggaac gcatgaaggt tatcgctgta cttaaccaga 13680

aaggcgggtc aggcaagacg accatcgcaa cccatctagc ccgcgccctg caactcgccg 13740

gggccgatgt tctgttagtc gattccgatc cccagggcag tgcccgcgat tgggcggccg 13800

tgcgggaaga tcaaccgcta accgttgtcg gcatcgaccg cccgacgatt gaccgcgacg 13860

tgaaggccat cggccggcgc gacttcgtag tgatcgacgg agcgccccag gcggcggact 13920

tggctgtgtc cgcgatcaag gcagccgact tcgtgctgat tccggtgcag ccaagccctt 13980

acgacatatg ggccaccgcc gacctggtgg agctggttaa gcagcgcatt gaggtcacgg 14040

atggaaggct acaagcggcc tttgtcgtgt cgcgggcgat caaaggcacg cgcatcggcg 14100

gtgaggttgc cgaggcgctg gccgggtacg agctgcccat tcttgagtcc cgtatcacgc 14160

agcgcgtgag ctacccaggc actgccgccg ccggcacaac cgttcttgaa tcagaacccg 14220

agggcgacgc tgcccgcgag gtccaggcgc tggccgctga aattaaatca aaactcattt 14280

gagttaatga ggtaaagaga aaatgagcaa aagcacaaac acgctaagtg ccggccgtcc 14340

gagcgcacgc agcagcaagg ctgcaacgtt ggccagcctg gcagacacgc cagccatgaa 14400

gcgggtcaac tttcagttgc cggcggagga tcacaccaag ctgaagatgt acgcggtacg 14460

ccaaggcaag accattaccg agctgctatc tgaatacatc gcgcagctac cagagtaaat 14520

gagcaaatga ataaatgagt agatgaattt tagcggctaa aggaggcggc atggaaaatc 14580

aagaacaacc aggcaccgac gccgtggaat gccccatgtg tggaggaacg ggcggttggc 14640

caggcgtaag cggctgggtt gtctgccggc cctgcaatgg cactggaacc cccaagcccg 14700

aggaatcggc gtgagcggtc gcaaaccatc cggcccggta caaatcggcg cggcgctggg 14760

tgatgacctg gtggagaagt tgaaggcggc gcaggccgcc cagcggcaac gcatcgaggc 14820

agaagcacgc cccggtgaat cgtggcaagc ggccgctgat cgaatccgca aagaatcccg 14880

gcaaccgccg gcagccggtg cgccgtcgat taggaagccg cccaagggcg acgagcaacc 14940

agattttttc gttccgatgc tctatgacgt gggcacccgc gatagtcgca gcatcatgga 15000

cgtggccgtt ttccgtctgt cgaagcgtga ccgacgagct ggcgaggtga tccgctacga 15060

gcttccagac gggcacgtag aggtttccgc agggccggcc ggcatggcga gtgtgtggga 15120

ttacgacctg gtactgatgg cggtttccca tctaaccgaa tccatgaacc gataccggga 15180

agggaaggga gacaagcccg gccgcgtgtt ccgtccacac gttgcggacg tactcaagtt 15240

ctgccggcga gccgatggcg gaaagcagaa agacgacctg gtagaaacct gcattcggtt 15300

aaacaccacg cacgttgcca tgcagcgtac gaagaaggcc aagaacggcc gcctggtgac 15360

ggtatccgag ggtgaagcct tgattagccg ctacaagatc gtaaagagcg aaaccgggcg 15420

gccggagtac atcgagatcg agctagctga ttggatgtac cgcgagatca cagaaggcaa 15480

gaacccggac gtgctgacgg ttcaccccga ttactttttg atcgatcccg gcatcggccg 15540

ttttctctac cgcctggcac gccgcgccgc aggcaaggca gaagccagat ggttgttcaa 15600

gacgatctac gaacgcagtg gcagcgccgg agagttcaag aagttctgtt tcaccgtgcg 15660

caagctgatc gggtcaaatg acctgccgga gtacgatttg aaggaggagg cggggcaggc 15720

tggcccgatc ctagtcatgc gctaccgcaa cctgatcgag ggcgaagcat ccgccggttc 15780

ctaatgtacg gagcagatgc tagggcaaat tgccctagca ggggaaaaag gtcgaaaagg 15840

tctctttcct gtggatagca cgtacattgg gaacccaaag ccgtacattg ggaaccggaa 15900

cccgtacatt gggaacccaa agccgtacat tgggaaccgg tcacacatgt aagtgactga 15960

tataaaagag aaaaaaggcg atttttccgc ctaaaactct ttaaaactta ttaaaactct 16020

taaaacccgc ctggcctgtg cataactgtc tggccagcgc acagccgaag agctgcaaaa 16080

agcgcctacc cttcggtcgc tgcgctccct acgccccgcc gcttcgcgtc ggcctatcgc 16140

ggccgctggc cgctcaaaaa tggctggcct acggccaggc aatctaccag ggcgcggaca 16200

agccgcgccg tcgccactcg accgccggcg cccacatcaa ggcaccggtg ggtatgcctg 16260

acgatgcgtg gagaccgaaa ccttgcgctc gttcgccagc caggacagaa atgcctcgac 16320

ttcgctgctg cccaaggttg ccgggtgacg cacaccgtgg aaacggatga aggcacgaac 16380

ccagtggaca taagcctgtt cggttcgtaa gctgtaatgc aagtagcgta tgcgctcacg 16440

caactggtcc agaaccttga ccgaacgcag cggtggtaac ggcgcagtgg cggttttcat 16500

ggcttgttat gactgttttt ttggggtaca gtctatgcct cgggcatcca agcagcaagc 16560

gcgttacgcc gtgggtcgat gtttgatgtt atggagcagc aacgatgtta cgcagcaggg 16620

cagtcgccct aaaacaaagt taaacatcat gagggaagcg gtgatcgccg aagtatcgac 16680

tcaactatca gaggtagttg gcgtcatcga gcgccatctc gaaccgacgt tgctggccgt 16740

acatttgtac ggctccgcag tggatggcgg cctgaagcca cacagtgata ttgatttgct 16800

ggttacggtg accgtaaggc ttgatgaaac aacgcggcga gctttgatca acgacctttt 16860

ggaaacttcg gcttcccctg gagagagcga gattctccgc gctgtagaag tcaccattgt 16920

tgtgcacgac gacatcattc cgtggcgtta tccagctaag cgcgaactgc aatttggaga 16980

atggcagcgc aatgacattc ttgcaggtat cttcgagcca gccacgatcg acattgatct 17040

ggctatcttg ctgacaaaag caagagaaca tagcgttgcc ttggtaggtc cagcggcgga 17100

ggaactcttt gatccggttc ctgaacagga tctatttgag gcgctaaatg aaaccttaac 17160

gctatggaac tcgccgcccg actgggctgg cgatgagcga aatgtagtgc ttacgttgtc 17220

ccgcatttgg tacagcgcag taaccggcaa aatcgcgccg aaggatgtcg ctgccgactg 17280

ggcaatggag cgcctgccgg cccagtatca gcccgtcata cttgaagcta gacaggctta 17340

tcttggacaa gaagaagatc gcttggcctc gcgcgcagat cagttggaag aatttgtcca 17400

ctacgtgaaa ggcgagatca ccaaggtagt cggcaaataa tgtctaacaa ttcgttcaag 17460

ccgacgccgc ttcgcggcgc ggcttaactc aagcgttaga tgcactaagc acataattgc 17520

tcacagccaa actatcaggt caagtctgct tttattattt ttaagcgtgc ataataagcc 17580

ctacacaaat tgggagatat atcatgaaag gctggctttt tcttgttatc gcaatagttg 17640

gcgaagtaat cgcaacatag cttgcttggt cgttccgcgt gaacgtcggc tcgattgtac 17700

ctgcgttcaa atactttgcg atcgtgttgc gcgcctgccc ggtgcgtcgg ctgatctcac 17760

ggatcgactg cttctctcgc aacgccatcc gacggatgat gtttaaaagt cccatgtgga 17820

tcactccgtt gccccgtcgc tcaccgtgtt ggggggaagg tgcacatggc tcagttctca 17880

atggaaatta tctgcctaac cggctcagtt ctgcgtagaa accaacatgc aagctccacc 17940

gggtgcaaag cggcagcggc ggcaggatat attcaattgt aaatggcttc atgtccggga 18000

aatctacatg gatcagcaat gagtatgatg gtcaatatgg agaaaaagaa agagtaatta 18060

ccaatttttt ttcaattcaa aaatgtagat gtccgcagcg ttattataaa atgaaagtac 18120

attttgataa aacgacaaat tacgatccgt cgtatttata ggcgaaagca ataaacaaat 18180

tattctaatt cggaaatctt tatttcgacg tgtctacatt cacgtccaaa tgggggctta 18240

gatgagaaac ttcacgatcg gctctagttc gagcgatcta gtaacataga tgacaccgcg 18300

cgcgataatt tatcctagtt tgcgcgctat attttgtttt ctatcgcgta ttaaatgtat 18360

aattgcggga ctctaatcat aaaaacccat ctcataaata acgtcatgca ttacatgtta 18420

attattacat gcttaacgta attcaacaga aattatatga taatcatcgc aagaccggca 18480

acaggattca atcttaagaa actttattgc caaatgtttg aacgatcggg gaaattcgtt 18540

tctatgcttc aattctgctc tatagatgtt cttctagtct taacttttag aaggcacaac 18600

atttgaaatg ttggtaattt ttctgctggg tagtgaacca ttgtaatttg tgtttgctta 18660

tattatccgt tgggtttctc tatgttatct gtggggccat atcccagaac tggttgagtc 18720

ggtccaacac ctgggtgcca atcatgtcga tggtggggta tggt 18764

<210> 13
<211> 19389
<212> DNA
<213> Artificial Sequence

<220>
<223> pZFNnptII-BCTIV-Repl/LbCpf1_tDT-crRNA_ALS2_RT_ALS-mutated_2x100 -
binary vector for Agro-transformation coding for the replicon
(BCTIV) including GE components (SSN, crRNA and the repair
template (ALS)) and 2*100 nt homology arms; the crRNA is embedded

<400> 13
gccctacaca aattgggaga tatatcatga aaggctggct ttttcttgtt atcgcaatag 60

ttggcgaagt aatcgcaaca tagcttgctt ggtcgttccg cgtgaacgtc ggctcgattg 120

tacctgcgtt caaatacttt gcgatcgtgt tgcgcgcctg cccggtgcgt cggctgatct 180

cacggatcga ctgcttctct cgcaacgcca tccgacggat gatgtttaaa agtcccatgt 240

ggatcactcc gttgccccgt cgctcaccgt gttgggggga aggtgcacat ggctcagttc 300

tcaatggaaa ttatctgcct aaccggctca gttctgcgta gaaaccaaca tgcaagctcc 360

accgggtgca aagcggcagc ggcggcagga tatattcaat tgtaaatggc ttcatgtccg 420

ggaaatctac atggatcagc aatgagtatg atggtcaata tggagaaaaa gaaagagtaa 480

ttaccaattt tttttcaatt caaaaatgta gatgtccgca gcgttattat aaaatgaaag 540

tacattttga taaaacgaca aattacgatc cgtcgtattt ataggcgaaa gcaataaaca 600

aattattcta attcggaaat ctttatttcg acgtgtctac attcacgtcc aaatgggggc 660

ttagatgaga aacttcacga tcggctctag ttcgagcgat ctagtaacat agatgacacc 720

gcgcgcgata atttatccta gtttgcgcgc tatattttgt tttctatcgc gtattaaatg 780

tataattgcg ggactctaat cataaaaacc catctcataa ataacgtcat gcattacatg 840

ttaattatta catgcttaac gtaattcaac agaaattata tgataatcat cgcaagaccg 900

gcaacaggat tcaatcttaa gaaactttat tgccaaatgt ttgaacgatc ggggaaattc 960

gtttctatgc ttcaattctg ctctatagat gttcttctag tcttaacttt tagaaggcac 1020

aacatttgaa atgttggtaa tttttctgct gggtagtgaa ccattgtaat ttgtgtttgc 1080

ttatattatc cgttgggttt ctctatgtta tctgtggggc catatcccag aactggttga 1140

gtcggtccaa cacctgggtg ccaatcatgt cgatggtggg gtatggtccg gattgaaaga 1200

aatatagttt aaatatttat tgataaaata acaagtcagg tattatagtc caagcaaaaa 1260

cataaattta ttgatgcaag tttaaattca gaaatatttc aataactgat tatatcagct 1320

ggtacattgc cgtagatgaa agactgagtg cgatattatg tgtaatacat aaattgatga 1380

tatagctagc ttagctcatc gggtcagaag aactcgtcaa gaaggcgata gaaggcgatg 1440

cgctgcgaat cgggagcggc gataccgtaa agcacgagga agcggtcagc ccattcgccg 1500

ccaagctctt cagcaatatc acgggtagcc aacgctatgt cctgatagcg gtccgccaca 1560

cccagccggc cacagtcgat gaatccagaa aagcggccat tttccaccat gatattcggc 1620

aagcaggcat cgccatgagt cacgacgaga tcctcgccgt cgggcatgcg cgccttgagc 1680

ctggcgaaca gttcggctgg cgcgagcccc tgatgctctt cgtccagatc atcctgatcg 1740

acaagaccgg cttccatccg agtacgtgct cgctcgatgc gatgtttcgc ttggtggtcg 1800

aatgggcagg tagccggatc aagcgtatgc agccgccgca ttgcatcagc catgatggat 1860

actttctcgg caggagcaag gtgagatgac aggagatcct gccccggcac ttcgcccaat 1920

agcagccagt cccttcccgc ttcagtgaca acgtcgagca cagctgcgca aggaacgccc 1980

gtcgtggcca gccacgatag ccgcgctgcc tcgtcctgaa gttcattcag ggcaccggac 2040

aggtcggtct tgacaaaaag aaccgggcgc ccctgcgctg acagccggaa cacggcggca 2100

tcagagcagc cgattgtctg ttgtgcccag tcatagccga atagcctctc cacccaagcg 2160

gccggagaac ctgcgtgcaa tccatcttgt tcaatccaag ctcccatttt ctgtgatttt 2220

ggatgtgttt ttgtatggat tgagagtgaa tatgagactc taattggata ccgaggggaa 2280

tttatggaac gtcagtggag catttttgac aagaaatatt tgctagctga tagtgacctt 2340

aggcgacttt tgaacgcgca ataatggttt ctgacgtatg tgcttagctc attaaactcc 2400

agaaacccgc ggctgagtgg ctccttcaac gttgcggttc tgtcagttcc aaacgtaaaa 2460

cggcttgtcc cgcgtcatcg gcgggggtca ttccggactg tggggccata tcccagaact 2520

ggttgagtcg gtccaacacc tgggtgccaa tcatgtcgat ggtggggtat ggccaatttt 2580

ttttcaattc aaaaatgtag atgtccgcag cgttattata aaatgaaagt acattttgat 2640

aaaacgacaa attacgatcc gtcgtattta taggcgaaag caataaacaa attattctaa 2700

ttcggaaatc tttatttcga cgtgtctaca ttcacgtcca aatgggggct tagatgagaa 2760

acttcacgat cggctctaga ggccatgggt ttgtttttgt ttccctcttc cgtacaatga 2820

atgaaaagaa attgtatata tgagaggaag aggaacttaa gaggaatgag gaactgaaga 2880

gggaggtaga gggataaata ggtaaacgcg gataagattc cccgcgttta cctttccacg 2940

tggcgtatgc agagtgggcg gacggttgag tggggaacac gctttacttt aatttaaagt 3000

aaagtagcac taagtgggac ccacttttta tttctttaaa gacttaatta ttaagtcttc 3060

tagatgcgta tattatacgt ataaactagt ggccttggcg gccgctcgag cgatctagta 3120

acatagatga caccgcgcgc gataatttat cctagtttgc gcgctatatt ttgttttcta 3180

tcgcgtatta aatgtataat tgcgggactc taatcataaa aacccatctc ataaataacg 3240

tcatgcatta catgttaatt attacatgct taacgtaatt caacagaaat tatatgataa 3300

tcatcgcaag accggcaaca ggattcaatc ttaagaaact ttattgccaa atgtttgaac 3360

gatcggggaa attcgatcta cacttagtag aaattaatct cccagttaag ataatgctgc 3420

atctacactt agtagaaatt atcaggtacc tctagacttg tacagctcgt ccatgccgta 3480

caggaacagg tggtggcggc cctcggagcg ctcgtactgt tccacgatgg tgtagtcctc 3540

gttgtgggag gtgatgtcca gcttggtgtc cacgtagtag tagccgggca gttgcacggg 3600

cttcttggcc atgtagatgg tcttgaactc caccaggtag tggccgccgt ccttcagctt 3660

cagggcctgg tggatctcgc ccttcagcac gccgtcgcgg gggtacaggc gctcggtgga 3720

ggcctcccag cccatggtct tcttctgcat tacggggccg tcggggggga agttggtgcc 3780

gcgcatcttc accttgtaga tcagcgtgcc gtcctgcagg gaggagtcct gggtcacggt 3840

caccagaccg ccgtcctcga agttcatcac gcgctcccac ttgaagccct cggggaagga 3900

cagcttcttg taatcgggga tgtcggcggg gtgcttcacg tacgccttgg agccgtacat 3960

gaactggggg gacaggatgt cccaggcgaa gggcaggggg ccgcccttgg tcaccttcag 4020

cttggcggtc tgggtgccct cgtaggggcg gccctcgccc tcgccctcga tctcgaactc 4080

gtggccgttc atggagccct ccatgcgcac cttgaagcgc atgaactctt tgatgacctc 4140

ctcgcccttg ctcaccatgg atcccctctc caaatgaaat gaacttcctt atatagagga 4200

agggtcttgc gaaggatagt gggattgtgc gtcatccctt acgtcagtgg agatatcaca 4260

tcaatccact tgctttgaag acgtggttgg aacgtcttct ttttccacga tgttcctcgt 4320

gggtgggggt ccatctttgg gaccactgtc ggtagaggca tcttgaacga tagcctttcc 4380

tttatcgcaa tgatggcatt tgtagaagcc atcttccttt tctactgtcc tttcgatgaa 4440

gtgacagata gctgggcaat ggaatccgag gaggtttccc gatattaccc tttgttgaaa 4500

agtctcaata gccctctggt cttctgagac tgtatctttg atattcttgg agtagacgag 4560

agtgtcgtgc tccaccatgt atcacatcaa tccacttgct ttgaagacgt ggttggaacg 4620

tcttcttttt ccacgatgtt cctcgtgggt gggggtccat ctttgggacc actgtcggta 4680

gaggcatctt gaacgatagc ctttccttta tcgcaatgat ggcatttgta gaagccatct 4740

tccttttcta ctgtcctttc gatgaagtga cagatagctg ggcaatggaa tccgaggagg 4800

tttcccgata ttaccctttg ttgaaaagtc tcaatagccc tctggtcttc tgaacactag 4860

taaggcctta agggccaccc gggctgcagg aattcgatct ggcacgacag gtttcccgac 4920

tggaaagcgg gcagtgagcg caacgcaatt aatgtgagtt agctcactca ttaggcaccc 4980

caggctttac actttatgct tccggctcgt atgttgtgtg gaattgtgag cggataacaa 5040

tttcacacag gaaacagcta tgacatgatt acgaattcaa aaattacgga tatgaatata 5100

ggcatatccg tatccgaatt atccgtttga cagctagcaa cgattgtaca attgcttctt 5160

taaaaaagga agaaagaaag aaagaaaaga atcaacatca gcgttaacaa acggccccgt 5220

tacggcccaa acggtcatat agagtaacgg cgttaagcgt tgaaagactc ctatcgaaat 5280

acgtaaccgc aaacgtgtca tagtcagatc ccctcttcct tcaccgcctc aaacacaaaa 5340

ataatcttct acagcctata tatacaaccc ccccttctat ctctcctttc tcacaattca 5400

tcatctttct ttctctaccc ccaattttaa gaaatcctct cttctcctct tcattttcaa 5460

ggtaaatctc tctctctctc tctctctctg ttattccttg ttttaattag gtatgtatta 5520

ttgctagttt gttaatctgc ttatcttatg tatgccttat gtgaatatct ttatcttgtt 5580

catctcatcc gtttagaagc tataaatttg ttgatttgac tgtgtatcta cacgtggtta 5640

tgtttatatc taatcagata tgaatttctt catattgttg cgtttgtgtg taccaatccg 5700

aaatcgttga tttttttcat ttaatcgtgt agctaattgt acgtatacat atggatctac 5760

gtatcaattg ttcatctgtt tgtgtttgta tgtatacaga tctgaaaaca tcacttctct 5820

catctgattg tgttgttaca tacatagata tagatctgtt atatcatttt ttttattaat 5880

tgtgtatata tatatgtgca tagatctgga ttacatgatt gtgattattt acatgatttt 5940
gttatttacg tatgtatata tgtagatctg gactttttgg agttgttgac ttgattgtat 6000
ttgtgtgtgt atatgtgtgt tctgatcttg atatgttatg tatgtgcagc gaattcggcg 6060
cgccaaacaa tggcttcctc catggctcct aagaagaaga ggaaggttag caagctcgag 6120
aagtttacca actgctacag cctctctaag accctcaggt tcaaggctat ccctgtggga 6180
aagacccaag agaatatcga caacaagagg ctcctcgtcg aggatgagaa gagagctgaa 6240
gattacaagg gcgtgaagaa gctcctcgac aggtactacc tcagcttcat caacgatgtg 6300
ctccacagca tcaagctcaa gaacctcaac aactacatca gcctcttccg taagaaaacc 6360
aggaccgaga aagagaacaa agagcttgag aacctcgaga tcaacctccg taaagagatc 6420
gccaaggctt tcaagggaaa cgagggatac aagagcctct tcaagaagga tattatcgag 6480
acaatcctgc ctgagttcct ggacgataag gatgagatcg ctctcgtgaa cagcttcaac 6540
ggattcacta ctgccttcac cggattcttc gacaacaggg aaaacatgtt cagcgaagag 6600
gccaagagca cctctatcgc tttcagatgc atcaacgaga acctcacgcg ttacatcagc 6660
aacatggaca tcttcgagaa ggtggacgcc atcttcgata agcacgaggt gcaagaaatc 6720
aaagagaaga tcctcaacag cgactacgac gtcgaggact tttttgaagg ggagttcttc 6780
aacttcgttc tcacccaaga gggcatcgac gtgtacaacg ctattatcgg aggattcgtg 6840
accgagtctg gggagaagat taagggactc aacgagtaca tcaacctgta caaccagaaa 6900
acgaagcaga agctcccgaa gttcaagccg ctctacaagc aggttctctc tgatcgtgag 6960
agcctctcat tttacggtga gggttacacc tctgacgagg aagtgcttga ggttttccgt 7020
aacaccctca acaagaacag cgagatcttc tcgtccatca agaagttgga gaaacttttc 7080
aagaacttcg acgagtacag cagcgctggg atcttcgtta agaacggacc tgctatcagc 7140
accatcagca aggatatttt cggcgagtgg aacgtgatca gggacaagtg gaatgctgag 7200
tacgatgaca tccacctcaa gaagaaggct gtcgtcactg agaagtacga ggatgacagg 7260
cgtaagtcgt tcaagaagat cggctctttc agcctcgagc agcttcaaga atacgctgat 7320
gctgatctca gcgtggtcga gaagctcaaa gagatcatca tccagaaggt cgacgagatc 7380
tacaaggtgt acgggtcctc tgagaagttg ttcgatgctg atttcgtcct cgagaagagt 7440
ctgaagaaga acgacgctgt cgtcgcgatc atgaaggatt tgctcgacag cgtgaagtcc 7500
ttcgagaact atatcaaggc cttcttcgga gagggcaaag agactaatag ggacgagtct 7560
ttctacgggg atttcgtgct cgcttacgat atcctcctca aggtggacca tatctacgac 7620
gccatcagaa actacgtgac ccagaagcct tacagcaagg acaagttcaa gttgtacttt 7680
cagaacccgc agttcatggg cggatgggac aaagacaaag agacagatta cagggccacc 7740
atcctcaggt acgggtctaa gtactacctg gccatcatgg acaagaaata cgccaagtgc 7800

ctccaaaaga tcgacaagga tgacgtgaac gggaactatg agaagatcaa ctacaagctc 7860

cttccgggac cgaacaagat gcttcctaag gtgttcttca gcaagaaatg gatggcctac 7920

tacaacccgt ctgaggacat ccagaaaatc tacaagaacg ggaccttcaa gaaaggcgac 7980

atgttcaacc tcaacgactg ccacaagctc atcgatttct tcaaggacag catctcgcgt 8040

tacccgaagt ggtctaacgc ttacgacttt aacttcagcg agacagaaaa gtacaaggat 8100

atcgccgggt tctaccgtga ggttgaggaa cagggttaca aggttagctt cgagagcgcc 8160

tccaagaaag aggttgacaa gttggtcgaa gagggcaagc tctacatgtt ccagatctat 8220

aacaaggact tctccgacaa gagccacgga actcctaacc tccatacgat gtacttcaag 8280

ctgcttttcg acgagaacaa ccacgggcag atcagacttt ctggtggtgc tgaactcttc 8340

atgcgtaggg cctcactcaa gaaagaagag ttggttgttc acccggccaa ctctccaatc 8400

gctaacaaga atcctgacaa cccgaaaaag accaccacgc tgtcttacga cgtctacaag 8460

gacaaaaggt tcagcgagga ccagtacgag cttcatatcc cgatcgctat caacaagtgc 8520

ccgaagaaca tcttcaagat caataccgag gtgagggtgc tgctcaagca cgatgataac 8580

ccttacgtga tcggaatcga tcgtggtgag agaaacctcc tctacatcgt tgtggtggac 8640

ggaaagggaa acatcgtcga gcagtacagc ctgaacgaga ttatcaacaa tttcaacggc 8700

atcaggatca agaccgacta ccactcactc ctcgataaga aagaaaaaga gcgtttcgag 8760

gccaggcaga actggacttc tatcgaaaac atcaaagagt tgaaggccgg ctacatctct 8820

caggtggtgc ataagatctg cgagctggtg gaaaagtacg atgctgtgat cgctcttgag 8880

gacctcaact ctgggttcaa gaacagtaga gtgaaggttg agaagcaggt ctaccaaaag 8940

ttcgagaaga tgctcatcga caagctcaac tacatggtgg acaaaaagag caacccttgc 9000

gctaccggtg gtgctcttaa gggataccag atcacgaaca agttcgagtc cttcaagagc 9060

atgagcaccc agaacggctt catcttctat atccctgctt ggctcaccag caagatcgat 9120

ccttctactg gtttcgtgaa cctgctcaag accaagtaca cctcgatcgc cgacagcaag 9180

aagttcatct cgtctttcga caggatcatg tacgtgccgg aagaggatct tttcgagttc 9240

gctctcgact ataagaactt cagcaggacc gacgccgact acattaagaa gtggaagctc 9300

tactcctacg ggaaccgtat caggatcttc cgaaatccga agaaaaacaa cgtgttcgac 9360

tgggaagaag tgtgcctcac ctctgcctac aaagaactgt tcaacaagta cggcatcaac 9420

taccagcagg gtgatatcag ggctcttttg tgcgagcaga gcgacaaggc attctacagc 9480

tcattcatgg ccctcatgtc tctcatgctc cagatgagga actctatcac cggaaggacc 9540

gatgtggact tccttatctc tccggtcaag aactctgacg ggatcttcta cgacagccgt 9600

aactatgagg ctcaagagaa cgctatcctg ccgaagaatg ctgatgcaaa cggggcttac 9660

aacattgcga gaaaggttct ctgggctatc gggcagttta agaaagcgga agatgagaag 9720

ctcgacaagg tgaagatcgc catctccaac aaagagtggc ttgagtacgc tcagacctcc 9780

gttaagcaca agaggcctgc tgctactaag aaagctggcc aggccaaaaa gaagaagtga 9840

ggcgcgccga gctccaggcc tcccagcttt cgtccgtatc atcggtttcg acaacgttcg 9900

tcaagttcaa tgcatcagtt tcattgccca cacaccagaa tcctactaag tttgagtatt 9960

atggcattgg aaaagctgtt ttcttctatc atttgttctg cttgtaattt actgtgttct 10020

ttcagttttt gttttcggac atcaaaatgc aaatggatgg ataagagtta ataaatgata 10080

tggtcctttt gttcattctc aaattattat tatctgttgt ttttacttta atgggttgaa 10140

tttaagtaag aaaggaacta acagtgtgat attaaggtgc aatgttagac atataaaaca 10200

gtctttcacc tctctttggt tatgtcttga attggtttgt ttcttcactt atctgtgtaa 10260

tcaagtttac tatgagtcta tgatcaagta attatgcaat caagttaagt acagtatagg 10320

cttgagctcc ctaggatcaa gctttatggg gtttgggcta ccagccgcca ttggagctgc 10380

agttgctcga ccagatgcag tggttgtcga tattgatggg gatggcagtt ttattatgaa 10440

tgttcaggaa cttgcaacta taagagttga gaaccttcct gtgaaaatta tgcttctcaa 10500

taaccagcac ttgggaatgg tggttcagtt ggaggacaga ttttacaagg caaatcgcgc 10560

tcacacttat ttgggtaatc catctaagtc tgctgatatc ttccctgata tgctcaaatt 10620

cgctgaggca tgtgatattc cttctgcccg tgttagcaac gtggctgatt tgagggccgc 10680

cattcaaaaa gcttatcgat accgtcgacc tcgagggggg gcccggtacc actagtattg 10740

tagggcttat ttctattgat tattagaaat gcccaatgat gttgaagttt aataaaaaat 10800

ttattattaa atttgtttgt gtaagattga aatgtagata tgtaaatatc tagtataatt 10860

gccttggaaa acaaaaaacg aaaaaatgat tgccggaaaa cgaaaaaaaa acccctcaga 10920

aattagaccc ccggaaaagc aatattttta tattaatctg cagcccatcc acgaagtgga 10980

tgttacatat ttcgaagaaa taacttaact ataccaaact tcatcacttg ctaagtaata 11040

tacatcgcaa ttagcgtaaa accaatcttt catagattca ctcatacaag gtatccagtc 11100

catatctgga ttagtaatag caatacaagg tcttccgcct ggaatgacta agtttggttt 11160

atgttttatc caaacactat aatcttcgtt acatccgata atagatttaa acaactcctg 11220

ttgtattttt tgatagcgga tatcgtctat tatatggtat gtagtgtggt ctatatcgta 11280

tagagaccaa tctaatacca tgccagacat atagtggtgt cggcctagag atcttgccca 11340

ctgggtcttt cctgttctgg atggtccgca tatgtagaga cttcttggtc ggactggtct 11400

gttgtctggc tcctggacaa attaagtacg ttagtgtctt cactacatgc tagatcgcga 11460

ctaagtcgat ctaggtggaa gtattcttca agagtcattt gtctgtcttg aagaattgct 11520

cccctgcaat tctcacatat ttcgtaatgt aaacaatctg aacttacgaa atatatattt 11580

tgttccgccc actctttgat agtgtcaggt agcctagaga actccgtcca tctgggtgtg 11640

taaggttgca caactggcct gaaatgatag ttggcgaagg atagaatgga aggccatctg 11700

agaacgaaat ctgttgggca ggactctcgt atgttactga agaactcctc cttggtgttg 11760

gatgaatcaa gaattcgtct ccattccacg ttgctgttgc caatactgtt tggagagcgc 11820

cctcccagtc gaagctctcc aaatgttcta gggctcgtgt cctccttggt gatatactcg 11880

aatgctcgct ttggggcgtt gagtccgtca attcttgggt gaatcgttcg accgtctggg 11940

agtatgatgt tgaaaaacag gttcccattg acgtcgagtt tcttgtcaca ctggataata 12000

cagtgtgtgt ggaatcctcc ttcaggttgt tcttcagtag gctgatgatg ttctagtgat 12060

actgcaagga atagaatcac atatgattct aataaggttt tcagtttctc gaatatataa 12120

tctttaaaat ccccccctat ttgggaatat gttagaaaag catttttttt ttgaaaacgt 12180

aaataaccgg gttggttagg ggtaggtctg tttctgtttg gagcacgtgg catgtttgtt 12240

tttgtttccc tcttccgtac aatgaatgaa aagaaattgt atatatgaga ggaagaggaa 12300

cttaagagga atgaggaact gaagagggag gtagagggat aaataggtaa acgcggataa 12360

gattccccgc gtttaccttt ccacgtggcg tatgcagagt gggcggacgg ttgagtgggg 12420

aacacgcttt actttaattt aaagtaaagt agcactaagt gggacccact ttttatttct 12480

ttaaagactt aattattaag tcttctagat gcgtatatta tacgtataag tttaaactat 12540

cagtgtttga caggatatat tggcgggtaa acctaagaga aaagagcgtt tattagaata 12600

atcggatatt taaaagggcg tgaaaaggtt tatccgttcg tccatttgta tgtgcatgcc 12660

aaccacaggg ttcccctcgg gagtgcttgg cattccgtgc gataatgact tctgttcaac 12720

cacccaaacg tcggaaagcc tgacgacgga gcagcattcc aaaaagatcc cttggctcgt 12780

ctgggtcggc tagaaggtcg agtgggctgc tgtggcttga tccctcaacg cggtcgcgga 12840

cgtagcgcag cgccgaaaaa tcctcgatcg caaatccgac gctgtcgaaa agcgtgatct 12900

gcttgtcgct ctttcggccg acgtcctggc cagtcatcac gcgccaaagt tccgtcacag 12960

gatgatctgg cgcgagttgc tggatctcgc cttcaatccg ggtctgtggc gggaactcca 13020

cgaaaatatc cgaacgcagc aagatatcgc ggtgcatctc ggtcttgcct gggcagtcgc 13080

cgccgacgcc gttgatgtgg acgccgaaaa ggatctaggt gaagatcctt tttgataatc 13140

tcatgaccaa aatcccttaa cgtgagtttt cgttccactg agcgtcagac cccgtagaaa 13200

agatcaaagg atcttcttga gatccttttt ttctgcgcgt aatctgctgc ttgcaaacaa 13260

aaaaaccacc gctaccagcg gtggtttgtt tgccggatca agagctacca actctttttc 13320

cgaaggtaac tggcttcagc agagcgcaga taccaaatac tgttcttcta gtgtagccgt 13380

agttaggcca ccacttcaag aactctgtag caccgcctac atacctcgct ctgctaatcc 13440

tgttaccagt ggctgctgcc agtggcgata agtcgtgtct taccgggttg gactcaagac 13500

gatagttacc ggataaggcg cagcggtcgg gctgaacggg gggttcgtgc acacagccca 13560

gcttggagcg aacgacctac accgaactga gatacctaca gcgtgagcta tgagaaagcg 13620

ccacgcttcc cgaagggaga aaggcggaca ggtatccggt aagcggcagg gtcggaacag 13680

gagagcgcac gagggagctt ccagggggaa acgcctggta tctttatagt cctgtcgggt 13740

ttcgccacct ctgacttgag cgtcgatttt tgtgatgctc gtcagggggg cggagcctat 13800

ggaaaaacgc cagcaacgcg gcctttttac ggttcctggc cttttgctgg ccttttgctc 13860

acatgttctt tcctgcgtta tcccctgatt ctgtggataa ccgtattacc gcctttgagtg 13920

agctgatacc gctcgccgca gccgaacgac cgagcgcagc gagtcagtga gcgaggaagc 13980

ggaagagcgc ctgatgcggt attttctcct tacgcatctg tgcggtattt cacaccgcat 14040

atggtgcact ctcagtacaa tctgctctga tgccgcatag ttaagccagt atacactccg 14100

ctatcgctac gtgactgggt catggctgcg ccccgacacc cgccaacacc cgctgacgcg 14160

ccctgacggg cttgtctgct cccggcatcc gcttacagac aagctgtgac cgtctccggg 14220

agctgcatgt gtcagaggtt ttcaccgtca tcaccgaaac gcgcgaggca ggggtacgtc 14280

gaggtcgatc caacccctcc gctgctatag tgcagtcggc ttctgacgtt cagtgcagcc 14340

gtcttctgaa aacgacatgt cgcacaagtc ctaagttacg cgacaggctg ccgccctgcc 14400

cttttcctgg cgttttcttg tcgcgtgttt tagtcgcata aagtagaata cttgcgacta 14460

gaaccggaga cattacgcca tgaacaagag cgccgccgct ggcctgctgg gctatgcccg 14520

cgtcagcacc gacgaccagg acttgaccaa ccaacgggcc gaactgcacg cggccggctg 14580

caccaagctg ttttccgaga agatcaccgg caccaggcgc gaccgcccgg agctggccag 14640

gatgcttgac cacctacgcc ctggcgacgt tgtgacagtg accaggctag accgcctggc 14700

ccgcagcacc cgcgacctac tggacattgc cgagcgcatc caggaggccg gcgcgggcct 14760

gcgtagcctg gcagagccgt gggccgacac caccacgccg gccggccgca tggtgttgac 14820

cgtgttcgcc ggcattgccg agttcgagcg ttccctaatc atcgaccgca cccggagcgg 14880

gcgcgaggcc gccaaggcgc gaggcgtgaa gtttggcccc cgccctaccc tcaccccggc 14940

acagatcgcg cacgcccgcg agctgatcga ccaggaaggc cgcaccgtga aagaggcggc 15000

tgcactgctt ggcgtgcatc gctcgaccct gtaccgcgca cttgagcgca gcgaggaagt 15060

gacgcccacc gaggccaggc ggcgcggtgc cttccgtgag gacgcattga ccgaggccga 15120

cgccctggcg gccgccgaga atgaacgcca agaggaacaa gcatgaaacc gcaccaggac 15180

ggccaggacg aaccgttttt cattaccgaa gagatcgagg cggagatgat cgcggccggg 15240

tacgtgttcg agccgcccgc gcacgtctca accgtgcggc tgcatgaaat cctggccggt 15300

ttgtctgatg ccaagctcgc ggcctggccg gcgagcttgg ccgctgaaga aaccgagcgc 15360

cgccgtctaa aaaggtgatg tgtatttgag taaaacagct tgcgtcatgc ggtcgctgcg 15420

tatatgatgc gatgagtaaa taaacaaata cgcaagggga acgcatgaag gttatcgctg 15480

tacttaacca gaaaggcggg tcaggcaaga cgaccatcgc aacccatcta gcccgcgccc 15540

tgcaactcgc cggggccgat gttctgttag tcgattccga tccccagggc agtgcccgcg 15600

attgggcggc cgtgcgggaa gatcaaccgc taaccgttgt cggcatcgac cgcccgacga 15660

ttgaccgcga cgtgaaggcc atcggccggc gcgacttcgt agtgatcgac ggagcgcccc 15720

aggcggcgga cttggctgtg tccgcgatca aggcagccga cttcgtgctg attccggtgc 15780

agccaagccc ttacgacata tgggccaccg ccgacctggt ggagctggtt aagcagcgca 15840

ttgaggtcac ggatggaagg ctacaagcgg cctttgtcgt gtcgcgggcg atcaaaggca 15900

cgcgcatcgg cggtgaggtt gccgaggcgc tggccgggta cgagctgccc attcttgagt 15960

cccgtatcac gcagcgcgtg agctacccag gcactgccgc cgccggcaca accgttcttg 16020

aatcagaacc cgagggcgac gctgcccgcg aggtccaggc gctggccgct gaaattaaat 16080

caaaactcat ttgagttaat gaggtaaaga gaaaatgagc aaaagcacaa acacgctaag 16140

tgccggccgt ccgagcgcac gcagcagcaa ggctgcaacg ttggccagcc tggcagacac 16200

gccagccatg aagcgggtca actttcagtt gccggcggag gatcacacca agctgaagat 16260

gtacgcggta cgccaaggca agaccattac cgagctgcta tctgaataca tcgcgcagct 16320

accagagtaa atgagcaaat gaataaatga gtagatgaat tttagcggct aaaggaggcg 16380

gcatggaaaa tcaagaacaa ccaggcaccg acgccgtgga atgccccatg tgtggaggaa 16440

cgggcggttg gccaggcgta agcggctggg ttgtctgccg gccctgcaat ggcactggaa 16500

cccccaagcc cgaggaatcg gcgtgagcgg tcgcaaacca tccggcccgg tacaaatcgg 16560

cgcggcgctg ggtgatgacc tggtggagaa gttgaaggcg gcgcaggccg cccagcggca 16620

acgcatcgag gcagaagcac gccccggtga atcgtggcaa gcggccgctg atcgaatccg 16680

caaagaatcc cggcaaccgc cggcagccgg tgcgccgtcg attaggaagc cgcccaaggg 16740

cgacgagcaa ccagattttt tcgttccgat gctctatgac gtgggcaccc gcgatagtcg 16800

cagcatcatg gacgtggccg ttttccgtct gtcgaagcgt gaccgacgag ctggcgaggt 16860

gatccgctac gagcttccag acgggcacgt agaggtttcc gcagggccgg ccggcatggc 16920

gagtgtgtgg gattacgacc tggtactgat ggcggtttcc catctaaccg aatccatgaa 16980

ccgataccgg gaagggaagg gagacaagcc cggccgcgtg ttccgtccac acgttgcgga 17040

cgtactcaag ttctgccggc gagccgatgg cggaaagcag aaagacgacc tggtagaaac 17100

ctgcattcgg ttaaacacca cgcacgttgc catgcagcgt acgaagaagg ccaagaacgg 17160

ccgcctggtg acggtatccg agggtgaagc cttgattagc cgctacaaga tcgtaaagag 17220

cgaaaccggg cggccggagt acatcgagat cgagctagct gattggatgt accgcgagat 17280

cacagaaggc aagaacccgg acgtgctgac ggttcacccc gattactttt tgatcgatcc 17340

cggcatcggc cgttttctct accgcctggc acgccgcgcc gcaggcaagg cagaagccag 17400

atggttgttc aagacgatct acgaacgcag tggcagcgcc ggagagttca agaagttctg 17460

tttcaccgtg cgcaagctga tcgggtcaaa tgacctgccg gagtacgatt tgaaggagga 17520

ggcggggcag gctggcccga tcctagtcat gcgctaccgc aacctgatcg agggcgaagc 17580

atccgccggt tcctaatgta cggagcagat gctagggcaa attgccctag caggggaaaa 17640

aggtcgaaaa ggtctctttc ctgtggatag cacgtacatt gggaacccaa agccgtacat 17700

tgggaaccgg aacccgtaca ttgggaaccc aaagccgtac attgggaacc ggtcacacat 17760

gtaagtgact gatataaaag agaaaaaagg cgatttttcc gcctaaaact ctttaaaact 17820

tattaaaact cttaaaaccc gcctggcctg tgcataactg tctggccagc gcacagccga 17880

agagctgcaa aaagcgccta cccttcggtc gctgcgctcc ctacgccccg ccgcttcgcg 17940

tcggcctatc gcggccgctg gccgctcaaa aatggctggc ctacggccag gcaatctacc 18000

agggcgcgga caagccgcgc cgtcgccact cgaccgccgg cgcccacatc aaggcaccgg 18060

tgggtatgcc tgacgatgcg tggagaccga aaccttgcgc tcgttcgcca gccaggacag 18120

aaatgcctcg acttcgctgc tgcccaaggt tgccgggtga cgcacaccgt ggaaacggat 18180

gaaggcacga acccagtgga cataagcctg ttcggttcgt aagctgtaat gcaagtagcg 18240

tatgcgctca cgcaactggt ccagaacctt gaccgaacgc agcggtggta acggcgcagt 18300

ggcggttttc atggcttgtt atgactgttt ttttggggta cagtctatgc ctcgggcatc 18360

caagcagcaa gcgcgttacg ccgtgggtcg atgtttgatg ttatggagca gcaacgatgt 18420

tacgcagcag ggcagtcgcc ctaaaacaaa gttaaacatc atgagggaag cggtgatcgc 18480

cgaagtatcg actcaactat cagaggtagt tggcgtcatc gagcgccatc tcgaaccgac 18540

gttgctggcc gtacatttgt acggctccgc agtggatggc ggcctgaagc cacacagtga 18600

tattgatttg ctggttacgg tgaccgtaag gcttgatgaa acaacgcggc gagctttgat 18660

caacgacctt ttggaaactt cggcttcccc tggagagagc gagattctcc gcgctgtaga 18720

agtcaccatt gttgtgcacg acgacatcat tccgtggcgt tatccagcta agcgcgaact 18780

gcaatttgga gaatggcagc gcaatgacat tcttgcaggt atcttcgagc cagccacgat 18840

cgacattgat ctggctatct tgctgacaaa agcaagagaa catagcgttg ccttggtagg 18900

tccagcggcg gaggaactct ttgatccggt tcctgaacag gatctatttg aggcgctaaa 18960

tgaaacctta acgctatgga actcgccgcc cgactgggct ggcgatgagc gaaatgtagt 19020

gcttacgttg tcccgcattt ggtacagcgc agtaaccggc aaaatcgcgc cgaaggatgt 19080

cgctgccgac tgggcaatgg agcgcctgcc ggcccagtat cagcccgtca tacttgaagc 19140

tagacaggct tatcttggac aagaagaaga tcgcttggcc tcgcgcgcag atcagttgga 19200

agaatttgtc cactacgtga aaggcgagat caccaaggta gtcggcaaat aatgtctaac 19260

aattcgttca agccgacgcc gcttcgcggc gcggcttaac tcaagcgtta gatgcactaa 19320

gcacataatt gctcacagcc aaactatcag gtcaagtctg cttttattat ttttaagcgt 19380

gcataataa 19389

<210> 14
<211> 18930
<212> DNA
<213> Artificial Sequence

<220>
<223> pZFNnptII-BSCTV-Repl/LbCpf1_RibocrRNA_ALS4_RT_ALS-mutated_2x100 -
      binary vector for Agro-transformation coding for the replicon
      (BSCTV) including GE components (SSN, crRNA and the repair
      template (ALS)) and 2*100 nt homology arms; crRNA is produced

<400> 14
   ccggattgaa agaaatatag tttaaatatt tattgataaa ataacaagtc aggtattata 60
   gtccaagcaa aaacataaat ttattgatgc aagtttaaat tcagaaatat ttcaataact 120
   gattatatca gctggtacat tgccgtagat gaaagactga gtgcgatatt atgtgtaata 180
   cataaattga tgatatagct agcttagctc atcgggtcag aagaactcgt caagaaggcg 240
   atagaaggcg atgcgctgcg aatcgggagc ggcgataccg taaagcacga ggaagcggtc 300
   agcccattcg ccgccaagct cttcagcaat atcacgggta gccaacgcta tgtcctgata 360
   gcggtccgcc acacccagcc ggccacagtc gatgaatcca gaaaagcggc cattttccac 420
   catgatattc ggcaagcagg catcgccatg agtcacgacg agatcctcgc cgtcgggcat 480
   gcgcgccttg agcctggcga acagttcggc tggcgcgagc ccctgatgct cttcgtccag 540
   atcatcctga tcgacaagac cggcttccat ccgagtacgt gctcgctcga tgcgatgttt 600
   cgcttggtgg tcgaatgggc aggtagccgg atcaagcgta tgcagccgcc gcattgcatc 660
   agccatgatg gatactttct cggcaggagc aaggtgagat gacaggagat cctgccccgg 720
   cacttcgccc aatagcagcc agtcccttcc cgcttcagtg acaacgtcga gcacagctgc 780
   gcaaggaacg cccgtcgtgg ccagccacga tagccgcgct gcctcgtcct gaagttcatt 840
   cagggcaccg gacaggtcgg tcttgacaaa aagaaccggg cgcccctgcg ctgacagccg 900
   gaacacggcg gcatcagagc agccgattgt ctgttgtgcc cagtcatagc cgaatagcct 960
   ctccacccaa gcggccggag aacctgcgtg caatccatct tgttcaatcc aagctcccat 1020
   tttctgtgat tttggatgtg tttttgtatg gattgagagt gaatatgaga ctctaattgg 1080
   ataccgaggg gaatttatgg aacgtcagtg gagcattttt gacaagaaat atttgctagc 1140
   tgatagtgac cttaggcgac ttttgaacgc gcaataatgg tttctgacgt atgtgcttag 1200
   ctcattaaac tccagaaacc cgcggctgag tggctccttc aacgttgcgg ttctgtcagt 1260
   tccaaacgta aaacggcttg tcccgcgtca tcggcggggg tcattccgga ctgtggggcc 1320
   atatcccaga actggttgag tcggtccaac acctgggtgc caatcatgtc gatggtgggg 1380
   tatggccaat tttttttcaa ttcaaaaatg tagatgtccg cagcgttatt ataaaatgaa 1440
   agtacatttt gataaaacga caaattacga tccgtcgtat ttataggcga aagcaataaa 1500
   caaattattc taattcggaa atctttattt cgacgtgtct acattcacgt ccaaatgggg 1560
   gcttagatga gaaacttcac gatcggctct agaggccatg ggtcgacatt gaatcgggct 1620
   ctcttcaaat cccctatcaa ttgggtgctt tgggtgctct taaataccac caaggggcca 1680
   tccgcattaa tattaccgga tggcccccaa aaaatacgtg gcccaatgaa aatatgccac 1740
   gtggaaagct aaagatgttg atgtgatgga tttgggcggg aaacttcctg aagaagtttt 1800
   cattcccgcc cctttttttg acttagtcaa aaaagtagcc gacatactgt ttgaaataaa 1860
   gttaaaagta gatcattgtg gtccccacaa gaaacttgct aagcaagttt cgaattattt 1920
   ctgtttaaat acgttatttt tacatgtata tgtacttata aactagtggc cttggcggcc 1980
   gctcgagcga tctagtaaca tagatgacac cgcgcgcgat aatttatcct agtttgcgcg 2040
   ctatattttg ttttctatcg cgtattaaat gtataattgc gggactctaa tcataaaaac 2100
   ccatctcata aataacgtca tgcattacat gttaattatt acatgcttaa cgtaattcaa 2160
   cagaaattat atgataatca tcgcaagacc ggcaacagga ttcaatctta agaaacttta 2220
   ttgccaaatg tttgaacgat cgcctctctg cttggctcct ctttcttgtg aaagagggtt 2280
   cccaggcgac accacgagga ggtgcagagt aggactgacg ccctaaccgg gcacatacat 2340
   accttgatct acacttagta gaaattagac gagcttactc gtttcgtcct cacggactca 2400
   tcagtaattt ggatcccctc tccaaatgaa atgaacttcc ttatatagag gaagggtctt 2460
   gcgaaggata gtgggattgt gcgtcatccc ttacgtcagt ggagatatca catcaatcca 2520
   cttgctttga agacgtggtt ggaacgtctt ctttttccac gatgttcctc gtgggtgggg 2580
   gtccatcttt gggaccactg tcggtagagg catcttgaac gatagccttt cctttatcgc 2640
   aatgatggca tttgtagaag ccatcttcct tttctactgt cctttcgatg aagtgacaga 2700
   tagctgggca atggaatccg aggaggtttc ccgatattac cctttgttga aaagtctcaa 2760
   tagccctctg gtcttctgag actgtatctt tgatattctt ggagtagacg agagtgtcgt 2820
   gctccaccat gtatcacatc aatccacttg ctttgaagac gtggttggaa cgtcttcttt 2880
   ttccacgatg ttcctcgtgg gtgggggtcc atctttggga ccactgtcgg tagaggcatc 2940
   ttgaacgata gcctttcctt tatcgcaatg atggcatttg tagaagccat cttccttttc 3000
   tactgtcctt tcgatgaagt gacagatagc tgggcaatgg aatccgagga ggtttcccga 3060
   tattaccctt tgttgaaaag tctcaatagc cctctggtct tctgaacact agtaaggcct 3120
   taagggccac ccgggctgca ggaattcgat ctggcacgac aggtttcccg actggaaagc 3180
   gggcagtgag cgcaacgcaa ttaatgtgag ttagctcact cattaggcac cccaggcttt 3240
   acactttatg cttccggctc gtatgttgtg tggaattgtg agcggataac aatttcacac 3300
   aggaaacagc tatgacatga ttacgaattc aaaaattacg gatatgaata taggcatatc 3360
   cgtatccgaa ttatccgttt gacagctagc aacgattgta caattgcttc tttaaaaaag 3420
   gaagaaagaa agaaagaaaa gaatcaacat cagcgttaac aaacggcccc gttacggccc 3480
   aaacggtcat atagagtaac ggcgttaagc gttgaaagac tcctatcgaa atacgtaacc 3540
   gcaaacgtgt catagtcaga tcccctcttc cttcaccgcc tcaaacacaa aaataatctt 3600
   ctacagccta tatatacaac ccccccttct atctctcctt tctcacaatt catcatcttt 3660
   ctttctctac ccccaatttt aagaaatcct ctcttctcct cttcattttc aaggtaaatc 3720
   tctctctctc tctctctctc tgttattcct tgttttaatt aggtatgtat tattgctagt 3780
   ttgttaatct gcttatctta tgtatgcctt atgtgaatat ctttatcttg ttcatctcat 3840
   ccgtttagaa gctataaatt tgttgatttg actgtgtatc tacacgtggt tatgtttata 3900
   tctaatcaga tatgaatttc ttcatattgt tgcgtttgtg tgtaccaatc cgaaatcgtt 3960
   gatttttttc atttaatcgt gtagctaatt gtacgtatac atatggatct acgtatcaat 4020
   tgttcatctg tttgtgtttg tatgtataca gatctgaaaa catcacttct ctcatctgat 4080
   tgtgttgtta catacataga tatagatctg ttatatcatt tttttattta attgtgtata 4140
   tatatatgtg catagatctg gattacatga ttgtgattat ttacatgatt ttgttattta 4200
   cgtatgtata tatgtagatc tggacttttt ggagttgttg acttgattgt atttgtgtgt 4260
   gtatatgtgt gttctgatct tgatatgtta tgtatgtgca gcgaattcgg cgcgccaaac 4320
   aatggcttcc tccatggctc ctaagaagaa gaggaaggtt agcaagctcg agaagtttac 4380
   caactgctac agcctctcta agaccctcag gttcaaggct atccctgtgg gaaagaccca 4440
   agagaatatc gacaacaaga ggctcctcgt cgaggatgag aagagagctg aagattacaa 4500
   gggcgtgaag aagctcctcg acaggtacta cctcagcttc atcaacgatg tgctccacag 4560
   catcaagctc aagaacctca acaactacat cagcctcttc cgtaagaaaa ccaggaccga 4620
   gaaagagaac aaagagcttg agaacctcga gatcaacctc cgtaaagaga tcgccaaggc 4680
   tttcaaggga aacgagggat acaagagcct cttcaagaag gatattatcg agacaatcct 4740
   gcctgagttc ctggacgata aggatgagat cgctctcgtg aacagcttca acggattcac 4800
   tactgccttc accggattct tcgacaacag ggaaaacatg ttcagcgaag aggccaagag 4860
   cacctctatc gctttcagat gcatcaacga gaacctcacg cgttacatca gcaacatgga 4920
   catcttcgag aaggtggacg ccatcttcga taagcacgag gtgcaagaaa tcaaagagaa 4980
   gatcctcaac agcgactacg acgtcgagga cttttttgaa ggggagttct tcaacttcgt 5040
   tctcacccaa gagggcatcg acgtgtacaa cgctattatc ggaggattcg tgaccgagtc 5100
   tggggagaag attaagggac tcaacgagta catcaacctg tacaaccaga aaacgaagca 5160
   gaagctcccg aagttcaagc cgctctacaa gcaggttctc tctgatcgtg agagcctctc 5220
   attttacggt gagggttaca cctctgacga ggaagtgctt gaggttttcc gtaacaccct 5280
   caacaagaac agcgagatct tctcgtccat caagaagttg gagaaacttt tcaagaactt 5340
   cgacgagtac agcagcgctg ggatcttcgt taagaacgga cctgctatca gcaccatcag 5400
   caaggatatt ttcggcgagt ggaacgtgat cagggacaag tggaatgctg agtacgatga 5460
   catccacctc aagaagaagg ctgtcgtcac tgagaagtac gaggatgaca ggcgtaagtc 5520
   gttcaagaag atcggctctt tcagcctcga gcagcttcaa gaatacgctg atgctgatct 5580
   cagcgtggtc gagaagctca aagagatcat catccagaag gtcgacgaga tctacaaggt 5640
   gtacgggtcc tctgagaagt tgttcgatgc tgatttcgtc ctcgagaaga gtctgaagaa 5700
   gaacgacgct gtcgtcgcga tcatgaagga tttgctcgac agcgtgaagt ccttcgagaa 5760
   ctatatcaag gccttcttcg gagagggcaa agagactaat agggacgagt ctttctacgg 5820
   ggatttcgtg ctcgcttacg atatcctcct caaggtggac catatctacg acgccatcag 5880
   aaactacgtg acccagaagc cttacagcaa ggacaagttc aagttgtact ttcagaaccc 5940
   gcagttcatg ggcggatggg acaaagacaa agagacagat tacagggcca ccatcctcag 6000
   gtacgggtct aagtactacc tggccatcat ggacaagaaa tacgccaagt gcctccaaaa 6060
   gatcgacaag gatgacgtga acgggaacta tgagaagatc aactacaagc tccttccggg 6120
   accgaacaag atgcttccta aggtgttctt cagcaagaaa tggatggcct actacaaccc 6180
   gtctgaggac atccagaaaa tctacaagaa cgggaccttc aagaaaggcg acatgttcaa 6240
   cctcaacgac tgccacaagc tcatcgattt cttcaaggac agcatctcgc gttacccgaa 6300
   gtggtctaac gcttacgact ttaacttcag cgagacagaa aagtacaagg atatcgccgg 6360
   gttctaccgt gaggttgagg aacagggtta caaggttagc ttcgagagcg cctccaagaa 6420
   agaggttgac aagttggtcg aagagggcaa gctctacatg ttccagatct ataacaagga 6480
   cttctccgac aagagccacg gaactcctaa cctccatacg atgtacttca agctgctttt 6540
   cgacgagaac aaccacgggc agatcagact ttctggtggt gctgaactct tcatgcgtag 6600
   ggcctcactc aagaaagaag agttggttgt tcacccggcc aactctccaa tcgctaacaa 6660
   gaatcctgac aacccgaaaa agaccaccac gctgtcttac gacgtctaca aggacaaaag 6720
   gttcagcgag gaccagtacg agcttcatat cccgatcgct atcaacaagt gcccgaagaa 6780
   catcttcaag atcaataccg aggtgagggt gctgctcaag cacgatgata acccttacgt 6840
   gatcggaatc gatcgtggtg agagaaacct cctctacatc gttgtggtgg acggaaaggg 6900
   aaacatcgtc gagcagtaca gcctgaacga gattatcaac aatttcaacg gcatcaggat 6960
   caagaccgac taccactcac tcctcgataa gaaagaaaaa gagcgtttcg aggccaggca 7020
   gaactggact tctatcgaaa acatcaaaga gttgaaggcc ggctacatct ctcaggtggt 7080
   gcataagatc tgcgagctgg tggaaaagta cgatgctgtg atcgctcttg aggacctcaa 7140
   ctctgggttc aagaacagta gagtgaaggt tgagaagcag gtctaccaaa agttcgagaa 7200
   gatgctcatc gacaagctca actacatggt ggacaaaaag agcaaccctt gcgctaccgg 7260
   tggtgctctt aagggatacc agatcacgaa caagttcgag tccttcaaga gcatgagcac 7320
   ccagaacggc ttcatcttct atatccctgc ttggctcacc agcaagatcg atccttctac 7380
   tggtttcgtg aacctgctca agaccaagta cacctcgatc gccgacagca agaagttcat 7440
   ctcgtctttc gacaggatca tgtacgtgcc ggaagaggat cttttcgagt tcgctctcga 7500
   ctataagaac ttcagcagga ccgacgccga ctacattaag aagtggaagc tctactccta 7560
   cgggaaccgt atcaggatct tccgaaatcc gaagaaaaac aacgtgttcg actgggaaga 7620
   agtgtgcctc acctctgcct acaaagaact gttcaacaag tacggcatca actaccagca 7680
   gggtgatatc agggctcttt tgtgcgagca gagcgacaag gcattctaca gctcattcat 7740
   ggccctcatg tctctcatgc tccagatgag gaactctatc accggaagga ccgatgtgga 7800
   cttccttatc tctccggtca agaactctga cgggatcttc tacgacagcc gtaactatga 7860
   ggctcaagag aacgctatcc tgccgaagaa tgctgatgca aacggggctt acaacattgc 7920
   gagaaaggtt ctctgggcta tcgggcagtt taagaaagcg gaagatgaga agctcgacaa 7980
   ggtgaagatc gccatctcca acaaagagtg gcttgagtac gctcagacct ccgttaagca 8040
   caagaggcct gctgctacta agaaagctgg ccaggccaaa aagaagaagt gaggcgcgcc 8100
   gagctccagg cctcccagct ttcgtccgta tcatcggttt cgacaacgtt cgtcaagttc 8160
   aatgcatcag tttcattgcc cacacaccag aatcctacta agtttgagta ttatggcatt 8220
   ggaaaagctg ttttcttcta tcatttgttc tgcttgtaat ttactgtgtt ctttcagttt 8280
   ttgttttcgg acatcaaaat gcaaatggat ggataagagt taataaatga tatggtcctt 8340
   ttgttcattc tcaaattatt attatctgtt gtttttactt taatgggttg aatttaagta 8400
   agaaaggaac taacagtgtg atattaaggt gcaatgttag acatataaaa cagtctttca 8460
   cctctctttg gttatgtctt gaattggttt gtttcttcac ttatctgtgt aatcaagttt 8520
   actatgagtc tatgatcaag taattatgca atcaagttaa gtacagtata ggcttgagct 8580
   ccctaggatc aagctttatg gggtttgggc taccagccgc cattggagct gcagttgctc 8640
   gaccagatgc agtggttgtc gatattgatg gggatggcag ttttattatg aatgttcagg 8700
   aacttgcaac tataagagtt gagaaccttc ctgtgaaaat tatgcttctc aataaccagc 8760
   acttgggaat ggtggttcag ttggaggaca gattttacaa ggcaaatcgc gctcacactt 8820
   atttgggtaa tccatctaag tctgctgata tcttccctga tatgctcaaa ttcgctgagg 8880
   catgtgatat tccttctgcc cgtgttagca acgtggctga tttgagggcc gccattcaaa 8940
   aagcttacta gtcggataat actaatatgt atgccacttt gtttggcaat tgtagatgtt 9000
   atttttatta ataaaaatat tattttttta atacaatttc attgcaacac tctggttctg 9060
   aattacacta gatacaaaat taaattcttc atacaggaca tgatttaaac ctctaattac 9120
   attattaata caaattaaac ctaaattatc caagtatatc tctagtctct ttttcaagac 9180
   tttcaaaaaa cggtccccag ttaatgtcct gtgcgatcca gtgatcgtca aatctatcca 9240
   gcacttgtga agattcaagt atttgcggag gttgtggttg aatcttatct ggactttcag 9300
   ttgataaact ggcccggaac ggaagaagtc ctggagtttg aggtacaatg gattcggtac 9360
   caagtccacg ggtatggaat tcgtcgcttg ttgcagcgtg atgggttcct ctgtgcgaaa 9420
   atccctgatt acattcatga tgaattgtga aatgacactt acaaggaagt ttgatcttgc 9480
   gaggacgctt cttgattgtt atcaaagaga ggacttgtga gtttggcgaa gactgaattt 9540
   tgtaatgtcc aggacctaag ggcttcattt tctgatttat tgaggaagtc ctggtaagag 9600
   ctgccttcgc ctggattgca taatataata ctgggaatac cacctttaat gacacgtggt 9660
   tttccatact ttaagtttgt ctgccactct ctttgtgcgc ctataaggtg cttccaatgc 9720
   ttcatcttta agtaattggg atctacgtca tcaatgacgt tatacagaac attatcatta 9780
   taagttttta aactaaaatc taaatgacca gtaatataat tatgtggacc taaacacctg 9840
   gcccacattg ttttaccagt tcttgaatca ccctctatga ttaaactatt atatctaaaa 9900
   ggccgcgcag cgggatccaa accaaaataa gaatcggccc actcttgaat aatttctgga 9960
   actctagtga aagaagaaag aggaaaaggt ggttgataaa gatctggagg aggaagaaaa 10020
   atagcctcta agttaggttt gaggttgtga tactgaaaaa tatatttttc tgggagtttc 10080
   tcccttatta tctgcagagc ttctaatgca ttacctgcat ttaatgcctc tgctgcagca 10140
   tcattagccg tctgttgacc tccgcgtgca gatcttccat cgacctgaaa ttcaccccag 10200
   tcgatgtgat ctccgtcctt tgagacgtag gacttgacgt cggaactgga tttagctccc 10260
   tgaatattgc agtggaattg tttgctggta cttcgatgtt gcagatcgaa gtaacgggca 10320
   ttacggatct ggacttttcc ttcgaattga ataagggcat gcagatgtgg ttccccattt 10380
   tcatgtaatt ctctgcagat gcgaatatat tttttattcg aaggtgtatt tatagcgagg 10440
   agctgttcta aggcgtcttc tttggttact gaacattgag ggtatgtaag gaaaaaattt 10500
   ttggcttttt tgtaaaaagg catattgaat cggctctctt tcaaatcccc tatcaattgg 10560
   gtgctttggg tgctcttaaa taccaccaag gggccatccg cattaatatt accggatggc 10620
   ccccaaaaaa tacgtggccc aatgaaaata tgccacgtgg aaagctaaag atgttgatgt 10680
   gatggatttg ggcgggaaac ttcctgaaga agttttcatt cccgcccctt tttttgactt 10740
   agtcaaaaaa gtagccgaca tactgtttga aataaagtta aaagtagatc attgtggtcc 10800
   ccacaagaaa cttgctaagc aagtttcgaa ttatttctgt ttaaatacgt tatttttaca 10860
   tgtatatgta cttataagtt taaacgggcc ctatcagtgt ttgacaggat atattggcgg 10920
   gtaaacctaa gagaaaagag cgtttattag aataatcgga tatttaaaag ggcgtgaaaa 10980
   ggtttatccg ttcgtccatt tgtatgtgca tgccaaccac agggttcccc tcgggagtgc 11040
   ttggcattcc gtgcgataat gacttctgtt caaccaccca aacgtcggaa agcctgacga 11100
   cggagcagca ttccaaaaag atcccttggc tcgtctgggt cggctagaag gtcgagtggg 11160
   ctgctgtggc ttgatccctc aacgcggtcg cggacgtagc gcagcgccga aaaatcctcg 11220
   atcgcaaatc cgacgctgtc gaaaagcgtg atctgcttgt cgctctttcg gccgacgtcc 11280
   tggccagtca tcacgcgcca aagttccgtc acaggatgat ctggcgcgag ttgctggatc 11340
   tcgccttcaa tccgggtctg tggcgggaac tccacgaaaa tatccgaacg cagcaagata 11400
   tcgcggtgca tctcggtctt gcctgggcag tcgccgccga cgccgttgat gtggacgccg 11460
   aaaaggatct aggtgaagat cctttttgat aatctcatga ccaaaatccc ttaacgtgag 11520
   ttttcgttcc actgagcgtc agaccccgta gaaaagatca aaggatcttc ttgagatcct 11580
   ttttttctgc gcgtaatctg ctgcttgcaa acaaaaaaac caccgctacc agcggtggtt 11640
   tgtttgccgg atcaagagct accaactctt tttccgaagg taactggctt cagcagagcg 11700
   cagataccaa atactgttct tctagtgtag ccgtagttag gccaccactt caagaactct 11760
   gtagcaccgc ctacatacct cgctctgcta atcctgttac cagtggctgc tgccagtggc 11820
   gataagtcgt gtcttaccgg gttggactca agacgatagt taccggataa ggcgcagcgg 11880
   tcgggctgaa cggggggttc gtgcacacag cccagcttgg agcgaacgac ctacaccgaa 11940
   ctgagatacc tacagcgtga gctatgagaa agcgccacgc ttcccgaagg gagaaaggcg 12000
   gacaggtatc cggtaagcgg cagggtcgga acaggagagc gcacgaggga gcttccaggg 12060
   ggaaacgcct ggtatcttta tagtcctgtc gggtttcgcc acctctgact tgagcgtcga 12120
   tttttgtgat gctcgtcagg ggggcggagc ctatggaaaa acgccagcaa cgcggccttt 12180
   ttacggttcc tggccttttg ctggcctttt gctcacatgt tctttcctgc gttatcccct 12240
   gattctgtgg ataaccgatt accgcctttg agtgagctga taccgctcgc cgcagccgaa 12300
   cgaccgagcg cagcgagtca gtgagcgagg aagcggaaga gcgcctgatg cggtattttc 12360
   tccttacgca tctgtgcggt atttcacacc gcatatggtg cactctcagt acaatctgct 12420
   ctgatgccgc atagttaagc cagtatacac tccgctatcg ctacgtgact gggtcatggc 12480
   tgcgccccga cacccgccaa cacccgctga cgcgccctga cgggcttgtc tgctcccggc 12540
   atccgcttac agacaagctg tgaccgtctc cgggagctgc atgtgtcaga ggttttcacc 12600
   gtcatcaccg aaacgcgcga ggcaggggta cgtcgaggtc gatccaaccc ctccgctgct 12660
   atagtgcagt cggcttctga cgttcagtgc agccgtcttc tgaaaacgac atgtcgcaca 12720
   agtcctaagt tacgcgacag gctgccgccc tgcccttttc ctggcgtttt cttgtcgcgt 12780
   gttttagtcg cataaagtag aatacttgcg actagaaccg gagacattac gccatgaaca 12840
   agagcgccgc cgctggcctg ctgggctatg cccgcgtcag caccgacgac caggacttga 12900
   ccaaccaacg ggccgaactg cacgcggccg gctgcaccaa gctgttttcc gagaagatca 12960
   ccggcaccag gcgcgaccgc ccggagctgg ccaggatgct tgaccaccta cgccctggcg 13020
   acgttgtgac agtgaccagg ctagaccgcc tggcccgcag cacccgcgac ctactggaca 13080
   ttgccgagcg catccaggag gccggcgcgg gcctgcgtag cctggcagag ccgtgggccg 13140
   acaccaccac gccggccggc cgcatggtgt tgaccgtgtt cgccggcatt gccgagttcg 13200
   agcgttccct aatcatcgac cgcacccgga gcgggcgcga ggccgccaag gcgcgaggcg 13260
   tgaagtttgg cccccgccct accctcaccc cggcacagat cgcgcacgcc cgcgagctga 13320
   tcgaccagga aggccgcacc gtgaaagagg cggctgcact gcttggcgtg catcgctcga 13380
   ccctgtaccg cgcacttgag cgcagcgagg aagtgacgcc caccgaggcc aggcggcgcg 13440
   gtgccttccg tgaggacgca ttgaccgagg ccgacgccct ggcggccgcc gagaatgaac 13500
   gccaagagga acaagcatga aaccgcacca ggacggccag gacgaaccgt ttttcattac 13560
   cgaagagatc gaggcggaga tgatcgcggc cgggtacgtg ttcgagccgc ccgcgcacgt 13620
   ctcaaccgtg cggctgcatg aaatcctggc cggtttgtct gatgccaagc tcgcggcctg 13680
   gccggcgagc ttggccgctg aagaaaccga gcgccgccgt ctaaaaaggt gatgtgtatt 13740
   tgagtaaaac agcttgcgtc atgcggtcgc tgcgtatatg atgcgatgag taaataaaca 13800
   aatacgcaag gggaacgcat gaaggttatc gctgtactta accagaaagg cgggtcaggc 13860
   aagacgacca tcgcaaccca tctagcccgc gccctgcaac tcgccggggc cgatgttctg 13920
   ttagtcgatt ccgatcccca gggcagtgcc cgcgattggg cggccgtgcg ggaagatcaa 13980
   ccgctaaccg ttgtcggcat cgaccgcccg acgattgacc gcgacgtgaa ggccatcggc 14040
   cggcgcgact tcgtagtgat cgacggagcg ccccaggcgg cggacttggc tgtgtccgcg 14100
   atcaaggcag ccgacttcgt gctgattccg gtgcagccaa gcccttacga catatgggcc 14160
   accgccgacc tggtggagct ggttaagcag cgcattgagg tcacggatgg aaggctacaa 14220
   gcggcctttg tcgtgtcgcg ggcgatcaaa ggcacgcgca tcggcggtga ggttgccgag 14280
   gcgctggccg ggtacgagct gcccattctt gagtcccgta tcacgcagcg cgtgagctac 14340
   ccaggcactg ccgccgccgg cacaaccgtt cttgaatcag aacccgaggg cgacgctgcc 14400
   cgcgaggtcc aggcgctggc cgctgaaatt aaatcaaaac tcatttgagt taatgaggta 14460
   aagagaaaat gagcaaaagc acaaacacgc taagtgccgg ccgtccgagc gcacgcagca 14520
   gcaaggctgc aacgttggcc agcctggcag acacgccagc catgaagcgg gtcaactttc 14580
   agttgccggc ggaggatcac accaagctga agatgtacgc ggtacgccaa ggcaagacca 14640
   ttaccgagct gctatctgaa tacatcgcgc agctaccaga gtaaatgagc aaatgaataa 14700
   atgagtagat gaattttagc ggctaaagga ggcggcatgg aaaatcaaga acaaccaggc 14760
   accgacgccg tggaatgccc catgtgtgga ggaacgggcg gttggccagg cgtaagcggc 14820
   tgggttgtct gccggccctg caatggcact ggaaccccca agcccgagga atcggcgtga 14880
   gcggtcgcaa accatccggc ccggtacaaa tcggcgcggc gctgggtgat gacctggtgg 14940
   agaagttgaa ggcggcgcag gccgcccagc ggcaacgcat cgaggcagaa gcacgccccg 15000
   gtgaatcgtg gcaagcggcc gctgatcgaa tccgcaaaga atcccggcaa ccgccggcag 15060
   ccggtgcgcc gtcgattagg aagccgccca agggcgacga gcaaccagat tttttcgttc 15120
   cgatgctcta tgacgtgggc acccgcgata gtcgcagcat catggacgtg gccgttttcc 15180
   gtctgtcgaa gcgtgaccga cgagctggcg aggtgatccg ctacgagctt ccagacgggc 15240
   acgtagaggt ttccgcaggg ccggccggca tggcgagtgt gtgggattac gacctggtac 15300
   tgatggcggt ttcccatcta accgaatcca tgaaccgata ccgggaaggg aagggagaca 15360
   agcccggccg cgtgttccgt ccacacgttg cggacgtact caagttctgc cggcgagccg 15420
   atggcggaaa gcagaaagac gacctggtag aaacctgcat tcggttaaac accacgcacg 15480
   ttgccatgca gcgtacgaag aaggccaaga acggccgcct ggtgacggta tccgagggtg 15540
   aagccttgat tagccgctac aagatcgtaa agagcgaaac cgggcggccg gagtacatcg 15600
   agatcgagct agctgattgg atgtaccgcg agatcacaga aggcaagaac ccggacgtgc 15660
   tgacggttca ccccgattac tttttgatcg atcccggcat cggccgtttt ctctaccgcc 15720
   tggcacgccg cgccgcaggc aaggcagaag ccagatggtt gttcaagacg atctacgaac 15780
   gcagtggcag cgccggagag ttcaagaagt tctgtttcac cgtgcgcaag ctgatcgggt 15840
   caaatgacct gccggagtac gatttgaagg aggaggcggg gcaggctggc ccgatcctag 15900
   tcatgcgcta ccgcaacctg atcgagggcg aagcatccgc cggttcctaa tgtacggagc 15960
   agatgctagg gcaaattgcc ctagcagggg aaaaaggtcg aaaaggtctc tttcctgtgg 16020
   atagcacgta cattgggaac ccaaagccgt acattgggaa ccggaacccg tacattggga 16080
   acccaaagcc gtacattggg aaccggtcac acatgtaagt gactgatata aaagagaaaa 16140
   aaggcgattt ttccgcctaa aactctttaa aacttattaa aactcttaaa acccgcctgg 16200
   cctgtgcata actgtctggc cagcgcacag ccgaagagct gcaaaaagcg cctacccttc 16260
   ggtcgctgcg ctccctacgc cccgccgctt cgcgtcggcc tatcgcggcc gctggccgct 16320
   caaaaatggc tggcctacgg ccaggcaatc taccagggcg cggacaagcc gcgccgtcgc 16380
   cactcgaccg ccggcgccca catcaaggca ccggtgggta tgcctgacga tgcgtggaga 16440
   ccgaaacctt gcgctcgttc gccagccagg acagaaatgc ctcgacttcg ctgctgccca 16500
   aggttgccgg gtgacgcaca ccgtggaaac ggatgaaggc acgaacccag tggacataag 16560
   cctgttcggt tcgtaagctg taatgcaagt agcgtatgcg ctcacgcaac tggtccagaa 16620
   ccttgaccga acgcagcggt ggtaacggcg cagtggcggt tttcatggct tgttatgact 16680
   gtttttttgg ggtacagtct atgcctcggg catccaagca gcaagcgcgt tacgccgtgg 16740
   gtcgatgttt gatgttatgg agcagcaacg atgttacgca gcagggcagt cgccctaaaa 16800
   caaagttaaa catcatgagg gaagcggtga tcgccgaagt atcgactcaa ctatcagagg 16860
   tagttggcgt catcgagcgc catctcgaac cgacgttgct ggccgtacat ttgtacggct 16920
   ccgcagtgga tggcggcctg aagccacaca gtgatattga tttgctggtt acggtgaccg 16980
   taaggcttga tgaaacaacg cggcgagctt tgatcaacga ccttttggaa acttcggctt 17040
   cccctggaga gagcgagatt ctccgcgctg tagaagtcac cattgttgtg cacgacgaca 17100
   tcattccgtg gcgttatcca gctaagcgcg aactgcaatt tggagaatgg cagcgcaatg 17160
   acattcttgc aggtatcttc gagccagcca cgatcgacat tgatctggct atcttgctga 17220
   caaaagcaag agaacatagc gttgccttgg taggtccagc ggcggaggaa ctctttgatc 17280
   cggttcctga acaggatcta tttgaggcgc taaatgaaac cttaacgcta tggaactcgc 17340
   cgcccgactg ggctggcgat gagcgaaatg tagtgcttac gttgtcccgc atttggtaca 17400
   gcgcagtaac cggcaaaatc gcgccgaagg atgtcgctgc cgactgggca atggagcgcc 17460
   tgccggccca gtatcagccc gtcatacttg aagctagaca ggcttatctt ggacaagaag 17520
   aagatcgctt ggcctcgcgc gcagatcagt tggaagaatt tgtccactac gtgaaaggcg 17580
   agatcaccaa ggtagtcggc aaataatgtc taacaattcg ttcaagccga cgccgcttcg 17640
   cggcgcggct taactcaagc gttagatgca ctaagcacat aattgctcac agccaaacta 17700
   tcaggtcaag tctgctttta ttatttttaa gcgtgcataa taagccctac acaaattggg 17760
   agatatatca tgaaaggctg gctttttctt gttatcgcaa tagttggcga agtaatcgca 17820
   acatagcttg cttggtcgtt ccgcgtgaac gtcggctcga ttgtacctgc gttcaaatac 17880
   tttgcgatcg tgttgcgcgc ctgcccggtg cgtcggctga tctcacggat cgactgcttc 17940
   tctcgcaacg ccatccgacg gatgatgttt aaaagtccca tgtggatcac tccgttgccc 18000
   cgtcgctcac cgtgttgggg ggaaggtgca catggctcag ttctcaatgg aaattatctg 18060
   cctaaccggc tcagttctgc gtagaaacca acatgcaagc tccaccgggt gcaaagcggc 18120
   agcggcggca ggatatattc aattgtaaat ggcttcatgt ccgggaaatc tacatggatc 18180
   agcaatgagt atgatggtca atatggagaa aaagaaagag taattaccaa ttttttttca 18240
   attcaaaaat gtagatgtcc gcagcgttat tataaaatga aagtacattt tgataaaacg 18300
   acaaattacg atccgtcgta tttataggcg aaagcaataa acaaattatt ctaattcgga 18360
   aatctttatt tcgacgtgtc tacattcacg tccaaatggg ggcttagatg agaaacttca 18420
   cgatcggctc tagttcgagc gatctagtaa catagatgac accgcgcgcg ataatttatc 18480
   ctagtttgcg cgctatattt tgttttctat cgcgtattaa atgtataatt gcgggactct 18540
   aatcataaaa acccatctca taaataacgt catgcattac atgttaatta ttacatgctt 18600
   aacgtaattc aacagaaatt atatgataat catcgcaaga ccggcaacag gattcaatct 18660
   taagaaactt tattgccaaa tgtttgaacg atcggggaaa ttcgtttcta tgcttcaatt 18720
   ctgctctata gatgttcttc tagtcttaac ttttagaagg cacaacattt gaaatgttgg 18780
   taatttttct gctgggtagt gaaccattgt aatttgtgtt tgcttatatt atccgttggg 18840
   tttctctatg ttatctgtgg ggccatatcc cagaactggt tgagtcggtc caacacctgg 18900
   gtgccaatca tgtcgatggt ggggtatggt 18930

<210> 15
<211> 19555
<212> DNA
<213> Artificial Sequence

<220>
<223> pZFNnptII-BSCTV-Repl/LbCpf1_tDT-crRNA_ALS2_RT_ALS-mutated_2x100 -
 binary vector for Agro-transformation coding for the replicon
 (BSCTV) including GE components (SSN, crRNA and the repair
 template (ALS)) and 2*100 nt homology arms; the crRNA is embedded

<400> 15
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg ggtcgacatt gaatcgggct 1620

ctcttcaaat cccctatcaa ttgggtgctt tgggtgctct taaataccac caaggggcca 1680

tccgcattaa tattaccgga tggcccccaa aaaatacgtg gcccaatgaa aatatgccac 1740

gtggaaagct aaagatgttg atgtgatgga tttgggcggg aaacttcctg aagaagtttt 1800

cattcccgcc cctttttttg acttagtcaa aaaagtagcc gacatactgt ttgaaataaa 1860

gttaaaagta gatcattgtg gtccccacaa gaaacttgct aagcaagttt cgaattattt 1920

ctgtttaaat acgttatttt tacatgtata tgtacttata aactagtggc cttggcggcc 1980

gctcgagcga tctagtaaca tagatgacac cgcgcgcgat aatttatcct agtttgcgcg 2040

ctatattttg ttttctatcg cgtattaaat gtataattgc gggactctaa tcataaaaac 2100

ccatctcata aataacgtca tgcattacat gttaattatt acatgcttaa cgtaattcaa 2160

cagaaattat atgataatca tcgcaagacc ggcaacagga ttcaatctta agaaacttta 2220

ttgccaaatg tttgaacgat cggggaaatt cgatctacac ttagtagaaa ttaatctccc 2280

agttaagata atgctgcatc tacacttagt agaaattatc aggtacctct agacttgtac 2340

agctcgtcca tgccgtacag gaacaggtgg tggcggccct cggagcgctc gtactgttcc 2400

acgatggtgt agtcctcgtt gtgggaggtg atgtccagct tggtgtccac gtagtagtag 2460

ccgggcagtt gcacgggctt cttggccatg tagatggtct tgaactccac caggtagtgg 2520

ccgccgtcct tcagcttcag ggcctggtgg atctcgccct tcagcacgcc gtcgcggggg 2580

tacaggcgct cggtggaggc ctcccagccc atggtcttct tctgcattac ggggccgtcg 2640

ggggggaagt tggtgccgcg catcttcacc ttgtagatca gcgtgccgtc ctgcagggag 2700

gagtcctggg tcacggtcac cagaccgccg tcctcgaagt tcatcacgcg ctcccacttg 2760

aagccctcgg ggaaggacag cttcttgtaa tcggggatgt cggcggggtg cttcacgtac 2820

gccttggagc cgtacatgaa ctggggggac aggatgtccc aggcgaaggg cagggggccg 2880

cccttggtca ccttcagctt ggcggtctgg gtgccctcgt aggggcggcc ctcgccctcg 2940

ccctcgatct cgaactcgtg gccgttcatg gagccctcca tgcgcacctt gaagcgcatg 3000

aactctttga tgacctcctc gcccttgctc accatggatc ccctctccaa atgaaatgaa 3060

cttccttata tagaggaagg gtcttgcgaa ggatagtggg attgtgcgtc atcccttacg 3120

tcagtggaga tatcacatca atccacttgc tttgaagacg tggttggaac gtcttctttt 3180

tccacgatgt tcctcgtggg tgggggtcca tctttgggac cactgtcggt agaggcatct 3240

tgaacgatag cctttccttt atcgcaatga tggcatttgt agaagccatc ttccttttct 3300

actgtccttt cgatgaagtg acagatagct gggcaatgga atccgaggag gtttcccgat 3360

attacccttt gttgaaaagt ctcaatagcc ctctggtctt ctgagactgt atctttgata 3420

ttcttggagt agacgagagt gtcgtgctcc accatgtatc acatcaatcc acttgctttg 3480

aagacgtggt tggaacgtct tctttttcca cgatgttcct cgtgggtggg ggtccatctt 3540

tgggaccact gtcggtagag gcatcttgaa cgatagcctt tcctttatcg caatgatggc 3600

atttgtagaa gccatcttcc ttttctactg tcctttcgat gaagtgacag atagctgggc 3660

aatggaatcc gaggaggttt cccgatatta ccctttgttg aaaagtctca atagccctct 3720

ggtcttctga acactagtaa ggccttaagg gccacccggg ctgcaggaat tcgatctggc 3780

acgacaggtt tcccgactgg aaagcgggca gtgagcgcaa cgcaattaat gtgagttagc 3840

tcactcatta ggcaccccag gctttacact ttatgcttcc ggctcgtatg ttgtgtggaa 3900

ttgtgagcgg ataacaattt cacacaggaa acagctatga catgattacg aattcaaaaa 3960

ttacggatat gaatataggc atatccgtat ccgaattatc cgtttgacag ctagcaacga 4020

ttgtacaatt gcttctttaa aaaaggaaga aagaaagaaa gaaaagaatc aacatcagcg 4080

ttaacaaacg gccccgttac ggcccaaacg gtcatataga gtaacggcgt taagcgttga 4140

aagactccta tcgaaatacg taaccgcaaa cgtgtcatag tcagatcccc tcttccttca 4200

ccgcctcaaa cacaaaaata atcttctaca gcctatatat acaacccccc cttctatctc 4260

tcctttctca caattcatca tctttctttc tctaccccca attttaagaa atcctctctt 4320

ctcctcttca ttttcaaggt aaatctctct ctctctctct ctctctgtta ttccttgttt 4380

taattaggta tgtattattg ctagtttgtt aatctgctta tcttatgtat gccttatgtg 4440

aatatcttta tcttgttcat ctcatccgtt tagaagctat aaatttgttg atttgactgt 4500

gtatctacac gtggttatgt ttatatctaa tcagatatga atttcttcat attgttgcgt 4560

ttgtgtgtac caatccgaaa tcgttgattt ttttcattta atcgtgtagc taattgtacg 4620

tatacatatg gatctacgta tcaattgttc atctgtttgt gtttgtatgt atacagatct 4680

gaaaacatca cttctctcat ctgattgtgt tgttacatac atagatatag atctgttata 4740

tcattttttt tattaattgt gtatatatat atgtgcatag atctggatta catgattgtg 4800

attatttaca tgattttgtt atttacgtat gtatatatgt agatctggac tttttggagt 4860

tgttgacttg attgtatttg tgtgtgtata tgtgtgttct gatcttgata tgttatgtat 4920

gtgcagcgaa ttcggcgcgc caaacaatgg cttcctccat ggctcctaag aagaagagga 4980

aggttagcaa gctcgagaag tttaccaact gctacagcct ctctaagacc ctcaggttca 5040

aggctatccc tgtgggaaag acccaagaga atatcgacaa caagaggctc ctcgtcgagg 5100

atgagaagag agctgaagat tacaagggcg tgaagaagct cctcgacagg tactacctca 5160

gcttcatcaa cgatgtgctc cacagcatca agctcaagaa cctcaacaac tacatcagcc 5220

tcttccgtaa gaaaaccagg accgagaaag agaacaaaga gcttgagaac ctcgagatca 5280

acctccgtaa agagatcgcc aaggctttca agggaaacga gggatacaag agcctcttca 5340

agaaggatat tatcgagaca atcctgcctg agttcctgga cgataaggat gagatcgctc 5400

tcgtgaacag cttcaacgga ttcactactg ccttcaccgg attcttcgac aacagggaaa 5460

acatgttcag cgaagaggcc aagagcacct ctatcgcttt cagatgcatc aacgagaacc 5520

tcacgcgtta catcagcaac atggacatct tcgagaaggt ggacgccatc ttcgataagc 5580

acgaggtgca agaaatcaaa gagaagatcc tcaacagcga ctacgacgtc gaggactttt 5640

ttgaagggga gttcttcaac ttcgttctca cccaagaggg catcgacgtg tacaacgcta 5700

ttatcggagg attcgtgacc gagtctgggg agaagattaa gggactcaac gagtacatca 5760

acctgtacaa ccagaaaacg aagcagaagc tcccgaagtt caagccgctc tacaagcagg 5820

ttctctctga tcgtgagagc ctctcatttt acggtgaggg ttacacctct gacgaggaag 5880

tgcttgaggt tttccgtaac accctcaaca agaacagcga gatcttctcg tccatcaaga 5940

agttggagaa acttttcaag aacttcgacg agtacagcag cgctgggatc ttcgttaaga 6000

acggacctgc tatcagcacc atcagcaagg atattttcgg cgagtggaac gtgatcaggg 6060

acaagtggaa tgctgagtac gatgacatcc acctcaagaa gaaggctgtc gtcactgaga 6120

agtacgagga tgacaggcgt aagtcgttca agaagatcgg ctctttcagc ctcgagcagc 6180

ttcaagaata cgctgatgct gatctcagcg tggtcgagaa gctcaaagag atcatcatcc 6240

agaaggtcga cgagatctac aaggtgtacg ggtcctctga gaagttgttc gatgctgatt 6300

tcgtcctcga gaagagtctg aagaagaacg acgctgtcgt cgcgatcatg aaggatttgc 6360

tcgacagcgt gaagtccttc gagaactata tcaaggcctt cttcggagag ggcaaagaga 6420

ctaataggga cgagtctttc tacggggatt tcgtgctcgc ttacgatatc ctcctcaagg 6480

tggaccatat ctacgacgcc atcagaaact acgtgaccca gaagccttac agcaaggaca 6540

agttcaagtt gtactttcag aacccgcagt tcatgggcgg atgggacaaa gacaaagaga 6600

cagattacag ggccaccatc ctcaggtacg ggtctaagta ctacctggcc atcatggaca 6660

agaaatacgc caagtgcctc caaaagatcg acaaggatga cgtgaacggg aactatgaga 6720

agatcaacta caagctcctt ccgggaccga acaagatgct tcctaaggtg ttcttcagca 6780

agaaatggat ggcctactac aacccgtctg aggacatcca gaaaatctac aagaacggga 6840

ccttcaagaa aggcgacatg ttcaacctca acgactgcca caagctcatc gatttcttca 6900

aggacagcat ctcgcgttac ccgaagtggt ctaacgctta cgactttaac ttcagcgaga 6960

cagaaaagta caaggatatc gccgggttct accgtgaggt tgaggaacag ggttacaagg 7020

ttagcttcga gagcgcctcc aagaaagagg ttgacaagtt ggtcgaagag ggcaagctct 7080

acatgttcca gatctataac aaggacttct ccgacaagag ccacggaact cctaacctcc 7140

atacgatgta cttcaagctg cttttcgacg agaacaacca cgggcagatc agactttctg 7200

gtggtgctga actcttcatg cgtagggcct cactcaagaa agaagagttg gttgttcacc 7260

cggccaactc tccaatcgct aacaagaatc ctgacaaccc gaaaaagacc accacgctgt 7320

cttacgacgt ctacaaggac aaaaggttca gcgaggacca gtacgagctt catatcccga 7380

tcgctatcaa caagtgcccg aagaacatct tcaagatcaa taccgaggtg agggtgctgc 7440

tcaagcacga tgataaccct tacgtgatcg gaatcgatcg tggtgagaga aacctcctct 7500

acatcgttgt ggtggacgga aagggaaaca tcgtcgagca gtacagcctg aacgagatta 7560

tcaacaattt caacggcatc aggatcaaga ccgactacca ctcactcctc gataagaaag 7620

aaaaagagcg tttcgaggcc aggcagaact ggacttctat cgaaaacatc aaagagttga 7680

aggccggcta catctctcag gtggtgcata agatctgcga gctggtggaa aagtacgatg 7740

ctgtgatcgc tcttgaggac ctcaactctg ggttcaagaa cagtagagtg aaggttgaga 7800

agcaggtcta ccaaaagttc gagaagatgc tcatcgacaa gctcaactac atggtggaca 7860

aaaagagcaa cccttgcgct accggtggtg ctcttaaggg ataccagatc acgaacaagt 7920

tcgagtcctt caagagcatg agcacccaga acggcttcat cttctatatc cctgcttggc 7980

tcaccagcaa gatcgatcct tctactggtt tcgtgaacct gctcaagacc aagtacacct 8040

cgatcgccga cagcaagaag ttcatctcgt ctttcgacag gatcatgtac gtgccggaag 8100

aggatctttt cgagttcgct ctcgactata agaacttcag caggaccgac gccgactaca 8160

ttaagaagtg gaagctctac tcctacggga accgtatcag gatcttccga aatccgaaga 8220

aaaacaacgt gttcgactgg gaagaagtgt gcctcacctc tgcctacaaa gaactgttca 8280

acaagtacgg catcaactac cagcagggtg atatcagggc tcttttgtgc gagcagagcg 8340

acaaggcatt ctacagctca ttcatggccc tcatgtctct catgctccag atgaggaact 8400

ctatcaccgg aaggaccgat gtggacttcc ttatctctcc ggtcaagaac tctgacggga 8460

tcttctacga cagccgtaac tatgaggctc aagagaacgc tatcctgccg aagaatgctg 8520

atgcaaacgg ggcttacaac attgcgagaa aggttctctg ggctatcggg cagtttaaga 8580

aagcggaaga tgagaagctc gacaaggtga agatcgccat ctccaacaaa gagtggcttg 8640

agtacgctca gacctccgtt aagcacaaga ggcctgctgc tactaagaaa gctggccagg 8700

ccaaaaagaa gaagtgaggc gcgccgagct ccaggcctcc cagctttcgt ccgtatcatc 8760

ggtttcgaca acgttcgtca agttcaatgc atcagtttca ttgcccacac accagaatcc 8820

tactaagttt gagtattatg gcattggaaa agctgttttc ttctatcatt tgttctgctt 8880

gtaatttact gtgttctttc agtttttgtt ttcggacatc aaaatgcaaa tggatggata 8940

agagttaata aatgatatgg tccttttgtt cattctcaaa ttattattat ctgttgtttt 9000

tactttaatg ggttgaattt aagtaagaaa ggaactaaca gtgtgatatt aaggtgcaat 9060

gttagacata taaaacagtc tttcacctct ctttggttat gtcttgaatt ggtttgtttc 9120

ttcacttatc tgtgtaatca agtttactat gagtctatga tcaagtaatt atgcaatcaa 9180

gttaagtaca gtataggctt gagctcccta ggatcaagct ttatggggtt tgggctacca 9240

gccgccattg gagctgcagt tgctcgacca gatgcagtgg ttgtcgatat tgatggggat 9300

ggcagtttta ttatgaatgt tcaggaactt gcaactataa gagttgagaa ccttcctgtg 9360

aaaattatgc ttctcaataa ccagcacttg ggaatggtgg ttcagttgga ggacagattt 9420

tacaaggcaa atcgcgctca cacttatttg ggtaatccat ctaagtctgc tgatatcttc 9480

cctgatatgc tcaaattcgc tgaggcatgt gatattcctt ctgcccgtgt tagcaacgtg 9540

gctgatttga gggccgccat tcaaaaagct tactagtcgg ataatactaa tatgtatgcc 9600

actttgtttg gcaattgtag atgttatttt tattaataaa aatattattt ttttaataca 9660

atttcattgc aacactctgg ttctgaatta cactagatac aaaattaaat tcttcataca 9720

ggacatgatt taaacctcta attacattat taatacaaat taaacctaaa ttatccaagt 9780

atatctctag tctctttttc aagactttca aaaaacggtc cccagttaat gtcctgtgcg 9840

atccagtgat cgtcaaatct atccagcact tgtgaagatt caagtatttg cggaggttgt 9900

ggttgaatct tatctggact ttcagttgat aaactggccc ggaacggaag aagtcctgga 9960

gtttgaggta caatggattc ggtaccaagt ccacgggtat ggaattcgtc gcttgttgca 10020

gcgtgatggg ttcctctgtg cgaaatcccc tgattacatt catgatgaat tgtgaaatga 10080

cacttacaag gaagtttgat cttgcgagga cgcttcttga ttgttatcaa agagaggact 10140

tgtgagtttg gcgaagactg aattttgtaa tgtccaggac ctaagggctt cattttctga 10200

tttattgagg aagtcctggt aagagctgcc ttcgcctgga ttgcataata taatactggg 10260

aataccacct ttaatgacac gtggttttcc atactttaag tttgtctgcc actctctttg 10320

tgcgcctata aggtgcttcc aatgcttcat ctttaagtaa ttgggatcta cgtcatcaat 10380

gacgttatac agaacattat cattataagt ttttaaacta aaatctaaat gaccagtaat 10440

ataattatgt ggacctaaac acctggccca cattgtttta ccagttcttg aatcaccctc 10500

tatgattaaa ctattatatc taaaaggccg cgcagcggga tccaaaccaa aataagaatc 10560

ggcccactct tgaataattt ctggaactct agtgaaagaa gaaagaggaa aaggtggttg 10620

ataaagatct ggaggaggaa gaaaaatagc ctctaagtta ggtttgaggt tgtgatactg 10680

aaaaatatat ttttctggga gtttctccct tattatctgc agagcttcta atgcattacc 10740

tgcatttaat gcctctgctg cagcatcatt agccgtctgt tgacctccgc gtgcagatct 10800

tccatcgacc tgaaattcac cccagtcgat gtgatctccg tcctttgaga cgtaggactt 10860

gacgtcggaa ctggatttag ctccctgaat attgcagtgg aattgtttgc tggtacttcg 10920

atgttgcaga tcgaagtaac gggcattacg gatctggact tttccttcga attgaataag 10980

ggcatgcaga tgtggttccc cattttcatg taattctctg cagatgcgaa tatatttttt 11040

attcgaaggt gtatttatag cgaggagctg ttctaaggcg tcttctttgg ttactgaaca 11100

ttgagggtat gtaaggaaaa aatttttggc ttttttgtaa aaaggcatat tgaatcgggc 11160

tctcttcaaa tcccctatca attgggtgct ttgggtgctc ttaaatacca ccaaggggcc 11220

atccgcatta atattaccgg atggccccca aaaaatacgt ggcccaatga aaatatgcca 11280

cgtggaaagc taaagatgtt gatgtgatgg atttgggcgg gaaacttcct gaagaagttt 11340

tcattcccgc cccttttttt gacttagtca aaaaagtagc cgacatactg tttgaaataa 11400

agttaaaagt agatcattgt ggtccccaca agaaacttgc taagcaagtt tcgaattatt 11460

tctgtttaaa tacgttattt ttacatgtat atgtacttat aagtttaaac gggccctatc 11520

agtgtttgac aggatatatt ggcgggtaaa cctaagagaa aagagcgttt attagaataa 11580

tcggatattt aaaagggcgt gaaaaggttt atccgttcgt ccatttgtat gtgcatgcca 11640

accacagggt tcccctcggg agtgcttggc attccgtgcg ataatgactt ctgttcaacc 11700

acccaaacgt cggaaagcct gacgacggag cagcattcca aaaagatccc ttggctcgtc 11760

tgggtcggct agaaggtcga gtgggctgct gtggcttgat ccctcaacgc ggtcgcggac 11820

gtagcgcagc gccgaaaaat cctcgatcgc aaatccgacg ctgtcgaaaa gcgtgatctg 11880

cttgtcgctc tttcggccga cgtcctggcc agtcatcacg cgccaaagtt ccgtcacagg 11940

atgatctggc gcgagttgct ggatctcgcc ttcaatccgg gtctgtggcg ggaactccac 12000

gaaaatatcc gaacgcagca agatatcgcg gtgcatctcg gtcttgcctg ggcagtcgcc 12060

gccgacgccg ttgatgtgga cgccgaaaag gatctaggtg aagatccttt ttgataatct 12120

catgaccaaa atcccttaac gtgagttttc gttccactga gcgtcagacc ccgtagaaaa 12180

gatcaaagga tcttcttgag atcctttttt tctgcgcgta atctgctgct tgcaaacaaa 12240

aaaaccaccg ctaccagcgg tggtttgttt gccggatcaa gagctaccaa ctctttttcc 12300

gaaggtaact ggcttcagca gagcgcagat accaaatact gttcttctag tgtagccgta 12360

gttaggccac cacttcaaga actctgtagc accgcctaca tacctcgctc tgctaatcct 12420

gttaccagtg gctgctgcca gtggcgataa gtcgtgtctt accgggttgg actcaagacg 12480

atagttaccg gataaggcgc agcggtcggg ctgaacgggg ggttcgtgca cacagcccag 12540

cttggagcga acgacctaca ccgaactgag atacctacag cgtgagctat gagaaagcgc 12600

cacgcttccc gaagggagaa aggcggacag gtatccggta agcggcaggg tcggaacagg 12660

agagcgcacg agggagcttc cagggggaaa cgcctggtat ctttatagtc ctgtcgggtt 12720

tcgccacctc tgacttgagc gtcgattttt gtgatgctcg tcaggggggc ggagcctatg 12780

gaaaaacgcc agcaacgcgg cctttttacg gttcctggcc ttttgctggc cttttgctca 12840

catgttcttt cctgcgttat cccctgattc tgtggataac cgattaccgc ctttgagtga 12900

gctgataccg ctcgccgcag ccgaacgacc gagcgcagcg agtcagtgag cgaggaagcg 12960

gaagagcgcc tgatgcggta ttttctcctt acgcatctgt gcggtatttc acaccgcata 13020

tggtgcactc tcagtacaat ctgctctgat gccgcatagt taagccagta tacactccgc 13080

tatcgctacg tgactgggtc atggctgcgc cccgacaccc gccaacaccc gctgacgcgc 13140

cctgacgggc ttgtctgctc ccggcatccg cttacagaca agctgtgacc gtctccggga 13200

gctgcatgtg tcagaggttt tcaccgtcat caccgaaacg cgcgaggcag gggtacgtcg 13260

aggtcgatcc aacccctccg ctgctatagt gcagtcggct tctgacgttc agtgcagccg 13320

tcttctgaaa acgacatgtc gcacaagtcc taagttacgc gacaggctgc cgccctgccc 13380

ttttcctggc gttttcttgt cgcgtgtttt agtcgcataa agtagaatac ttgcgactag 13440

aaccggagac attacgccat gaacaagagc gccgccgctg gcctgctggg ctatgcccgc 13500

gtcagcaccg acgaccagga cttgaccaac caacgggccg aactgcacgc ggccggctgc 13560

accaagctgt tttccgagaa gatcaccggc accaggcgcg accgcccgga gctggccagg 13620

atgcttgacc acctacgccc tggcgacgtt gtgacagtga ccaggctaga ccgcctggcc 13680

cgcagcaccc gcgacctact ggacattgcc gagcgcatcc aggaggccgg cgcgggcctg 13740

cgtagcctgg cagagccgtg ggccgacacc accacgccgg ccggccgcat ggtgttgacc 13800

gtgttcgccg gcattgccga gttcgagcgt tccctaatca tcgaccgcac ccggagcggg 13860

cgcgaggccg ccaaggcgcg aggcgtgaag tttggccccc gccctaccct caccccggca 13920

cagatcgcgc acgcccgcga gctgatcgac caggaaggcc gcaccgtgaa agaggcggct 13980

gcactgcttg gcgtgcatcg ctcgaccctg taccgcgcac ttgagcgcag cgaggaagtg 14040

acgcccaccg aggccaggcg gcgcggtgcc ttccgtgagg acgcattgac cgaggccgac 14100

gccctggcgg ccgccgagaa tgaacgccaa gaggaacaag catgaaaccg caccaggacg 14160

gccaggacga accgtttttc attaccgaag agatcgaggc ggagatgatc gcggccgggt 14220

acgtgttcga gccgcccgcg cacgtctcaa ccgtgcggct gcatgaaatc ctggccggtt 14280

tgtctgatgc caagctcgcg gcctggccgg cgagcttggc cgctgaagaa accgagcgcc 14340

gccgtctaaa aaggtgatgt gtatttgagt aaaacagctt gcgtcatgcg gtcgctgcgt 14400

atatgatgcg atgagtaaat aaacaaatac gcaaggggaa cgcatgaagg ttatcgctgt 14460

acttaaccag aaaggcgggt caggcaagac gaccatcgca acccatctag cccgcgccct 14520

gcaactcgcc ggggccgatg ttctgttagt cgattccgat ccccagggca gtgcccgcga 14580

ttgggcggcc gtgcgggaag atcaaccgct aaccgttgtc ggcatcgacc gcccgacgat 14640

tgaccgcgac gtgaaggcca tcggccggcg cgacttcgta gtgatcgacg gagcgcccca 14700

ggcggcggac ttggctgtgt ccgcgatcaa ggcagccgac ttcgtgctga ttccggtgca 14760

gccaagccct tacgacatat gggccaccgc cgacctggtg gagctggtta agcagcgcat 14820

tgaggtcacg gatggaaggc tacaagcggc ctttgtcgtg tcgcgggcga tcaaaggcac 14880

gcgcatcggc ggtgaggttg ccgaggcgct ggccgggtac gagctgccca ttcttgagtc 14940

ccgtatcacg cagcgcgtga gctacccagg cactgccgcc gccggcacaa ccgttcttga 15000

atcagaaccc gagggcgacg ctgcccgcga ggtccaggcg ctggccgctg aaattaaatc 15060

aaaactcatt tgagttaatg aggtaaagag aaaatgagca aaagcacaaa cacgctaagt 15120

gccggccgtc cgagcgcacg cagcagcaag gctgcaacgt tggccagcct ggcagacacg 15180

ccagccatga agcgggtcaa ctttcagttg ccggcggagg atcacaccaa gctgaagatg 15240

tacgcggtac gccaaggcaa gaccattacc gagctgctat ctgaatacat cgcgcagcta 15300

ccagagtaaa tgagcaaatg aataaatgag tagatgaatt ttagcggcta aaggaggcgg 15360

catggaaaat caagaacaac caggcaccga cgccgtggaa tgccccatgt gtggaggaac 15420

gggcggttgg ccaggcgtaa gcggctgggt tgtctgccgg ccctgcaatg gcactggaac 15480

ccccaagccc gaggaatcgg cgtgagcggt cgcaaaccat ccggcccggt acaaatcggc 15540

gcggcgctgg gtgatgacct ggtggagaag ttgaaggcgg cgcaggccgc ccagcggcaa 15600

cgcatcgagg cagaagcacg ccccggtgaa tcgtggcaag cggccgctga tcgaatccgc 15660

aaagaatccc ggcaaccgcc ggcagccggt gcgccgtcga ttaggaagcc gcccaagggc 15720

gacgagcaac cagatttttt cgttccgatg ctctatgacg tgggcacccg cgatagtcgc 15780

agcatcatgg acgtggccgt tttccgtctg tcgaagcgtg accgacgagc tggcgaggtg 15840

atccgctacg agcttccaga cgggcacgta gaggtttccg cagggccggc cggcatggcg 15900

agtgtgtggg attacgacct ggtactgatg gcggtttccc atctaaccga atccatgaac 15960

cgataccggg aagggaaggg agacaagccc ggccgcgtgt tccgtccaca cgttgcggac 16020

gtactcaagt tctgccggcg agccgatggc ggaaagcaga aagacgacct ggtagaaacc 16080

tgcattcggt taaacaccac gcacgttgcc atgcagcgta cgaagaaggc caagaacggc 16140

cgcctggtga cggtatccga gggtgaagcc ttgattagcc gctacaagat cgtaaagagc 16200

gaaaccgggc ggccggagta catcgagatc gagctagctg attggatgta ccgcgagatc 16260

acagaaggca agaacccgga cgtgctgacg gttcaccccg attacttttt gatcgatccc 16320

ggcatcggcc gttttctcta ccgcctggca cgccgcgccg caggcaaggc agaagccaga 16380

tggttgttca agacgatcta cgaacgcagt ggcagcgccg gagagttcaa gaagttctgt 16440

ttcaccgtgc gcaagctgat cgggtcaaat gacctgccgg agtacgattt gaaggaggag 16500

gcggggcagg ctggcccgat cctagtcatg cgctaccgca acctgatcga gggcgaagca 16560

tccgccggtt cctaatgtac ggagcagatg ctagggcaaa ttgccctagc aggggaaaaa 16620

ggtcgaaaag gtctctttcc tgtggatagc acgtacattg ggaacccaaa gccgtacatt 16680

gggaaccgga acccgtacat tgggaaccca aagccgtaca ttgggaaccg gtcacacatg 16740

taagtgactg atataaaaga gaaaaaaggc gatttttccg cctaaaactc tttaaaactt 16800

attaaaactc ttaaaacccg cctggcctgt gcataactgt ctggccagcg cacagccgaa 16860

gagctgcaaa aagcgcctac ccttcggtcg ctgcgctccc tacgccccgc cgcttcgcgt 16920

cggcctatcg cggccgctgg ccgctcaaaa atggctggcc tacggccagg caatctacca 16980

gggcgcggac aagccgcgcc gtcgccactc gaccgccggc gcccacatca aggcaccggt 17040

gggtatgcct gacgatgcgt ggagaccgaa accttgcgct cgttcgccag ccaggacaga 17100

aatgcctcga cttcgctgct gcccaaggtt gccgggtgac gcacaccgtg gaaacggatg 17160

aaggcacgaa cccagtggac ataagcctgt tcggttcgta agctgtaatg caagtagcgt 17220

atgcgctcac gcaactggtc cagaaccttg accgaacgca gcggtggtaa cggcgcagtg 17280

gcggttttca tggcttgtta tgactgtttt tttggggtac agtctatgcc tcgggcatcc 17340

aagcagcaag cgcgttacgc cgtgggtcga tgtttgatgt tatggagcag caacgatgtt 17400

acgcagcagg gcagtcgccc taaaacaaag ttaaacatca tgagggaagc ggtgatcgcc 17460

gaagtatcga ctcaactatc agaggtagtt ggcgtcatcg agcgccatct cgaaccgacg 17520

ttgctggccg tacatttgta cggctccgca gtggatggcg gcctgaagcc acacagtgat 17580

attgatttgc tggttacggt gaccgtaagg cttgatgaaa caacgcggcg agctttgatc 17640

aacgaccttt tggaaacttc ggcttcccct ggagagagcg agattctccg cgctgtagaa 17700

gtcaccattg ttgtgcacga cgacatcatt ccgtggcgtt atccagctaa gcgcgaactg 17760

caatttggag aatggcagcg caatgacatt cttgcaggta tcttcgagcc agccacgatc 17820

gacattgatc tggctatctt gctgacaaaa gcaagagaac atagcgttgc cttggtaggt 17880

ccagcggcgg aggaactctt tgatccggtt cctgaacagg atctatttga ggcgctaaat 17940

gaaaccttaa cgctatggaa ctcgccgccc gactgggctg gcgatgagcg aaatgtagtg 18000

cttacgttgt cccgcatttg gtacagcgca gtaaccggca aaatcgcgcc gaaggatgtc 18060

gctgccgact gggcaatgga gcgcctgccg gcccagtatc agcccgtcat acttgaagct 18120

agacaggctt atcttggaca agaagaagat cgcttggcct cgcgcgcaga tcagttggaa 18180

gaatttgtcc actacgtgaa aggcgagatc accaaggtag tcggcaaata atgtctaaca 18240

attcgttcaa gccgacgccg cttcgcggcg cggcttaact caagcgttag atgcactaag 18300

cacataattg ctcacagcca aactatcagg tcaagtctgc ttttattatt tttaagcgtg 18360

cataataagc cctacacaaa ttgggagata tatcatgaaa ggctggcttt ttcttgttat 18420

cgcaatagtt ggcgaagtaa tcgcaacata gcttgcttgg tcgttccgcg tgaacgtcgg 18480

ctcgattgta cctgcgttca aatactttgc gatcgtgttg cgcgcctgcc cggtgcgtcg 18540

gctgatctca cggatcgact gcttctctcg caacgccatc cgacggatga tgtttaaaag 18600

tcccatgtgg atcactccgt tgccccgtcg ctcaccgtgt tggggggaag gtgcacatgg 18660

ctcagttctc aatggaaatt atctgcctaa ccggctcagt tctgcgtaga aaccaacatg 18720

caagctccac cgggtgcaaa gcggcagcgg cggcaggata tattcaattg taaatggctt 18780

catgtccggg aaatctacat ggatcagcaa tgagtatgat ggtcaatatg gagaaaaaga 18840

aagagtaatt accaattttt tttcaattca aaaatgtaga tgtccgcagc gttattataa 18900

aatgaaagta cattttgata aaacgacaaa ttacgatccg tcgtatttat aggcgaaagc 18960

aataaacaaa ttattctaat tcggaaatct ttatttcgac gtgtctacat tcacgtccaa 19020

atgggggctt agatgagaaa cttcacgatc ggctctagtt cgagcgatct agtaacatag 19080

atgacaccgc gcgcgataat ttatcctagt ttgcgcgcta tattttgttt tctatcgcgt 19140

attaaatgta taattgcggg actctaatca taaaaaccca tctcataaat aacgtcatgc 19200

attacatgtt aattattaca tgcttaacgt aattcaacag aaattatatg ataatcatcg 19260

caagaccggc aacaggattc aatcttaaga aactttattg ccaaatgttt gaacgatcgg 19320

ggaaattcgt ttctatgctt caattctgct ctatagatgt tcttctagtc ttaactttta 19380

gaaggcacaa catttgaaat gttggtaatt tttctgctgg gtagtgaacc attgtaattt 19440

gtgtttgctt atattatccg ttgggtttct ctatgttatc tgtgggccca tatcccagaa 19500

ctggttgagt cggtccaaca cctgggtgcc aatcatgtcg atggtggggt atggt 19555

<210>  16
<211>  24
<212>  DNA
<213>  Artificial Sequence

<220>
<223>  crRNA_ALS#1 - PAM TTTC + protospacer within the target gene BvALS

<400>  16
caccctaatt gtagccaact cttg                                              24


<210>  17
<211>  24
<212>  DNA
<213>  Artificial Sequence

<220>
<223>  crRNA_ALS#2 - PAM TTTA + protospacer within the target gene BvALS

<400>  17
gcagcattat cttaactggg agat                                              24


<210>  18
<211>  24
<212>  DNA
<213>  Artificial Sequence

<220>
<223>  crRNA_ALS#3 - PAM TTTA + protospacer within the target gene BvALS

<400>  18
ggtatggttg tccaatggga agat                                              24


<210>  19
<211>  24
<212>  DNA
<213>  Artificial Sequence

<220>
<223>  crRNA_ALS#4 - PAM TTTC + protospacer within the target gene BvALS

<400>  19
caaggtatgt atgtgcccgg ttag                                              24


<210>  20
<211>  24
<212>  DNA
<213>  Artificial Sequence

<220>
<223>  crRNA_ALS#5 - PAM TTTG + protospacer within the target gene BvALS

<400>  20
gaagggtttc caaggtatgt atgt                                              24


<210>  21
<211>  368

<212>  DNA
<213>  Artificial Sequence

<220>
<223>  RT_ALS-mutated_2x100bp arms_HindIII - full sequence of the ALS
       repair template

<400>  21
gaggacaagc tttatggggt ttgggctacc agccgccatt ggagctgcag ttgctcgacc      60

agatgcagtg gttgtcgata ttgatgggga tggcagtttt attatgaatg ttcaggaact     120

tgcaactata agagttgaga accttcctgt gaaaattatg cttctcaata accagcactt     180

gggaatggtg gttcagttgg aggacagatt ttacaaggca aatcgcgctc acacttattt     240

gggtaatcca tctaagtctg ctgatatctt ccctgatatg ctcaaattcg ctgaggcatg     300

tgatattcct tctgcccgtg ttagcaacgt ggctgatttg agggccgcca ttcaaaaagc     360

ttaacggt                                                              368


<210>  22
<211>  2843
<212>  DNA
<213>  Becurtovirus Beet Curly Top Iran Virus

<400>  22
ccccgcgttt acctttccac gtggcgtatg cagagtgggc ggacggttga gtggggaaca      60

cgctttactt taatttaaag taaagtagca ctaagtggga cccacttttt atttctttaa     120

agacttaatt attaagtctt ctagatgcgt atattatacg tataaatgat ggtgtgtatt     180

cccgactggt tatttctact cttcgtattt agtacgattc ttcagtcggg aattaatttc     240

tatggtacct ttcagagtga gcgaatttcc gcgaagctat ccagccttgc tagccgtttc     300

gacgagctgt ttcttgcgtt acaacaagtg gtgtttggtc ggaatcgagc ctcaagttcg     360

cgagccgttg actctagagg aaggcgagga ctttcttcag ttccagaagg aagtgaagaa     420

attgctgaag cttaaggtta gttttaagcg taagtgtagt ttgtacgagg acatatacaa     480

gaagtatggc ggttcagagt caaaagagaa agtatactcc tcccgccagt tggacgagga     540

agaggaagac gaccggtggg aggacagttt cgaagaaata ccaatggaag agaccggtgc     600

gaagtaaccg ggctgtgaag ttgaagatgt atgacgacat gttaggtgca tctggagttg     660

gatctaccat cagtaatgat gggatgatca ctatgttgaa caactatgtt caaggaattg     720

gtgatagcca gaggtcaaac aacttgaccg ttaccaggca tttgaagttt gatatgtctc     780

ttatggctag tggtgcattt tgggcgagtc ctaactatat gactcaatat cattgggtta     840

ttgtggatcg tgatgttggt tctacgtttc cgactcagtt gagtactatc tttgatattc     900

cgactggtgg tcaggctatg ccttccactt atcggattag gagggatgcg aatgaacgat     960

ttatcgtgaa gaagaagtgg cagactcatt tgatgtctac tggtactgat tatggaggaa    1020

agcagaccta taaggcgcct tcgatgccta attataagaa agctatgaac atcaatattc 1080

ggaatatcaa tgtcaagaca ttgtggaagg acactggagg tggcaaatat gaagatgtga 1140

aggagaatgc tattctttac gtagtggtta atgataatac tgataataca aatatgtatg 1200

ctactttgtt tggtaattgt agggcttatt tctattgatt attagaaatg cccaatgatg 1260

ttgaagttta ataaaaaatt tattattaaa tttgtttgtg taagattgaa atgtagatat 1320

gtaaatatct agtataattg ccttggaaaa caaaaaacga aaaaatgatt gccggaaaac 1380

gaaaaaaaaa cccctcagaa attagacccc cggaaaagca atatttttat attaatctgc 1440

agcccatcca cgaagtggat gttacatatt tcgaagaaat aacttaacta taccaaactt 1500

catcacttgc taagtaatat acatcgcaat tagcgtaaaa ccaatctttc atagattcac 1560

tcatacaagg tatccagtcc atatctggat tagtaatagc aatacaaggt cttccgcctg 1620

gaatgactaa gtttggttta tgttttatcc aaacactata atcttcgtta catccgataa 1680

tagatttaaa caactcctgt tgtatttttt gatagcggat atcgtctatt atatggtatg 1740

tagtgtggtc tatatcgtat agagaccaat ctaataccat gccagacata tagtggtgtc 1800

ggcctagaga tcttgcccac tgggtctttc ctgttctgga tggtccgcat atgtagagac 1860

ttcttggtcg gactggtctg ttgtctggct cctggacaaa ttaagtacgt tagtgtcttc 1920

actacatgct agatcgcgac taagtcgatc taggtggaag tattcttcaa gagtcatttg 1980

tctgtcttga agaattgctc ccctgcaatt ctcacatatt tcgtaatgta aacaatctga 2040

acttacgaaa tatatatttt gttccgccca ctctttgata gtgtcaggta gcctagagaa 2100

ctccgtccat ctgggtgtgt aaggttgcac aactggcctg aaatgatagt tggcgaagga 2160

tagaatggaa ggccatctga gaacgaaatc tgttgggcag gactctcgta tgttactgaa 2220

gaactcctcc ttggtgttgg atgaatcaag aattcgtctc cattccacgt tgctgttgcc 2280

aatactgttt ggagagcgcc ctcccagtcg aagctctcca aatgttctag ggctcgtgtc 2340

ctccttggtg atatactcga atgctcgctt tggggcgttg agtccgtcaa ttcttgggtg 2400

aatcgttcga ccgtctggga gtatgatgtt gaaaaacagg ttcccattga cgtcgagttt 2460

cttgtcacac tggataatac agtgtgtgtg gaatcctcct tcaggttgtt cttcagtagg 2520

ctgatgatgt tctagtgata ctgcaaggaa tagaatcaca tatgattcta ataaggtttt 2580

cagtttctcg aatatataat ctttaaaatc ccccctattt tgggaatatg ttagaaaagc 2640

attttttttt tgaaaacgta aataaccggg ttggttaggg gtaggtctgt ttctgtttgg 2700

agcacgtggc atgtttgttt ttgtttccct cttccgtaca atgaatgaaa agaaattgta 2760

tatatgagag gaagaggaac ttaagaggaa tgaggaactg aagagggagg tagagggata 2820

aataggtaaa cgcggataag att 2843

<210>  23
<211>  2927
<212>  DNA
<213>  Curtovirus Beet Severe Curly Top Virus

<400>  23
attgaatcgg gctctcttca aatcccctat caattgggtg ctttgggtgc tcttaaatac      60

caccaagggg ccatccgcat taatattacc ggatggcccc caaaaaatac gtggcccaat     120

gaaaatatgc cacgtggaaa gctaaagatg ttgatgtgat ggatttgggc gggaaacttc     180

ctgaagaagt tttcattccc gccccttttt ttgacttagt caaaaaagta gccgacatac     240

tgtttgaaat aaagttaaaa gtagatcatt gtggtcccca caagaaactt gctaagcaag     300

tttcgaatta tttctgttta aatacgttat ttttacatgt atatgtactt ataaatgatg     360

gtctgtctac cagactggtt atttttgcta tttatcttca gtattctcct gcaatcaggt     420

acgaactttt atgggacctt tcagagtgga tcaatttcca gacaattatc cagcctttct     480

agcagtatcg accagttgtt tcttaaggta caacaagtgg tgtatactag gtatccatca     540

agagatagag gctctgaccc tagaagaagg cgcggtcttt cttcaattcc agaaggaagt     600

gaagaagcta ctgaggcgta aggttaattt tcataggaag tgttcgttgt atgaggaaat     660

atacaagaaa tacgtataca atgtcccaga aaagaaaggt gaatcctcaa agtgcgtggc     720

cgaagaagag gaggactact acgatttcga ggaaatacca atggaggaga cctgtgacaa     780

aaaacaggac tctgaagtta aagatgtatg atgatatgtt gggtgctggt ggtataggat     840

ctaccattag taataatggt atgattacta tgttgaataa ttatgtccag ggtattggtg     900

atagtcagag agcgaagaac gttactgtga cgaagcattt gaagtttgat atggctctta     960

tggggagttc tccgttctgg gagactccta attatatgac ccaatatcat tggattatca    1020

tagacaagga tgttgggtca gtgtttccta ctaagttatc gagtatattt gatattcccg    1080

ataacggtca ggctatgccg tctacttatc gtattcgaag agatatgaac gagaggttta    1140

ttgtgaagaa gaaatggaga actcatttga tgtctactgg tactggatat ggagggaagg    1200

agacttacaa ggctccttca atgccaaatt acaagaaacc gatgaatatc aatgtccgca    1260

atctgaacat gaggactatt tggaaagaca ccggtggtgg gaagtatgaa gacgtgaagg    1320

agaatgcttt actctatgtt gttgttaatg ataatacgga taatactaat atgtatgcca    1380

ctttgtttgg caattgtaga tgttattttt attaataaaa atattatttt tttaatacaa    1440

tttcattgca acactctggt tctgaattac actagataca aaattaaatt cttcatacag    1500

gacatgattt aaacctctaa ttacattatt aatacaaatt aaacctaaat tatccaagta    1560

tatctctagt ctctttttca agactttcaa aaaacggtcc ccagttaatg tcctgtgcga    1620

tccagtgatc gtcaaatcta tccagcactt gtgaagattc aagtatttgc ggaggttgtg    1680

gttgaatctt atctggactt tcagttgata aactggcccg gaacggaaga agtcctggag    1740

tttgaggtac aatggattcg gtaccaagtc cacgggtatg gaattcgtcg cttgttgcag 1800

cgtgatgggt tcctctgtgc gaaaatccct gattacattc atgatgaatt gtgaaatgac 1860

acttacaagg aagtttgatc ttgcgaggac gcttcttgat tgttatcaaa gagaggactt 1920

gtgagtttgg cgaagactga attttgtaat gtccaggacc taagggcttc attttctgat 1980

ttattgagga agtcctggta agagctgcct tcgcctggat tgcataatat aatactggga 2040

ataccacctt taatgacacg tggttttcca tactttaagt ttgtctgcca ctctctttgt 2100

gcgcctataa ggtgcttcca atgcttcatc tttaagtaat tgggatctac gtcatcaatg 2160

acgttataca gaacattatc attataagtt tttaaactaa aatctaaatg accagtaata 2220

taattatgtg gacctaaaca cctggcccac attgttttac cagttcttga atcaccctct 2280

atgattaaac tattatatct aaaaggccgc gcagcgggat ccaaaccaaa ataagaatcg 2340

gcccactctt gaataatttc tggaactcta gtgaaagaag aaagaggaaa aggtggttga 2400

taaagatctg gaggaggaag aaaaatagcc tctaagttag gtttgaggtt gtgatactga 2460

aaaatatatt tttctgggag tttctccctt attatctgca gagcttctaa tgcattacct 2520

gcatttaatg cctctgctgc agcatcatta gccgtctgtt gacctccgcg tgcagatctt 2580

ccatcgacct gaaattcacc ccagtcgatg tgatctccgt cctttgagac gtaggacttg 2640

acgtcggaac tggatttagc tccctgaata ttgcagtgga attgtttgct ggtacttcga 2700

tgttgcagat cgaagtaacg ggcattacgg atctggactt ttccttcgaa ttgaataagg 2760

gcatgcagat gtggttcccc attttcatgt aattctctgc agatgcgaat atatttttta 2820

ttcgaaggtg tatttatagc gaggagctgt tctaaggcgt cttctttggt tactgaacat 2880

tgagggtatg taaggaaaaa atttttggct tttttgtaaa aaggcat 2927

<210> 24
<211> 3771
<212> DNA
<213> Artificial Sequence

<220>
<223> LbCpf1 optimized for Arabidopsis thaliana

<400> 24
atggcttcct ccatggctcc taagaagaag aggaaggtta gcaagctcga gaagtttacc 60

aactgctaca gcctctctaa gaccctcagg ttcaaggcta tccctgtggg aaagacccaa 120

gagaatatcg acaacaagag gctcctcgtc gaggatgaga agagagctga agattacaag 180

ggcgtgaaga agctcctcga caggtactac ctcagcttca tcaacgatgt gctccacagc 240

atcaagctca agaacctcaa caactacatc agcctcttcc gtaagaaaac caggaccgag 300

aaagagaaca aagagcttga gaacctcgag atcaacctcc gtaaagagat cgccaaggct 360

ttcaagggaa acgagggata caagagcctc ttcaagaagg atattatcga gacaatcctg 420

cctgagttcc tggacgataa ggatgagatc gctctcgtga acagcttcaa cggattcact 480

actgccttca ccggattctt cgacaacagg gaaaacatgt tcagcgaaga ggccaagagc 540

acctctatcg ctttcagatg catcaacgag aacctcacgc gttacatcag caacatggac 600

atcttcgaga aggtggacgc catcttcgat aagcacgagg tgcaagaaat caaagagaag 660

atcctcaaca gcgactacga cgtcgaggac ttttttgaag gggagttctt caacttcgtt 720

ctcacccaag agggcatcga cgtgtacaac gctattatcg gaggattcgt gaccgagtct 780

ggggagaaga ttaagggact caacgagtac atcaacctgt acaaccagaa aacgaagcag 840

aagctcccga agttcaagcc gctctacaag caggttctct ctgatcgtga gagcctctca 900

ttttacggtg agggttacac ctctgacgag gaagtgcttg aggttttccg taacaccctc 960

aacaagaaca gcgagatctt ctcgtccatc aagaagttgg agaaactttt caagaacttc 1020

gacgagtaca gcagcgctgg gatcttcgtt aagaacggac ctgctatcag caccatcagc 1080

aaggatattt tcggcgagtg gaacgtgatc agggacaagt ggaatgctga gtacgatgac 1140

atccacctca agaagaaggc tgtcgtcact gagaagtacg aggatgacag gcgtaagtcg 1200

ttcaagaaga tcggctcttt cagcctcgag cagcttcaag aatacgctga tgctgatctc 1260

agcgtggtcg agaagctcaa agagatcatc atccagaagg tcgacgagat ctacaaggtg 1320

tacgggtcct ctgagaagtt gttcgatgct gatttcgtcc tcgagaagag tctgaagaag 1380

aacgacgctg tcgtcgcgat catgaaggat ttgctcgaca gcgtgaagtc cttcgagaac 1440

tatatcaagg ccttcttcgg agagggcaaa gagactaata gggacgagtc tttctacggg 1500

gatttcgtgc tcgcttacga tatcctcctc aaggtggacc atatctacga cgccatcaga 1560

aactacgtga cccagaagcc ttacagcaag gacaagttca agttgtactt tcagaacccg 1620

cagttcatgg gcggatggga caaagacaaa gagacagatt acagggccac catcctcagg 1680

tacgggtcta agtactacct ggccatcatg gacaagaaat acgccaagtg cctccaaaag 1740

atcgacaagg atgacgtgaa cgggaactat gagaagatca actacaagct ccttccggga 1800

ccgaacaaga tgcttcctaa ggtgttcttc agcaagaaat ggatggccta ctacaacccg 1860

tctgaggaca tccagaaaat ctacaagaac gggaccttca agaaaggcga catgttcaac 1920

ctcaacgact gccacaagct catcgatttc ttcaaggaca gcatctcgcg ttacccgaag 1980

tggtctaacg cttacgactt taacttcagc gagacagaaa agtacaagga tatcgccggg 2040

ttctaccgtg aggttgagga acagggttac aaggttagct tcgagagcgc ctccaagaaa 2100

gaggttgaca agttggtcga agagggcaag ctctacatgt tccagatcta taacaaggac 2160

ttctccgaca agagccacgg aactcctaac ctccatacga tgtacttcaa gctgcttttc 2220

gacgagaaca accacgggca gatcagactt tctggtggtg ctgaactctt catgcgtagg 2280

gcctcactca agaaagaaga gttggttgtt cacccggcca actctccaat cgctaacaag 2340

aatcctgaca acccgaaaaa gaccaccacg ctgtcttacg acgtctacaa ggacaaaagg 2400

ttcagcgagg accagtacga gcttcatatc ccgatcgcta tcaacaagtg cccgaagaac 2460

atcttcaaga tcaataccga ggtgagggtg ctgctcaagc acgatgataa cccttacgtg 2520

atcggaatcg atcgtggtga gagaaacctc ctctacatcg ttgtggtgga cggaaaggga 2580

aacatcgtcg agcagtacag cctgaacgag attatcaaca atttcaacgg catcaggatc 2640

aagaccgact accactcact cctcgataag aaagaaaaag agcgtttcga ggccaggcag 2700

aactggactt ctatcgaaaa catcaaagag ttgaaggccg gctacatctc tcaggtggtg 2760

cataagatct gcgagctggt ggaaaagtac gatgctgtga tcgctcttga ggacctcaac 2820

tctgggttca agaacagtag agtgaaggtt gagaagcagg tctaccaaaa gttcgagaag 2880

atgctcatcg acaagctcaa ctacatggtg gacaaaaaga gcaacccttg cgctaccggt 2940

ggtgctctta agggatacca gatcacgaac aagttcgagt ccttcaagag catgagcacc 3000

cagaacggct tcatcttcta tatccctgct tggctcacca gcaagatcga tccttctact 3060

ggtttcgtga acctgctcaa gaccaagtac acctcgatcg ccgacagcaa gaagttcatc 3120

tcgtctttcg acaggatcat gtacgtgccg gaagaggatc ttttcgagtt cgctctcgac 3180

tataagaact tcagcaggac cgacgccgac tacattaaga agtggaagct ctactcctac 3240

gggaaccgta tcaggatctt ccgaaatccg aagaaaaaca acgtgttcga ctgggaagaa 3300

gtgtgcctca cctctgccta caaagaactg ttcaacaagt acggcatcaa ctaccagcag 3360

ggtgatatca gggctctttt gtgcgagcag agcgacaagg cattctacag ctcattcatg 3420

gccctcatgt ctctcatgct ccagatgagg aactctatca ccggaaggac cgatgtggac 3480

ttccttatct ctccggtcaa gaactctgac gggatcttct acgacagccg taactatgag 3540

gctcaagaga acgctatcct gccgaagaat gctgatgcaa acggggctta caacattgcg 3600

agaaaggttc tctgggctat cgggcagttt aagaaagcgg aagatgagaa gctcgacaag 3660

gtgaagatcg ccatctccaa caaagagtgg cttgagtacg ctcagacctc cgttaagcac 3720

aagaggcctg ctgctactaa gaaagctggc caggccaaaa agaagaagtg a 3771

<210> 25
<211> 8828
<212> DNA
<213> Artificial Sequence

<220>
<223> LbCpf1_tDTcrRNA_ALS#1

<400> 25
gattgaaaga aatatagttt aaatatttat tgataaaata acaagtcagg tattatagtc 60

caagcaaaaa cataaattta ttgatgcaag tttaaattca gaaatatttc aataactgat 120

tatatcagct ggtacattgc cgtagatgaa agactgagtg cgatattatg tgtaatacat 180

aaattgatga tatagctagc ttagctcatc gggtcagaag aactcgtcaa gaaggcgata 240

gaaggcgatg cgctgcgaat cgggagcggc gataccgtaa agcacgagga agcggtcagc 300

ccattcgccg ccaagctctt cagcaatatc acgggtagcc aacgctatgt cctgatagcg 360

gtccgccaca cccagccggc cacagtcgat gaatccagaa aagcggccat tttccaccat 420

gatattcggc aagcaggcat cgccatgagt cacgacgaga tcctcgccgt cgggcatgcg 480

cgccttgagc ctggcgaaca gttcggctgg cgcgagcccc tgatgctctt cgtccagatc 540

atcctgatcg acaagaccgg cttccatccg agtacgtgct cgctcgatgc gatgtttcgc 600

ttggtggtcg aatgggcagg tagccggatc aagcgtatgc agccgccgca ttgcatcagc 660

catgatggat actttctcgg caggagcaag gtgagatgac aggagatcct gccccggcac 720

ttcgcccaat agcagccagt cccttcccgc ttcagtgaca acgtcgagca cagctgcgca 780

aggaacgccc gtcgtggcca gccacgatag ccgcgctgcc tcgtcctgaa gttcattcag 840

ggcaccggac aggtcggtct tgacaaaaag aaccgggcgc ccctgcgctg acagccggaa 900

cacggcggca tcagagcagc cgattgtctg ttgtgcccag tcatagccga atagcctctc 960

cacccaagcg gccggagaac ctgcgtgcaa tccatcttgt tcaatccaag ctcccatttt 1020

ctgtgatttt ggatgtgttt ttgtatggat tgagagtgaa tatgagactc taattggata 1080

ccgaggggaa tttatggaac gtcagtggag catttttgac aagaaatatt tgctagctga 1140

tagtgacctt aggcgacttt tgaacgcgca ataatggttt ctgacgtatg tgcttagctc 1200

attaaactcc agaaacccgc ggctgagtgg ctccttcaac gttgcggttc tgtcagttcc 1260

aaacgtaaaa cggcttgtcc cgcgtcatcg gcgggggtca ttccggactg tggggccata 1320

tcccagaact ggttgagtcg gtccaacacc tgggtgccaa tcatgtcgat ggtggggtat 1380

ggccaatttt ttttcaattc aaaaatgtag atgtccgcag cgttattata aaatgaaagt 1440

acattttgat aaaacgacaa attacgatcc gtcgtattta taggcgaaag caataaacaa 1500

attattctaa ttcggaaatc tttatttcga cgtgtctaca ttcacgtcca aatgggggct 1560

tagatgagaa acttcacgat cggctctaga ggccatggcg gccgctcgag cgatctagta 1620

acatagatga caccgcgcgc gataatttat cctagtttgc gcgctatatt ttgttttcta 1680

tcgcgtatta aatgtataat tgcgggactc taatcataaa aacccatctc ataaataacg 1740

tcatgcatta catgttaatt attacatgct taacgtaatt caacagaaat tatatgataa 1800

tcatcgcaag accggcaaca ggattcaatc ttaagaaact ttattgccaa atgtttgaac 1860

gatcggggaa attcgatcta cacttagtag aaattacaag agttggctac aattagggtg 1920

atctacactt agtagaaatt atcaggtacc tctagacttg tacagctcgt ccatgccgta 1980

caggaacagg tggtggcggc cctcggagcg ctcgtactgt tccacgatgg tgtagtcctc 2040

gttgtgggag gtgatgtcca gcttggtgtc cacgtagtag tagccgggca gttgcacggg 2100

cttcttggcc atgtagatgg tcttgaactc caccaggtag tggccgccgt ccttcagctt 2160

cagggcctgg tggatctcgc ccttcagcac gccgtcgcgg gggtacaggc gctcggtgga 2220

ggcctcccag cccatggtct tcttctgcat tacggggccg tcggggggga agttggtgcc 2280

gcgcatcttc accttgtaga tcagcgtgcc gtcctgcagg gaggagtcct gggtcacggt 2340

caccagaccg ccgtcctcga agttcatcac gcgctcccac ttgaagccct cggggaagga 2400

cagcttcttg taatcgggga tgtcggcggg gtgcttcacg tacgccttgg agccgtacat 2460

gaactggggg gacaggatgt cccaggcgaa gggcaggggg ccgcccttgg tcaccttcag 2520

cttggcggtc tgggtgccct cgtaggggcg gccctcgccc tcgccctcga tctcgaactc 2580

gtggccgttc atggagccct ccatgcgcac cttgaagcgc atgaactctt tgatgacctc 2640

ctcgcccttg ctcaccatgg atcccctctc caaatgaaat gaacttcctt atatagagga 2700

agggtcttgc gaaggatagt gggattgtgc gtcatccctt acgtcagtgg agatatcaca 2760

tcaatccact tgctttgaag acgtggttgg aacgtcttct ttttccacga tgttcctcgt 2820

gggtgggggt ccatctttgg gaccactgtc ggtagaggca tcttgaacga tagcctttcc 2880

tttatcgcaa tgatggcatt tgtagaagcc atcttccttt tctactgtcc tttcgatgaa 2940

gtgacagata gctgggcaat ggaatccgag gaggtttccc gatattaccc tttgttgaaa 3000

agtctcaata gccctctggt cttctgagac tgtatctttg atattcttgg agtagacgag 3060

agtgtcgtgc tccaccatgt atcacatcaa tccacttgct ttgaagacgt ggttggaacg 3120

tcttcttttt ccacgatgtt cctcgtgggt gggggtccat ctttgggacc actgtcggta 3180

gaggcatctt gaacgatagc ctttccttta tcgcaatgat ggcatttgta gaagccatct 3240

tccttttcta ctgtcctttc gatgaagtga cagatagctg ggcaatggaa tccgaggagg 3300

tttcccgata ttaccctttg ttgaaaagtc tcaatagccc tctggtcttc tgaacactag 3360

taaggcctta agggccagat cccccgggct gcaggaattc gatctggcac gacaggtttc 3420

ccgactggaa agcgggcagt gagcgcaacg caattaatgt gagttagctc actcattagg 3480

caccccaggc tttacacttt atgcttccgg ctcgtatgtt gtgtggaatt gtgagcggat 3540

aacaatttca cacaggaaac agctatgaca tgattacgaa ttcaaaaatt acggatatga 3600

atataggcat atccgtatcc gaattatccg tttgacagct agcaacgatt gtacaattgc 3660

ttctttaaaa aaggaagaaa gaaagaaaga aaagaatcaa catcagcgtt aacaaacggc 3720

cccgttacgg cccaaacggt catatagagt aacggcgtta agcgttgaaa gactcctatc 3780

gaaatacgta accgcaaacg tgtcatagtc agatcccctc ttccttcacc gcctcaaaca 3840

caaaaataat cttctacagc ctatatatac aaccccccct tctatctctc ctttctcaca 3900

attcatcatc tttctttctc tacccccaat tttaagaaat cctctcttct cctcttcatt 3960

ttcaaggtaa atctctctct ctctctctct ctctgttatt ccttgtttta attaggtatg 4020

tattattgct agtttgttaa tctgcttatc ttatgtatgc cttatgtgaa tatctttatc 4080

ttgttcatct catccgttta gaagctataa atttgttgat ttgactgtgt atctacacgt 4140

ggttatgttt atatctaatc agatatgaat ttcttcatat tgttgcgttt gtgtgtacca 4200

atccgaaatc gttgattttt ttcatttaat cgtgtagcta attgtacgta tacatatgga 4260

tctacgtatc aattgttcat ctgtttgtgt ttgtatgtat acagatctga aaacatcact 4320

tctctcatct gattgtgttg ttacatacat agatatagat ctgttatatc atttttttta 4380

ttaattgtgt atatatatat gtgcatagat ctggattaca tgattgtgat tatttacatg 4440

attttgttat ttacgtatgt atatatgtag atctggactt tttggagttg ttgacttgat 4500

tgtatttgtg tgtgtatatg tgtgttctga tcttgatatg ttatgtatgt gcagcgaatt 4560

cggcgcgcca aacaatggct tcctccatgg ctcctaagaa gaagaggaag gttagcaagc 4620

tcgagaagtt taccaactgc tacagcctct ctaagaccct caggttcaag gctatccctg 4680

tgggaaagac ccaagagaat atcgacaaca agaggctcct cgtcgaggat gagaagagag 4740

ctgaagatta caagggcgtg aagaagctcc tcgacaggta ctacctcagc ttcatcaacg 4800

atgtgctcca cagcatcaag ctcaagaacc tcaacaacta catcagcctc ttccgtaaga 4860

aaaccaggac cgagaaagag aacaaagagc ttgagaacct cgagatcaac ctccgtaaag 4920

agatcgccaa ggctttcaag ggaaacgagg gatacaagag cctcttcaag aaggatatta 4980

tcgagacaat cctgcctgag ttcctggacg ataaggatga gatcgctctc gtgaacagct 5040

tcaacggatt cactactgcc ttcaccggat tcttcgacaa cagggaaaac atgttcagcg 5100

aagaggccaa gagcacctct atcgctttca gatgcatcaa cgagaacctc acgcgttaca 5160

tcagcaacat ggacatcttc gagaaggtgg acgccatctt cgataagcac gaggtgcaag 5220

aaatcaaaga gaagatcctc aacagcgact acgacgtcga ggactttttt gaaggggagt 5280

tcttcaactt cgttctcacc caagagggca tcgacgtgta caacgctatt atcggaggat 5340

tcgtgaccga gtctggggag aagattaagg gactcaacga gtacatcaac ctgtacaacc 5400

agaaaacgaa gcagaagctc ccgaagttca agccgctcta caagcaggtt ctctctgatc 5460

gtgagagcct ctcattttac ggtgagggtt acacctctga cgaggaagtg cttgaggttt 5520

tccgtaacac cctcaacaag aacagcgaga tcttctcgtc catcaagaag ttggagaaac 5580

ttttcaagaa cttcgacgag tacagcagcg ctgggatctt cgttaagaac ggacctgcta 5640

tcagcaccat cagcaaggat attttcggcg agtggaacgt gatcagggac aagtggaatg 5700

ctgagtacga tgacatccac ctcaagaaga aggctgtcgt cactgagaag tacgaggatg 5760

acaggcgtaa gtcgttcaag aagatcggct ctttcagcct cgagcagctt caagaatacg 5820

ctgatgctga tctcagcgtg gtcgagaagc tcaaagagat catcatccag aaggtcgacg 5880

agatctacaa ggtgtacggg tcctctgaga agttgttcga tgctgatttc gtcctcgaga 5940

agagtctgaa gaagaacgac gctgtcgtcg cgatcatgaa ggatttgctc gacagcgtga 6000

agtccttcga gaactatatc aaggccttct tcggagaggg caaagagact aatagggacg 6060

agtctttcta cggggatttc gtgctcgctt acgatatcct cctcaaggtg gaccatatct 6120

acgacgccat cagaaactac gtgacccaga agccttacag caaggacaag ttcaagttgt 6180

actttcagaa cccgcagttc atgggcggat gggacaaaga caaagagaca gattacaggg 6240

ccaccatcct caggtacggg tctaagtact acctggccat catggacaag aaatacgcca 6300

agtgcctcca aaagatcgac aaggatgacg tgaacgggaa ctatgagaag atcaactaca 6360

agctccttcc gggaccgaac aagatgcttc ctaaggtgtt cttcagcaag aaatggatgg 6420

cctactacaa cccgtctgag gacatccaga aaatctacaa gaacgggacc ttcaagaaag 6480

gcgacatgtt caacctcaac gactgccaca agctcatcga tttcttcaag gacagcatct 6540

cgcgttaccc gaagtggtct aacgcttacg actttaactt cagcgagaca gaaaagtaca 6600

aggatatcgc cgggttctac cgtgaggttg aggaacaggg ttacaaggtt agcttcgaga 6660

gcgcctccaa gaaagaggtt gacaagttgg tcgaagaggg caagctctac atgttccaga 6720

tctataacaa ggacttctcc gacaagagcc acggaactcc taacctccat acgatgtact 6780

tcaagctgct tttcgacgag aacaaccacg ggcagatcag actttctggt ggtgctgaac 6840

tcttcatgcg tagggcctca ctcaagaaag aagagttggt tgttcacccg gccaactctc 6900

caatcgctaa caagaatcct gacaacccga aaaagaccac cacgctgtct tacgacgtct 6960

acaaggacaa aaggttcagc gaggaccagt acgagcttca tatcccgatc gctatcaaca 7020

agtgcccgaa gaacatcttc aagatcaata ccgaggtgag ggtgctgctc aagcacgatg 7080

ataaccctta cgtgatcgga atcgatcgtg gtgagagaaa cctcctctac atcgttgtgg 7140

tggacggaaa gggaaacatc gtcgagcagt acagcctgaa cgagattatc aacaatttca 7200

acggcatcag gatcaagacc gactaccact cactcctcga taagaaagaa aaagagcgtt 7260

tcgaggccag gcagaactgg acttctatcg aaaacatcaa agagttgaag gccggctaca 7320

tctctcaggt ggtgcataag atctgcgagc tggtggaaaa gtacgatgct gtgatcgctc 7380

ttgaggacct caactctggg ttcaagaaca gtagagtgaa ggttgagaag caggtctacc 7440

aaaagttcga gaagatgctc atcgacaagc tcaactacat ggtggacaaa aagagcaacc 7500

cttgcgctac cggtggtgct cttaagggat accagatcac gaacaagttc gagtccttca 7560

agagcatgag cacccagaac ggcttcatct tctatatccc tgcttggctc accagcaaga 7620

tcgatccttc tactggtttc gtgaacctgc tcaagaccaa gtacacctcg atcgccgaca 7680

gcaagaagtt catctcgtct ttcgacagga tcatgtacgt gccggaagag gatcttttcg 7740

agttcgctct cgactataag aacttcagca ggaccgacgc cgactacatt aagaagtgga 7800

agctctactc ctacgggaac cgtatcagga tcttccgaaa tccgaagaaa aacaacgtgt 7860

tcgactggga agaagtgtgc ctcacctctg cctacaaaga actgttcaac aagtacggca 7920

tcaactacca gcagggtgat atcagggctc ttttgtgcga gcagagcgac aaggcattct 7980

acagctcatt catggccctc atgtctctca tgctccagat gaggaactct atcaccggaa 8040

ggaccgatgt ggacttcctt atctctccgg tcaagaactc tgacgggatc ttctacgaca 8100

gccgtaacta tgaggctcaa gagaacgcta tcctgccgaa gaatgctgat gcaaacgggg 8160

cttacaacat tgcgagaaag gttctctggg ctatcgggca gtttaagaaa gcggaagatg 8220

agaagctcga caaggtgaag atcgccatct ccaacaaaga gtggcttgag tacgctcaga 8280

cctccgttaa gcacaagagg cctgctgcta ctaagaaagc tggccaggcc aaaaagaaga 8340

agtgaggcgc gccgagctcc aggcctccca gctttcgtcc gtatcatcgg tttcgacaac 8400

gttcgtcaag ttcaatgcat cagtttcatt gcccacacac cagaatccta ctaagtttga 8460

gtattatggc attggaaaag ctgttttctt ctatcatttg ttctgcttgt aatttactgt 8520

gttctttcag tttttgtttt cggacatcaa aatgcaaatg gatggataag agttaataaa 8580

tgatatggtc cttttgttca ttctcaaatt attattatct gttgttttta ctttaatggg 8640

ttgaatttaa gtaagaaagg aactaacagt gtgatattaa ggtgcaatgt tagacatata 8700

aaacagtctt tcacctctct ttggttatgt cttgaattgg tttgtttctt cacttatctg 8760

tgtaatcaag tttactatga gtctatgatc aagtaattat gcaatcaagt taagtacagt 8820

ataggctt 8828

<210> 26
<211> 8201
<212> DNA
<213> Artificial Sequence

<220>
<223> LbCpf1_RibocrRNA_ALS#1

<400> 26
ttgaaagaaa tatagtttaa atatttattg ataaaataac aagtcaggta ttatagtcca 60

agcaaaaaca taaatttatt gatgcaagtt taaattcaga aatatttcaa taactgatta 120

tatcagctgg tacattgccg tagatgaaag actgagtgcg atattatgtg taatacataa 180

attgatgata tagctagctt agctcatcgg gtcagaagaa ctcgtcaaga aggcgataga 240

aggcgatgcg ctgcgaatcg ggagcggcga taccgtaaag cacgaggaag cggtcagccc 300

attcgccgcc aagctcttca gcaatatcac gggtagccaa cgctatgtcc tgatagcggt 360

ccgccacacc cagccggcca cagtcgatga atccagaaaa gcggccattt tccaccatga 420

tattcggcaa gcaggcatcg ccatgagtca cgacgagatc ctcgccgtcg ggcatgcgcg 480

ccttgagcct ggcgaacagt tcggctggcg cgagcccctg atgctcttcg tccagatcat 540

cctgatcgac aagaccggct tccatccgag tacgtgctcg ctcgatgcga tgtttcgctt 600

ggtggtcgaa tgggcaggta gccggatcaa gcgtatgcag ccgccgcatt gcatcagcca 660

tgatggatac tttctcggca ggagcaaggt gagatgacag gagatcctgc cccggcactt 720

cgcccaatag cagccagtcc cttcccgctt cagtgacaac gtcgagcaca gctgcgcaag 780

gaacgcccgt cgtggccagc cacgatagcc gcgctgcctc gtcctgaagt tcattcaggg 840

caccggacag gtcggtcttg acaaaaagaa ccgggcgccc ctgcgctgac agccggaaca 900

cggcggcatc agagcagccg attgtctgtt gtgcccagtc atagccgaat agcctctcca 960

cccaagcggc cggagaacct gcgtgcaatc catcttgttc aatccaagct cccatttctt 1020

gtgattttgg atgtgttttt gtatggattg agagtgaata tgagactcta attggatacc 1080

gaggggaatt tatggaacgt cagtggagca tttttgacaa gaaatatttg ctagctgata 1140

gtgaccttag gcgacttttg aacgcgcaat aatggtttct gacgtatgtg cttagctcat 1200

taaactccag aaacccgcgg ctgagtggct ccttcaacgt tgcggttctg tcagttccaa 1260

acgtaaaacg gcttgtcccg cgtcatcggc gggggtcatt ccggactgtg gggccatatc 1320

ccagaactgg ttgagtcggt ccaacacctg ggtgccaatc atgtcgatgg tggggtatgg 1380

ccaatttttt ttcaattcaa aaatgtagat gtccgcagcg ttattataaa atgaaagtac 1440

attttgataa aacgacaaat tacgatccgt cgtatttata ggcgaaagca ataaacaaat 1500

tattctaatt cggaaatctt tatttcgacg tgtctacatt cacgtccaaa tgggggctta 1560

gatgagaaac ttcacgatcg gctctagagg ccatggcggc cgctcgagcg atctagtaac 1620

atagatgaca ccgcgcgcga taatttatcc tagtttgcgc gctatatttt gttttctatc 1680

gcgtattaaa tgtataattg cgggactcta atcataaaaa cccatctcat aaataacgtc 1740

atgcattaca tgttaattat tacatgctta acgtaattca acagaaatta tatgataatc 1800

atcgcaagac cggcaacagg attcaatctt aagaaacttt attgccaaat gtttgaacga 1860

tcgcctctct gcttggctcc tctttcttgt gaaagagggt tcccaggcga caccacgagg 1920

aggtgcagag taggactgac gcccaagagt tggctacaat tagggtgatc tacacttagt 1980

agaaattaga cgagcttact cgtttcgtcc tcacggactc atcagtaatt tggatcccct 2040

ctccaaatga aatgaacttc cttatataga ggaagggtct tgcgaaggat agtgggattg 2100

tgcgtcatcc cttacgtcag tggagatatc acatcaatcc acttgctttg aagacgtggt 2160

tggaacgtct tctttttcca cgatgttcct cgtgggtggg ggtccatctt tgggaccact 2220

gtcggtagag gcatcttgaa cgatagcctt tcctttatcg caatgatggc atttgtagaa 2280

gccatcttcc ttttctactg tcctttcgat gaagtgacag atagctgggc aatggaatcc 2340

gaggaggttt cccgatatta ccctttgttg aaaagtctca atagccctct ggtcttctga 2400

gactgtatct ttgatattct tggagtagac gagagtgtcg tgctccacca tgtatcacat 2460

caatccactt gctttgaaga cgtggttgga acgtcttctt tttccacgat gttcctcgtg 2520

ggtgggggtc catctttggg accactgtcg gtagaggcat cttgaacgat agcctttcct 2580

ttatcgcaat gatggcattt gtagaagcca tcttcctttt ctactgtcct ttcgatgaag 2640

tgacagatag ctgggcaatg gaatccgagg aggtttcccg atattaccct ttgttgaaaa 2700

gtctcaatag ccctctggtc ttctgaacac tagtaaggcc ttaagggcca gatcccccgg 2760

gctgcaggaa ttcgatctgg cacgacaggt ttcccgactg gaaagcgggc agtgagcgca 2820

acgcaattaa tgtgagttag ctcactcatt aggcacccca ggctttacac tttatgcttc 2880

cggctcgtat gttgtgtgga attgtgagcg gataacaatt tcacacagga aacagctatg 2940

acatgattac gaattcaaaa attacggata tgaatatagg catatccgta tccgaattat 3000

ccgtttgaca gctagcaacg attgtacaat tgcttcttta aaaaaggaag aaagaaagaa 3060

agaaaagaat caacatcagc gttaacaaac ggccccgtta cggcccaaac ggtcatatag 3120

agtaacggcg ttaagcgttg aaagactcct atcgaaatac gtaaccgcaa acgtgtcata 3180

gtcagatccc ctcttccttc accgcctcaa acacaaaaat aatcttctac agcctatata 3240

tacaaccccc ccttctatct ctcctttctc acaattcatc atctttcttt ctctaccccc 3300

aattttaaga aatcctctct tctcctcttc attttcaagg taaatctctc tctctctctc 3360

tctctctgtt attccttgtt ttaattaggt atgtattatt gctagtttgt taatctgctt 3420

atcttatgta tgccttatgt gaatatcttt atcttgttca tctcatccgt ttagaagcta 3480

taaatttgtt gatttgactg tgtatctaca cgtggttatg tttatatcta atcagatatg 3540

aatttcttca tattgttgcg tttgtgtgta ccaatccgaa atcgttgatt tttttcattt 3600

aatcgtgtag ctaattgtac gtatacatat ggatctacgt atcaattgtt catctgtttg 3660

tgtttgtatg tatacagatc tgaaaacatc acttctctca tctgattgtg ttgttacata 3720

catagatata gatctgttat atcatttttt ttattaattg tgtatatata tatgtgcata 3780

gatctggatt acatgattgt gattatttac atgattttgt tatttacgta tgtatatatg 3840

tagatctgga ctttttggag ttgttgactt gattgtattt gtgtgtgtat atgtgtgttc 3900

tgatcttgat atgttatgta tgtgcagcga attcggcgcg ccaaacaatg gcttcctcca 3960

tggctcctaa gaagaagagg aaggttagca agctcgagaa gtttaccaac tgctacagcc 4020

tctctaagac cctcaggttc aaggctatcc ctgtgggaaa gacccaagag aatatcgaca 4080

acaagaggct cctcgtcgag gatgagaaga gagctgaaga ttacaagggc gtgaagaagc 4140

tcctcgacag gtactacctc agcttcatca acgatgtgct ccacagcatc aagctcaaga 4200

acctcaacaa ctacatcagc ctcttccgta agaaaaccag gaccgagaaa gagaacaaag 4260

agcttgagaa cctcgagatc aacctccgta aagagatcgc caaggctttc aagggaaacg 4320

agggatacaa gagcctcttc aagaaggata ttatcgagac aatcctgcct gagttcctgg 4380

acgataagga tgagatcgct ctcgtgaaca gcttcaacgg attcactact gccttcaccg 4440

gattcttcga caacagggaa aacatgttca gcgaagaggc caagagcacc tctatcgctt 4500

tcagatgcat caacgagaac ctcacgcgtt acatcagcaa catggacatc ttcgagaagg 4560

tggacgccat cttcgataag cacgaggtgc aagaaatcaa agagaagatc ctcaacagcg 4620

actacgacgt cgaggacttt tttgaagggg agttcttcaa cttcgttctc acccaagagg 4680

gcatcgacgt gtacaacgct attatcggag gattcgtgac cgagtctggg gagaagatta 4740

agggactcaa cgagtacatc aacctgtaca accagaaaac gaagcagaag ctcccgaagt 4800

tcaagccgct ctacaagcag gttctctctg atcgtgagag cctctcattt tacggtgagg 4860

gttacacctc tgacgaggaa gtgcttgagg ttttccgtaa caccctcaac aagaacagcg 4920

agatcttctc gtccatcaag aagttggaga aacttttcaa gaacttcgac gagtacagca 4980

gcgctgggat cttcgttaag aacggacctg ctatcagcac catcagcaag gatattttcg 5040

gcgagtggaa cgtgatcagg gacaagtgga atgctgagta cgatgacatc cacctcaaga 5100

agaaggctgt cgtcactgag aagtacgagg atgacaggcg taagtcgttc aagaagatcg 5160

gctctttcag cctcgagcag cttcaagaat acgctgatgc tgatctcagc gtggtcgaga 5220

agctcaaaga gatcatcatc cagaaggtcg acgagatcta caaggtgtac gggtcctctg 5280

agaagttgtt cgatgctgat ttcgtcctcg agaagagtct gaagaagaac gacgctgtcg 5340

tcgcgatcat gaaggatttg ctcgacagcg tgaagtcctt cgagaactat atcaaggcct 5400

tcttcggaga gggcaaagag actaataggg acgagtcttt ctacggggat ttcgtgctcg 5460

cttacgatat cctcctcaag gtggaccata tctacgacgc catcagaaac tacgtgaccc 5520

agaagcctta cagcaaggac aagttcaagt tgtactttca gaacccgcag ttcatgggcg 5580

gatgggacaa agacaaagag acagattaca gggccaccat cctcaggtac gggtctaagt 5640

actacctggc catcatggac aagaaatacg ccaagtgcct ccaaaagatc gacaaggatg 5700

acgtgaacgg gaactatgag aagatcaact acaagctcct tccgggaccg aacaagatgc 5760

ttcctaaggt gttcttcagc aagaaatgga tggcctacta caacccgtct gaggacatcc 5820

agaaaatcta caagaacggg accttcaaga aaggcgacat gttcaacctc aacgactgcc 5880

acaagctcat cgatttcttc aaggacagca tctcgcgtta cccgaagtgg tctaacgctt 5940

acgactttaa cttcagcgag acagaaaagt acaaggatat cgccgggttc taccgtgagg 6000

ttgaggaaca gggttacaag gttagcttcg agagcgcctc caagaaagag gttgacaagt 6060

tggtcgaaga gggcaagctc tacatgttcc agatctataa caaggacttc tccgacaaga 6120

gccacggaac tcctaacctc catacgatgt acttcaagct gcttttcgac gagaacaacc 6180

acgggcagat cagactttct ggtggtgctg aactcttcat gcgtagggcc tcactcaaga 6240

aagaagagtt ggttgttcac ccggccaact ctccaatcgc taacaagaat cctgacaacc 6300

cgaaaaagac caccacgctg tcttacgacg tctacaagga caaaaggttc agcgaggacc 6360

agtacgagct tcatatcccg atcgctatca acaagtgccc gaagaacatc ttcaagatca 6420

ataccgaggt gagggtgctg ctcaagcacg atgataaccc ttacgtgatc ggaatcgatc 6480

gtggtgagag aaacctcctc tacatcgttg tggtggacgg aaagggaaac atcgtcgagc 6540

agtacagcct gaacgagatt atcaacaatt tcaacggcat caggatcaag accgactacc 6600

actcactcct cgataagaaa gaaaaagagc gtttcgaggc caggcagaac tggacttcta 6660

tcgaaaacat caaagagttg aaggccggct acatctctca ggtggtgcat aagatctgcg 6720

agctggtgga aaagtacgat gctgtgatcg ctcttgagga cctcaactct gggttcaaga 6780

acagtagagt gaaggttgag aagcaggtct accaaaagtt cgagaagatg ctcatcgaca 6840

agctcaacta catggtggac aaaaagagca acccttgcgc taccggtggt gctcttaagg 6900

gataccagat cacgaacaag ttcgagtcct tcaagagcat gagcacccag aacggcttca 6960

tcttctatat ccctgcttgg ctcaccagca agatcgatcc ttctactggt ttcgtgaacc 7020

tgctcaagac caagtacacc tcgatcgccg acagcaagaa gttcatctcg tctttcgaca 7080

ggatcatgta cgtgccggaa gaggatcttt tcgagttcgc tctcgactat aagaacttca 7140

gcaggaccga cgccgactac attaagaagt ggaagctcta ctcctacggg aaccgtatca 7200

ggatcttccg aaatccgaag aaaaacaacg tgttcgactg ggaagaagtg tgcctcacct 7260

ctgcctacaa agaactgttc aacaagtacg gcatcaacta ccagcagggt gatatcaggg 7320

ctcttttgtg cgagcagagc gacaaggcat tctacagctc attcatggcc ctcatgtctc 7380

tcatgctcca gatgaggaac tctatcaccg gaaggaccga tgtggacttc cttatctctc 7440

cggtcaagaa ctctgacggg atcttctacg acagccgtaa ctatgaggct caagagaacg 7500

ctatcctgcc gaagaatgct gatgcaaacg gggcttacaa cattgcgaga aaggttctct 7560

gggctatcgg gcagtttaag aaagcggaag atgagaagct cgacaaggtg aagatcgcca 7620

tctccaacaa agagtggctt gagtacgctc agacctccgt taagcacaag aggcctgctg 7680

ctactaagaa agctggccag gccaaaaaga agaagtgagg cgcgccgagc tccaggcctc 7740

ccagctttcg tccgtatcat cggtttcgac aacgttcgtc aagttcaatg catcagtttc 7800

attgcccaca caccagaatc ctactaagtt tgagtattat ggcattggaa aagctgtttt 7860

cttctatcat ttgttctgct tgtaatttac tgtgttcttt cagtttttgt tttcggacat 7920

caaaatgcaa atggatggat aagagttaat aaatgatatg gtccttttgt tcattctcaa 7980

attattatta tctgttgttt ttactttaat gggttgaatt taagtaagaa aggaactaac 8040

agtgtgatat taaggtgcaa tgttagacat ataaaacagt ctttcacctc tctttggtta 8100

tgtcttgaat tggtttgttt cttcacttat ctgtgtaatc aagtttacta tgagtctatg 8160

atcaagtaat tatgcaatca agttaagtac agtataggct t 8201

<210> 27
<211> 2930
<212> DNA
<213> Curtovirus Beet mild curly top virus

<400> 27
accggatggc cccgaaaatt ttgccccccc aattgtaacg tgacacgtgt tatcgcatta 60

tacgttgtcg gtgcttggaa acttcctatg taagtttcct tctctaacaa gttttgacta 120

gtcaaaagtt gcggccgaca actaccttta cttttttaaa gtaaagtaag tttattattc 180

aaaacttgct gtgtaagttt ggaatatttt tctatttaaa tagctatacg aatacgtata 240

ggtatttata aatgatggtc tgtttaccag actggttatt tttgctattt atcttcagta 300

ttctactgca atcaggtacg aatttttatg ggacctttca gagtggatca atttccagac 360

aattatccag cctttctagc agtatcgacc agttgtttct taaggtacaa caggtggtgt 420

atactaggta tccatcaaga gatagaggct ctgaccctag aagaaggcga ggtctttctt 480

caattccaga aggaagtgaa gaagctactg aggtgtaagg tcaattttca taggaagtgt 540

tcgttgtatg aggaaatata caagaaatac gtatacaatg tcccagaaaa gaaaggtgaa 600

tcctcaaagt gcttggccga agaagaggag gactactacg acttcgagga aataccaatg 660

gaggagacct gtgacaaaaa acaggactcc gaagttaaag atgtatgatg atatgcttgg 720

tgctggtggt ataggatcta ccattagtaa taatggtatg attactatgc tgaataatta 780

tgtccagggt attggtgata gtcagagagc gaacaacgtt actgtgacga agcatttgaa 840

gtttgatatg gctcttatgg ggagttctcc gttctgggag actcctaatt atatgaccca 900

atatcattgg attatcatag acaaggatgt tgggtcagtg ttccctacta agttatccag 960

tatatttgat attccggata acggtcaggc tatgccgtct acttatcgta ttcgaagaga 1020

tatgaacgag aggttcattg tgaagaagaa gtggagaact catttgatgt ctactggtac 1080

tggctatgga ggcaaggaga cttacaaggc tccttcaatg ccaaattaca agaaaccgat 1140

gaatatcaat gtccgtaatc tgaacatgag gactatttgg aaagataccg gaggtgggaa 1200

gtatgaagac gtgaaggaga atgctttact ctatgttgtt gttaatgata atactgataa 1260

tactaatatg tatgccactt tgtttggcaa ttgtagatgt tatttctatt aataaaacta 1320

ttttttattt aatacaactt cattgcaata ctagtatatt gtattacact attgacgaaa 1380

ttgaagacag aatacaatac atgatttaat atttctatta caatgttaat tgagagagaa 1440

gatcgttttt tgatttccct gtcgacttgg ttcttcaaga ctctcaaaaa acggtctcca 1500

gtcaatgtcc tgtgggatcc agttatcgtc atatcgatcc aacacttgtg caggttgagc 1560

gatttgcgga ggttgtggtt gaatctcatc tggactttga gttgataaat cgttccgtat 1620

ctctcgaacc ataggagttt gaagtagagt ggattcggaa ccgatgttgt tggtgttgat 1680

ttcgtcgcct gttccagggt aataggtagt tccgtgcgaa aatccgtgat ggcagtcatg 1740

atgaattgtg aagtgacact tacaggggag attgaccttg cgaggacgct tctgtatctt 1800

tatcaaagag agggccggag agtttaacga aggttgaatt ctgtatagtc caggacttaa 1860

gggcttcatt ttctgattta tctaggaagt cctggtaaga gctgccatct cctgggttgc 1920

ataatataat actgggaata ccacctttaa tgacacgtgg ttttccatac tttaagtttg 1980

tctgccactc tctttgtgcg cctatgaggt gtttccaatg cttcatcttt aagtaagctg 2040

ggtctacgtc atcaatgacg ttatataaaa catcatcgtg atatgttttt aaactaaaat 2100

ctaaatgccc tgaaatgtaa ttatgaggtc ctaatgatct agcccacaaa gtcttacccg 2160

ttctagaatc accctctatg attatactat tatatctaaa aggccgcgca gcggcatcca 2220

ccccgaaata agaatctgcc cactcttgaa caatttctgg aacttgagtg aaagaagatt 2280

gtgggaatgg aggttgataa atatctggtg gaggaagaaa aatggcttct aagttaggtt 2340

taaggttgtg atactgaaaa atatattttt ctgggagttt ctcccttatt atttgcagtg 2400

cttcagctgc attacctgca tttaatgctt ctgctgctgc atcattagcc gtctgctggc 2460

ctcctctagc agatcttccg tcgacttgaa atgtacccca gtcgacgtaa tcaccgtcct 2520

tctcgatgta ttgtttaaca tcggatgcag attttgctcc ttggaagttg gggtggaagg 2580

tggagcttga ggaaggatga gtgatgtcga agtgtctagg gtttctgaat tgtgctttac 2640

ctttgaattg gatgagggcg tggagatgca gagacccatc ctgatgtttt tcctgggata 2700

ctctaataaa taatttatca gatgggcaag gaatattttt caatatttct agagcatctt 2760

cttttataac tgaacatcgt gggtatgtta gaaagatatt tttggcttta atttgaaatg 2820

atggtgaacg aggcatggtt actattccta ttgggggctc tcgtaaactt gtatgaatgg 2880

ggtgctatgg gagcccttat atacctccag gggccatccg caataatatt 2930

<210> 28
<211> 2993
<212> DNA
<213> Curtovirus Beet Curly Top Virus

<400> 28
accggatggc ccgaaaaaaa tgggcaccca atcaaaacgt gacacgtgga aggggactgt 60

tgaatgatgt gacgtttttg agcgggaaac ttcctgaaga agattcctgc gggaaacttc 120

ctgaagaaga ttcctttcag ataagatttg ttgactggtc aaaagaaggg gacaacttta 180

ttaaagtaac tttactttag taaaagtaaa gtaagtgtgc cccacaggaa acttgctcag 240

caagttttga attatgtcgt tttatatacg ttatttttac atgtatatgt aattataaat 300

gatggtctgt ctaccagact ggttattttt gctatttatc ttcagtattc tactgcaatc 360

aggtacgaac ttttatggga cctttcagag tggatcaatt tccagacaat tatccagcct 420

ttctagcagt atcgaccagt tgtttcttaa ggtacaacag gtggtgtata ctaggtatcc 480

atcaagagat agggtctctg accctagaag aaggcgaggt ctttcgtcaa ttccagaagg 540

aagtgaagaa gctactgagg tgtaaggtta attttcatag gaagtgttcg ttgtatgagg 600

aaatatacaa gaaatacgta tacaatgtcc cagaaaagaa aggtgaatcc tcaaagtgcg 660

tggccgaaga agaggaggac tagtacgact tcgaggaaat accaatggag gagacctgtg 720

acaaaaaaca ggactctgaa gttaaagatg tatgatgata tgttgggtgc tggtggtata 780

ggatctacca ttagtaataa tggtatgatt actatgttga ataattatgt ccagggtatt 840

ggtgatagtc agagagcgaa gaacgttact gtgacgaagc atttgaagtt tgatatggct 900

cttatgggga gttctcagtt ctgggagact cctaattata tgacccaata tcattggatt 960

atcatagaca aggatgttgg gtcagtgttt cctactaagt tatcgagtat atttgatatt 1020

cccgataacg gtcaggctat gccgtctact tatcgtattc gaagagatat gaacgagagg 1080

tttattggga agaagaaatg gagaactcat tggatgtcta ctggtactgg atatggaggg 1140

aaggagactt acaaggctcc ttcaatgcca aattacaaga aaccgatgaa tatcaatgtg 1200

cgcaatttga acatgaggac tatttggaaa gacaccggtg gtgggaagta tgaagacgtg 1260

aaggagaatg ctttactcta tgttgttgtt aatgataata cggataatac taatatgtat 1320

gccacattat ttggcaattg tagatgctat ttttattaat aatatttatt attaataatg 1380

aaattagtac aagttcattg caacactctg gttctgaatt acacttgata caaaagtaaa 1440

ttcttcatac aggacatgat ttaaacctct aattacatta ttaatacaaa ttaaacctag 1500

attatccaag tatatctcta gtctcttttt caagaccttc aagaaacggg gtccagtcaa 1560

tatcctgttc gatccagtta tcgtcagctc tatccagcac ttgtgaagat tcaacgctct 1620

ccggaggttg tagttgaatc tgatttgtat cttcagttga tagattggcc cgtatctctc 1680

gaaccatagg agtttcatat acagtggatt cggtacattc tcgaattcga cggaatttgt 1740

tgcctgatgg agagtaatag gttcccctgt gcgaaaatcc tctattacat tcatggtgta 1800

ttgtgaagtg gcagttacag ggtagagtca ccttgcgagg atgctgctgc ttggttatta 1860

aagagcggac cttggagttt gcagaaggtt gaattatgta atgtccagga cttaagggct 1920

tcattttctg gtttttcaag aaattgttgg tatgagctct caggtcctgg attgcataat 1980

ataatgcagg ggataccacc tttaatgaca cgtggttttc catactttaa gtttgtctgc 2040

cactcctttt gtgctccaat aaggtgtttc cagtgtttca tctttaagta agtgggatct 2100

acgtcatcaa tgacgttgta ttccacttca tcataatacg ttctagggct aaaatctaag 2160

tgccctgtga tgtaattgtg ggcccctaaa gatctagccc acatagtctt ccctgttctt 2220

gaatcaccct ctactatgat actattatat cttaaaggcc gcgcagcggc atcaaccccg 2280

aaatatgcat cagcccattc ttgcatttcc tcaggaacgt ttgtgaatga ggacagagga 2340

aatagtggag tccatggatc aggtggcctc tgaaatatct tttgagcatt gttcaaaaga 2400

ttgtgatgtt gaaggaagta atcctttggt tgttcttcct tcaatatttg aagtgcttgg 2460

tcaagagaag ttgcgtttaa cgccttggca tatgagtcgt tagctgtctg ttgacctcct 2520

ctagcacttc taccgtcgat ctggaattct ccccattcaa ttgtgtctcc gtccttgtcg 2580

acgtaggact tgacgtcgga gctggattta gctctctgaa tgtttggatg gaaatgtgct 2640

gacctggttg gggataccag gtcgaataat ctgatatttg tgatctgaac ttttccttcg 2700

agttgaagca ggacgtggag atgaggttgc ccatcttcgt gtagctctct ggcaatttta 2760

atatattttt tattagatga aagttgtatt ctttgaattt gctcaagagc ttcttctttt 2820

gaaagagaac actgaggata tgtaagaaat atgttttttg cttgaatacg aaatctttta 2880

gtaggaggca ttgtactccg atgacgtggc ttagcatatt aacatatcta ttggagtatt 2940

ggagtattat atatattagt acaactttca taagggccat ccgttataat att 2993

<210> 29
<211> 792
<212> DNA
<213> Artificial Sequence

<220>
<223> mGFP5-ER as expressed in the transgenic Nicotiana benthamiana 16c
      line [Philips et al., 2017]

<400> 29
atgaagacta atctttttct ctttctcatc ttttcacttc tcctatcatt atcctcggcc 60

gaattcagta aaggagaaga acttttcact ggagttgtcc caattcttgt tgaattagat 120

ggtgatgtta atgggtacaa attttctgtc agtggagagg gtgaaggtga tgcaacatac 180

ggaaaactta cccttaaatt tatttgcact actggaaaac tacctgttcc atggccaaca 240

cttgtcacta ctttctctta tggtgttcaa tgcttttcaa gatacccaga tcatatgaag 300

cggcacgact tcttcaagag cgccatgcct gagggatacg tgcaggagag gaccatcttc 360

ttcaaggacg acgggaacta caagacacgt gctgaagtca agtttgaggg agacaccctc 420

gtcaacagga tcgagcttaa gggaatcgat ttcaaggagg acggaaacat cctcggccac 480

aagttggaat acaactacaa ctcccacaac gtatacatca tggccgacaa gcaaaagaac 540

ggcatcaaag ccaacttcaa gacccgccac aacatcgaag acggcggcgt gcaactcgct 600

gatcattatc aacaaaatac tccaattggc gatggccctg tccttttacc agacaaccat 660

tacctgtcca cacaatctgc cctttcgaaa gatcccaacg aaaagagaga ccacatggtc 720

cttcttgagt ttgtaacagc tgctgggatt acacatggca tggatgaact atacaaacat 780

gatgagcttt aa 792

<210> 30
<211> 263
<212> PRT
<213> Artificial sequence

<220>
<223> mGFP5-ER protein sequence as expressed in the transgenic
      Nicotiana benthamiana 16c line [Philips et al., 2017]

<400> 30

Met Lys Thr Asn Leu Phe Leu Phe Leu Ile Phe Ser Leu Leu Leu Ser
1               5                   10                  15

Leu Ser Ser Ala Glu Phe Ser Lys Gly Glu Glu Leu Phe Thr Gly Val
            20                  25                  30

Val Pro Ile Leu Val Glu Leu Asp Gly Asp Val Asn Gly Tyr Lys Phe
        35                  40                  45

Ser Val Ser Gly Glu Gly Glu Gly Asp Ala Thr Tyr Gly Lys Leu Thr
    50                  55                  60

Leu Lys Phe Ile Cys Thr Thr Gly Lys Leu Pro Val Pro Trp Pro Thr
65                  70                  75                  80

Leu Val Thr Thr Phe Ser Tyr Gly Val Gln Cys Phe Ser Arg Tyr Pro
                85                  90                  95

Asp His Met Lys Arg His Asp Phe Phe Lys Ser Ala Met Pro Glu Gly
            100                 105                 110

Tyr Val Gln Glu Arg Thr Ile Phe Phe Lys Asp Asp Gly Asn Tyr Lys
        115                 120                 125

Thr Arg Ala Glu Val Lys Phe Glu Gly Asp Thr Leu Val Asn Arg Ile
    130                 135                 140

Glu Leu Lys Gly Ile Asp Phe Lys Glu Asp Gly Asn Ile Leu Gly His
145                 150                 155                 160

Lys Leu Glu Tyr Asn Tyr Asn Ser His Asn Val Tyr Ile Met Ala Asp

165                 170                 175

Lys Gln Lys Asn Gly Ile Lys Ala Asn Phe Lys Thr Arg His Asn Ile
            180                 185                 190

Glu Asp Gly Gly Val Gln Leu Ala Asp His Tyr Gln Gln Asn Thr Pro
        195                 200                 205

Ile Gly Asp Gly Pro Val Leu Leu Pro Asp Asn His Tyr Leu Ser Thr
    210                 215                 220

Gln Ser Ala Leu Ser Lys Asp Pro Asn Glu Lys Arg Asp His Met Val
225                 230                 235                 240

Leu Leu Glu Phe Val Thr Ala Ala Gly Ile Thr His Gly Met Asp Glu
                245                 250                 255

Leu Tyr Lys His Asp Glu Leu
            260

<210>  31
<211>  238
<212>  DNA
<213>  Artificial Sequence

<220>
<223>  GFP to YFP repair template

<400>  31
cttcaagacc cgccacaaca tcgaggacgg cggcgtgcaa ctcgctgatc attatcaaca      60

aaatactcca attggcgatg gccctgtcct gcttcctgat aatcactatc tcagctacca     120

gtcagctctg agcaaggacc ctaatgagaa gagtgaccac atggtccttc ttgagtttgt     180

aacagctgct gggattacac atggcatgga tgaactatac aaacatgatg agctttaa       238

<210>  32
<211>  17436
<212>  DNA
<213>  Artificial Sequence

<220>
<223>  pZFNnptII/LbCpf1_tDT-crRNA_mGFP#1+2+3_GFPtoYFP template

<400>  32
gggagctgca tgtgtcagag gttttcaccg tcatcaccga aacgcgcgag gcaggggtac      60

gtcgaggtcg atccaacccc tccgctgcta tagtgcagtc ggcttctgac gttcagtgca     120

gccgtcttct gaaaacgaca tgtcgcacaa gtcctaagtt acgcgacagg ctgccgccct     180

gcccttttcc tggcgttttc ttgtcgcgtg ttttagtcgc ataaagtaga atacttgcga      240

ctagaaccgg agacattacg ccatgaacaa gagcgccgcc gctggcctgc tgggctatgc     300

ccgcgtcagc accgacgacc aggacttgac caaccaacgg gccgaactgc acgcggccgg 360
ctgcaccaag ctgttttccg agaagatcac cggcaccagg cgcgaccgcc cggagctggc 420
caggatgctt gaccacctac gccctggcga cgttgtgaca gtgaccaggc tagaccgcct 480
ggcccgcagc acccgcgacc tactggacat tgccgagcgc atccaggagg ccggcgcggg 540
cctgcgtagc ctggcagagc cgtgggccga caccaccacg ccggccggcc gcatggtgtt 600
gaccgtgttc gccggcattg ccgagttcga gcgttcccta atcatcgacc gcacccggag 660
cgggcgcgag gccgccaagg cgcgaggcgt gaagtttggc ccccgcccta ccctcacccc 720
ggcacagatc gcgcacgccc gcgagctgat cgaccaggaa ggccgcaccg tgaaagaggc 780
ggctgcactg cttggcgtgc atcgctcgac cctgtaccgc gcacttgagc gcagcgagga 840
agtgacgccc accgaggcca ggcggcgcgg tgccttccgt gaggacgcat tgaccgaggc 900
cgacgccctg gcggccgccg agaatgaacg ccaagaggaa caagcatgaa accgcaccag 960
gacggccagg acgaaccgtt tttcattacc gaagagatcg aggcggagat gatcgcggcc 1020
gggtacgtgt tcgagccgcc cgcgcacgtc tcaaccgtgc ggctgcatga aatcctggcc 1080
ggtttgtctg atgccaagct cgcggcctgg ccggcgagct tggccgctga agaaaccgag 1140
cgccgccgtc taaaaaggtg atgtgtattt gagtaaaaca gcttgcgtca tgcggtcgct 1200
gcgtatatga tgcgatgagt aaataaacaa atacgcaagg ggaacgcatg aaggttatcg 1260
ctgtacttaa ccagaaaggc gggtcaggca agacgaccat cgcaacccat ctagcccgcg 1320
ccctgcaact cgccggggcc gatgttctgt tagtcgattc cgatccccag ggcagtgccc 1380
gcgattgggc ggccgtgcgg gaagatcaac cgctaaccgt tgtcggcatc gaccgcccga 1440
cgattgaccg cgacgtgaag gccatcggcc ggcgcgactt cgtagtgatc gacggagcgc 1500
cccaggcggc ggacttggct gtgtccgcga tcaaggcagc cgacttcgtg ctgattccgg 1560
tgcagccaag cccttacgac atatgggcca ccgccgacct ggtggagctg gttaagcagc 1620
gcattgaggt cacggatgga aggctacaag cggcctttgt cgtgtcgcgg gcgatcaaag 1680
gcacgcgcat cggcggtgag gttgccgagg cgctggccgg gtacgagctg cccattcttg 1740
agtcccgtat cacgcagcgc gtgagctacc caggcactgc cgccgccggc acaaccgttc 1800
ttgaatcaga acccgagggc gacgctgccc gcgaggtcca ggcgctggcc gctgaaatta 1860
aatcaaaact catttgagtt aatgaggtaa agagaaaatg agcaaaagca caaacacgct 1920
aagtgccggc cgtccgagcg cacgcagcag caaggctgca acgttggcca gcctggcaga 1980
cacgccagcc atgaagcggg tcaactttca gttgccggcg gaggatcaca ccaagctgaa 2040
gatgtacgcg gtacgccaag gcaagaccat taccgagctg ctatctgaat acatcgcgca 2100
gctaccagag taaatgagca aatgaataaa tgagtagatg aattttagcg gctaaaggag 2160

gcggcatgga aaatcaagaa caaccaggca ccgacgccgt ggaatgcccc atgtgtggag 2220

gaacgggcgg ttggccaggc gtaagcggct gggttgtctg ccggccctgc aatggcactg 2280

gaacccccaa gcccgaggaa tcggcgtgag cggtcgcaaa ccatccggcc cggtacaaat 2340

cggcgcggcg ctgggtgatg acctggtgga gaagttgaag gcggcgcagg ccgcccagcg 2400

gcaacgcatc gaggcagaag cacgccccgg tgaatcgtgg caagcggccg ctgatcgaat 2460

ccgcaaagaa tcccggcaac cgccggcagc cggtgcgccg tcgattagga agccgcccaa 2520

gggcgacgag caaccagatt ttttcgttcc gatgctctat gacgtgggca cccgcgatag 2580

tcgcagcatc atggacgtgg ccgttttccg tctgtcgaag cgtgaccgac gagctggcga 2640

ggtgatccgc tacgagcttc cagacgggca cgtagaggtt tccgcagggc cggccggcat 2700

ggcgagtgtg tgggattacg acctggtact gatggcggtt tcccatctaa ccgaatccat 2760

gaaccgatac cgggaaggga agggagacaa gcccggccgc gtgttccgtc cacacgttgc 2820

ggacgtactc aagttctgcc ggcgagccga tggcggaaag cagaaagacg acctggtaga 2880

aacctgcatt cggttaaaca ccacgcacgt tgccatgcag cgtacgaaga aggccaagaa 2940

cggccgcctg gtgacggtat ccgagggtga agccttgatt agccgctaca agatcgtaaa 3000

gagcgaaacc gggcggccgg agtacatcga gatcgagcta gctgattgga tgtaccgcga 3060

gatcacagaa ggcaagaacc cggacgtgct gacggttcac cccgattact ttttgatcga 3120

tcccggcatc ggccgttttc tctaccgcct ggcacgccgc gccgcaggca aggcagaagc 3180

cagatggttg ttcaagacga tctacgaacg cagtggcagc gccggagagt tcaagaagtt 3240

ctgtttcacc gtgcgcaagc tgatcgggtc aaatgacctg ccggagtacg atttgaagga 3300

ggaggcgggg caggctggcc cgatcctagt catgcgctac cgcaacctga tcgagggcga 3360

agcatccgcc ggttcctaat gtacggagca gatgctaggg caaattgccc tagcagggga 3420

aaaaggtcga aaaggtctct ttcctgtgga tagcacgtac attgggaacc caaagccgta 3480

cattgggaac cggaacccgt acattgggaa cccaaagccg tacattggga accggtcaca 3540

catgtaagtg actgatataa aagagaaaaa aggcgatttt tccgcctaaa actctttaaa 3600

acttattaaa actcttaaaa cccgcctggc ctgtgcataa ctgtctggcc agcgcacagc 3660

cgaagagctg caaaaagcgc ctacccttcg gtcgctgcgc tccctacgcc ccgccgcttc 3720

gcgtcggcct atcgcggccg ctggccgctc aaaaatggct ggcctacggc caggcaatct 3780

accagggcgc ggacaagccg cgccgtcgcc actcgaccgc cggcgcccac atcaaggcac 3840

cggtgggtat gcctgacgat gcgtggagac cgaaaccttg cgctcgttcg ccagccagga 3900

cagaaatgcc tcgacttcgc tgctgcccaa ggttgccggg tgacgcacac cgtggaaacg 3960

gatgaaggca cgaacccagt ggacataagc ctgttcggtt cgtaagctgt aatgcaagta 4020

gcgtatgcgc tcacgcaact ggtccagaac cttgaccgaa cgcagcggtg gtaacggcgc 4080

agtggcggtt ttcatggctt gttatgactg tttttttggg gtacagtcta tgcctcgggc 4140

atccaagcag caagcgcgtt acgccgtggg tcgatgtttg atgttatgga gcagcaacga 4200

tgttacgcag cagggcagtc gccctaaaac aaagttaaac atcatgaggg aagcggtgat 4260

cgccgaagta tcgactcaac tatcagaggt agttggcgtc atcgagcgcc atctcgaacc 4320

gacgttgctg gccgtacatt tgtacggctc cgcagtggat ggcggcctga agccacacag 4380

tgatattgat ttgctggtta cggtgaccgt aaggcttgat gaaacaacgc ggcgagcttt 4440

gatcaacgac cttttggaaa cttcggcttc ccctggagag agcgagattc tccgcgctgt 4500

agaagtcacc attgttgtgc acgacgacat cattccgtgg cgttatccag ctaagcgcga 4560

actgcaattt ggagaatggc agcgcaatga cattcttgca ggtatcttcg agccagccac 4620

gatcgacatt gatctggcta tcttgctgac aaaagcaaga gaacatagcg ttgccttggt 4680

aggtccagcg gcggaggaac tctttgatcc ggttcctgaa caggatctat ttgaggcgct 4740

aaatgaaacc ttaacgctat ggaactcgcc gcccgactgg gctggcgatg agcgaaatgt 4800

agtgcttacg ttgtcccgca tttggtacag cgcagtaacc ggcaaaatcg cgccgaagga 4860

tgtcgctgcc gactgggcaa tggagcgcct gccggcccag tatcagcccg tcatacttga 4920

agctagacag gcttatcttg gacaagaaga agatcgcttg gcctcgcgcg cagatcagtt 4980

ggaagaattt gtccactacg tgaaaggcga gatcaccaag gtagtcggca aataatgtct 5040

aacaattcgt tcaagccgac gccgcttcgc ggcgcggctt aactcaagcg ttagatgcac 5100

taagcacata attgctcaca gccaaactat caggtcaagt ctgcttttat tatttttaag 5160

cgtgcataat aagccctaca caaattggga gatatatcat gaaaggctgg ctttttcttg 5220

ttatcgcaat agttggcgaa gtaatcgcaa catagcttgc ttggtcgttc cgcgtgaacg 5280

tcggctcgat tgtacctgcg ttcaaatact ttgcgatcgt gttgcgcgcc tgcccggtgc 5340

gtcggctgat ctcacggatc gactgcttct ctcgcaacgc catccgacgg atgatgttta 5400

aaagtcccat gtggatcact ccgttgcccc gtcgctcacc gtgttggggg gaaggtgcac 5460

atggctcagt tctcaatgga aattatctgc ctaaccggct cagttctgcg tagaaaccaa 5520

catgcaagct ccaccgggtg caaagcggca gcggcggcag gatatattca attgtaaatg 5580

gcttcatgtc cgggaaatct acatggatca gcaatgagta tgatggtcaa tatggagaaa 5640

aagaaagagt aattaccaat tttttttcaa ttcaaaaatg tagatgtccg cagcgttatt 5700

ataaaatgaa agtacatttt gataaaacga caaattacga tccgtcgtat ttataggcga 5760

aagcaataaa caaattattc taattcggaa atctttattt cgacgtgtct acattcacgt 5820

ccaaatgggg gcttagatga gaaacttcac gatcggctct agttcgagcg atctagtaac 5880

atagatgaca ccgcgcgcga taatttatcc tagtttgcgc gctatatttt gttttctatc 5940

gcgtattaaa tgtataattg cgggactcta atcataaaaa cccatctcat aaataacgtc 6000

atgcattaca tgttaattat tacatgctta acgtaattca acagaaatta tatgataatc 6060

atcgcaagac cggcaacagg attcaatctt aagaaacttt attgccaaat gtttgaacga 6120

tcggggaaat tcgtttctat gcttcaattc tgctctatag atgttcttct agtcttaact 6180

tttagaaggc acaacatttg aaatgttggt aatttttctg ctgggtagtg aaccattgta 6240

atttgtgttt gcttatatta tccgttgggt ttctctatgt tatctgtggg gccatatccc 6300

agaactggtt gagtcggtcc aacacctggg tgccaatcat gtcgatggtg gggtatggtc 6360

cggattgaaa gaaatatagt ttaaatattt attgataaaa taacaagtca ggtattatag 6420

tccaagcaaa aacataaatt tattgatgca agtttaaatt cagaaatatt tcaataactg 6480

attatatcag ctggtacatt gccgtagatg aaagactgag tgcgatatta tgtgtaatac 6540

ataaattgat gatatagcta gcttagctca tcgggtcaga agaactcgtc aagaaggcga 6600

tagaaggcga tgcgctgcga atcgggagcg gcgataccgt aaagcacgag gaagcggtca 6660

gcccattcgc cgccaagctc ttcagcaata tcacgggtag ccaacgctat gtcctgatag 6720

cggtccgcca cacccagccg gccacagtcg atgaatccag aaaagcggcc attttccacc 6780

atgatattcg gcaagcaggc atcgccatga gtcacgacga gatcctcgcc gtcgggcatg 6840

cgcgccttga gcctggcgaa cagttcggct ggcgcgagcc cctgatgctc ttcgtccaga 6900

tcatcctgat cgacaagacc ggcttccatc cgagtacgtg ctcgctcgat gcgatgtttc 6960

gcttggtggt cgaatgggca ggtagccgga tcaagcgtat gcagccgccg cattgcatca 7020

gccatgatgg atactttctc ggcaggagca aggtgagatg acaggagatc ctgccccggc 7080

acttcgccca atagcagcca gtcccttccc gcttcagtga caacgtcgag cacagctgcg 7140

caaggaacgc ccgtcgtggc cagccacgat agccgcgctg cctcgtcctg aagttcattc 7200

agggcaccgg acaggtcggt cttgacaaaa agaaccgggc gcccctgcgc tgacagccgg 7260

aacacggcgg catcagagca gccgattgtc tgttgtgccc agtcatagcc gaatagcctc 7320

tccacccaag cggccggaga acctgcgtgc aatccatctt gttcaatcca agctcccatt 7380

ttctgtgatt ttggatgtgt ttttgtatgg attgagagtg aatatgagac tctaattgga 7440

taccgagggg aatttatgga acgtcagtgg agcatttttg acaagaaata tttgctagct 7500

gatagtgacc ttaggcgact tttgaacgcg caataatggt ttctgacgta tgtgcttagc 7560

tcattaaact ccagaaaccc gcggctgagt ggctccttca acgttgcggt tctgtcagtt 7620

ccaaacgtaa aacggcttgt cccgcgtcat cggcgggggt cattccggac tgtgggccca 7680

tatcccagaa ctggttgagt cggtccaaca cctgggtgcc aatcatgtcg atggtggggt 7740

atggccaatt ttttttcaat tcaaaaatgt agatgtccgc agcgttatta taaaatgaaa 7800

gtacattttg ataaaacgac aaattacgat ccgtcgtatt tataggcgaa agcaataaac 7860

aaattattct aattcggaaa tctttatttc gacgtgtcta cattcacgtc caaatggggg 7920

cttagatgag aaacttcacg atcggctcta gaggccatgg cggccgctcg agcgatctag 7980

taacatagat gacaccgcgc gcgataattt atcctagttt gcgcgctata ttttgttttc 8040

tatcgcgtat taaatgtata attgcgggac tctaatcata aaaacccatc tcataaataa 8100

cgtcatgcat tacatgttaa ttattacatg cttaacgtaa ttcaacagaa attatatgat 8160

aatcatcgca agaccggcaa caggattcaa tcttaagaaa ctttattgcc aaatgtttga 8220

acgatcgggg aaattcgatc tacacttagt agaaattatc tgccctttcg aaagatccca 8280

acatctacac ttagtagaaa ttacctgtcc acacaatctg ccctttcatc tacacttagt 8340

agaaattagt gtggacaggt aatggttgtc tgatctacac ttagtagaaa ttatcaggta 8400

cctctagact tgtacagctc gtccatgccg tacaggaaca ggtggtggcg gccctcggag 8460

cgctcgtact gttccacgat ggtgtagtcc tcgttgtggg aggtgatgtc cagcttggtg 8520

tccacgtagt agtagccggg cagttgcacg ggcttcttgg ccatgtagat ggtcttgaac 8580

tccaccaggt agtggccgcc gtccttcagc ttcagggcct ggtggatctc gcccttcagc 8640

acgccgtcgc gggggtacag gcgctcggtg gaggcctccc agcccatggt cttcttctgc 8700

attacggggc cgtcgggggg gaagttggtg ccgcgcatct tcaccttgta gatcagcgtg 8760

ccgtcctgca gggaggagtc ctgggtcacg gtcaccagac cgccgtcctc gaagttcatc 8820

acgcgctccc acttgaagcc ctcggggaag gacagcttct tgtaatcggg gatgtcggcg 8880

gggtgcttca cgtacgcctt ggagccgtac atgaactggg gggacaggat gtcccaggcg 8940

aagggcaggg ggccgccctt ggtcaccttc agcttggcgg tctgggtgcc ctcgtagggg 9000

cggccctcgc cctcgccctc gatctcgaac tcgtggccgt tcatggagcc ctccatgcgc 9060

accttgaagc gcatgaactc tttgatgacc tcctcgccct tgctcaccat ggatcccctc 9120

tccaaatgaa atgaacttcc ttatatagag gaagggtctt gcgaaggata gtgggattgt 9180

gcgtcatccc ttacgtcagt ggagatatca catcaatcca cttgctttga agacgtggtt 9240

ggaacgtctt ctttttccac gatgttcctc gtgggtgggg gtccatcttt gggaccactg 9300

tcggtagagg catcttgaac gatagccttt cctttatcgc aatgatggca tttgtagaag 9360

ccatcttcct tttctactgt cctttcgatg aagtgacaga tagctgggca atggaatccg 9420

aggaggtttc ccgatattac cctttgttga aaagtctcaa tagccctctg gtcttctgag 9480

actgtatctt tgatattctt ggagtagacg agagtgtcgt gctccaccat gtatcacatc 9540

aatccacttg ctttgaagac gtggttggaa cgtcttcttt ttccacgatg ttcctcgtgg 9600

gtgggggtcc atctttggga ccactgtcgg tagaggcatc ttgaacgata gcctttcctt 9660

tatcgcaatg atggcatttg tagaagccat cttccttttc tactgtcctt tcgatgaagt 9720

gacagatagc tgggcaatgg aatccgagga ggtttcccga tattaccctt tgttgaaaag 9780

tctcaatagc cctctggtct tctgaacact agtaaggcct taagggccag atcccccggg 9840

ctgcaggaat tcgatctggc acgacaggtt tcccgactgg aaagcgggca gtgagcgcaa 9900

cgcaattaat gtgagttagc tcactcatta ggcaccccag gctttacact ttatgcttcc 9960

ggctcgtatg ttgtgtggaa ttgtgagcgg ataacaattt cacacaggaa acagctatga 10020

catgattacg aattcaaaaa ttacggatat gaatataggc atatccgtat ccgaattatc 10080

cgtttgacag ctagcaacga ttgtacaatt gcttctttaa aaaaggaaga aagaaagaaa 10140

gaaaagaatc aacatcagcg ttaacaaacg gccccgttac ggcccaaacg gtcatataga 10200

gtaacggcgt taagcgttga aagactccta tcgaaatacg taaccgcaaa cgtgtcatag 10260

tcagatcccc tcttccttca ccgcctcaaa cacaaaaata atcttctaca gcctatatat 10320

acaacccccc cttctatctc tcctttctca caattcatca tctttctttc tctaccccca 10380

attttaagaa atcctctctt ctcctcttca ttttcaaggt aaatctctct ctctctctct 10440

ctctctgtta ttccttgttt taattaggta tgtattattg ctagtttgtt aatctgctta 10500

tcttatgtat gccttatgtg aatatcttta tcttgttcat ctcatccgtt tagaagctat 10560

aaatttgttg atttgactgt gtatctacac gtggttatgt ttatatctaa tcagatatga 10620

atttcttcat attgttgcgt ttgtgtgtac caatccgaaa tcgttgattt ttttcattta 10680

atcgtgtagc taattgtacg tatacatatg gatctacgta tcaattgttc atctgtttgt 10740

gtttgtatgt atacagatct gaaaacatca cttctctcat ctgattgtgt tgttacatac 10800

atagatatag atctgttata tcattttttt tattaattgt gtatatatat atgtgcatag 10860

atctggatta catgattgtg attatttaca tgattttgtt atttacgtat gtatatatgt 10920

agatctggac tttttggagt tgttgacttg attgtatttg tgtgtgtata tgtgtgttct 10980

gatcttgata tgttatgtat gtgcagcgaa ttcggcgcgc caaacaatgg cttcctccat 11040

ggctcctaag aagaagagga aggttagcaa gctcgagaag tttaccaact gctacagcct 11100

ctctaagacc ctcaggttca aggctatccc tgtgggaaag acccaagaga atatcgacaa 11160

caagaggctc ctcgtcgagg atgagaagag agctgaagat tacaagggcg tgaagaagct 11220

cctcgacagg tactacctca gcttcatcaa cgatgtgctc cacagcatca agctcaagaa 11280

cctcaacaac tacatcagcc tcttccgtaa gaaaaccagg accgagaaag agaacaaaga 11340

gcttgagaac ctcgagatca acctccgtaa agagatcgcc aaggctttca agggaaacga 11400

gggatacaag agcctcttca agaaggatat tatcgagaca atcctgcctg agttcctgga 11460

cgataaggat gagatcgctc tcgtgaacag cttcaacgga ttcactactg ccttcaccgg 11520

attcttcgac aacaggaaaa acatgttcag cgaagaggcc aagagcacct ctatcgcttt 11580

cagatgcatc aacgagaacc tcacgcgtta catcagcaac atggacatct tcgagaaggt 11640

ggacgccatc ttcgataagc acgaggtgca agaaatcaaa gagaagatcc tcaacagcga 11700

ctacgacgtc gaggactttt ttgaagggga gttcttcaac ttcgttctca cccaagaggg 11760

catcgacgtg tacaacgcta ttatcggagg attcgtgacc gagtctgggg agaagattaa 11820

gggactcaac gagtacatca acctgtacaa ccagaaaacg aagcagaagc tcccgaagtt 11880

caagccgctc tacaagcagg ttctctctga tcgtgagagc ctctcatttt acggtgaggg 11940

ttacacctct gacgaggaag tgcttgaggt tttccgtaac accctcaaca agaacagcga 12000

gatcttctcg tccatcaaga agttggagaa acttttcaag aacttcgacg agtacagcag 12060

cgctgggatc ttcgttaaga acggacctgc tatcagcacc atcagcaagg atattttcgg 12120

cgagtggaac gtgatcaggg acaagtggaa tgctgagtac gatgacatcc acctcaagaa 12180

gaaggctgtc gtcactgaga agtacgagga tgacaggcgt aagtcgttca agaagatcgg 12240

ctctttcagc ctcgagcagc ttcaagaata cgctgatgct gatctcagcg tggtcgagaa 12300

gctcaaagag atcatcatcc agaaggtcga cgagatctac aaggtgtacg ggtcctctga 12360

gaagttgttc gatgctgatt tcgtcctcga gaagagtctg aagaagaacg acgctgtcgt 12420

cgcgatcatg aaggatttgc tcgacagcgt gaagtccttc gagaactata tcaaggcctt 12480

cttcggagag ggcaaagaga ctaataggga cgagtctttc tacggggatt tcgtgctcgc 12540

ttacgatatc ctcctcaagg tggaccatat ctacgacgcc atcagaaact acgtgaccca 12600

gaagccttac agcaaggaca agttcaagtt gtactttcag aacccgcagt tcatgggcgg 12660

atgggacaaa gacaaagaga cagattacag ggccaccatc ctcaggtacg ggtctaagta 12720

ctacctggcc atcatggaca agaaatacgc caagtgcctc caaaagatcg acaaggatga 12780

cgtgaacggg aactatgaga agatcaacta caagctcctt ccgggaccga acaagatgct 12840

tcctaaggtg ttcttcagca agaaatggat ggcctactac aacccgtctg aggacatcca 12900

gaaaatctac aagaacggga ccttcaagaa aggcgacatg ttcaacctca acgactgcca 12960

caagctcatc gatttcttca aggacagcat ctcgcgttac ccgaagtggt ctaacgctta 13020

cgactttaac ttcagcgaga cagaaaagta caaggatatc gccgggttct accgtgaggt 13080

tgaggaacag ggttacaagg ttagcttcga gagcgcctcc aagaaagagg ttgacaagtt 13140

ggtcgaagag ggcaagctct acatgttcca gatctataac aaggacttct ccgacaagag 13200

ccacggaact cctaacctcc atacgatgta cttcaagctg cttttcgacg agaacaacca 13260

cgggcagatc agactttctg gtggtgctga actcttcatg cgtagggcct cactcaagaa 13320

agaagagttg gttgttcacc cggccaactc tccaatcgct aacaagaatc ctgacaaccc 13380

gaaaaagacc accacgctgt cttacgacgt ctacaaggac aaaaggttca gcgaggacca 13440

gtacgagctt catatcccga tcgctatcaa caagtgcccg aagaacatct tcaagatcaa 13500

taccgaggtg agggtgctgc tcaagcacga tgataaccct tacgtgatcg gaatcgatcg 13560
tggtgagaga aacctcctct acatcgttgt ggtggacgga aagggaaaca tcgtcgagca 13620
gtacagcctg aacgagatta tcaacaattt caacggcatc aggatcaaga ccgactacca 13680
ctcactcctc gataagaaag aaaaagagcg tttcgaggcc aggcagaact ggacttctat 13740
cgaaaacatc aaagagttga aggccggcta catctctcag gtggtgcata agatctgcga 13800
gctggtggaa aagtacgatg ctgtgatcgc tcttgaggac ctcaactctg ggttcaagaa 13860
cagtagagtg aaggttgaga agcaggtcta ccaaaagttc gagaagatgc tcatcgacaa 13920
gctcaactac atggtggaca aaaagagcaa cccttgcgct accggtggtg ctcttaaggg 13980
ataccagatc acgaacaagt tcgagtcctt caagagcatg agcacccaga acggcttcat 14040
cttctatatc cctgcttggc tcaccagcaa gatcgatcct tctactggtt tcgtgaacct 14100
gctcaagacc aagtacacct cgatcgccga cagcaagaag ttcatctcgt ctttcgacag 14160
gatcatgtac gtgccggaag aggatctttt cgagttcgct ctcgactata agaacttcag 14220
caggaccgac gccgactaca ttaagaagtg gaagctctac tcctacggga accgtatcag 14280
gatcttccga aatccgaaga aaaacaacgt gttcgactgg gaagaagtgt gcctcacctc 14340
tgcctacaaa gaactgttca acaagtacgg catcaactac cagcagggtg atatcagggc 14400
tcttttgtgc gagcagagcg acaaggcatt ctacagctca ttcatggccc tcatgtctct 14460
catgctccag atgaggaact ctatcaccgg aaggaccgat gtggacttcc ttatctctcc 14520
ggtcaagaac tctgacggga tcttctacga cagccgtaac tatgaggctc aagagaacgc 14580
tatcctgccg aagaatgctg atgcaaacgg ggcttacaac attgcgagaa aggttctctg 14640
ggctatcggg cagtttaaga aagcggaaga tgagaagctc gacaaggtga agatcgccat 14700
ctccaacaaa gagtggcttg agtacgctca gacctccgtt aagcacaaga ggcctgctgc 14760
tactaagaaa gctggccagg ccaaaaagaa gaagtgaggc gcgccgagct ccaggcctcc 14820
cagctttcgt ccgtatcatc ggtttcgaca acgttcgtca agttcaatgc atcagtttca 14880
ttgcccacac accagaatcc tactaagttt gagtattatg gcattggaaa agctgttttc 14940
ttctatcatt tgttctgctt gtaatttact gtgttctttc agtttttgtt ttcggacatc 15000
aaaatgcaaa tggatggata agagttaata aatgatatgg tccttttgtt cattctcaaa 15060
ttattattat ctgttgtttt tactttaatg ggttgaattt aagtaagaaa ggaactaaca 15120
gtgtgatatt aaggtgcaat gttagacata taaaacagtc tttcacctct ctttggttat 15180
gtcttgaatt ggtttgtttc ttcacttatc tgtgtaatca agtttactat gagtctatga 15240
tcaagtaatt atgcaatcaa gttaagtaca gtataggctt gagctcccta ggatcaagct 15300
atatagtcct gcaggtggaa aacaaggatg catatatgag ggcccatgaa cgatgcagag 15360
gcaatgccga tggtttcggg tcttcgagaa gaccttaatg cttcaagacc cgccacaaca 15420

tcgaggacgg cggcgtgcaa ctcgctgatc attatcaaca aaatactcca attggcgatg 15480

gccctgtcct gcttcctgat aatcactatc tcagctacca gtcagctctg agcaaggacc 15540

ctaatgagaa gagtgaccac atggtccttc ttgagtttgt aacagctgct gggattacac 15600

atggcatgga tgaactatac aaacatgatg agctttaact ttcggagacg gagctcagtc 15660

tgaccgcggc gtctcctaat agcttatcga taccgtcgac ctcgaggggg ggcccggtac 15720

ccgatcagat tgtcgtttcc cgccttcggt ttaaactatc agtgtttgac aggatatatt 15780

ggcgggtaaa cctaagagaa aagagcgttt attagaataa tcggatattt aaaagggcgt 15840

gaaaaggttt atccgttcgt ccatttgtat gtgcatgcca accacagggt tcccctcggg 15900

agtgcttggc attccgtgcg ataatgactt ctgttcaacc acccaaacgt cggaaagcct 15960

gacgacggag cagcattcca aaaagatccc ttggctcgtc tgggtcggct agaaggtcga 16020

gtgggctgct gtggcttgat ccctcaacgc ggtcgcggac gtagcgcagc gccgaaaaat 16080

cctcgatcgc aaatccgacg ctgtcgaaaa gcgtgatctg cttgtcgctc tttcggccga 16140

cgtcctggcc agtcatcacg cgccaaagtt ccgtcacagg atgatctggc gcgagttgct 16200

ggatctcgcc ttcaatccgg gtctgtggcg ggaactccac gaaaatatcc gaacgcagca 16260

agatatcgcg gtgcatctcg gtcttgcctg ggcagtcgcc gccgacgccg ttgatgtgga 16320

cgccgaaaag gatctaggtg aagatccttt ttgataatct catgaccaaa atcccttaac 16380

gtgagttttc gttccactga gcgtcagacc ccgtagaaaa gatcaaagga tcttcttgag 16440

atcctttttt tctgcgcgta atctgctgct tgcaaacaaa aaaaccaccg ctaccagcgg 16500

tggtttgttt gccggatcaa gagctaccaa ctctttttcc gaaggtaact ggcttcagca 16560

gagcgcagat accaaatact gttcttctag tgtagccgta gttaggccac cacttcaaga 16620

actctgtagc accgcctaca tacctcgctc tgctaatcct gttaccagtg gctgctgcca 16680

gtggcgataa gtcgtgtctt accgggttgg actcaagacg atagttaccg gataaggcgc 16740

agcggtcggg ctgaacgggg ggttcgtgca cacagcccag cttggagcga acgacctaca 16800

ccgaactgag atacctacag cgtgagctat gagaaagcgc cacgcttccc gaagggagaa 16860

aggcggacag gtatccggta agcggcaggg tcggaacagg agagcgcacg agggagcttc 16920

cagggggaaa cgcctggtat ctttatagtc ctgtcgggtt tcgccacctc tgacttgagc 16980

gtcgattttt gtgatgctcg tcaggggggc ggagcctatg gaaaaacgcc agcaacgcgg 17040

cctttttacg gttcctggcc ttttgctggc cttttgctca catgttcttt cctgcgttat 17100

cccctgattc tgtggataac cgattaccgc ctttgagtga gctgataccg ctcgccgcag 17160

ccgaacgacc gagcgcagcg agtcagtgag cgaggaagcg gaagagcgcc tgatgcggta 17220

ttttctcctt acgcatctgt gcggtatttc acaccgcata tggtgcactc tcagtacaat 17280

ctgctctgat gccgcatagt taagccagta tacactccgc tatcgctacg tgactgggtc 17340

atggctgcgc cccgacaccc gccaacaccc gctgacgcgc cctgacgggc ttgtctgctc 17400

ccggcatccg cttacagaca agctgtgacc gtctcc 17436

<210> 33
<211> 19726
<212> DNA
<213> Artificial Sequence

<220>
<223> pZFNnptII-BSCTV-Repl/LbCpf1_tDT-crRNA _mGFP#1+2+3 _GFPtoYFP
      Template

<400> 33
   ccggattgaa agaaatatag tttaaatatt tattgataaa ataacaagtc aggtattata 60
   gtccaagcaa aaacataaat ttattgatgc aagtttaaat tcagaaatat ttcaataact 120
   gattatatca gctggtacat tgccgtagat gaaagactga gtgcgatatt atgtgtaata 180
   cataaattga tgatatagct agcttagctc atcgggtcag aagaactcgt caagaaggcg 240
   atagaaggcg atgcgctgcg aatcgggagc ggcgataccg taaagcacga ggaagcggtc 300
   agcccattcg ccgccaagct cttcagcaat atcacgggta gccaacgcta tgtcctgata 360
   gcggtccgcc acacccagcc ggccacagtc gatgaatcca gaaaagcggc cattttccac 420
   catgatattc ggcaagcagg catcgccatg agtcacgacg agatcctcgc cgtcgggcat 480
   gcgcgccttg agcctggcga acagttcggc tggcgcgagc ccctgatgct cttcgtccag 540
   atcatcctga tcgacaagac cggcttccat ccgagtacgt gctcgctcga tgcgatgttt 600
   cgcttggtgg tcgaatgggc aggtagccgg atcaagcgta tgcagccgcc gcattgcatc 660
   agccatgatg gatactttct cggcaggagc aaggtgagat gacaggagat cctgccccgg 720
   cacttcgccc aatagcagcc agtcccttcc cgcttcagtg acaacgtcga gcacagctgc 780
   gcaaggaacg cccgtcgtgg ccagccacga tagccgcgct gcctcgtcct gaagttcatt 840
   cagggcaccg gacaggtcgg tcttgacaaa aagaaccggg cgcccctgcg ctgacagccg 900
   gaacacggcg gcatcagagc agccgattgt ctgttgtgcc cagtcatagc cgaatagcct 960
   ctccacccaa gcggccggag aacctgcgtg caatccatct tgttcaatcc aagctcccat 1020
   tttctgtgat tttggatgtg tttttgtatg gattgagagt gaatatgaga ctctaattgg 1080
   ataccgaggg gaatttatgg aacgtcagtg gagcattttt gacaagaaat atttgctagc 1140
   tgatagtgac cttaggcgac ttttgaacgc gcaataatgg tttctgacgt atgtgcttag 1200
   ctcattaaac tccagaaacc cgcggctgag tggctccttc aacgttgcgg ttctgtcagt 1260
   tccaaacgta aaacggcttg tcccgcgtca tcggcggggg tcattccgga ctgtggggcc 1320
   atatcccaga actggttgag tcggtccaac acctgggtgc caatcatgtc gatggtgggg 1380
   tatggccaat tttttttcaa ttcaaaaatg tagatgtccg cagcgttatt ataaaatgaa 1440
   agtacatttt gataaaacga caaattacga tccgtcgtat ttataggcga aagcaataaa 1500
   caaattattc taattcggaa atctttattt cgacgtgtct acattcacgt ccaaatgggg 1560
   gcttagatga gaaacttcac gatcggctct agaggccatg ggtcgacatt gaatcgggct 1620
   ctcttcaaat cccctatcaa ttgggtgctt tgggtgctct taaataccac caaggggcca 1680
   tccgcattaa tattaccgga tggcccccaa aaaatacgtg gcccaatgaa aatatgccac 1740
   gtggaaagct aaagatgttg atgtgatgga tttgggcggg aaacttcctg aagaagtttt 1800
   cattcccgcc cctttttttg acttagtcaa aaaagtagcc gacatactgt ttgaaataaa 1860
   gttaaaagta gatcattgtg gtccccacaa gaaacttgct aagcaagttt cgaattattt 1920
   ctgtttaaat acgttatttt tacatgtata tgtacttata aactagtggc cttggcggcc 1980
   gctcgagcga tctagtaaca tagatgacac cgcgcgcgat aatttatcct agtttgcgcg 2040
   ctatattttg ttttctatcg cgtattaaat gtataattgc gggactctaa tcataaaaac 2100
   ccatctcata aataacgtca tgcattacat gttaattatt acatgcttaa cgtaattcaa 2160
   cagaaattat atgataatca tcgcaagacc ggcaacagga ttcaatctta agaaacttta 2220
   ttgccaaatg tttgaacgat cggggaaatt cgatctacac ttagtagaaa ttatctgccc 2280
   tttcgaaaga tcccaacatc tacacttagt agaaattacc tgtccacaca atctgccctt 2340
   tcatctacac ttagtagaaa ttagtgtgga caggtaatgg ttgtctgatc tacacttagt 2400
   agaaattatc aggtacctct agacttgtac agctcgtcca tgccgtacag gaacaggtgg 2460
   tggcggccct cggagcgctc gtactgttcc acgatggtgt agtcctcgtt gtgggaggtg 2520
   atgtccagct tggtgtccac gtagtagtag ccgggcagtt gcacgggctt cttggccatg 2580
   tagatggtct tgaactccac caggtagtgg ccgccgtcct tcagcttcag ggcctggtgg 2640
   atctcgccct tcagcacgcc gtcgcggggg tacaggcgct cggtggaggc ctcccagccc 2700
   atggtcttct tctgcattac ggggccgtcg ggggggaagt tggtgccgcg catcttcacc 2760
   ttgtagatca gcgtgccgtc ctgcagggag gagtcctggg tcacggtcac cagaccgccg 2820
   tcctcgaagt tcatcacgcg ctcccacttg aagccctcgg ggaaggacag cttcttgtaa 2880
   tcggggatgt cggcggggtg cttcacgtac gccttggagc cgtacatgaa ctggggggac 2940
   aggatgtccc aggcgaaggg cagggggccg cccttggtca ccttcagctt ggcggtctgg 3000
   gtgccctcgt aggggcggcc ctcgccctcg ccctcgatct cgaactcgtg gccgttcatg 3060
   gagccctcca tgcgcacctt gaagcgcatg aactctttga tgacctcctc gcccttgctc 3120
   accatggatc ccctctccaa atgaaatgaa cttccttata tagaggaagg gtcttgcgaa 3180
   ggatagtggg attgtgcgtc atcccttacg tcagtggaga tatcacatca atccacttgc 3240
   tttgaagacg tggttggaac gtcttctttt tccacgatgt tcctcgtggg tgggggtcca 3300
   tctttgggac cactgtcggt agaggcatct tgaacgatag cctttccttt atcgcaatga 3360
   tggcatttgt agaagccatc ttccttttct actgtccttt cgatgaagtg acagatagct 3420
   gggcaatgga atccgaggag gtttcccgat attacccttt gttgaaaagt ctcaatagcc 3480
   ctctggtctt ctgagactgt atctttgata ttcttggagt agacgagagt gtcgtgctcc 3540
   accatgtatc acatcaatcc acttgctttg aagacgtggt tggaacgtct tctttttcca 3600
   cgatgttcct cgtgggtggg ggtccatctt tgggaccact gtcggtagag gcatcttgaa 3660
   cgatagcctt tcctttatcg caatgatggc atttgtagaa gccatcttcc ttttctactg 3720
   tcctttcgat gaagtgacag atagctgggc aatggaatcc gaggaggttt cccgatatta 3780
   ccctttgttg aaaagtctca atagccctct ggtcttctga acactagtaa ggccttaagg 3840
   gccacccggg ctgcaggaat tcgatctggc acgacaggtt tcccgactgg aaagcgggca 3900
   gtgagcgcaa cgcaattaat gtgagttagc tcactcatta ggcaccccag gctttacact 3960
   ttatgcttcc ggctcgtatg ttgtgtggaa ttgtgagcgg ataacaattt cacacaggaa 4020
   acagctatga catgattacg aattcaaaaa ttacggatat gaatataggc atatccgtat 4080
   ccgaattatc cgtttgacag ctagcaacga ttgtacaatt gcttctttaa aaaaggaaga 4140
   aagaaagaaa gaaaagaatc aacatcagcg ttaacaaacg gccccgttac ggcccaaacg 4200
   gtcatataga gtaacggcgt taagcgttga aagactccta tcgaaatacg taaccgcaaa 4260
   cgtgtcatag tcagatcccc tcttccttca ccgcctcaaa cacaaaaata atcttctaca 4320
   gcctatatat acaacccccc cttctatctc tcctttctca caattcatca tctttctttc 4380
   tctaccccca attttaagaa atcctctctt ctcctcttca ttttcaaggt aaatctctct 4440
   ctctctctct ctctctgtta ttccttgttt taattaggta tgtattattg ctagtttgtt 4500
   aatctgctta tcttatgtat gccttatgtg aatatcttta tcttgttcat ctcatccgtt 4560
   tagaagctat aaatttgttg atttgactgt gtatctacac gtggttatgt ttatatctaa 4620
   tcagatatga atttcttcat attgttgcgt ttgtgtgtac caatccgaaa tcgttgattt 4680
   ttttcattta atcgtgtagc taattgtacg tatacatatg gatctacgta tcaattgttc 4740
   atctgtttgt gtttgtatgt atacagatct gaaaacatca cttctctcat ctgattgtgt 4800
   tgttacatac atagatatag atctgttata tcattttttt tattaattgt gtatatatat 4860
   atgtgcatag atctggatta catgattgtg attatttaca tgattttgtt atttacgtat 4920
   gtatatatgt agatctggac tttttggagt tgttgacttg attgtatttg tgtgtgtata 4980
   tgtgtgttct gatcttgata tgttatgtat gtgcagcgaa ttcggcgcgc caaacaatgg 5040
   cttcctccat ggctcctaag aagaagagga aggttagcaa gctcgagaag tttaccaact 5100
   gctacagcct ctctaagacc ctcaggttca aggctatccc tgtgggaaag acccaagaga 5160
   atatcgacaa caagaggctc ctcgtcgagg atgagaagag agctgaagat tacaagggcg 5220
   tgaagaagct cctcgacagg tactacctca gcttcatcaa cgatgtgctc cacagcatca 5280
   agctcaagaa cctcaacaac tacatcagcc tcttccgtaa gaaaaccagg accgagaaag 5340
   agaacaaaga gcttgagaac ctcgagatca acctccgtaa agagatcgcc aaggctttca 5400
   agggaaacga gggatacaag agcctcttca agaaggatat tatcgagaca atcctgcctg 5460
   agttcctgga cgataaggat gagatcgctc tcgtgaacag cttcaacgga ttcactactg 5520
   ccttcaccgg attcttcgac aacagggaaa acatgttcag cgaagaggcc aagagcacct 5580
   ctatcgcttt cagatgcatc aacgagaacc tcacgcgtta catcagcaac atggacatct 5640
   tcgagaaggt ggacgccatc ttcgataagc acgaggtgca agaaatcaaa gagaagatcc 5700
   tcaacagcga ctacgacgtc gaggactttt ttgaagggga gttcttcaac ttcgttctca 5760
   cccaagaggg catcgacgtg tacaacgcta ttatcggagg attcgtgacc gagtctgggg 5820
   agaagattaa gggactcaac gagtacatca acctgtacaa ccagaaaacg aagcagaagc 5880
   tcccgaagtt caagccgctc tacaagcagg ttctctctga tcgtgagagc ctctcatttt 5940
   acggtgaggg ttacacctct gacgaggaag tgcttgaggt tttccgtaac accctcaaca 6000
   agaacagcga gatcttctcg tccatcaaga agttggagaa acttttcaag aacttcgacg 6060
   agtacagcag cgctgggatc ttcgttaaga acggacctgc tatcagcacc atcagcaagg 6120
   atattttcgg cgagtggaac gtgatcaggg acaagtggaa tgctgagtac gatgacatcc 6180
   acctcaagaa gaaggctgtc gtcactgaga agtacgagga tgacaggcgt aagtcgttca 6240
   agaagatcgg ctctttcagc ctcgagcagc ttcaagaata cgctgatgct gatctcagcg 6300
   tggtcgagaa gctcaaagag atcatcatcc agaaggtcga cgagatctac aaggtgtacg 6360
   ggtcctctga gaagttgttc gatgctgatt tcgtcctcga gaagagtctg aagaagaacg 6420
   acgctgtcgt cgcgatcatg aaggatttgc tcgacagcgt gaagtccttc gagaactata 6480
   tcaaggcctt cttcggagag ggcaaagaga ctaataggga cgagtctttc tacggggatt 6540
   tcgtgctcgc ttacgatatc ctcctcaagg tggaccatat ctacgacgcc atcagaaact 6600
   acgtgaccca gaagccttac agcaaggaca agttcaagtt gtactttcag aacccgcagt 6660
   tcatgggcgg atgggacaaa gacaaagaga cagattacag ggccaccatc ctcaggtacg 6720
   ggtctaagta ctacctggcc atcatggaca agaaatacgc caagtgcctc caaaagatcg 6780
   acaaggatga cgtgaacggg aactatgaga agatcaacta caagctcctt ccgggaccga 6840
   acaagatgct tcctaaggtg ttcttcagca agaaatggat ggcctactac aacccgtctg 6900
   aggacatcca gaaaatctac aagaacggga ccttcaagaa aggcgacatg ttcaacctca 6960
   acgactgcca caagctcatc gatttcttca aggacagcat ctcgcgttac ccgaagtggt 7020
   ctaacgctta cgactttaac ttcagcgaga cagaaaagta caaggatatc gccgggttct 7080
   accgtgaggt tgaggaacag ggttacaagg ttagcttcga gagcgcctcc aagaaagagg 7140
   ttgacaagtt ggtcgaagag ggcaagctct acatgttcca gatctataac aaggacttct 7200
   ccgacaagag ccacggaact cctaacctcc atacgatgta cttcaagctg cttttcgacg 7260
   agaacaacca cgggcagatc agactttctg gtggtgctga actcttcatg cgtagggcct 7320
   cactcaagaa agaagagttg gttgttcacc cggccaactc tccaatcgct aacaagaatc 7380
   ctgacaaccc gaaaaagacc accacgctgt cttacgacgt ctacaaggac aaaaggttca 7440
   gcgaggacca gtacgagctt catatcccga tcgctatcaa caagtgcccg aagaacatct 7500
   tcaagatcaa taccgaggtg agggtgctgc tcaagcacga tgataaccct tacgtgatcg 7560
   gaatcgatcg tggtgagaga aacctcctct acatcgttgt ggtggacgga aagggaaaca 7620
   tcgtcgagca gtacagcctg aacgagatta tcaacaattt caacggcatc aggatcaaga 7680
   ccgactacca ctcactcctc gataagaaag aaaaagagcg tttcgaggcc aggcagaact 7740
   ggacttctat cgaaaacatc aaagagttga aggccggcta catctctcag gtggtgcata 7800
   agatctgcga gctggtggaa aagtacgatg ctgtgatcgc tcttgaggac ctcaactctg 7860
   ggttcaagaa cagtagagtg aaggttgaga agcaggtcta ccaaaagttc gagaagatgc 7920
   tcatcgacaa gctcaactac atggtggaca aaaagagcaa cccttgcgct accggtggtg 7980
   ctcttaaggg ataccagatc acgaacaagt tcgagtcctt caagagcatg agcacccaga 8040
   acggcttcat cttctatatc cctgcttggc tcaccagcaa gatcgatcct tctactggtt 8100
   tcgtgaacct gctcaagacc aagtacacct cgatcgccga cagcaagaag ttcatctcgt 8160
   ctttcgacag gatcatgtac gtgccggaag aggatctttt cgagttcgct ctcgactata 8220
   agaacttcag caggaccgac gccgactaca ttaagaagtg gaagctctac tcctacggga 8280
   accgtatcag gatcttccga aatccgaaga aaaacaacgt gttcgactgg gaagaagtgt 8340
   gcctcacctc tgcctacaaa gaactgttca acaagtacgg catcaactac cagcagggtg 8400
   atatcagggc tcttttgtgc gagcagagcg acaaggcatt ctacagctca ttcatggccc 8460
   tcatgtctct catgctccag atgaggaact ctatcaccgg aaggaccgat gtggacttcc 8520
   ttatctctcc ggtcaagaac tctgacggga tcttctacga cagccgtaac tatgaggctc 8580
   aagagaacgc tatcctgccg aagaatgctg atgcaaacgg ggcttacaac attgcgagaa 8640
   aggttctctg ggctatcggg cagtttaaga aagcggaaga tgagaagctc gacaaggtga 8700
   agatcgccat ctccaacaaa gagtggcttg agtacgctca gacctccgtt aagcacaaga 8760
   ggcctgctgc tactaagaaa gctggccagg ccaaaaagaa gaagtgaggc gcgccgagct 8820
   ccaggcctcc cagctttcgt ccgtatcatc ggtttcgaca acgttcgtca agttcaatgc 8880
   atcagtttca ttgcccacac accagaatcc tactaagttt gagtattatg gcattggaaa 8940
   agctgttttc ttctatcatt tgttctgctt gtaatttact gtgttctttc agtttttgtt 9000
   ttcggacatc aaaatgcaaa tggatggata agagttaata aatgatatgg tccttttgtt 9060
   cattctcaaa ttattattat ctgttgtttt tactttaatg ggttgaattt aagtaagaaa 9120
   ggaactaaca gtgtgatatt aaggtgcaat gttagacata taaaacagtc tttcacctct 9180
   ctttggttat gtcttgaatt ggtttgtttc ttcacttatc tgtgtaatca agtttactat 9240
   gagtctatga tcaagtaatt atgcaatcaa gttaagtaca gtataggctt gagctcccta 9300
   ggatcaagct atatagtcct gcaggtggaa aacaaggatg catatatgag ggcccatgaa 9360
   cgatgcagag gcaatgccga tggtttcggg tcttcgagaa gaccttaatg cttcaagacc 9420
   cgccacaaca tcgaggacgg cggcgtgcaa ctcgctgatc attatcaaca aaatactcca 9480
   attggcgatg gccctgtcct gcttcctgat aatcactatc tcagctacca gtcagctctg 9540
   agcaaggacc ctaatgagaa gagtgaccac atggtccttc ttgagtttgt aacagctgct 9600
   gggattacac atggcatgga tgaactatac aaacatgatg agctttaact ttcggagacg 9660
   gagctcagtc tgaccgcggc gtctcctaat agcttggtac cactagtcgg ataatactaa 9720
   tatgtatgcc actttgtttg gcaattgtag atgttatttt tattaataaa aatattattt 9780
   ttttaataca atttcattgc aacactctgg ttctgaatta cactagatac aaaattaaat 9840
   tcttcataca ggacatgatt taaacctcta attacattat taatacaaat taaacctaaa 9900
   ttatccaagt atatctctag tctctttttc aagactttca aaaaacggtc cccagttaat 9960
   gtcctgtgcg atccagtgat cgtcaaatct atccagcact tgtgaagatt caagtatttg 10020
   cggaggttgt ggttgaatct tatctggact ttcagttgat aaactggccc ggaacggaag 10080
   aagtcctgga gtttgaggta caatggattc ggtaccaagt ccacgggtat ggaattcgtc 10140
   gcttgttgca gcgtgatggg ttcctctgtg cgaaaatccc tgattacatt catgatgaat 10200
   tgtgaaatga cacttacaag gaagtttgat cttgcgagga cgcttcttga ttgttatcaa 10260
   agagaggact tgtgagtttg gcgaagactg aattttgtaa tgtccaggac ctaagggctt 10320
   cattttctga tttattgagg aagtcctggt aagagctgcc ttcgcctgga ttgcataata 10380
   taatactggg aataccacct ttaatgacac gtggttttcc atactttaag tttgtctgcc 10440
   actctctttg tgcgcctata aggtgcttcc aatgcttcat ctttaagtaa ttgggatcta 10500
   cgtcatcaat gacgttatac agaacattat cattataagt ttttaaacta aaatctaaat 10560
   gaccagtaat ataattatgt ggacctaaac acctggccca cattgtttta ccagttcttg 10620
   aatcaccctc tatgattaaa ctattatatc taaaaggccg cgcagcggga tccaaaccaa 10680
   aataagaatc ggcccactct tgaataattt ctggaactct agtgaaagaa gaaagaggaa 10740
   aaggtggttg ataaagatct ggaggaggaa gaaaaatagc ctctaagtta ggtttgaggt 10800
   tgtgatactg aaaaatatat ttttctggga gtttctccct tattatctgc agagcttcta 10860
   atgcattacc tgcatttaat gcctctgctg cagcatcatt agccgtctgt tgacctccgc 10920
   gtgcagatct tccatcgacc tgaaattcac cccagtcgat gtgatctccg tcctttgaga 10980
   cgtaggactt gacgtcggaa ctggatttag ctccctgaat attgcagtgg aattgtttgc 11040
   tggtacttcg atgttgcaga tcgaagtaac gggcattacg gatctggact tttccttcga 11100
   attgaataag ggcatgcaga tgtggttccc cattttcatg taattctctg cagatgcgaa 11160
   tatatttttt attcgaaggt gtatttatag cgaggagctg ttctaaggcg tcttctttgg 11220
   ttactgaaca ttgagggtat gtaaggaaaa aatttttggc ttttttgtaa aaaggcatat 11280
   tgaatcgggc tctcttcaaa tcccctatca attgggtgct ttgggtgctc ttaaatacca 11340
   ccaaggggcc atccgcatta atattaccgg atggccccca aaaaatacgt ggcccaatga 11400
   aaatatgcca cgtggaaagc taaagatgtt gatgtgatgg atttgggcgg gaaacttcct 11460
   gaagaagttt tcattcccgc cccttttttt gacttagtca aaaaagtagc cgacatactg 11520
   tttgaaataa agttaaaagt agatcattgt ggtccccaca agaaacttgc taagcaagtt 11580
   tcgaattatt tctgtttaaa tacgttattt ttacatgtat atgtacttat aagtttaaac 11640
   gggcccggta cccgatcaga ttgtcgtttc ccgccttcgg tttaaactat cagtgtttga 11700
   caggatatat tggcgggtaa acctaagaga aaagagcgtt tattagaata atcggatatt 11760
   taaaagggcg tgaaaaggtt tatccgttcg tccatttgta tgtgcatgcc aaccacaggg 11820
   ttcccctcgg gagtgcttgg cattccgtgc gataatgact tctgttcaac cacccaaacg 11880
   tcggaaagcc tgacgacgga gcagcattcc aaaaagatcc cttggctcgt ctgggtcggc 11940
   tagaaggtcg agtgggctgc tgtggcttga tccctcaacg cggtcgcgga cgtagcgcag 12000
   cgccgaaaaa tcctcgatcg caaatccgac gctgtcgaaa agcgtgatct gcttgtcgct 12060
   ctttcggccg acgtcctggc cagtcatcac gcgccaaagt tccgtcacag gatgatctgg 12120
   cgcgagttgc tggatctcgc cttcaatccg ggtctgtggc gggaactcca cgaaaatatc 12180
   cgaacgcagc aagatatcgc ggtgcatctc ggtcttgcct gggcagtcgc cgccgacgcc 12240
   gttgatgtgg acgccgaaaa ggatctaggt gaagatcctt tttgataatc tcatgaccaa 12300
   aatcccttaa cgtgagtttt cgttccactg agcgtcagac cccgtagaaa agatcaaagg 12360
   atcttcttga gatccttttt ttctgcgcgt aatctgctgc ttgcaaacaa aaaaaccacc 12420
   gctaccagcg gtggtttgtt tgccggatca agagctacca actctttttc cgaaggtaac 12480
   tggcttcagc agagcgcaga taccaaatac tgttcttcta gtgtagccgt agttaggcca 12540
   ccacttcaag aactctgtag caccgcctac atacctcgct ctgctaatcc tgttaccagt 12600
   ggctgctgcc agtggcgata agtcgtgtct taccgggttg gactcaagac gatagttacc 12660
   ggataaggcg cagcggtcgg gctgaacggg gggttcgtgc acacagccca gcttggagcg 12720
   aacgacctac accgaactga gatacctaca gcgtgagcta tgagaaagcg ccacgcttcc 12780
   cgaagggaga aaggcggaca ggtatccggt aagcggcagg gtcggaacag gagagcgcac 12840
   gagggagctt ccagggggaa acgcctggta tctttatagt cctgtcgggt ttcgccacct 12900
   ctgacttgag cgtcgatttt tgtgatgctc gtcagggggg cggagcctat ggaaaaacgc 12960
   cagcaacgcg gcctttttac ggttcctggc cttttgctgg ccttttgctc acatgttctt 13020
   tcctgcgtta tcccctgatt ctgtggataa ccgtattacc gcctttgagt agctgatacc 13080
   gctcgccgca gccgaacgac cgagcgcagc gagtcagtga gcgaggaagc ggaagagcgc 13140
   ctgatgcggt attttctcct tacgcatctg tgcggtattt cacaccgcat atggtgcact 13200
   ctcagtacaa tctgctctga tgccgcatag ttaagccagt atacactccg ctatcgctac 13260
   gtgactgggt catggctgcg ccccgacacc cgccaacacc cgctgacgcg ccctgacggg 13320
   cttgtctgct cccggcatcc gcttacagac aagctgtgac cgtctccggg agctgcatgt 13380
   gtcagaggtt ttcaccgtca tcaccgaaac gcgcgaggca ggggtacgtc gaggtcgatc 13440
   caacccctcc gctgctatag tgcagtcggc ttctgacgtt cagtgcagcc gtcttctgaa 13500
   aacgacatgt cgcacaagtc ctaagttacg cgacaggctg ccgccctgcc cttttcctgg 13560
   cgttttcttg tcgcgtgttt tagtcgcata aagtagaata cttgcgacta gaaccggaga 13620
   cattacgcca tgaacaagag cgccgccgct ggcctgctgg gctatgcccg cgtcagcacc 13680
   gacgaccagg acttgaccaa ccaacgggcc gaactgcacg cggccggctg caccaagctg 13740
   ttttccgaga agatcaccgg caccaggcgc gaccgcccgg agctggccag gatgcttgac 13800
   cacctacgcc ctggcgacgt tgtgacagtg accaggctag accgcctggc ccgcagcacc 13860
   cgcgacctac tggacattgc cgagcgcatc caggaggccg gcgcgggcct gcgtagcctg 13920
   gcagagccgt gggccgacac caccacgccg gccggccgca tggtgttgac cgtgttcgcc 13980
   ggcattgccg agttcgagcg ttccctaatc atcgaccgca cccggagcgg gcgcgaggcc 14040
   gccaaggcgc gaggcgtgaa gtttggcccc cgccctaccc tcaccccggc acagatcgcg 14100
   cacgcccgcg agctgatcga ccaggaaggc cgcaccgtga aagaggcggc tgcactgctt 14160
   ggcgtgcatc gctcgaccct gtaccgcgca cttgagcgca gcgaggaagt gacgcccacc 14220
   gaggccaggc ggcgcggtgc cttccgtgag gacgcattga ccgaggccga cgccctggcg 14280
   gccgccgaga atgaacgcca agaggaacaa gcatgaaacc gcaccaggac ggccaggacg 14340
   aaccgttttt cattaccgaa gagatcgagg cggagatgat cgcggccggg tacgtgttcg 14400
   agccgcccgc gcacgtctca accgtgcggc tgcatgaaat cctggccggt ttgtctgatg 14460
   ccaagctcgc ggcctggccg gcgagcttgg ccgctgaaga aaccgagcgc cgccgtctaa 14520
   aaaggtgatg tgtatttgag taaaacagct tgcgtcatgc ggtcgctgcg tatatgatgc 14580
   gatgagtaaa taaacaaata cgcaagggga acgcatgaag gttatcgctg tacttaacca 14640
   gaaaggcggg tcaggcaaga cgaccatcgc aacccatcta gcccgcgccc tgcaactcgc 14700
   cggggccgat gttctgttag tcgattccga tccccagggc agtgcccgcg attgggcggc 14760
   cgtgcgggaa gatcaaccgc taaccgttgt cggcatcgac cgcccgacga ttgaccgcga 14820
   cgtgaaggcc atcggccggc gcgacttcgt agtgatcgac ggagcgcccc aggcggcgga 14880
   cttggctgtg tccgcgatca aggcagccga cttcgtgctg attccggtgc agccaagccc 14940
   ttacgacata tgggccaccg ccgacctggt ggagctggtt aagcagcgca ttgaggtcac 15000
   ggatggaagg ctacaagcgg cctttgtcgt gtcgcgggcg atcaaaggca cgcgcatcgg 15060
   cggtgaggtt gccgaggcgc tggccgggta cgagctgccc attcttgagt cccgtatcac 15120
   gcagcgcgtg agctacccag gcactgccgc cgccggcaca accgttcttg aatcagaacc 15180
   cgagggcgac gctgcccgcg aggtccaggc gctggccgct gaaattaaat caaaactcat 15240
   ttgagttaat gaggtaaaga gaaaatgagc aaaagcacaa acacgctaag tgccggccgt 15300
   ccgagcgcac gcagcagcaa ggctgcaacg ttggccagcc tggcagacac gccagccatg 15360
   aagcgggtca actttcagtt gccggcggag gatcacacca agctgaagat gtacgcggta 15420
   cgccaaggca agaccattac cgagctgcta tctgaataca tcgcgcagct accagagtaa 15480
   atgagcaaat gaataaatga gtagatgaat tttagcggct aaaggaggcg gcatggaaaa 15540
   tcaagaacaa ccaggcaccg acgccgtgga atgccccatg tgtggaggaa cgggcggttg 15600
   gccaggcgta agcggctggg ttgtctgccg gccctgcaat ggcactggaa cccccaagcc 15660
   cgaggaatcg gcgtgagcgg tcgcaaacca tccggcccgg tacaaatcgg cgcggcgctg 15720
   ggtgatgacc tggtggagaa gttgaaggcg gcgcaggccg cccagcggca acgcatcgag 15780
   gcagaagcac gccccggtga atcgtggcaa gcggccgctg atcgaatccg caaagaatcc 15840
   cggcaaccgc cggcagccgg tgcgccgtcg attaggaagc cgcccaaggg cgacgagcaa 15900
   ccagattttt tcgttccgat gctctatgac gtgggcaccc gcgatagtcg cagcatcatg 15960
   gacgtggccg ttttccgtct gtcgaagcgt gaccgacgag ctggcgaggt gatccgctac 16020
   gagcttccag acgggcacgt agaggtttcc gcagggccgg ccggcatggc gagtgtgtgg 16080
   gattacgacc tggtactgat ggcggtttcc catctaaccg aatccatgaa ccgataccgg 16140
   gaagggaagg gagacaagcc cggccgcgtg ttccgtccac acgttgcgga cgtactcaag 16200
   ttctgccggc gagccgatgg cggaaagcag aaagacgacc tggtagaaac ctgcattcgg 16260
   ttaaacacca cgcacgttgc catgcagcgt acgaagaagg ccaagaacgg ccgcctggtg 16320
   acggtatccg agggtgaagc cttgattagc cgctacaaga tcgtaaagag cgaaaccggg 16380
   cggccggagt acatcgagat cgagctagct gattggatgt accgcgagat cacagaaggc 16440
   aagaacccgg acgtgctgac ggttcacccc gattactttt tgatcgatcc cggcatcggc 16500
   cgttttctct accgcctggc acgccgcgcc gcaggcaagg cagaagccag atggttgttc 16560
   aagacgatct acgaacgcag tggcagcgcc ggagagttca agaagttctg tttcaccgtg 16620
   cgcaagctga tcgggtcaaa tgacctgccg gagtacgatt tgaaggagga ggcggggcag 16680
   gctggcccga tcctagtcat gcgctaccgc aacctgatcg agggcgaagc atccgccggt 16740
   tcctaatgta cggagcagat gctagggcaa attgccctag caggggaaaa aggtcgaaaa 16800
   ggtctctttc ctgtggatag cacgtacatt gggaacccaa agccgtacat tgggaaccgg 16860
   aacccgtaca ttgggaaccc aaagccgtac attgggaacc ggtcacacat gtaagtgact 16920
   gatataaaag agaaaaaagg cgatttttcc gcctaaaact ctttaaaact tattaaaact 16980
   cttaaaaccc gcctggcctg tgcataactg tctggccagc gcacagccga agagctgcaa 17040
   aaagcgccta cccttcggtc gctgcgctcc ctacgccccg ccgcttcgcg tcggcctatc 17100
   gcggccgctg gccgctcaaa aatggctggc ctacggccag gcaatctacc agggcgcgga 17160
   caagccgcgc cgtcgccact cgaccgccgg cgcccacatc aaggcaccgg tgggtatgcc 17220
   tgacgatgcg tggagaccga aaccttgcgc tcgttcgcca gccaggacag aaatgcctcg 17280
   acttcgctgc tgcccaaggt tgccgggtga cgcacaccgt ggaaacggat gaaggcacga 17340
   acccagtgga cataagcctg ttcggttcgt aagctgtaat gcaagtagcg tatgcgctca 17400
   cgcaactggt ccagaacctt gaccgaacgc agcggtggta acggcgcagt ggcggttttc 17460
   atggcttgtt atgactgttt ttttggggta cagtctatgc ctcgggcatc caagcagcaa 17520
   gcgcgttacg ccgtgggtcg atgtttgatg ttatggagca gcaacgatgt tacgcagcag 17580
   ggcagtcgcc ctaaaacaaa gttaaacatc atgagggaag cggtgatcgc cgaagtatcg 17640
   actcaactat cagaggtagt tggcgtcatc gagcgccatc tcgaaccgac gttgctggcc 17700
   gtacatttgt acggctccgc agtggatggc ggcctgaagc cacacagtga tattgatttg 17760
   ctggttacgg tgaccgtaag gcttgatgaa acaacgcggc gagctttgat caacgacctt 17820
   ttggaaactt cggcttcccc tggagagagc gagattctcc gcgctgtaga agtcaccatt 17880
   gttgtgcacg acgacatcat tccgtggcgt tatccagcta agcgcgaact gcaatttgga 17940
   gaatggcagc gcaatgacat tcttgcaggt atcttcgagc cagccacgat cgacattgat 18000
   ctggctatct tgctgacaaa agcaagagaa catagcgttg ccttggtagg tccagcggcg 18060
   gaggaactct ttgatccggt tcctgaacag gatctatttg aggcgctaaa tgaaacctta 18120
   acgctatgga actcgccgcc cgactgggct ggcgatgagc gaaatgtagt gcttacgttg 18180
   tcccgcattt ggtacagcgc agtaaccggc aaaatcgcgc cgaaggatgt cgctgccgac 18240
   tgggcaatgg agcgcctgcc ggcccagtat cagcccgtca tacttgaagc tagacaggct 18300
   tatcttggac aagaagaaga tcgcttggcc tcgcgcgcag atcagttgga agaatttgtc 18360
   cactacgtga aaggcgagat caccaaggta gtcggcaaat aatgtctaac aattcgttca 18420
   agccgacgcc gcttcgcggc gcggcttaac tcaagcgtta gatgcactaa gcacataatt 18480
   gctcacagcc aaactatcag gtcaagtctg cttttattat ttttaagcgt gcataataag 18540
   ccctacacaa attgggagat atatcatgaa aggctggctt tttcttgtta tcgcaatagt 18600
   tggcgaagta atcgcaacat agcttgcttg gtcgttccgc gtgaacgtcg gctcgattgt 18660
   acctgcgttc aaatactttg cgatcgtgtt gcgcgcctgc ccggtgcgtc ggctgatctc 18720
   acggatcgac tgcttctctc gcaacgccat ccgacggatg atgtttaaaa gtcccatgtg 18780
   gatcactccg ttgccccgtc gctcaccgtg ttggggggaa ggtgcacatg gctcagttct 18840
   caatggaaat tatctgccta accggctcag ttctgcgtag aaaccaacat gcaagctcca 18900
   ccgggtgcaa agcggcagcg gcggcaggat atattcaatt gtaaatggct tcatgtccgg 18960
   gaaatctaca tggatcagca atgagtatga tggtcaatat ggagaaaaag aaagagtaat 19020
   taccaatttt ttttcaattc aaaaatgtag atgtccgcag cgttattata aaatgaaagt 19080
   acattttgat aaaacgacaa attacgatcc gtcgtattta taggcgaaag caataaacaa 19140
   attattctaa ttcggaaatc tttatttcga cgtgtctaca ttcacgtcca aatgggggct 19200
   tagatgagaa acttcacgat cggctctagt tcgagcgatc tagtaacata gatgacaccg 19260
   cgcgcgataa tttatcctag tttgcgcgct atattttgtt ttctatcgcg tattaaatgt 19320
   ataattgcgg gactctaatc ataaaaaccc atctcataaa taacgtcatg cattacatgt 19380
   taattattac atgcttaacg taattcaaca gaaattatat gataatcatc gcaagaccgg 19440
   caacaggatt caatcttaag aaactttatt gccaaatgtt tgaacgatcg gggaaattcg 19500
   tttctatgct tcaattctgc tctatagatg ttcttctagt cttaactttt agaaggcaca 19560
   acatttgaaa tgttggtaat ttttctgctg ggtagtgaac cattgtaatt tgtgtttgct 19620
   tatattatcc gttgggtttc tctatgttat ctgtggggcc atatcccaga actggttgag 19680
   tcggtccaac acctgggtgc caatcatgtc gatggtgggg tatggt 19726

<210> 34
<211> 19524
<212> DNA
<213> Artificial Sequence

<220>
<223> pZFNnptII-BSCTV-Repl/ tDT-crRNA_mGFP#1+2+3-RT GFPtoYFP200-Out
 LbCpf1

<400> 34
ccggattgaa agaaatatag tttaaatatt tattgataaa ataacaagtc aggtattata 60

gtccaagcaa aaacataaat ttattgatgc aagtttaaat tcagaaatat ttcaataact 120

gattatatca gctggtacat tgccgtagat gaaagactga gtgcgatatt atgtgtaata 180

cataaattga tgatatagct agcttagctc atcgggtcag aagaactcgt caagaaggcg 240

atagaaggcg atgcgctgcg aatcgggagc ggcgataccg taaagcacga ggaagcggtc 300

agcccattcg ccgccaagct cttcagcaat atcacgggta gccaacgcta tgtcctgata 360

gcggtccgcc acacccagcc ggccacagtc gatgaatcca gaaaagcggc cattttccac 420

catgatattc ggcaagcagg catcgccatg agtcacgacg agatcctcgc cgtcgggcat 480

gcgcgccttg agcctggcga acagttcggc tggcgcgagc ccctgatgct cttcgtccag 540

atcatcctga tcgacaagac cggcttccat ccgagtacgt gctcgctcga tgcgatgttt 600

cgcttggtgg tcgaatgggc aggtagccgg atcaagcgta tgcagccgcc gcattgcatc 660

agccatgatg gatactttct cggcaggagc aaggtgagat gacaggagat cctgccccgg 720

cacttcgccc aatagcagcc agtcccttcc cgcttcagtg acaacgtcga gcacagctgc 780

gcaaggaacg cccgtcgtgg ccagccacga tagccgcgct gcctcgtcct gaagttcatt 840

cagggcaccg gacaggtcgg tcttgacaaa aagaaccggg cgcccctgcg ctgacagccg 900

gaacacggcg gcatcagagc agccgattgt ctgttgtgcc cagtcatagc cgaatagcct 960

ctccacccaa gcggccggag aacctgcgtg caatccatct tgttcaatcc aagctcccat 1020

tttctgtgat tttggatgtg tttttgtatg gattgagagt gaatatgaga ctctaattgg 1080

ataccgaggg gaatttatgg aacgtcagtg gagcattttt gacaagaaat atttgctagc 1140

tgatagtgac cttaggcgac ttttgaacgc gcaataatgg tttctgacgt atgtgcttag 1200

ctcattaaac tccagaaacc cgcggctgag tggctccttc aacgttgcgg ttctgtcagt 1260

tccaaacgta aaacggcttg tcccgcgtca tcggcggggg tcattccgga ctgtggggcc 1320

atatcccaga actggttgag tcggtccaac acctgggtgc caatcatgtc gatggtgggg 1380

tatggccaat tttttttcaa ttcaaaaatg tagatgtccg cagcgttatt ataaaatgaa 1440

agtacatttt gataaaacga caaattacga tccgtcgtat ttataggcga aagcaataaa 1500

caaattattc taattcggaa atctttattt cgacgtgtct acattcacgt ccaaatgggg 1560

gcttagatga gaaacttcac gatcggctct agaggccatg ggtcgacatt gaatcgggct 1620

ctcttcaaat cccctatcaa ttgggtgctt tgggtgctct taaataccac caagggggca 1680

tccgcattaa tattaccgga tggcccccaa aaaatacgtg gcccaatgaa aatatgccac 1740

gtggaaagct aaagatgttg atgtgatgga tttgggcggg aaacttcctg aagaagtttt 1800

cattcccgcc cctttttttg acttagtcaa aaaagtagcc gacatactgt ttgaaataaa 1860

gttaaaagta gatcattgtg gtccccacaa gaaacttgct aagcaagttt cgaattattt 1920

ctgtttaaat acgttatttt tacatgtata tgtacttata aactagtggc cttggcggcc 1980

gctcgagcga tctagtaaca tagatgacac cgcgcgcgat aatttatcct agtttgcgcg 2040

ctatattttg ttttctatcg cgtattaaat gtataattgc gggactctaa tcataaaaac 2100

ccatctcata aataacgtca tgcattacat gttaattatt acatgcttaa cgtaattcaa 2160

cagaaattat atgataatca tcgcaagacc ggcaacagga ttcaatctta agaaacttta 2220

ttgccaaatg tttgaacgat cggggaaatt cgatctacac ttagtagaaa ttatctgccc 2280

tttcgaaaga tcccaacatc tacacttagt agaaattacc tgtccacaca atctgccctt 2340

tcatctacac ttagtagaaa ttagtgtgga caggtaatgg ttgtctgatc tacacttagt 2400

agaaattatc aggtacctct agacttgtac agctcgtcca tgccgtacag gaacaggtgg 2460

tggcggccct cggagcgctc gtactgttcc acgatggtgt agtcctcgtt gtgggaggtg 2520

atgtccagct tggtgtccac gtagtagtag ccgggcagtt gcacgggctt cttggccatg 2580

tagatggtct tgaactccac caggtagtgg ccgccgtcct tcagcttcag ggcctggtgg 2640

atctcgccct tcagcacgcc gtcgcggggg tacaggcgct cggtggaggc ctcccagccc 2700

atggtcttct tctgcattac ggggccgtcg ggggggaagt tggtgccgcg catcttcacc 2760

ttgtagatca gcgtgccgtc ctgcagggag gagtcctggg tcacggtcac cagaccgccg 2820

tcctcgaagt tcatcacgcg ctcccacttg aagccctcgg ggaaggacag cttcttgtaa 2880

tcggggatgt cggcggggtg cttcacgtac gccttggagc cgtacatgaa ctggggggac 2940

aggatgtccc aggcgaaggg cagggggccg cccttggtca ccttcagctt ggcggtctgg 3000

gtgccctcgt aggggcggcc ctcgccctcg ccctcgatct cgaactcgtg gccgttcatg 3060

gagccctcca tgcgcacctt gaagcgcatg aactctttga tgacctcctc gcccttgctc 3120

accatggatc ccctctccaa atgaaatgaa cttccttata tagaggaagg gtcttgcgaa 3180

ggatagtggg attgtgcgtc atcccttacg tcagtggaga tatcacatca atccacttgc 3240

tttgaagacg tggttggaac gtcttctttt tccacgatgt tcctcgtggg tgggggtcca 3300

tctttgggac cactgtcggt agaggcatct tgaacgatag cctttccttt atcgcaatga 3360

tggcatttgt agaagccatc ttccttttct actgtccttt cgatgaagtg acagatagct 3420

gggcaatgga atccgaggag gtttcccgat attacccttt gttgaaaagt ctcaatagcc 3480

ctctggtctt ctgagactgt atctttgata ttcttggagt agacgagagt gtcgtgctcc 3540

accatgtatc acatcaatcc acttgctttg aagacgtggt tggaacgtct tctttttcca 3600

cgatgttcct cgtgggtggg ggtccatctt tgggaccact gtcggtagag gcatcttgaa 3660

cgatagcctt tcctttatcg caatgatggc atttgtagaa gccatcttcc ttttctactg 3720

tcctttcgat gaagtgacag atagctgggc aatggaatcc gaggaggttt cccgatatta 3780

ccctttgttg aaaagtctca atagccctct ggtcttctga acactagtaa ggccttaagg 3840

gccacccggg agtactaagc tatatagtcc tgcaggtgga aaacaaggat gcatatatga 3900

gggcccatga acgatgcaga ggcaatgccg atggtttcgg gtcttcgaga agaccttaat 3960

gcttcaagac ccgccacaac atcgaggacg gcggcgtgca actcgctgat cattatcaac 4020

aaaatactcc aattggcgat ggccctgtcc tgcttcctga taatcactat ctcagctacc 4080

agtcagctct gagcaaggac cctaatgaga agagtgacca catggtcctt cttgagtttg 4140

taacagctgc tgggattaca catggcatgg atgaactata caaacatgat gagctttaac 4200

tttcggagac ggagctcagt ctgaccgcgg cgtctcctaa tagcttacta gtcggataat 4260

actaatatgt atgccacttt gtttggcaat tgtagatgtt atttttatta ataaaaatat 4320

tattttttta atacaatttc attgcaacac tctggttctg aattacacta gatacaaaat 4380

taaattcttc atacaggaca tgatttaaac ctctaattac attattaata caaattaaac 4440

ctaaattatc caagtatatc tctagtctct ttttcaagac tttcaaaaaa cggtccccag 4500

ttaatgtcct gtgcgatcca gtgatcgtca aatctatcca gcacttgtga agattcaagt 4560

atttgcggag gttgtggttg aatcttatct ggactttcag ttgataaact ggcccggaac 4620

ggaagaagtc ctggagtttg aggtacaatg gattcggtac caagtccacg ggtatggaat 4680

tcgtcgcttg ttgcagcgtg atgggttcct ctgtgcgaaa atccctgatt acattcatga 4740

tgaattgtga aatgacactt acaaggaagt ttgatcttgc gaggacgctt cttgattgtt 4800

atcaaagaga ggacttgtga gtttggcgaa gactgaattt tgtaatgtcc aggacctaag 4860

ggcttcattt tctgatttat tgaggaagtc ctggtaagag ctgccttcgc ctggattgca 4920

taatataata ctgggaatac cacctttaat gacacgtggt tttccatact ttaagtttgt 4980

ctgccactct ctttgtgcgc ctataaggtg cttccaatgc ttcatcttta agtaattggg 5040

atctacgtca tcaatgacgt tatacagaac attatcatta taagttttta aactaaaatc 5100

taaatgacca gtaatataat tatgtggacc taaacacctg gcccacattg ttttaccagt 5160

tcttgaatca ccctctatga ttaaactatt atatctaaaa ggccgcgcag cgggatccaa 5220

accaaaataa gaatcggccc actcttgaat aatttctgga actctagtga aagaagaaag 5280

aggaaaaggt ggttgataaa gatctggagg aggaagaaaa atagcctcta agttaggttt 5340

gaggttgtga tactgaaaaa tatatttttc tgggagtttc tcccttatta tctgcagagc 5400

ttctaatgca ttacctgcat ttaatgcctc tgctgcagca tcattagccg tctgttgacc 5460

tccgcgtgca gatcttccat cgacctgaaa ttcaccccag tcgatgtgat ctccgtcctt 5520

tgagacgtag gacttgacgt cggaactgga tttagctccc tgaatattgc agtggaattg 5580

tttgctggta cttcgatgtt gcagatcgaa gtaacgggca ttacggatct ggacttttcc 5640

ttcgaattga ataagggcat gcagatgtgg ttccccattt tcatgtaatt ctctgcagat 5700

gcgaatatat tttttattcg aaggtgtatt tatagcgagg agctgttcta aggcgtcttc 5760

tttggttact gaacattgag ggtatgtaag gaaaaaattt ttggcttttt tgtaaaaagg 5820

catattgaat cgggctctct tcaaatcccc tatcaattgg gtgctttggg tgctcttaaa 5880

taccaccaag gggccatccg cattaatatt accggatggc ccccaaaaaa tacgtggccc 5940

aatgaaaata tgccacgtgg aaagctaaag atgttgatgt gatggatttg ggcgggaaac 6000

ttcctgaaga agttttcatt cccgcccctt tttttgactt agtcaaaaaa gtagccgaca 6060

tactgtttga aataaagtta aaagtagatc attgtggtcc ccacaagaaa cttgctaagc 6120

aagtttcgaa ttatttctgt ttaaatacgt tatttttaca tgtatatgta cttataagtt 6180

taaacaaaaa ttacggatat gaatataggc atatccgtat ccgaattatc cgtttgacag 6240

ctagcaacga ttgtacaatt gcttctttaa aaaaggaaga aagaaagaaa gaaaagaatc 6300

aacatcagcg ttaacaaacg gccccgttac ggcccaaacg gtcatataga gtaacggcgt 6360

taagcgttga aagactccta tcgaaatacg taaccgcaaa cgtgtcatag tcagatcccc 6420

tcttccttca ccgcctcaaa cacaaaaata atcttctaca gcctatatat acaacccccc 6480

cttctatctc tcctttctca caattcatca tctttctttc tctaccccca attttaagaa 6540

atcctctctt ctcctcttca ttttcaaggt aaatctctct ctctctctct ctctctgtta 6600

ttccttgttt taattaggta tgtattattg ctagtttgtt aatctgctta tcttatgtat 6660

gccttatgtg aatatcttta tcttgttcat ctcatccgtt tagaagctat aaatttgttg 6720

atttgactgt gtatctacac gtggttatgt ttatatctaa tcagatatga atttcttcat 6780

attgttgcgt ttgtgtgtac caatccgaaa tcgttgattt ttttcattta atcgtgtagc 6840

taattgtacg tatacatatg gatctacgta tcaattgttc atctgtttgt gtttgtatgt 6900

atacagatct gaaaacatca cttctctcat ctgattgtgt tgttacatac atagatatag 6960

atctgttata tcattttttt tattaattgt gtatatatat atgtgcatag atctggatta 7020

catgattgtg attatttaca tgattttgtt atttacgtat gtatatatgt agatctggac 7080

tttttggagt tgttgacttg attgtatttg tgtgtgtata tgtgtgttct gatcttgata 7140

tgttatgtat gtgcagcgaa ttcggcgcgc caaacaatgg cttcctccat ggctcctaag 7200

aagaagagga aggttagcaa gctcgagaag tttaccaact gctacagcct ctctaagacc 7260

ctcaggttca aggctatccc tgtgggaaag acccaagaga atatcgacaa caagaggctc 7320

ctcgtcgagg atgagaagag agctgaagat tacaagggcg tgaagaagct cctcgacagg 7380

tactacctca gcttcatcaa cgatgtgctc cacagcatca agctcaagaa cctcaacaac 7440

tacatcagcc tcttccgtaa gaaaaccagg accgagaaag agaacaaaga gcttgagaac 7500

ctcgagatca acctccgtaa agagatcgcc aaggctttca agggaaacga gggatacaag 7560

agcctcttca agaaggatat tatcgagaca atcctgcctg agttcctgga cgataaggat 7620

gagatcgctc tcgtgaacag cttcaacgga ttcactactg ccttcaccgg attcttcgac 7680

aacaggaaaa acatgttcag cgaagaggcc aagagcacct ctatcgcttt cagatgcatc 7740

aacgagaacc tcacgcgtta catcagcaac atggacatct tcgagaaggt ggacgccatc 7800

ttcgataagc acgaggtgca agaaatcaaa gagaagatcc tcaacagcga ctacgacgtc 7860

gaggactttt ttgaagggga gttcttcaac ttcgttctca cccaagaggg catcgacgtg 7920

tacaacgcta ttatcggagg attcgtgacc gagtctgggg agaagattaa gggactcaac 7980

gagtacatca acctgtacaa ccagaaaacg aagcagaagc tcccgaagtt caagccgctc 8040

tacaagcagg ttctctctga tcgtgagagc ctctcatttt acggtgaggg ttacacctct 8100

gacgaggaag tgcttgaggt tttccgtaac accctcaaca agaacagcga gatcttctcg 8160

tccatcaaga agttggagaa acttttcaag aacttcgacg agtacagcag cgctgggatc 8220

ttcgttaaga acggacctgc tatcagcacc atcagcaagg atattttcgg cgagtggaac 8280

gtgatcaggg acaagtggaa tgctgagtac gatgacatcc acctcaagaa gaaggctgtc 8340

gtcactgaga agtacgagga tgacaggcgt aagtcgttca agaagatcgg ctctttcagc 8400

ctcgagcagc ttcaagaata cgctgatgct gatctcagcg tggtcgagaa gctcaaagag 8460

atcatcatcc agaaggtcga cgagatctac aaggtgtacg ggtcctctga gaagttgttc 8520

gatgctgatt tcgtcctcga gaagagtctg aagaagaacg acgctgtcgt cgcgatcatg 8580

aaggatttgc tcgacagcgt gaagtccttc gagaactata tcaaggcctt cttcggagag 8640

ggcaaagaga ctaataggga cgagtctttc tacggggatt tcgtgctcgc ttacgatatc 8700

ctcctcaagg tggaccatat ctacgacgcc atcagaaact acgtgaccca gaagccttac 8760

agcaaggaca agttcaagtt gtactttcag aacccgcagt tcatgggcgg atgggacaaa 8820

gacaaagaga cagattacag ggccaccatc ctcaggtacg ggtctaagta ctacctggcc 8880

atcatggaca agaaatacgc caagtgcctc caaaagatcg acaaggatga cgtgaacggg 8940

aactatgaga agatcaacta caagctcctt ccgggaccga acaagatgct tcctaaggtg 9000

ttcttcagca agaaatggat ggcctactac aacccgtctg aggacatcca gaaaatctac 9060

aagaacggga ccttcaagaa aggcgacatg ttcaacctca acgactgcca caagctcatc 9120

gatttcttca aggacagcat ctcgcgttac ccgaagtggt ctaacgctta cgactttaac 9180

ttcagcgaga cagaaaagta caaggatatc gccgggttct accgtgaggt tgaggaacag 9240

ggttacaagg ttagcttcga gagcgcctcc aagaaagagg ttgacaagtt ggtcgaagag 9300

ggcaagctct acatgttcca gatctataac aaggacttct ccgacaagag ccacggaact 9360

cctaacctcc atacgatgta cttcaagctg cttttcgacg agaacaacca cgggcagatc 9420

agactttctg gtggtgctga actcttcatg cgtagggcct cactcaagaa agaagagttg 9480

gttgttcacc cggccaactc tccaatcgct aacaagaatc ctgacaaccc gaaaaagacc 9540

accacgctgt cttacgacgt ctacaaggac aaaaggttca gcgaggacca gtacgagctt 9600

catatcccga tcgctatcaa caagtgcccg aagaacatct tcaagatcaa taccgaggtg 9660

agggtgctgc tcaagcacga tgataaccct tacgtgatcg gaatcgatcg tggtgagaga 9720

aacctcctct acatcgttgt ggtggacgga aagggaaaca tcgtcgagca gtacagcctg 9780

aacgagatta tcaacaattt caacggcatc aggatcaaga ccgactacca ctcactcctc 9840

gataagaaag aaaaagagcg tttcgaggcc aggcagaact ggacttctat cgaaaacatc 9900

aaagagttga aggccggcta catctctcag gtggtgcata agatctgcga gctggtggaa 9960

aagtacgatg ctgtgatcgc tcttgaggac ctcaactctg ggttcaagaa cagtagagtg 10020

aaggttgaga agcaggtcta ccaaaagttc gagaagatgc tcatcgacaa gctcaactac 10080

atggtggaca aaaagagcaa cccttgcgct accggtggtg ctcttaaggg ataccagatc 10140

acgaacaagt tcgagtcctt caagagcatg agcacccaga acggcttcat cttctatatc 10200

cctgcttggc tcaccagcaa gatcgatcct tctactggtt tcgtgaacct gctcaagacc 10260

aagtacacct cgatcgccga cagcaagaag ttcatctcgt ctttcgacag gatcatgtac 10320

gtgccggaag aggatctttt cgagttcgct ctcgactata agaacttcag caggaccgac 10380

gccgactaca ttaagaagtg gaagctctac tcctacggga accgtatcag gatcttccga 10440

aatccgaaga aaaacaacgt gttcgactgg gaagaagtgt gcctcacctc tgcctacaaa 10500

gaactgttca acaagtacgg catcaactac cagcagggtg atatcagggc tcttttgtgc 10560

gagcagagcg acaaggcatt ctacagctca ttcatggccc tcatgtctct catgctccag 10620

atgaggaact ctatcaccgg aaggaccgat gtggacttcc ttatctctcc ggtcaagaac 10680

tctgacggga tcttctacga cagccgtaac tatgaggctc aagagaacgc tatcctgccg 10740

aagaatgctg atgcaaacgg ggcttacaac attgcgagaa aggttctctg ggctatcggg 10800

cagtttaaga aagcggaaga tgagaagctc gacaaggtga agatcgccat ctccaacaaa 10860

gagtggcttg agtacgctca gacctccgtt aagcacaaga ggcctgctgc tactaagaaa 10920

gctggccagg ccaaaaagaa gaagtgaggc gcgccgagct ccaggcctcc cagctttcgt 10980

ccgtatcatc ggtttcgaca acgttcgtca agttcaatgc atcagtttca ttgcccacac 11040

accagaatcc tactaagttt gagtattatg gcattggaaa agctgttttc ttctatcatt 11100

tgttctgctt gtaatttact gtgttctttc agtttttgtt ttcggacatc aaaatgcaaa 11160

tggatggata agagttaata aatgatatgg tccttttgtt cattctcaaa ttattattat 11220

ctgttgtttt tactttaatg ggttgaattt aagtaagaaa ggaactaaca gtgtgatatt 11280

aaggtgcaat gttagacata taaaacagtc tttcacctct ctttggttat gtcttgaatt 11340

ggtttgtttc ttcacttatc tgtgtaatca agtttactat gagtctatga tcaagtaatt 11400

atgcaatcaa gttaagtaca gtataggctt gtttaaacgg gcccggtacc cgatcagatt 11460

gtcgtttccc gccttcggtt taaactatca gtgtttgaca ggatatattg gcgggtaaac 11520

ctaagagaaa agagcgttta ttagaataat cggatattta aaagggcgtg aaaaggttta 11580

tccgttcgtc catttgtatg tgcatgccaa ccacagggtt cccctcggga gtgcttggca 11640

ttccgtgcga taatgacttc tgttcaacca cccaaacgtc ggaaagcctg acgacggagc 11700

agcattccaa aaagatccct tggctcgtct gggtcggcta gaaggtcgag tgggctgctg 11760

tggcttgatc cctcaacgcg gtcgcggacg tagcgcagcg ccgaaaaatc ctcgatcgca 11820

aatccgacgc tgtcgaaaag cgtgatctgc ttgtcgctct ttcggccgac gtcctggcca 11880

gtcatcacgc gccaaagttc cgtcacagga tgatctggcg cgagttgctg gatctcgcct 11940

tcaatccggg tctgtggcgg gaactccacg aaaatatccg aacgcagcaa gatatcgcgg 12000

tgcatctcgg tcttgcctgg gcagtcgccg ccgacgccgt tgatgtggac gccgaaaagg 12060

atctaggtga agatcctttt tgataatctc atgaccaaaa tcccttaacg tgagttttcg 12120

ttccactgag cgtcagaccc cgtagaaaag atcaaaggat cttcttgaga tccttttttt 12180

ctgcgcgtaa tctgctgctt gcaaacaaaa aaaccaccgc taccagcggt ggtttgtttg 12240

ccggatcaag agctaccaac tctttttccg aaggtaactg gcttcagcag agcgcagata 12300

ccaaatactg ttcttctagt gtagccgtag ttaggccacc acttcaagaa ctctgtagca 12360

ccgcctacat acctcgctct gctaatcctg ttaccagtgg ctgctgccag tggcgataag 12420

tcgtgtctta ccgggttgga ctcaagacga tagttaccgg ataaggcgca gcggtcgggc 12480

tgaacggggg gttcgtgcac acagcccagc ttggagcgaa cgacctacac cgaactgaga 12540

tacctacagc gtgagctatg agaaagcgcc acgcttcccg aagggagaaa ggcggacagg 12600

tatccggtaa gcggcagggt cggaacagga gagcgcacga gggagcttcc agggggaaac 12660

gcctggtatc tttatagtcc tgtcgggttt cgccacctct gacttgagcg tcgatttttg 12720

tgatgctcgt caggggggcg gagcctatgg aaaaacgcca gcaacgcggc ctttttacgg 12780

ttcctggcct tttgctggcc ttttgctcac atgttctttc ctgcgttatc ccctgattct 12840

gtggataacc gattaccgcc tttgagtgag ctgataccgc tcgccgcagc cgaacgaccg 12900

agcgcagcga gtcagtgagc gaggaagcgg aagagcgcct gatgcggtat tttctcctta 12960

cgcatctgtg cggtatttca caccgcatat ggtgcactct cagtacaatc tgctctgatg 13020

ccgcatagtt aagccagtat acactccgct atcgctacgt gactgggtca tggctgcgcc 13080

ccgacacccg ccaacacccg ctgacgcgcc ctgacgggct tgtctgctcc cggcatccgc 13140

ttacagacaa gctgtgaccg tctccgggag ctgcatgtgt cagaggtttt caccgtcatc 13200

accgaaacgc gcgaggcagg ggtacgtcga ggtcgatcca acccctccgc tgctatagtg 13260

cagtcggctt ctgacgttca gtgcagccgt cttctgaaaa cgacatgtcg cacaagtcct 13320

aagttacgcg acaggctgcc gccctgccct tttcctggcg ttttcttgtc gcgtgtttta 13380

gtcgcataaa gtagaatact tgcgactaga accggagaca ttacgccatg aacaagagcg 13440

ccgccgctgg cctgctgggc tatgcccgcg tcagcaccga cgaccaggac ttgaccaacc 13500

aacgggccga actgcacgcg gccggctgca ccaagctgtt ttccgagaag atcaccggca 13560

ccaggcgcga ccgcccggag ctggccagga tgcttgacca cctacgccct ggcgacgttg 13620

tgacagtgac caggctagac cgcctggccc gcagcacccg cgacctactg gacattgccg 13680

agcgcatcca ggaggccggc gcgggcctgc gtagcctggc agagccgtgg gccgacacca 13740

ccacgccggc cggccgcatg gtgttgaccg tgttcgccgg cattgccgag ttcgagcgtt 13800

ccctaatcat cgaccgcacc cggagcgggc gcgaggccgc caaggcgcga ggcgtgaagt 13860

ttggcccccg ccctaccctc accccggcac agatcgcgca cgcccgcgag ctgatcgacc 13920

aggaaggccg caccgtgaaa gaggcggctg cactgcttgg cgtgcatcgc tcgaccctgt 13980

accgcgcact tgagcgcagc gaggaagtga cgcccaccga ggccaggcgg cgcggtgcct 14040

tccgtgagga cgcattgacc gaggccgacg ccctggcggc cgccgagaat gaacgccaag 14100

aggaacaagc atgaaaccgc accaggacgg ccaggacgaa ccgtttttca ttaccgaaga 14160

gatcgaggcg gagatgatcg cggccgggta cgtgttcgag ccgcccgcgc acgtctcaac 14220

cgtgcggctg catgaaatcc tggccggttt gtctgatgcc aagctcgcgg cctggccggc 14280

gagcttggcc gctgaagaaa ccgagcgccg ccgtctaaaa aggtgatgtg tatttgagta 14340

aaacagcttg cgtcatgcgg tcgctgcgta tatgatgcga tgagtaaata aacaaatacg 14400

caaggggaac gcatgaaggt tatcgctgta cttaaccaga aaggcgggtc aggcaagacg 14460

accatcgcaa cccatctagc ccgcgccctg caactcgccg gggccgatgt tctgttagtc 14520

gattccgatc cccagggcag tgcccgcgat tgggcggccg tgcgggaaga tcaaccgcta 14580

accgttgtcg gcatcgaccg cccgacgatt gaccgcgacg tgaaggccat cggccggcgc 14640

gacttcgtag tgatcgacgg agcgccccag gcggcggact tggctgtgtc cgcgatcaag 14700

gcagccgact tcgtgctgat tccggtgcag ccaagccctt acgacatatg ggccaccgcc 14760

gacctggtgg agctggttaa gcagcgcatt gaggtcacgg atggaaggct acaagcggcc 14820

tttgtcgtgt cgcgggcgat caaaggcacg cgcatcggcg gtgaggttgc cgaggcgctg 14880

gccgggtacg agctgcccat tcttgagtcc cgtatcacgc agcgcgtgag ctacccaggc 14940

actgccgccg ccggcacaac cgttcttgaa tcagaacccg agggcgacgc tgcccgcgag 15000

gtccaggcgc tggccgctga aattaaatca aaactcattt gagttaatga ggtaaagaga 15060

aaatgagcaa aagcacaaac acgctaagtg ccggccgtcc gagcgcacgc agcagcaagg 15120

ctgcaacgtt ggccagcctg gcagacacgc cagccatgaa gcgggtcaac tttcagttgc 15180

cggcggagga tcacaccaag ctgaagatgt acgcggtacg ccaaggcaag accattaccg 15240

agctgctatc tgaatacatc gcgcagctac cagagtaaat gagcaaatga ataaatgagt 15300

agatgaattt tagcggctaa aggaggcggc atggaaaatc aagaacaacc aggcaccgac 15360

gccgtggaat gccccatgtg tggaggaacg ggcggttggc caggcgtaag cggctgggtt 15420

gtctgccggc cctgcaatgg cactggaacc cccaagcccg aggaatcggc gtgagcggtc 15480

gcaaaccatc cggcccggta caaatcggcg cggcgctggg tgatgacctg gtggagaagt 15540

tgaaggcggc gcaggccgcc cagcggcaac gcatcgaggc agaagcacgc cccggtgaat 15600

cgtggcaagc ggccgctgat cgaatccgca aagaatcccg gcaaccgccg gcagccggtg 15660

cgccgtcgat taggaagccg cccaagggcg acgagcaacc agattttttc gttccgatgc 15720

tctatgacgt gggcacccgc gatagtcgca gcatcatgga cgtggccgtt ttccgtctgt 15780

cgaagcgtga ccgacgagct ggcgaggtga tccgctacga gcttccagac gggcacgtag 15840

aggtttccgc agggccggcc ggcatggcga gtgtgtggga ttacgacctg gtactgatgg 15900

cggtttccca tctaaccgaa tccatgaacc gataccggga agggaaggga gacaagcccg 15960

gccgcgtgtt ccgtccacac gttgcggacg tactcaagtt ctgccggcga gccgatggcg 16020

gaaagcagaa agacgacctg gtagaaacct gcattcggtt aaacaccacg cacgttgcca 16080

tgcagcgtac gaagaaggcc aagaacggcc gcctggtgac ggtatccgag ggtgaagcct 16140

tgattagccg ctacaagatc gtaaagagcg aaaccgggcg gccggagtac atcgagatcg 16200

agctagctga ttggatgtac cgcgagatca cagaaggcaa gaacccggac gtgctgacgg 16260

ttcaccccga ttactttttg atcgatcccg gcatcggccg ttttctctac cgcctggcac 16320

gccgcgccgc aggcaaggca gaagccagat ggttgttcaa gacgatctac gaacgcagtg 16380

gcagcgccgg agagttcaag aagttctgtt tcaccgtgcg caagctgatc gggtcaaatg 16440

acctgccgga gtacgatttg aaggaggagg cggggcaggc tggcccgatc ctagtcatgc 16500

gctaccgcaa cctgatcgag ggcgaagcat ccgccggttc ctaatgtacg gagcagatgc 16560

tagggcaaat tgccctagca ggggaaaaag gtcgaaaagg tctctttcct gtggatagca 16620

cgtacattgg gaacccaaag ccgtacattg ggaaccggaa cccgtacatt gggaacccaa 16680

agccgtacat tgggaaccgg tcacacatgt aagtgactga tataaaagag aaaaaaggcg 16740

atttttccgc ctaaaactct ttaaaactta ttaaaactct taaaacccgc ctggcctgtg 16800

cataactgtc tggccagcgc acagccgaag agctgcaaaa agcgcctacc cttcggtcgc 16860

tgcgctccct acgccccgcc gcttcgcgtc ggcctatcgc ggccgctggc cgctcaaaaa 16920

tggctggcct acggccaggc aatctaccag ggcgcggaca agccgcgccg tcgccactcg 16980

accgccggcg cccacatcaa ggcaccggtg ggtatgcctg acgatgcgtg gagaccgaaa 17040

ccttgcgctc gttcgccagc caggacagaa atgcctcgac ttcgctgctg cccaaggttg 17100

ccgggtgacg cacaccgtgg aaacggatga aggcacgaac ccagtggaca taagcctgtt 17160

cggttcgtaa gctgtaatgc aagtagcgta tgcgctcacg caactggtcc agaaccttga 17220

ccgaacgcag cggtggtaac ggcgcagtgg cggttttcat ggcttgttat gactgttttt 17280

ttggggtaca gtctatgcct cgggcatcca agcagcaagc gcgttacgcc gtgggtcgat 17340

gtttgatgtt atggagcagc aacgatgtta cgcagcaggg cagtcgccct aaaacaaagt 17400

taaacatcat gagggaagcg gtgatcgccg aagtatcgac tcaactatca gaggtagttg 17460

gcgtcatcga gcgccatctc gaaccgacgt tgctggccgt acatttgtac ggctccgcag 17520

tggatggcgg cctgaagcca cacagtgata ttgatttgct ggttacggtg accgtaaggc 17580

ttgatgaaac aacgcggcga gctttgatca acgacctttt ggaaacttcg gcttcccctg 17640

gagagagcga gattctccgc gctgtagaag tcaccattgt tgtgcacgac gacatcattc 17700

cgtggcgtta tccagctaag cgcgaactgc aatttggaga atggcagcgc aatgacattc 17760

ttgcaggtat cttcgagcca gccacgatcg acattgatct ggctatcttg ctgacaaaag 17820

caagagaaca tagcgttgcc ttggtaggtc cagcggcgga ggaactcttt gatccggttc 17880

ctgaacagga tctatttgag gcgctaaatg aaaccttaac gctatggaac tcgccgcccg 17940

actgggctgg cgatgagcga aatgtagtgc ttacgttgtc ccgcatttgg tacagcgcag 18000

taaccggcaa aatcgcgccg aaggatgtcg ctgccgactg ggcaatggag cgcctgccgg 18060

cccagtatca gcccgtcata cttgaagcta gacaggctta tcttggacaa gaagaagatc 18120

gcttggcctc gcgcgcagat cagttggaag aatttgtcca ctacgtgaaa ggcgagatca 18180

ccaaggtagt cggcaaataa tgtctaacaa ttcgttcaag ccgacgccgc ttcgcggcgc 18240

ggcttaactc aagcgttaga tgcactaagc acataattgc tcacagccaa actatcaggt 18300

caagtctgct tttattattt ttaagcgtgc ataataagcc ctacacaaat tgggagatat 18360

atcatgaaag gctggctttt tcttgttatc gcaatagttg gcgaagtaat cgcaacatag 18420

cttgcttggt cgttccgcgt gaacgtcggc tcgattgtac ctgcgttcaa atactttgcg 18480

atcgtgttgc gcgcctgccc ggtgcgtcgg ctgatctcac ggatcgactg cttctctcgc 18540

aacgccatcc gacggatgat gtttaaaagt cccatgtgga tcactccgtt gccccgtcgc 18600

tcaccgtgtt gggggaaggt tgcacatggc tcagttctca atggaaatta tctgcctaac 18660

cggctcagtt ctgcgtagaa accaacatgc aagctccacc gggtgcaaag cggcagcggc 18720

ggcaggatat attcaattgt aaatggcttc atgtccggga aatctacatg gatcagcaat 18780

gagtatgatg gtcaatatgg agaaaaagaa agagtaatta ccaatttttt ttcaattcaa 18840

aaatgtagat gtccgcagcg ttattataaa atgaaagtac attttgataa aacgacaaat 18900

tacgatccgt cgtatttata ggcgaaagca ataaacaaat tattctaatt cggaaatctt 18960

tatttcgacg tgtctacatt cacgtccaaa tgggggctta gatgagaaac ttcacgatcg 19020

gctctagttc gagcgatcta gtaacataga tgacaccgcg cgcgataatt tatcctagtt 19080

tgcgcgctat attttgtttt ctatcgcgta ttaaatgtat aattgcggga ctctaatcat 19140

aaaaacccat ctcataaata acgtcatgca ttacatgtta attattacat gcttaacgta 19200

attcaacaga aattatatga taatcatcgc aagaccggca acaggattca atcttaagaa 19260

actttattgc caaatgtttg aacgatcggg gaaattcgtt tctatgcttc aattctgctc 19320

tatagatgtt cttctagtct taacttttag aaggcacaac atttgaaatg ttggtaattt 19380

ttctgctggg tagtgaacca ttgtaatttg tgtttgctta tattatccgt tgggtttctc 19440

tatgttatct gtggggccat atcccagaac tggttgagtc ggtccaacac ctgggtgcca 19500

atcatgtcga tggtggggta tggt 19524